# EUROPEAN PATENT APPLICATION

(11) **EP 1 564 213 A1**
(43) Date of publication of application: **17.08.2005**
(21) Application number: 03774086.7
(22) Date of filing: 20.11.2003
(51) Int. Cl.: C07D 401/04, C07D 401/06, C07D 401/14, C07D 487/04, C07D 513/04, A61K 31/4188, A61K 31/429, A61K 31/437, A61K 31/454, A61K 31/4985, A61K 31/55, A61K 31/455, A61P 7/02, A61P 9/00, A61P 9/10, A61P 43/00

(54) **IMIDAZOLE DERIVATIVE, PROCESS FOR PRODUCING THE SAME, AND USE**

(30) Priority: 22.11.2002 JP 2002338939
(71) Applicant: Takeda Pharmaceutical Company Limited, Osaka-shi, Osaka 541-0045 (JP)
(72) Inventor: KUBO, Keiji c/o Takeda Pharmaceutical Company Ltd, Osaka-shi, Osaka (JP); KUROITA, Takanobu Takeda Pharmaceutical Comp. Ltd, Osaka-shi, Osaka (JP); IMAEDA, Yasuhiro Takeda Pharmaceutical Company Ltd, Osaka-shi, Osaka (JP); KAWAMURA, Masaki Takeda Pharmaceutical Company Ltd, Osaka-shi, Osaka (JP)
(74) Representative: Rickard, Timothy Mark Adrian
(86) International application number: PCT/JP2003/014793
(87) International publication number: WO 2004/048363

(57) **Abstract**

There is provided an imidazole derivative useful as a thrombosis treating agent, which is represented by the formula (I): 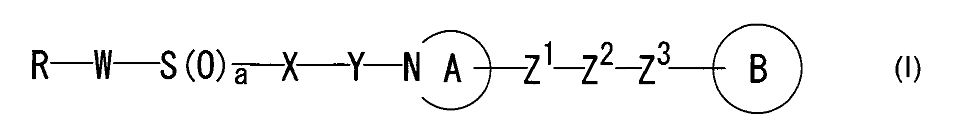 wherein R represents an optionally substituted cyclic hydrocarbon group or an optionally substituted heterocyclic group, W represents a bond or an optionally substituted divalent linear hydrocarbon group, X represents an optionally substituted divalent hydrocarbon group, Y represents -CO-, -S(O)-, -S(O)₂- or a bond, ring A represents an optionally substituted pyrrolidine ring, an optionally substituted piperidine ring or an optionally substituted perhydroazepine ring, Z¹ and Z³ independently represent a bond or an optionally substituted divalent linear hydrocarbon group, Z² represents -N(R¹)-, -O-, - S(O)-, -S(O)₂-, -CO-, -CH(R¹)- or a bond, ring B represents an optionally substituted imidazole ring, wherein a substituent which the optionally substituted imidazole ring represented by ring B may have may be taken together with R¹ to form an optionally substituted ring, and a represents 0, 1 or 2.

## Description

### Technical Field

The present invention relates to a novel imidazole derivative useful for preventing or treating arterial and venous thrombotic obstructive disease, inflammation, cancer and the like, which has anti-coagulation activity and anti-thrombosis activity by inhibiting activated blood coagulation factor X (FXa), and production and use thereof.

### Background Art

For preventing and treating myocardial infarction, cerebral thrombosis and the like, it is important to inhibit formation of thrombi and, an anti-thrombin agent, a platelet aggregation inhibitor and the like as a thrombosis inhibitor have been studied and developed variously. However, as well as a platelet aggregation inhibitor, an anti-thrombin agent not only has anti-coagulation activity but also inhibits aggregation of platelet. Thus these drugs tend to cause bleeding or the like as side effect and thereby have a problem of their safety. On the other hand, it is thought that an FXa inhibitor inhibits only a coagulation factor specifically and therefore it may be a safe anticoagulant.

To date, compounds having FXa inhibiting activity have been disclosed, for example, in JP-A 7-112970, JP-A 5-208946, WO 96/16940, WO 96/40679, WO 96/10022, WO 97/21437, WO 99/26919, WO 99/33805, WO 00/09480, WO 01/44172, WO 02/06234, US Patent Application Publication No. 2002/0045616 and Journal of Medicinal Chemistry, 1998, vol.41, p.3357.

### Objective of the Invention

There is a need for development of a novel compound useful as a thrombosis treating agent, which has improved drug efficacy, oral absorbability and duration of action and has fewer side effects, as compared with previous FXa inhibitors.

### Summary of the Invention

The present inventors studied intensively, considering that an imidazole derivative having high selectivity for and potent inhibitory activity on FXa may exert lasting and sufficient effect when orally administered and therefore it may be useful for preventing and treating arterial and venous thrombotic obstructive disease, inflammation and cancer.

As a result, the present inventors found that a novel imidazole derivative represented by the following formula (I) or a salt thereof [hereinafter referred to as Compound (I) in some cases] has selective and potent FXa inhibitory activity, is highly safe, and exerts lasting and sufficient effect when orally administered, and then completed the present invention.

That it, the present invention relates to:
(1) a compound represented by the formula (I): 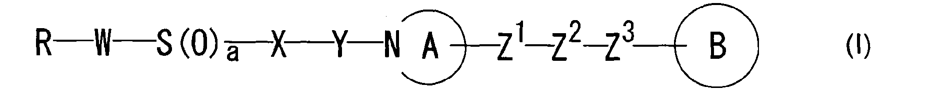 wherein R represents an optionally substituted cyclic hydrocarbon group or an optionally substituted heterocyclic group, W represents a bond or an optionally substituted divalent linear hydrocarbon group, X represents an optionally substituted divalent hydrocarbon group, Y represents -CO-, -S(O)-, -S(O)₂- or a bond, ring A represents an optionally substituted pyrrolidine ring, an optionally substituted piperidine ring or an optionally substituted perhydroazepine ring, Z¹ and Z³ independently represent a bond or an optionally substituted divalent linear hydrocarbon group, Z² represents -N(R¹)-, -O-, -S(O)-, -S(O)₂-, -CO-, -CH(R¹)- or a bond (R¹ represents a hydrogen atom, an optionally substituted hydrocarbon group, an optionally substituted acyl group, an optionally esterified carboxyl group or an optionally substituted carbamoyl group), ring B represents an optionally substituted imidazole ring, wherein a substituent which the optionally substituted imidazole ring represented by ring B may have may be taken together with R¹ to form an optionally substituted ring, and a represents 0, 1 or 2, or a salt thereof;
(2) a prodrug of the compound according to the above (1);
(3) the compound according to the above (1), wherein R is an optionally substituted aryl group;
(4) the compound according to the above (1), wherein R is naphthyl optionally substituted with a halogen atom or indolyl optionally substituted with a halogen atom;
(5) the compound according to the above (1), wherein W is a bond;
(6) the compound according to the above (1), wherein X is an optionally substituted divalent linear hydrocarbon group;
(7) the compound according to the above (1), wherein Y is -CO-;
(8) the compound according to the above (1), wherein ring A is an optionally substituted piperidine ring;
(9) the compound according to the above (1), wherein the formula: 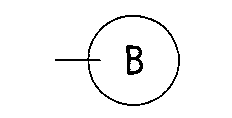 is the formula: 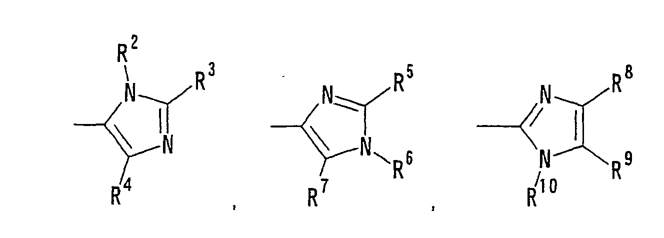 or 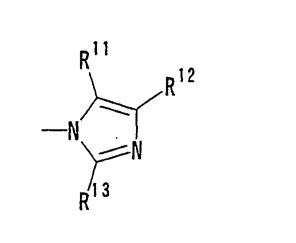 wherein R², R³, R^{4,} R⁵, R⁶, R⁷, R⁸, R⁹, R¹⁰, R¹¹, R¹² and R¹³ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group or an optionally substituted amino group, or R² and R³, R⁵ and R⁶, R⁶ and R⁷, R⁸ and R⁹ , R⁹ and R¹⁰, or R¹¹ and R¹² may be taken together to form an optionally substituted ring;
(10) the compound according to the above (1), wherein the formula: 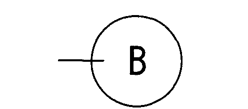 is the formula: 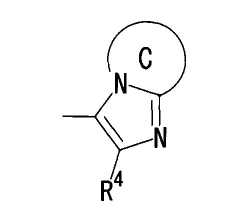 wherein ring C represents an optionally substituted nitrogen-containing heterocyclic ring, and other symbols are as defined in the above (9);
(11) the compound according to the above (1), wherein a substituent which the optionally substituted imidazole ring represented by ring B may have and R¹ do not form a ring;
(12) the compound according to the above (1), wherein Z² is -N (R¹) - or -CH (R¹) - (R¹ is as defined in the above (1)), and a substituent which the optionally substituted imidazole ring represented by ring B may have and R¹ are taken together to form an optionally substituted ring;
(13) the compound according to the above (1), wherein the formula (I) is the formula (Ia): 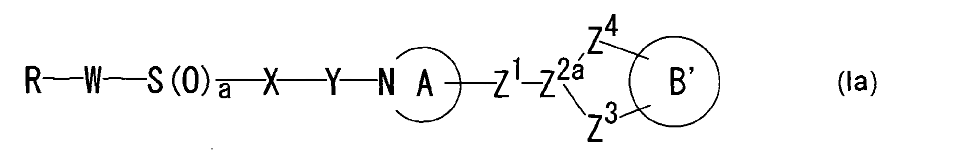 wherein ring B' represents an optionally further substituted imidazole ring, Z^{2a} represents N or CH, Z⁴ represents an optionally substituted divalent linear hydrocarbon group, and other symbols are as defined in the above (1);
(14) the compound according to the above (13), wherein Z^{2a} is a nitrogen atom;
(15) the compound according to the above (13), wherein Z³ and Z⁴ are independently a divalent linear hydrocarbon group optionally substituted with an oxo group;
(16) the compound according to the above (1), wherein the formula (I) is the formula (Ib): 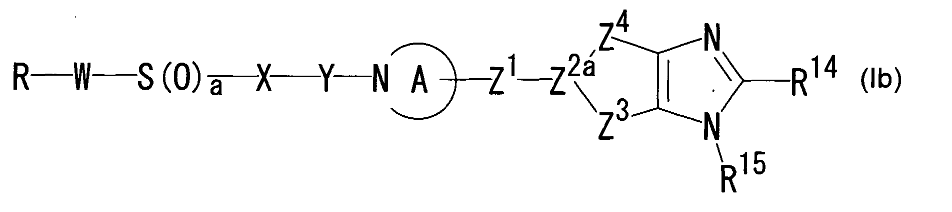 wherein R¹⁴ and R¹⁵ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group, or an optionally substituted amino group, or R¹⁴ and R¹⁵ may be taken together to form an optionally substituted ring, and other symbols are as defined in the above (1) or (13);
(17) the compound according to the above (1), wherein the formula (I) is the formula (Ic): 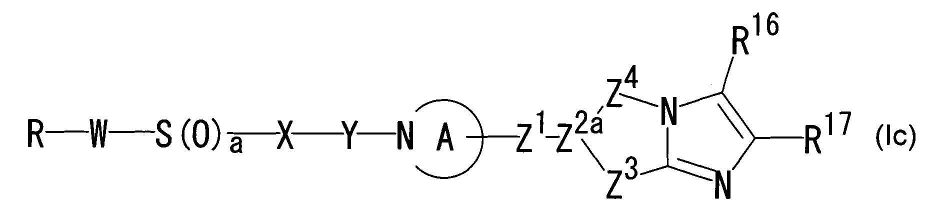 wherein R¹⁶ and R¹⁷ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group or an optionally substituted amino group, or R¹⁶ and R¹⁷ may be taken together to form an optionally substituted ring, and other symbols are as defined in the above (1) or (13);
(18) the compound according to the above (1), wherein the formula (I) is the formula (Id): 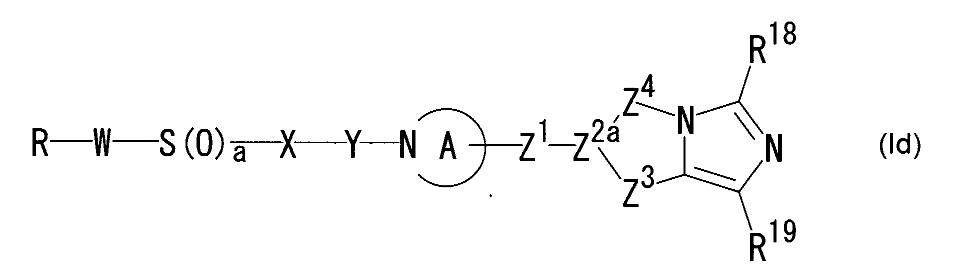 wherein R¹⁸ and R¹⁹ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group, or an optionally substituted amino group, and other symbols are as defined in the above (1) or (13);
(19) the compound according to the above (1), wherein a is 2;
(20) a compound selected from the group consisting of 7-(1-{3-[(6-chloro-2-naphthyl)sulfonyl]propanoyl}-4-piperidinyl)-3-methyl-6,7-dihydroimidazo[1,5-a]pyrazin-8(5H)-one, 7-(1-{3-[(6-choloro-2-naphthyl)sulfonyl]propanoyl}-4-piperidinyl)-1-methyl-6,7-dihydroimidazo[1,5-a]pyrazin-8(5H)-one, 2-(1-{3-[(6-choloro-2-naphthyl)sulfonyl]propanoyl}-4-piperidinyl)-5-methyl-1,2-dihydro-3H-imidazo[1,5-c]imidazol-3-one, 2-(1-{3-[(6-choloro-2-naphthyl)sulfonyl]propanoyl}-4-piperidinyl)-5,7-dimethyl-1,2-dihydro-3H-imidazo[1,5-c]imidazol-3-one, 2-(1-{3-[(7-choloro-2H-chromen-3-yl)sulfonyl]propanoyl}-4-piperidinyl)-5-methyl-1,2-dihydro-3H-imidazo[1,5-c]imidazol-3-one, 2-[1-(3-{[(E)-2-(4-cholorophenyl)vinyl]sulfonyl}propanoyl)-4-piperidinyl]-5-methyl-1,2-dihydro-3H-imidazo[1,5-c]imidazol-3-one, 2-(1-{3-[(5-chloro-1H-indol-2-yl)sulfonyl]propanoyl}-4-piperidinyl)-5-methyl-1,2-dihydro-3H-imidazo[1,5-c]imidazol-3-one, 2-(1-{3-[(6-chloro-2-naphthyl)sulfonyl]propanoyl}-4-piperidinyl)-5-(hydroxymethyl)-1,2-dihydro-3H-imidazo[1,5-c]imidazol-3-one, 2-(1-{(2S)-3-[(6-chloro-2-naphthyl)sulfonyl]-2-hydroxypropanoyl}-4-piperidinyl)-5-(hydroxymethyl)-1,2-dihydro-3H-imidazo[1,5-c]imidazol-3-one, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 1-acetylpiperidine-4-carboxylate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(2-oxo-1-pyrrolidinyl)propionate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl (2-oxo-1-pyrrolidinyl)acetate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 4-(acetylamino)butanoate, and 2-(1-{(2S)-3-[(6-chloro-2-naphthyl)sulfonyl]-2-hydroxypropanoyl}-4-piperidinyl)-5,7-dimethyl-1,2-dihydro-3H-imidazo[1,5-c]imidazol-3-one or a salt thereof;
(21) a pharmaceutical preparation which comprises the compound according to the above (1) or (2);
(22) the pharmaceutical preparation according to the above (21), which is an anticoagulant;
(23) the pharmaceutical preparation according to the above (21), which is an activated blood coagulation factor X inhibitor;
(24) the pharmaceutical preparation according to the above (21), which is an agent for preventing or treating myocardial infarction, cerebral infarction, deep venous thrombosis, pulmonary thromboembolism or arteriosclerotic obliterans;
(25) the pharmaceutical preparation according to the above (21), which is an agent for preventing or treating economy class syndrome, thromboembolism during or after an operation, or a secondary onset of deep venous thrombosis;
(26) a process for preparing the compound according to the above (1), which comprises reacting a compound represented by the formula (II): 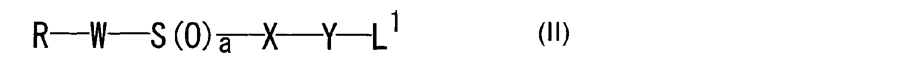 wherein L¹ represents a leaving group and other symbols are as defined in the above (1), or a salt thereof with a compound represented by the formula (III): 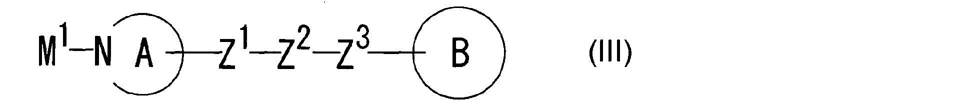 wherein M¹ represents a hydrogen atom, an alkaline metal, an alkaline earth metal or a leaving group, and other symbols are as defined in the above (1), or a salt thereof; or
   reacting a compound represented by the formula (IV): 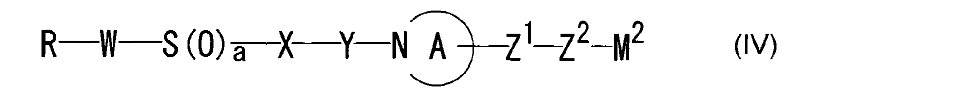 wherein M² represents a hydrogen atom, an alkaline metal, an alkaline earth metal or a leaving group, and other symbols are as defined in the above (1), or a salt thereof with a compound represented by the formula (V): 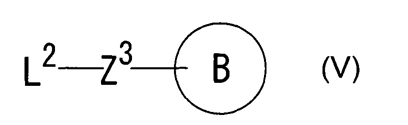 wherein L² represents a leaving group or a formyl group, and other symbols are as defined in the above (1), or a salt thereof; or
   reacting a compound represented by the formula (Ie): 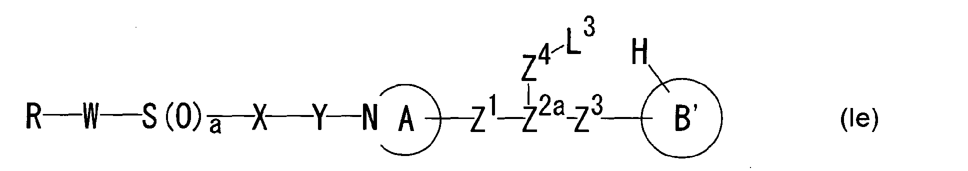 wherein L³ represents a leaving group and other symbols are as defined in the above (1) or (13), or a salt thereof with a base; or
   reacting a compound represented by the formula (If): 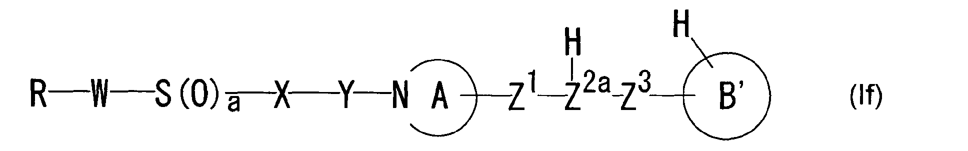 wherein symbols are as defined in the above (1) or (13), or a salt thereof with a compound represented by the formula (VI) :

   L⁴― Z⁴―L^{4,} (VI)

   wherein L⁴ and L^{4'} represent a leaving group and other symbols are as defined in the above (13), or a salt thereof; or
   oxidizing a compound represented by the formula (Ig): 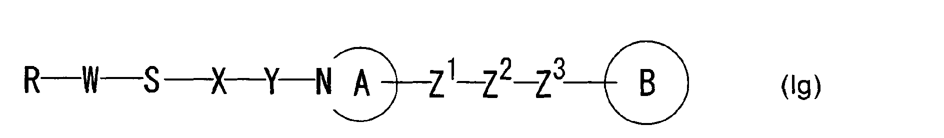 wherein symbols are as defined in the above (1), or a salt thereof, and optionally subjecting a compound obtained in the above reaction to hydrolysis, esterification, amidation, alkylation, acylation, reduction, oxidation or/and deprotection reaction;
(27) a method for inhibiting blood coagulation in a mammal, which comprises administering an effective amount of the compound according to the above (1) or a prodrug thereof to said mammal;
(28) a method for inhibiting activated blood coagulation factor X in a mammal, which comprises administering an effective amount of the compound according to the above (1) or a prodrug thereof to said mammal;
(29) a method for preventing or treating myocardial infarction, cerebral infarction, deep venous thrombosis, pulmonary thromboembolism or arteriosclerotic obliterans in a mammal, which comprises administering an effective amount of the compound according to the above (1) or a prodrug thereof to said mammal;
(30) use of the compound according to the above (1) or a prodrug thereof for manufacture of a medicament for inhibiting blood coagulation;
(31) use of the compound according to the above (1) or a prodrug thereof for manufacture of a medicament for inhibiting activated blood coagulation factor X;
(32) use of the compound according to the above (1) or a prodrug thereof for manufacture a medicament for preventing or treating myocardial infarction, cerebral infarction, deep venous thrombosis, pulmonary thromboembolism or arteriosclerotic obliterans; and the like.

### Detailed Description of the Invention

In the above formulas, R represents an optionally substituted cyclic hydrocarbon group, or an optionally substituted heterocyclic group (preferably optionally substituted aryl, or optionally substituted aromatic heterocyclic group).

The "cyclic hydrocarbon group" of the "optionally substituted cyclic hydrocarbon group" represented by R includes an alicyclic hydrocarbon group, an aryl group and the like and, among them, an aryl group is preferable.

The "alicyclic hydrocarbon group" as an example of a cyclic hydrocarbon group includes a saturated or unsaturated alicyclic hydrocarbon group such as a cycloalkyl group, a cycloalkenyl group, a cycloalkadienyl group, and the like.

Herein, the "cycloalkyl group" includes C₃₋₉ cycloalkyl such as cyclopropyl, cyclobutyl, cyclopentyl, cyclohexyl, cycloheptyl, cyclooctyl, cyclononyl, and the like.

The "cycloalkenyl group" includes a C₃₋₉ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, 1-cyclohexen-1-yl, 1-cyclohepten-1-yl and the like.

The "cycloalkadienyl group" includes a C₄₋₆ cycloalkadienyl group such as 2,4-cyclopentadien-1-yl, 2,4-cyclohexadien-1-yl, 2,5-cyclohexadien-1-yl and the like.

The "aryl group" as an example of a cyclic hydrocarbon group includes a monocyclic or fused polycyclic aromatic hydrocarbon group. For example, a C₆₋₁₄ aryl group such as phenyl, naphthyl, anthryl, phenanthryl, acenaphthylenyl and the like and, among them, phenyl, 1-naphthyl, 2-naphthyl and the like are particularly preferable.

In addition, a cyclic hydrocarbon group includes a dicyclic or tricyclic hydrocarbon group derived from fusion of same or different two to three rings (preferably two or more kinds of rings) selected from rings constituting the aforementioned alicyclic hydrocarbon group and aromatic hydrocarbon group, such as 1,2-dihydronaphthyl, 1,2,3,4-tetrahydronaphthyl, indenyl, dihydrobenzocycloheptenyl and fluorenyl.

The "heterocyclic group" of the "optionally substituted heterocyclic group" represented by R includes an aromatic heterocyclic group, a saturated or unsaturated non-aromatic heterocyclic group (aliphatic heterocyclic group) containing at least one (preferably one to four, more preferably one to two) 1 to 3 (preferably 1 to 2) kinds of heteroatoms selected from an oxygen atom, a sulfur atom, and a nitrogen atom as a ring system-constituting atom (ring atom) and the like.

The "aromatic heterocyclic group" includes a 5 to 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like, and a 8 to 16-membered (preferably 8 to 12-membered) aromatic fused heterocyclic group such as benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like, preferably, a heterocyclic ring in which 1 to 2 (preferably 1) of the aforementioned 5 to 6-membered aromatic monocyclic heterocyclic groups are fused with 1 to 2 (preferably 1) of benzene rings, or a heterocyclic ring in which 2 to 3 (preferably 2) of the same or different aforementioned 5 to 6-membered aromatic monocyclic heterocyclic groups are fused, more preferably a heterocyclic ring in which the aforementioned 5 to 6-membered aromatic monocyclic heterocyclic group is fused with a benzene ring, particularly preferably indolyl, benzofuranyl, benzo[b]thienyl, benzopyranyl.

The "non-aromatic heterocyclic group" includes a 3 to 8-membered (preferably 5 to 6-membered) saturated or unsaturated (preferably saturated) non-aromatic monocyclic heterocyclic group (aliphatic monocyclic heterocyclic group) such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl and the like, a heterocyclic group in which 1 to 2 (preferably 1) of the aforementioned non-aromatic monocyclic heterocyclic groups are fused with 1 to 2 (preferably 1) of benzene rings such as 1,3-dihydroisoindolyl and the like, a heterocyclic group in which 1 to 2 (preferably 1) of the aforementioned non-aromatic monocyclic heterocyclic groups are fused with 1 to 2 (preferably 1) of 5 to 6-membered aromatic monocyclic heterocyclic groups, and a non-aromatic heterocyclic group in which a part or all of the double bonds of the aforementioned aromatic monocyclic heterocyclic group or aromatic fused heterocyclic group is saturated, such as 1,2,3,4-tetrahydroquinolyl, 1,2,3,4-tetrahydroisoquinolyl.

Examples of a substituent for the "optionally substituted cyclic hydrocarbon group" and the "optionally substituted heterocyclic group" represented by R include optionally substituted alkyl, optionally substituted alkenyl, optionally substituted alkynyl, optionally substituted aryl, optionally substituted cycloalkyl, optionally substituted cycloalkenyl, optionally substituted heterocyclic group, optionally substituted amino, optionally substituted imidoyl (e.g. a group represented by the formula -C(U')=N-U, wherein U and U' represent a hydrogen atom or a substituent respectively (U represents preferably a hydrogen atom) etc.), optionally substituted amidino (e.g. a group represented by the formula - C(NT'T'')=N-T, wherein T, T' and T" represent a hydrogen atom or a substituent respectively (T represents preferably a hydrogen atom) etc.), an optionally substituted hydroxy group, an optionally substituted thiol group, optionally substituted alkylsulfinyl, optionally substituted alkylsulfonyl, optionally esterified carboxyl, optionally substituted carbamoyl, optionally substituted thiocarbamoyl, an optionally substituted sulfamoyl group, a halogen atom (e.g. fluorine, chlorine, bromine, iodine etc. preferably chlorine, bromine etc.), a cyano group, a nitro group, a sulfonic acid-derived acyl, carboxylic acid-derived acyl and the like. These optional substituents may be at 1 to 5 (preferably 1 to 3) substitutable positions. In addition, the "optionally substituted cyclic hydrocarbon group" and the "optionally substituted heterocyclic group" represented by R may have an oxo group or a thioxo group. For example, when R is benzopyranyl, R may form benzo-α-pyronyl or benzo-γ-pyronyl.

The "aryl" of the "optionally substituted aryl" exemplified as a substituent for the "optionally substituted cyclic hydrocarbon group" and the "optionally substituted heterocyclic group" represented by R includes C₆₋₁₄ aryl such as phenyl, naphthyl, anthryl, phenanthryl, acenaphthylenyl and the like. Herein, substituents for the aryl include a lower alkoxy group (e.g. C₁₋₆ alkoxy such as methoxy, ethoxy, propoxy etc.), a halogen atom (e.g. fluorine, chlorine, bromine, iodine etc.), lower alkyl (e.g. C₁₋₆ alkyl such as methyl, ethyl, propyl etc.), lower alkenyl (e.g. C₂₋₆ alkenyl such as vinyl, allyl etc.), lower alkynyl (e.g. C₂₋₆ alkynyl such as ethynyl, propargyl etc.), optionally substituted amino, an optionally substituted hydroxyl group, a cyano group, optionally substituted amidino, carboxy, a lower alkoxycarbonyl group (e.g. C₁₋₆ alkoxycarbonyl such as methoxycarbonyl, ethoxycarbonyl etc.), an optionally substituted carbamoyl group (e.g. a carbamoyl group which may be substituted with C₁₋₆ alkyl optionally substituted with 5 to 6-membered aromatic monocyclic heterocyclic group (e.g. pyridinyl etc.), acyl (e.g. formyl, C₂₋₆ alkanoyl, benzoyl, optionally halogenated C₁₋₆ alkylsulfonyl, benzenesulfonyl etc.) or optionally halogenated C₁₋₆ alkoxycarbonyl), 1-azetidinylcarbonyl, 1-pyrrolidinylcarbonyl, piperidinocarbonyl, morpholinocarbonyl, thiomorpholinocarbonyl (the sulfur atom may be oxidized), 1-piperazinylcarbonyl etc.). These optional substituents may be at 1 to 3 substitutable positions.

The "optionally substituted amino", "optionally substituted hydroxyl group" and "optionally substituted amidino" exemplified as a substituent for the "optionally substituted aryl" exemplified as a substituent for the "optionally substituted cyclic hydrocarbon group" and the "optionally substituted heterocyclic group" represented by R include the same groups as the "optionally substituted amino", "optionally substituted hydroxyl group" and "optionally substituted amidino" exemplified as a substituent for the "optionally substituted cyclic hydrocarbon group" and the "optionally substituted heterocyclic group" represented by R described later.

The "alkyl" of the "optionally substituted alkyl" exemplified as a substituent for the "optionally substituted cyclic hydrocarbon group" and the "optionally substituted heterocyclic group" represented by R includes C₁₋₆ alkyl such as methyl, ethyl, n-propyl, isopropyl, n-butyl, isobutyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl and the like. Herein, substituents for the alkyl include the same number of the same groups as those of a substituent for the aforementioned "optionally substituted aryl" and an oxo group, a thioxo group and the like.

The "alkenyl" of the "optionally substituted alkenyl" exemplified as a substituent for the "optionally substituted cyclic hydrocarbon group" and the "optionally substituted heterocyclic group" represented by R includes C₂₋₆ alkenyl such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like. Herein, substituents for the alkenyl include the same number of the same groups as those of a substituent in the aforementioned "optionally substituted aryl", and an oxo group, a thioxo group and the like.

The "alkynyl" of the "optionally substituted alkynyl" exemplified as a substituent for the "optionally substituted cyclic hydrocarbon group" and the "optionally substituted heterocyclic group" represented by R includes C₂₋₆ alkynyl such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. Herein, substituents for the alkynyl include the same number of the same substituents as those of a substituent for the aforementioned "optionally substituted aryl", and an oxo group, a thioxo group and the like.

The "cycloalkyl" of the "optionally substituted cycloalkyl" exemplified as a substituent for the "optionally substituted cyclic hydrocarbon group" and the "optionally substituted heterocyclic group" represented by R includes C₃₋₇ cycloclkyl such as cyclopropyl, cyclobutyl, cyclopentyl, cyclohexyl, cycloheptyl and the like. Herein, substituents for cycloalkyl include the same number of the same groups as those of a substituent for the aforementioned "optionally substituted aryl".

The "cycloalkenyl" of the "optionally substituted cycloalkenyl" exemplified as a substituent for the "optionally substituted cyclic hydrocarbon group" and the "optionally substituted hetercyclic group" represented by R includes C₃₋₆ cycloalkenyl such as cyclopropenyl, cyclobutenyl, cyclopentenyl, cyclohexenyl and the like. Herein, substituents for the cycloalkenyl include the same number of the same groups as those of a substituent for the aforementioned "optionally substituted aryl", and an oxo group, a thioxo group and the like.

The "heterocyclic group" of the "optionally substituted heterocyclic group" exemplified as a substituent for the "optionally substituted cyclic hydrocarbon group" and the "optionally substituted heterocyclic group" represented by R is the same as the heterocyclic group of the "optionally substituted heterocyclic group" represented by R.

Substituents for the "optionally substituted heterocyclic group" include the same number of the same groups as those of a substituent for the aforementioned "optionally substituted aryl", and an oxo group, a thioxo group and the like.

The "alkylsulfinyl" of the "optionally substituted alkylsulfinyl" exemplified as a substituent for the "optionally substituted cyclic hydrocarbon group" and the "optionally substituted heterocyclic group" represented by R includes linear or cyclic C₁₋₆ alkylsulfinyl such as methanesulfinyl, ethanesulfinyl, propanesulfinyl, cyclopropanesulfinyl, cyclopentanesulfinyl, cyclohexanesulfinyl and the like.

The "alkylsulfonyl" of the "optionally substituted alkylsulfonyl" exemplified as a substituent for the "optionally substituted cyclic hydrocarbon group" and the "optionally substituted heterocyclic group" represented by R includes linear or cyclic C₁₋₆ alkylsulfonyl such as methanesulfonyl, ethanesulfonyl, propanesulfonyl, cyclopropanesulfonyl, cyclopentanesulfonyl, cyclohexanesulfonyl and the like.

Substituents for the "optionally substituted amino", the "optionally substituted imidoyl", the "optionally substituted amidino", the "optionally substituted hydroxyl group", the "optionally substituted thiol group", the "optionally substituted alkylsulfinyl" and the "optionally substituted alkylsulfonyl" exemplified as a substituent for the "optionally substituted cyclic hydrocarbon group" and the "optionally substituted heterocyclic group" represented by R include (1) lower alkyl (e.g. C₁₋₆ alkyl such as methyl, ethyl, propyl, isopropyl, butyl, isobutyl, tert-butyl, pentyl, hexyl etc.) which may be substituted with a substituent selected from a halogen atom (e.g fluorine, chlorine, bromine, iodine etc.) and optionally halogenated C₁₋₆ alkoxy (e.g. methoxy, ethoxy, trifluoromethoxy, 2,2,2-trifluoroethoxy, trichloromethoxy, 2,2,2-tricholoroethoxy etc.), (2) acyl such as C₁₋₆ alkanoyl (e.g. formyl, acetyl, propionyl, valeroyl, pivaloyl etc.), benzoyl, C₁₋₆ alkylsulfonyl (e.g. methanesulfonyl etc.), benzenesulfonyl and the like; wherein the acyl may be substituted with amino optionally substituted with a substituent selected from C₁₋₆ lower alkyl (e.g. methyl, ethyl, butyl, isopropyl, cyclopropyl, cyclohexyl etc.), C₁₋₁₀ acyl (e.g. acetyl, propionyl, isopropionyl, benzoyl, p-cholorobenzoyl, 4-methylbenzoyl etc.) and methanesulfonyl, 2-oxo-1-pyrrolidinyl, 2-oxo-1-piperidinyl, 1-acetyl-4-piperidinyl, 1-propionyl-4-piperidinyl or the like (e.g. 2-aminopropionyl, 2-benzoylaminopropionyl, 2-(oxo-1-pyrrolidinyl)propionyl, 2-(2-oxo-1-piperidinyl)propionyl, 2-(1-acetyl-4-piperidinyl)propionyl etc.), (3) optionally halogenated C₁₋₆ alkoxycarbonyl (e.g. methoxycarbonyl, ethoxycarbonyl, trifluoromethoxycarbonyl, 2,2,2-trifluoroethoxycarbonyl, tricholoromethoxycarbonyl, 2,2,2-trichloroethoxycarbonyl etc.), C₁₋₆ alkoxycarbonyl optionally substituted with phenyl (e.g. benzyloxycarbonyl etc.), (4) a hetrocyclic group (the same group as the "heterocyclic group" of the "optionally substituted heterocyclic group" represented by R) and the like. The "amino" of the "optionally substituted amino" as a substituent may be substituted with optionally substituted imidoyl (e.g. C₁₋₆ alkylimidoyl (e.g. formylimidoyl, acetylimidoyl etc.), C₁₋₆ alkoxyimidoyl, C₁₋₆ alkylthioimidoyl, amidino etc.), amino which may be substituted with 1 to 2 C₁₋₆ alkyl groups, or the like, or two substituents may be taken together with a nitrogen atom to form cyclic amino. Such cyclic amino includes 3 to 8-membered (preferably 5 to 6-membered) cyclic amino such as 1-azetidinyl, 1-pyrrolidinyl, piperidino, thiomorpholino, morpholino, 1-piperazinyl, 1-piperazinyl which may have lower alkyl (e.g. C₁₋₆ alkyl such as methyl, ethyl, propyl, isopropyl, butyl, t-butyl, pentyl, hexyl and the like), aralkyl (e.g. C₇₋₁₀ aralkyl such as benzyl, phenethyl etc.), aryl (e.g. C₆₋₁₀ aryl such as phenyl, 1-naphthyl, 2-naphthyl etc.) or the like at the 4-position, 1-pyrrolyl, 1-imidazolyl and the like.

The "optionally esterified carboxyl" exemplified as a substituent for the "optionally substituted cyclic hydrocarbon group" and the "optionally substituted heterocyclic group" represented by R includes, in addition to free carboxyl, lower alkoxycarbonyl, aryloxycarbonyl, aralkyloxycarbonyl and the like.

The "lower alkoxycarbonyl" includes C₁₋₆ alkoxycarbonyl such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl and the like. Among them, C₁₋₃ alkoxycarbonyl such as methoxycarbonyl, ethoxycarbonyl, propoxycarbonyl and the like is preferable.

As the "aryloxycarbonyl", C₇₋₁₂ aryloxycarbonyl such as phenoxycarbonyl, 1-naphthoxycarbonyl, 2-naphthoxycarbonyl and the like is preferable.

As the "aralkyloxycarbonyl", C₇₋₁₀ aralkyloxycarbonyl such as benzyloxycarbonyl, phenethyloxycarbonyl and the like (preferably, C₆₋₁₀ aryl-C₁₋₄ alkoxy-carbonyl etc.) is preferable.

The "aryloxycarbonyl" and the "aralkyloxycarbonyl" may have a substituent and, such a substituent and the number thereof used are the same as a substituent for aryl or aralkyl exemplified as a substituent for N-mono-substituted carbamoyl described later.

The "optionally substituted carbamoyl" exemplified as a substituent for the "optionally substituted cyclic hydrocarbon group" and the "optionally substituted heterocyclic group" represented by R includes, in addition to non-substituted carbamoyl, N-mono-substituted carbamoyl and N,N-di-substituted carbamoyl.

A substituent for the "N-mono-substituted carbamoyl" includes lower alkyl (e.g. C₁₋₆ alkyl such as methyl, ethyl, propyl, isopropyl, butyl, isobutyl, tert-butyl, pentyl, hexyl etc.), lower alkenyl (e.g. C₂₋₆ alkenyl such as vinyl, allyl, isopropenyl, propenyl, butenyl, pentenyl, hexenyl etc.), cycloalkyl (e.g. C₃₋₆ cycloalkyl such as cyclopropyl, cyclobutyl, cyclopentyl, cyclohexyl etc.), aryl (e.g. C₆₋₁₀ aryl such as phenyl, 1-naphtyl, 2-naphthyl etc.), aralkyl (e.g. C₇₋₁₀ aralkyl such as benzyl, phenethyl etc., preferably phenyl-C₁₋₄ alkyl etc.), arylalkenyl (e.g. C₈₋₁₀ alylalkenyl such as cinnamyl etc., preferably phenyl-C₂₋₄ alkenyl etc.), a heterocyclic group (e.g. the same group as the "heterocyclic group" of the "optionally substituted heterocyclic group" represented by R), amino optionally substituted with 1 to 2 C₁₋₆ alkyl groups and the like. The lower alkyl, the lower alkenyl, the cycloalkyl, the aryl, the aralkyl, the arylalkenyl and the heterocyclic group may have a substituent. Such a substituent includes a hydroxyl group, optionally substituted amino [the amino may be substituted with 1 or 2 substituents such as lower alkyl (e.g. C₁₋₄ alkyl such as methyl, ethyl, propyl, isopropyl, butyl, isobutyl, tert-butyl, pentyl, hexyl etc.), acyl (e.g. C₁₋₆ alkanoyl such as formyl, acetyl, propionyl, pivaloyl etc., benzoyl etc.), carboxyl, C₁₋₆-alkoxycarbonyl etc.], a halogen atom (e.g. fluorine, chlorine, bromine, iodine etc.), a nitro group, a cyano group, lower alkyl optionally substituted with 1 to 5 halogen atoms (e.g. fluorine, chlorine, bromine, iodine etc.), lower alkoxy optionally substituted with 1 to 5 halogen atoms (e.g. fluorine, chlorine, bromine, iodine etc.) and the like. The lower alkyl includes C₁₋₆ alkyl such as methyl, ethyl, n-propyl, isopropyl, n-butyl, isobutyl, sec-butyl, tert-butyl, pentyl, hexyl and the like. Particularly, methyl, ethyl and the like are preferable. The lower alkoxy includes C₁₋₆ alkoxy such as methoxy, ethoxy, n-propoxy, isopropoxy, n-butoxy, isobutoxy, sec-butoxy, tert-butoxy and the like. In particular, methoxy, ethoxy and the like are preferable.

In addition, it is preferable that 1 or 2 or 3 (preferably 1 or 2) of these substituents, which may be the same or different each other, are used.

The "N,N-di-substituted carbamoyl" means a carbamoyl group having two substituents on the nitrogen atom. One of the substituents includes the same substituents as the aforementioned substituents for the "N-mono-substituted carbamoyl", and the other includes lower alkyl (e.g. C₁₋₆ alkyl such as methyl, ethyl, propyl, isopropyl, butyl, tert-butyl, pentyl, hexyl etc.), C₁₋₆ cycloalkyl (e.g. cyclopropyl, cyclobutyl, cyclopentyl, cyclohexyl etc.), C₇₋₁₀ aralkyl (e.g. benzyl, phenethyl etc., preferably phenyl-C₁₋₄ alkyl etc.) and the like. The two substituents may be taken together with the nitrogen atom to form a cyclic amino. Such cyclic aminocarbamoyl includes 3- to 8- membered (preferably 5 to 6-membered) cyclic amimocarbonyl such as 1-azetidinylcarbonyl, 1-pyrrolidinylcarbonyl, piperidinocarbonyl, morpholinocarbonyl, thiomorpholinocarbonyl (the sulfur atom may be oxidized), 1-piperazinylcarbonyl, 1-piperazinylcarbonyl optionally having lower alkyl (e.g. C₁₋₆ alkyl such as methyl, ethyl, propyl, isopropyl, butyl, tert-butyl, pentyl, hexyl etc.), aralkyl (e.g. C₇₋₁₀ aralkyl such as benzyl, phenethyl etc.), aryl (e.g. C₆₋₁₀ aryl such as phenyl, 1-naphthyl, 2-naphthyl etc.) and the like at the 4-position, and the like.

Substituents for the "optionally substituted thiocarbamoyl" and the "optionally substituted sulfamoyl" exemplified as a substituent for the "optionally substituted cyclic hydrocarbon group" and the "optionally substituted heterocyclic group" represented by R are the same as substituents for the aforementioned "optionally substituted carbamoyl".

The "sulfonic acid-derived acid" exemplified as a substituent for the "optionally substituted cyclic hydrocarbon group" and the "optionally substituted heterocyclic group" represented by R includes a group obtained by binding of one substituent on the nitrogen atom of the aforementioned "N-mono-substituted carbamoyl" and sulfonyl, and preferable examples include acyl such as C₁₋₆ alkylsulfonyl such as methanesulfonyl and ethanesulfonyl, benzenesulfonyl and p-toluenesulfonyl.

The "carboxylic acid-derived acyl" exemplified as a substituent for the "optionally substituted cyclic hydrocarbon group" and the "optionally substituted heterocyclic group" represented by R includes a group obtained by binding of a hydrogen atom or one substituent on the nitrogen atom of the aforementioned "N-mono-substituted carbamoyl" and carbonyl, and preferable examples include acyl such as C₁₋₆ alkanoyl such as formyl, acetyl, propionyl and pivaloyl, and benzoyl.

R may be preferably an aryl group which may be substituted with a substituent selected from a halogen atom, C₁₋₆ alkyl, C₂₋₆ alkenyl, C₂₋₆ alkynyl, optionally substituted amino, nitro, cyano, optionally substituted amidino and optionally esterified or amidated carbonyl; or a heterocyclic group which may be substituted with a substituent selected from a halogen atom, C₁₋₆ alkyl, C₂₋₆ alkenyl, C₂₋₆ alkynyl, optionally substituted amino, nitro, cyano, optionally substituted amidino and optionally esterified or amidated carboxyl.

Among them, preferably R may be optionally substituted aryl, especially, aryl (preferably, C₆₋₁₄ aryl such as phenyl, 1-naphthyl, 2-naphthyl etc.) which may be substituted with a halogen atom or C₂₋₄ alkenyl (preferably, a halogen atom).

In addition, preferably R may be an optionally substituted heterocyclic group, especially, a heterocyclic group (preferably, indolyl, benzofuranyl, benzopyranyl etc., more preferably indolyl) which may be substituted with a halogen atom.

Among them, R is preferably naphthyl optionally substituted with a halogen atom.

In the above formulas, W represents a bond or an optionally substituted divalent linear hydrocarbon group.

The "divalent linear hydrocarbon group" of the "optionally substituted divalent linear hydrocarbon group" represented by W includes C₁₋₆ alkylene (e.g. methylene, ethylene, trimethylene, tetramethylene etc.), C₂₋₆ alkenylene (e.g. vinylene, propylene, 1-or 2-butenylene, butadienylene etc.) and C₂₋₈ alkynylene (e.g. ethynylene, 1- or 2-propynylene, 1- or 2- butynylene etc.).

Substituents for the "optionally substituted divalent linear hydrocarbon group" represented by W are the same as substituents for the aforementioned "optionally substituted cyclic hydrocarbon group" represented by R.

As W, for example, a bond or C₁₋₆ alkenylene is preferable and, among them, a bond is more preferable.

In the above formulas, X represents an optionally substituted divalent hydrocarbon group.

The "divalent hydrocarbon group" of the "optionally substituted divalent hydrocarbon group" represented by X includes a "divalent linear hydrocarbon group", a "divalent cyclic hydrocarbon group" and a combination thereof.

The "divalent linear hydrocarbon group" is, for example, the same as the "divalent linear hydrocarbon group" of the "optionally substituted divalent linear hydrocarbon group" represented by W as described above.

The "divalent cyclic hydrocarbon group" includes a "divalent cyclic hydrocarbon group" formed by removing any one hydrogen atom form the "cyclic hydrocarbon group" of the "optionally substituted cyclic hydrocarbon group" represented by R, and among them, a divalent aryl group, particularly a phenylene group is preferable, and such a phenylene group includes 1,2-phenylene, 1,3-phenylene and 1,4-phenylene.

As the "optionally substituted divalent hydrocarbon group" represented by X, an optionally substituted divalent linear hydrocarbon group and an optionally substituted phenylene group are preferable and, among them, an optionally substituted divalent linear hydrocarbon group (particularly, optionally substituted C₁₋₆ alkylene) is preferable.

Substitutents for the "optionally substituted divalent hydrocarbon group" represented by X are the same as those for the aforementioned "optionally substituted cyclic hydrocarbon group" represented by R. Among them, preferred are lower alkyl (e.g. C₁₋₆ alkyl such as methyl, ethyl, propyl etc.), lower alkenyl (e.g. C₂₋₆ alkenyl such as vinyl, allyl etc.), lower alkynyl (e.g. C₂₋₆ alkynyl such as ethynyl, propargyl etc.), optionally substituted amino, an optionally substituted hydroxyl group, a cyano group, optionally substituted amidino, carboxy, lower alkoxycarbonyl (e.g. C₁₋₆ alkoxycarbonyl such as methoxycarbonyl, ethoxycarbonyl etc.), an optionally substituted carbamoyl group (e.g. a carbamoyl group optionally substituted with C₁₋₆ alkyl or acyl (e.g. formyl, C₂₋₆ alkanoyl, benzoyl, optionally halogenated C₁₋₆ alkoxycarbonyl, optionally halogenated C₁₋₆ alkylsulfonyl, benzenesulfonyl, p-toluenesulfonyl etc.) etc.) and an oxo group. These substituents may be at 1 to 3 optional substitutable positions.

As X, C₁₋₆ alkylene optionally substituted with a hydroxyl group is preferable. Among them, ethylene optionally substituted with a hydroxyl group (among them, ethylene in which a carbon atom adjacent to Y may be substituted with a hydroxyl group) is particularly preferable and, among them, non-substituted ethylene is particularly preferable.

In the above formulas, Y represents -CO-, -S(O)-, - S(O)₂- or a bond.

As Y, -CO- is preferable.

In the above formulas, ring A represents an optionally substituted pyrrolidine ring, an optionally substituted piperidine ring or an optionally substituted perhydroazepine ring.

Substituent for the "optionally substituted pyrrolidine ring", the "optionally substituted piperidine ring" and the "optionally substituted perhydroazepine ring" represented by ring A are the same group as those for the aforementioned "optionally substituted heterocyclic group" represented by R. These substituents may be at 1 to 5 (preferable 1 to 3) optional substitutable positions. Among them, a C₁₋₆ alkyl group (optionally substituted with a C₁₋₆ alkylsulfinyl group, a C₁₋₆ alkylsulfonyl group, a hydroxyl group or an optionally esterified or amidated carboxyl), a hydroxyl group, an optionally esterified or amidated carboxyl group and an oxo group are preferable.

As ring A, an optionally substituted piperidine ring is preferable, among them, it is preferable that the formula: 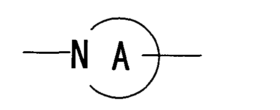 is 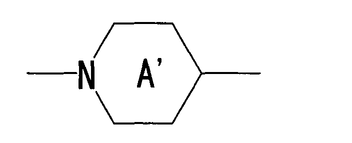 wherein ring A' may be substituted.

In the above formulas, Z¹ and Z³ independently represent a bond or an optionally substituted divalent linear hydrocarbon group.

The "divalent linear hydrocarbon group" of the "optionally substituted divalent linear hydrocarbon group" represented by Z¹ and Z³, respectively, is the same as the "divalent linear hydrocarbon group" of the "optionally substituted divalent linear hydrocarbon group" represented by W.

Substituents for the "optionally substituted divalent linear hydrocarbon group" represented by Z¹ and Z³, respectively, include the same groups and number as those of substituents for the "optionally substituted divalent linear hydrocarbon group" represented by W.

As Z¹, a bond and C₁₋₆ alkylene are preferable.

As Z³, a bond and C₁₋₆ alkylene are preferable.

In the above formulas, Z² represents -N(R¹)-, -O-, - S(O)-, S(O)₂- -CO-, -CH(R¹)- or a bond, wherein R¹ represents a hydrogen atom, an optionally substituted hydrocarbon group, an optionally substituted acyl group, an optionally esterified carboxyl group or an optionally substituted carbamoyl group.

The "hydrocarbon group" of the "optionally substituted hydrocarbon group" represented by R¹ includes alkyl, alkenyl, alkynyl, aryl, cycloalkyl, cycloalkenyl and aralkyl.

The alkyl, the alkenyl, the alkynyl, the aryl, the cycloalkyl and the cycloalkenyl are the same as alkyl, alkenyl, alkynyl, aryl, cycloalkyl and cycloalkenyl of the "optionally substituted alkyl", the "optionally substituted alkenyl", the "optionally substituted alkynyl", the "optionally substituted aryl", the "optionally substituted cycloalkyl" and the "optionally substituted cycloalkenyl" exemplified as substituents for the aforementioned "optionally substituted cyclic hydrocarbon group" represented by R, respectively.

The aralkyl includes a C₇₋₁₆ aralkyl group such as a phenyl-C₁₋₆ alkyl group such as benzyl, phenethyl, 3-phenylpropyl and 4-phenylbutyl, and naphthyl-C₁₋₆ alkyl group such as (1-naphthyl)methyl, 2-(1-naphthyl)ethyl and 2-(2-naphthyl)ethyl.

Substituents for the "optionally substituted hydrocarbon group" represented by R¹ include the same groups and the same number as those of substituents for the aforementioned "optionally substituted cyclic hydrocarbon group" represented by R.

The "optionally substituted acyl group" represented by R¹ includes the same groups as the "sulfonic acid-derived acyl" and the "carboxylic acid-derived acyl" exemplified as a substituent for the "optionally substituted cyclic hydrocarbon group" represented by R.

The "optionally esterified carboxyl group" represented by R¹ includes the same group as the "optionally esterified carboxyl" exemplified as a substituent for the "optionally substituted cyclic hydrocarbon group" represented by R.

The "optionally substituted carbamoyl group" represented by R¹ includes the same group as the "optionally substituted carbamoyl" exemplified as a substituent for the "optionally substituted cyclic hydrocarbon group" represented by R.

In addition, R¹ may be a phosphono group (e.g. (mono-or di-C₁₋₄ alkyl)phosphono which may form a ring, such as dimethylphosphono, diethylphosphono, diisopropylphosphono, dibutylphosphono and 2-oxide-1,3,2-dioxaphosphinan-2-yl).

As Z², -N(R¹)-, -CO- or a bond is preferable.

Ring B represents an optionally substituted imidazole ring.

Substituents for the optionally substituted imidazole ring are the same as those for the aforementioned "optionally substituted cyclic hydrocarbon group" represented by R (provided that an oxo group and a thioxo group are excluded). These optional substituents may be at 1 to 3 (preferably 1 to 2) substitutable positions.

When an imidazole ring represented by ring B has a substituent, the substituent and R¹ may be taken together to form an "optionally substituted ring". The "ring" of the "optionally substituted ring" may be homocyclic or heterocyclic.

The "homocyclic or heterocyclic" ring includes (i) an aromatic or non-aromatic heterocyclic ring containing, preferably one to three, 1 or 2 kinds of heteroatoms selected from a nitrogen atom, a sulfur atom and an oxygen atom in addition to carbon atoms, and (ii) cyclic hydrocarbon consisting of carbon atoms (homocyclic ring).

The "aromatic heterocyclic ring" includes a 5 to 6- membered aromatic heterocyclic ring containing 1 to 3 heteroatoms selected from a nitrogen atom, an oxygen atom and a sulfur atom in addition to carbon atoms (e.g. pyridine, pyrazine, pyrimidine, pyridazine, pyrrole, imidazole, pyrazole, triazole, thiophen, furan, thiazole, isothiazole, oxazole and isoxazole rings).

The "non-aromatic heterocyclic ring" includes a 5 to 9-membered (preferably 5 or 6-membered) non-aromatic heterocyclic ring containing 1 to 3 heteroatoms selected from a nitrogen atom, an oxygen atom and a sulfur atom in addition to carbon atoms (e.g. tetrahydropyridine, dihydropyridine, tetrahydropyrazine, tetrahydropyrimidine, tetrahydropyridazine, dihydropyran, dihydropyrrole, dihydrothiophen, dihydrofuran, piperidine, piperazine, hexahydropyrimidine, hexahydropyridazine, tetrahydropyran, morpholine, pyrrolidine, pyrazoline, imidazolidine, thiazoline, isothiazoline, oxazoline, isoxazoline, pyrazolidine, tetrahydrothiophen, tetrahydrofuran, tetrahydrothiazole, tetrahydroisothiazole, tetrahydrooxazole, tetrahydroisoxazole ring etc).

The "cyclic hydrocarbon (homocyclic ring)" includes a 3 to 10-membered (preferably 5 to 9-membered, more preferably 5 or 6-membered) cyclic hydrocarbon, for example, benzene, C₃₋₁₀ cycloalkene (e.g. cyclobutene, cyclopentene, cyclohexene, cycloheptene, cyclooctene etc.), C₃₋₁₀ cycloalkane (e.g.cyclobutane, cyclopentane, cyclohexane, cycloheptane, cyclooctane etc.) and the like. Cycloalkene may be preferably C₅₋₆ cycloalkene (e.g. cyclopentene, cyclohexne etc.) and cycloalkane may be preferably C₅₋₆ cycloalkane (e.g. cyclohexane, cyclopentane).

As a "ring" formed by binding of a substituent on an imidazole ring represented by ring B and R¹, for example, a 5 to 9-membered (preferably 5 or 6-membered) non-aromatic heterocyclic ring containing 1 to 2 (preferably 2) nitrogen atoms in addition to carbon atoms is preferable. Among them, more preferable examples thereof include tetrahydropyridine, tetrahydropyrazine, tetrahydropyrrole and tetrahydroimidazole.

Substituents for the "optionally substituted ring" formed by binding of a substituent on an imidazole ring represented by ring B and R¹ include the same group as substituents for the aforementioned "optionally substituted heterocyclic group" represented by R. These optional substituents may be at 1 to 4 (preferably 1 to 3) substitutable positions. As substituents for the "optionally substituted ring", among them, a C₁₋₆ alkyl group optionally substituted with a hydroxyl group, a hydroxyl group, an oxo group and the like are preferable.

In ring B, the formula: 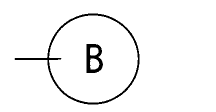 is preferably 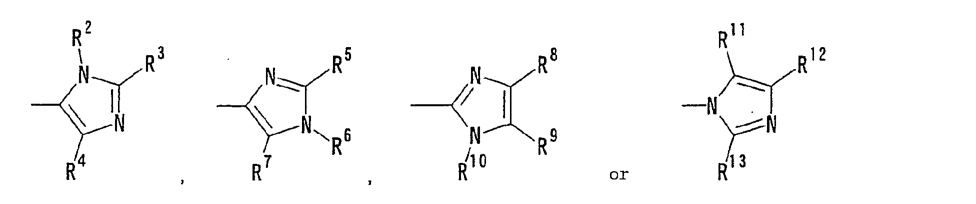 wherein R², R³, R⁴, R⁵, R⁶, R⁷, R⁸, R⁹, R¹⁰, R¹¹, R¹² and R¹³ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group or an optionally substituted amino group, or R² and R³, R⁵ and R⁶, R⁶ and R⁷, R⁸ and R⁹, R⁹ and R¹⁰, or R¹¹ and R¹² may be taken together to form an optionally substituted ring.

The "hydrocarbon group" of the "optionally substituted hydrocarbon group" represented by R², R³, R⁴, R⁵, R⁶, R⁷, R⁸, R⁹, R¹⁰, R¹¹, R¹² and R¹³ is, for example, the same as the "hydrocarbon group" of the aforementioned "optionally substituted hydrocarbon group" represented by R¹. Substituents for the "optionally substituted hydrocarbon group" include the same groups and number as those of a substituent for the aforementioned "optionally substituted hydrocarbon group" represented by R¹.

Substituents for the "optionally substituted hydroxyl group", the "optionally substituted thiol group", the "optionally substituted alkylsulfinyl group" and the "optionally substituted alkylsulfonyl group" represented by R², R³, R⁴, R⁵, R⁶, R⁷, R⁸, R⁹, R¹⁰, R¹¹, R¹² and R¹³ include the same groups as substituents for the "optionally substituted hydroxyl group", the "optionally substituted thiol group", the "optionally substituted alkylsulfinyl" and the "optionally substituted alkylsulfonyl" exemplified as a substituent for the "optionally substituted cyclic hydrocarbon group" represented by R, respectively.

The "optionally substituted acyl group" represented by R², R³, R⁴, R⁵, R⁶, R⁷, R⁸, R⁹, R¹⁰, R¹¹, R¹² and R¹³ includes the same groups as the "sulfonic acid-derived acyl" and the "carboxylic acid-derived acyl" exemplified as a substituent for the "optionally substituted cyclic hydrocarbon group" represented by R.

The "optionally esterified carboxyl group" represented by R², R³, R⁴, R⁵, R⁶, R⁷, R⁸, R⁹, R¹⁰, R¹¹, R¹² and R¹³ includes the same group as the "optionally esterified carboxyl" exemplified as a substituent for the "optionally substituted hydrocarbon group" represented R.

The "optionally substituted carbamoyl group" represented by R², R³, R⁴, R⁵, R⁶, R⁷, R⁸, R⁹, R¹⁰, R¹¹, R¹² and R¹³ includes the same group as the "optionally substituted carbamoyl" exemplified as a substituent for the aforementioned "optionally substituted cyclic hydrocarbon group" represented by R¹.

The "optionally substituted amino group" represented by R², R³, R⁴, R⁵, R⁶, R⁷, R⁸, R⁹, R¹⁰, R¹¹, R¹² and R¹³ includes the same group as the "optionally substituted amino" exemplified as a substituent for the aforementioned "optionally substituted cyclic hydrocarbon group" represented by R.

The "ring" of the "optionally substituted ring" which may be formed by binding of R² and R³, R⁵ and R⁶, R⁶ and R⁷, R⁸ and R⁹, R⁹ and R¹⁰, or R¹¹ and R¹² includes the same ring as the aforementioned "ring" formed by binding of a substituent on ring B and R¹. Substituents for the "optionally substituted ring" include the same group as substituents for the aforementioned "optionally substituted heterocyclic group" represented by R. These optional substituents may be at 1 to 5 (preferably 1 to 3) substitutable positions.

In addition, R², R³, R⁴, R⁵, R⁶ R⁷, R⁸ R⁹ R¹⁰ R¹¹ R¹² and R¹³ may be a phosphono group (e.g. (mono-or di-C₁₋₄ alkyl)phosphono which may form a ring, such as dimethylphosphono, diethylphosphono, diisopropyolphosphono, dibutylphosphono and 2-oxide-1,3,2-dioxaphosphinan-2-yl).

In a preferable aspect of ring B, for example, the formula: 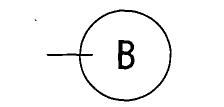 is 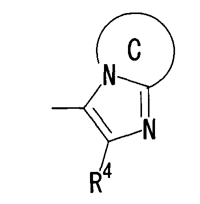 wherein ring C represents an optionally substituted nitrogen-containing heterocyclic ring, and other symbols are same as defined above.

The "nitrogen-containing heterocyclic ring" of the "optionally substituted nitrogen-containing heterocyclic ring" represented by ring C includes a non-aromatic nitrogen-containing heterocyclic ring (e.g. pyrrolidine, piperidine, perhydroazepine etc.) or a ring which may be fused with an imidazole ring to form an aromatic fused nitrogen-containing heterocyclic ring (e.g. a ring represented by the formula: 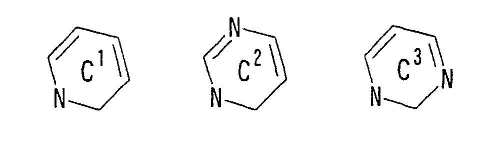 or 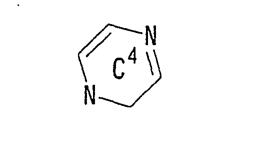 wherein rings C¹, C², C³ and C⁴ may be substituted). By fusion of such a ring with an imidazole ring, for example, a fused ring represented by the formula: 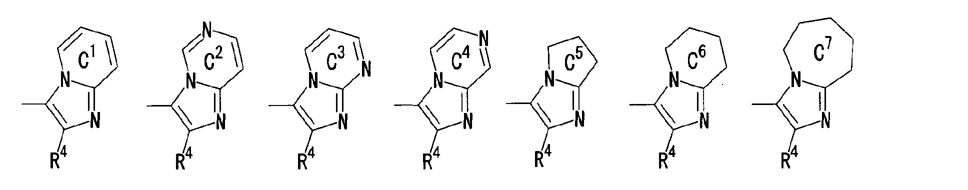 wherein rings C¹, C², C³, C⁴, C⁵, C⁶ and C⁷ may be substituted, may be formed.

Substituents for the "optionally substituted nitrogen-containing heterocyclic ring" represented by ring C (and substituents for rings C¹, C², C³, C⁴, C⁵, C⁶ and C⁷) include the same groups as substituents for the aforementioned "optionally substituted heterocyclic group" represented by R, and these optional substituents may be at 1 to 5 (preferably 1 to 3) substitutable positions.

In a preferable aspect of a compound represented by the formula (I), for example, a substituent of an imidazole ring represented by ring B and R¹ together do not form a ring.

In another preferable aspect of a compound represented by the formula (I), for example, Z² is -N (R¹) - or -CH (R¹) - (R¹ is as defined above) and a substituent of an imidazole ring represented by ring B is taken together with R¹ to form an optionally substituted ring.

In a preferable aspect of a compound represented by the formula (I), inter alia, the formula (I) is the formula (Ia) : 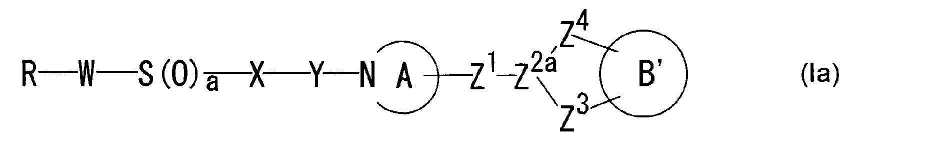 wherein ring B' represents an optionally further substituted imidazole ring, Z^{2a} represents N or CH, Z⁴ represents an optionally substituted divalent linear hydrocarbon group, and other symbols are as defined above.

Herein, Z^{2a} is preferably a nitrogen atom.

The "divalent linear hydrocarbon group" of the "optionally substituted divalent linear hydrocarbon group" represented by Z⁴ includes the same group as the "divalent linear hydrocarbon group" of the "optionally substituted divalent linear hydrocarbon group" represented by W.

Substituents for the "optionally substituted divalent linear hydrocarbon group" represented by Z⁴ include the same groups and number as those of substituents for the "optionally substituted divalent linear hydrocarbon group" represented by W.

Preferably, Z³ and Z⁴ are independently a divalent linear hydrocarbon group which may be substituted with an oxo group (preferably, C₁₋₆ alkylene which may be substituted with an oxo group).

In a preferable aspect of a compound represented by the formula (I), for example, the formula (I) is the formula (Ib): 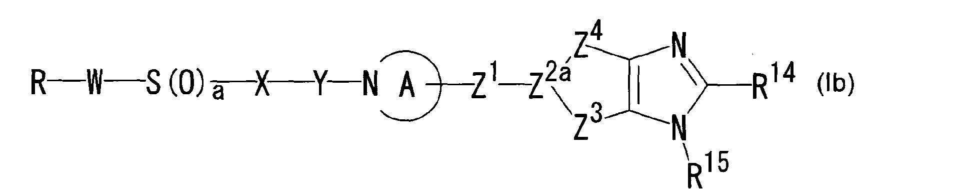 wherein R¹⁴ and R¹⁵ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group or an optionally substituted amino group, or R¹⁴ and R¹⁵ may be taken together to form an optionally substituted ring, and other symbols are as defined above.

The "hydrocarbon group" of the "optionally substituted hydrocarbon group" represented by R¹⁴ and R¹⁵ includes the same group as the "hydrocarbon group" of the aforementioned "optionally substituted hydrocarbon group" represented by R¹. Substituents for "optionally substituted hydrocarbon group" include the same groups and number as those of substituents for the aforementioned "optionally substituted hydrocarbon group" represented by R¹.

Substituents for the "optionally substituted hydroxyl group", the "optionally substituted thiol group", the "optionally substituted alkylsulfinyl group" and the "optionally substituted alkylsulfonyl group" represented by R¹⁴ and R¹⁵ include the same groups as substituents for the "optionally substituted hydroxyl group", the "optionally substituted thiol group", the "optionally substituted alkylsulfinyl" and the "optionally substituted alkylsulfonyl" exemplified as a substituent for the "optionally substituted cyclic hydrocarbon group" represented by R.

The "optionally substituted acyl group" represented by R¹⁴ and R¹⁵ includes the same groups as the "sulfonic acid-derived acyl" and the "carboxylic acid-derived acyl" exemplified as a substituent for the "optionally substituted cyclic hydrocarbon group" represented by R.

The "optionally esterified carboxyl group" represented by R¹⁴ and R¹⁵ includes the same group as the "optionally esterified carboxyl" exemplified as a substituent for the "optionally substituted cyclic hydrocarbon group" represented by R.

The "optionally substituted carbamoyl group" represented by R¹⁴ and R¹⁵ includes the same group as the "optionally substituted carbamoyl" exemplified as a substituent for the "optionally substituted cyclic hydrocarbon group" represented by R.

The "optionally substituted amino group" represented by R¹⁴ and R¹⁵ includes the same group as the "optionally substituted amino" exemplified as a substituent for the "optionally substituted cyclic hydrocarbon group" represented by R.

The "ring" of the "optionally substituted ring" which may be formed by binding of R¹⁴ and R¹⁵ includes the same ring as the aforementioned "ring" formed by binding a substituent on ring B and R¹. Substituents for the "optionally substituted ring" include the same groups as substituents for the aforementioned "optionally substituted heterocyclic group" represented by R. These optional substituents may be at 1 to 5 (preferably 1 to 3) substitutable positions.

Alternatively, R¹⁴ and R¹⁵ may be a phosphono group (e.g. (mono-or di-C₁₋₄ alkyl)phosphono which may form a ring, such as dimethylphosphono, diethylphosphono, diisopropylphosphono, dibutylphosphono and 2-oxide-1,3,2-dioxaphosphinan-2-yl).

In a preferable aspect of a compound represented by the formula (I), for example, the formula (I) is the formula (Ic): 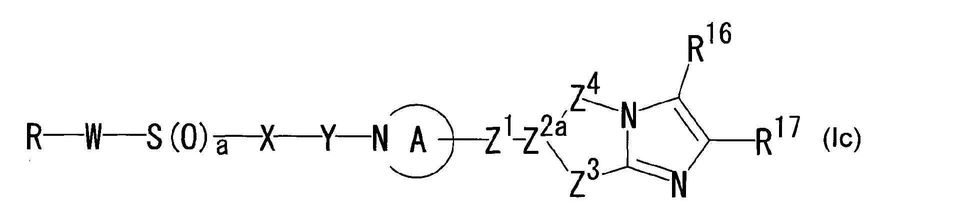 wherein R¹⁶ and R¹⁷ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group or an optionally substituted amino group, or R¹⁶ and R¹⁷ may be taken together to form an optionally substituted ring, and other symbols are as defined above.

The "hydrocarbon group" of the "optionally substituted hydrocarbon group" represented by R¹⁶ and R¹⁷ includes the same group as the "hydrocarbon group" of the aforementioned "optionally substituted hydrocarbon group" represented by R¹. Substituents for the "optionally substituted hydrocarbon group" include the same groups and number as those of substituents for the aforementioned "optionally substituted hydrocarbon group" represented by R¹.

Substituents for the "optionally substituted hydroxyl group", the "optionally substituted thiol group", the "optionally substituted alkylsulfinyl group" and the "optionally substituted alkylsulfonyl group" represented by R¹⁶ and R¹⁷ include the same groups as substituents for the "optionally substituted hydroxyl group", the "optionally substituted thiol group", the "optionally substituted alkylsulfinyl" and the "optionally substituted alkylsulfonyl" exemplified as a substituent for the "optionally substituted cyclic hydrocarbon group" represented by R, respectively.

The "optionally substituted acyl group" represented by R¹⁶ and R¹⁷ includes the same groups as the "sulfonic acid-derived acyl" and the "carboxylic acid-derived acyl" exemplified as a substituent for the "optionally substituted cyclic hydrocarbon group" represented by R.

The "optionally esterified carboxyl group" represented by R¹⁶ and R¹⁷ includes the same group as the "optionally esterified carboxyl" exemplified as a substituent for the "optionally substituted cyclic hydrocarbon group" represented by R.

The "optionally substituted carbamoyl group" represented by R¹⁶ and R¹⁷ includes the same group as the "optionally substituted carbamoyl" exemplified as a substituent for the "optionally substituted cyclic hydrocarbon group" represented by R.

The "optionally substituted amino group" represented by R¹⁶ and R¹⁷ includes the same group as the "optionally substituted amino" exemplified as a substituent for the "optionally substituted cyclic hydrocarbon group" represented by R.

The "ring" of the "optionally substituted ring" which may be formed by binding of R¹⁶ and R¹⁷ includes the same ring as the aforementioned "ring" formed by binding of a substituent on ring B and R¹. Substituents for the "optionally substituted ring" include the same group as substituents for the aforementioned "optionally substituted heterocyclic group" represented by R. These optional substituents may be at 1 to 5 (preferably 1 to 3) substitutable positions.

Alternatively, R¹⁶ and R¹⁷ may be a phosphono group (e.g. (mono-or di-C₁₋₄ alkyl)phosphono which may form a ring, such as dimethylphosphono, diethylphosphono, diisopropylphosphono, dibutylphosphono, 2-oxide-1,3,2-dioxaphosphinan-2-yl).

In a preferable aspect of a compound represented by the formula (I), for example, the formula (I) is the formula (Id): 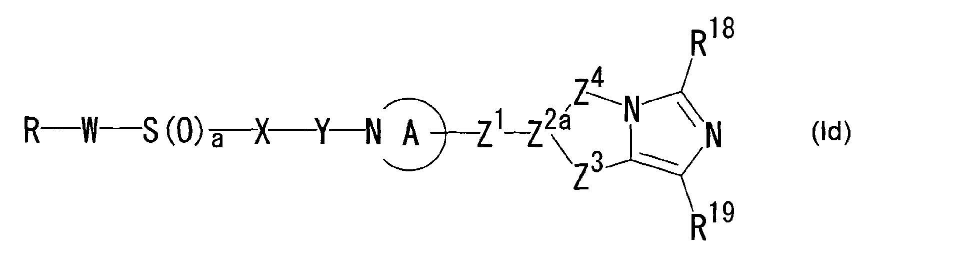 wherein R¹⁸ and R¹⁹ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group or an optionally substituted amino group, and other symbols are as defined above.

The "hydrocarbon group" of the "optionally substituted hydrocarbon group" represented by R¹⁸ and R¹⁹ includes the same group as the "hydrocarbon group" of the aforementioned "optionally substituted hydrocarbon group" represented by R¹. Substituents for the "optionally substituted hydrocarbon group" include the same groups and number as those of substituents for the aforementioned "optionally substituted hydrocarbon group" represented by R¹.

Substituents for the "optionally substituted hydroxyl group", the "optionally substituted thiol group", the "optionally substituted alkysulfinyl group" and the "optionally substituted alkylsulfonyl group" represented by R¹⁸ and R¹⁹ include the same groups as substituents for the "optionally substituted hydroxyl group", the "optionally substituted thiol group", the "optionally substituted alkylsulfinyl" and the "optionally substituted alkylsulfonyl" exemplified as a substituent for the "optionally substituted hydrocarbon group" represented by R, respectively.

The "optionally substituted acyl group" represented by R¹⁸ and R¹⁹ includes the same groups as the "sulfonic acid-derived acyl" and the "carboxylic acid-derived acyl" exemplified as a substituent for the "optionally substituted cyclic hydrocarbon group" represented by R.

The "optionally esterified carboxyl group" represented by R¹⁸ and R¹⁹ includes the same group as the "optionally esterified carboxyl" exemplified as a substituent for the "optionally substituted cyclic hydrocarbon group" represented by R.

The "optionally substituted carbamoyl group" represented by R¹⁸ and R¹⁹ includes the same group as the "optionally substituted carbamoyl" exemplified as a substituent for the "optionally substituted cyclic hydrocarbon group" represented by R.

The "optionally substituted amino group" represented by R¹⁸ and R¹⁹ includes the same group as the "optionally substituted amino" as a substituent for the aforementioned "optionally substituted cyclic hydrocarbon group" represented by R.

Alternatively, R¹⁸ and R¹⁹ may be a phosphono group (e.g. (mono-or di-C₁₋₄ alkyl)phosphono which may form a ring, such as dimethylphosphono, diethylphosphono, diisopropylphosphono, dibutylphosphono and 2-oxide-1,3,2-dioxaphosphinan-2-yl).

In an aspect of a compound represented by the formula (I), inter alia, the formula (I) is preferably the formula (Id), and more preferably, the formula (I) is the formula (Id) and Z^{2a} is a nitrogen atom.

In the above formulas, a indicates 0, 1 or 2 (preferably 2).

Compounds represented by the formula (I) in the present invention preferably include 7-(1-{3-[(6-chloro-2-naphthyl)sulfonyl]propanoyl}-4-piperidinyl)-3-methyl-6,7-dihydroimidazo[1,5-a]pyrazin-8(5H)-one, 7-(1-{3-[(6-chloro-2-naphthyl)sulfonyl]propanoyl}-4-piperidinyl)-1-methyl-6,7-dihydroimidazo[1,5-a]pyradin-8(5H)-one, 2-(1-{3-[(6-chloro-2-naphthyl)sulfonyl]propanoyl}-4-piperidinyl)-5-methyl-1,2-dihydro-3H-imidazo[1,5-c]imidazol-3-one, 2-(1-{3-[(6-chloro-2-naphthyl)sulfonyl]propanoyl}-4-piperidinyl)-5,7-dimethyl-1,2-dihydro-3H-imidazo[1,5-c]imidazol-3-one, 2-(1-{3-[(7-chloro-2H-chromen-3-yl)sulfonyl]propanoyl}-4-piperidinyl)-5-methyl-1,2-dihydro-3H-imidazo[1,5-c]imidazol-3-one, 2-[1-(3-{[(E)-2-(4-chlorophenyl)vinyl]sulfonyl}propanoyl)-4-piperidinyl]-5-methyl-1,2-dihydro-3H-imidazo[1,5-c]imidazol-3-one, 2-(1-{3-[(5-chloro-1H-indol-2-yl)sulfonyl]propanoyl}-4-piperidinyl)-5-methyl-1,2-dihydro-3H-imidazo[1,5-c]imidazol-3-one, 2-(1-{3-[(6-chloro-2-naphthyl)sulfonyl]propanoyl}-4-piperidinyl)-5-(hydroxymethyl)-1,2-dihydro-3H-imidazo[1,5-c]imidazol-3-one, 2-(1-{(2S)-3-[(6-chloro-2-naphthyl)sulfonyl]-2-hydroxypropanoyl}-4-piperidinyl)-5-(hydroxymethyl)-1,2-dihydro-3H-imidazo[1,5-c]imidazol-3-one, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 1-acetylpiperidine-4-carboxylate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(2-oxo-1-pyrrolidinyl)propionate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl (2-oxo-1-pyrrolidinyl)acetate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 4-(acetylamino)butanoate, and 2-(1-{(2S)-3-[(6-chloro-2-naphthyl)sulfonyl]-2-hydroxypropanoyl}-4-piperidinyl)-5,7-dimethyl-1,2-dihydro-3H-imidazo[1,5-c]imidazol-3-one.

A salt of a compound represented by the formula (I) (hereinafter, abbreviated as Compound (I) in some cases) includes pharmaceutically acceptable salts, for example, acid addition salts with acids such as trifluoroacetic acid, acetic acid, lactic acid, succinic acid, maleic acid, tartaric acid, citric acid, gluconic acid, ascorbic acid, benzoic acid, methanesulfonic acid, p-toluenesulfonic acid, cinnamic acid, fumaric acid, phosphonic acid, hydrochloric acid, nitric acid, hydrobromic acid, hydriodic acid, sulfamic acid, sulfuric acid and the like, metal salts such as sodium, potassium, magnesium, calcium and the like, and organic salts such as trimethylamine, triethylamine, pyridine, picoline, N-methylpyrrolidine, N-methylpiperidine, N-methylmorpholine and the like.

A prodrug of Compound (I) refers to a compound which is converted into Compound (I) by a reaction due to an enzyme or gastric acid under the physiological condition in a living body, that is, a compound which is changed into Compound (I) by enzymatic oxidation, reduction, hydrolysis or the like, or a compound which is changed into Compound (I) by hydrolysis with gastric acid. A prodrug of Compound (I) includes a compound obtained by acylating, alkylating or phosphorylating the amino group of Compound (I) (e.g. a compound obtained by eicosanoylating, alanylating, pentylaminocarbonylating, (5-methyl-2-oxo-1,3-dioxolen-4-yl)methoxycarbonylating, tetrahydrofuranylating, pyrrolidylmethylating, pivaloyloxymethylating or tert-butylating the amino group of Compound (I)), a compound obtained by acylating, alkylating, phosphorylating or borating the hydroxyl group of Compound (I) (e.g. a compound obtained by acetylating, palmitoylating, propanoylating, pivaloylating, succinylating, fumarylating, alanylating or dimethylaminomethylcarbonylating the hydroxyl group of Compound (I)) and a compound obtained by esterifying or amidating the carboxyl group of Compound (I) (e.g. a compound obtained by ethylesterifying, phenylesterifying, carboxymethylesterifying, dimethylaminomethylesterifying, pivaloyloxymethylesterifying, ethoxycarbonyloxyethylesterifying, phthalidylesterifying, (5-methyl-2-oxo-1,3-dioxolen-4-yl)methylesterifying, cyclohexyloxycarbonylethylesterifying or methylamidating the carboxyl group of Compound (I)). These compounds can be prepared from Compound (I) by a known method per se.

A prodrug of Compound (I) may be also a compound which is changed into Compound (I) under the physiological condition as described in "Development of Medicaments", vol. 7, Molecular Design, p.163-198 published by HIROKAWASHOTEN in 1990.

Compound (I) may be labeled with an isotope (e.g. ³H, ¹⁴C, ³⁵S, ¹²⁵I) or the like.

Compound (I) or a salt thereof can be prepared, for example, by the following methods (A) to (E). Each compound described in the following schemes may form a salt as far as it does not inhibit the reaction. Such a salt includes the same salts as those of Compound (I).

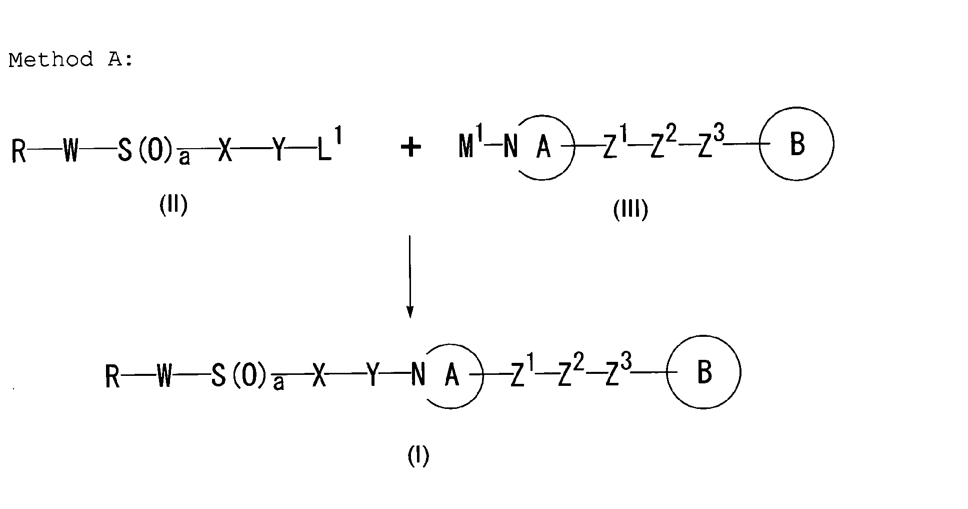

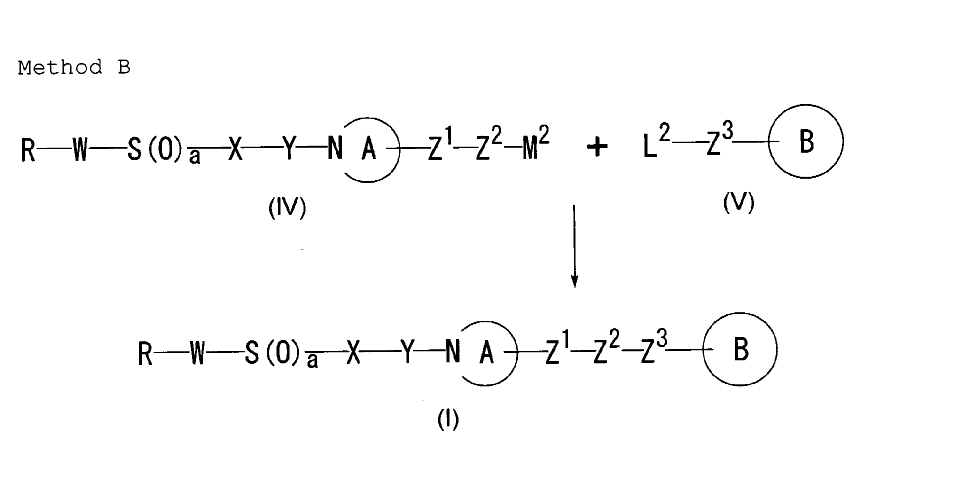

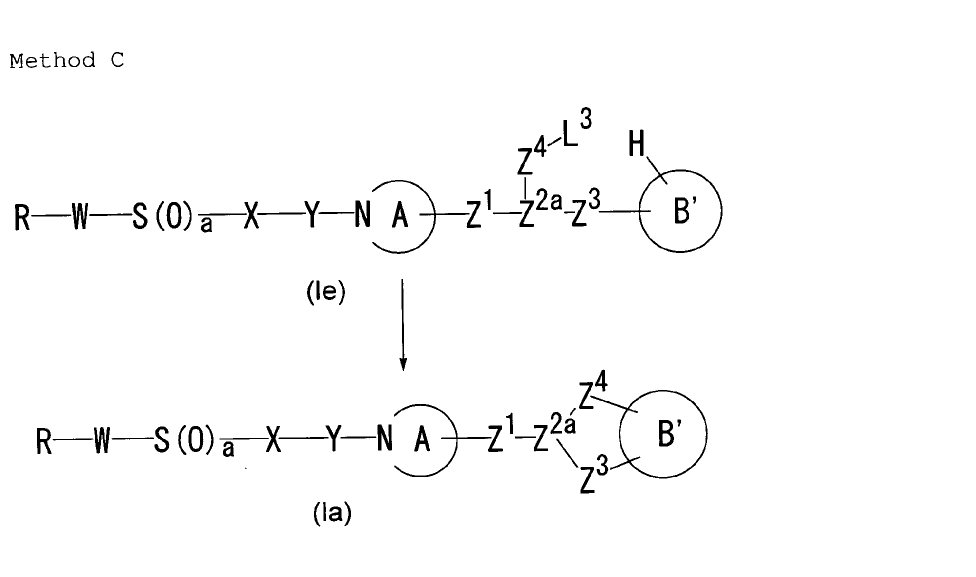

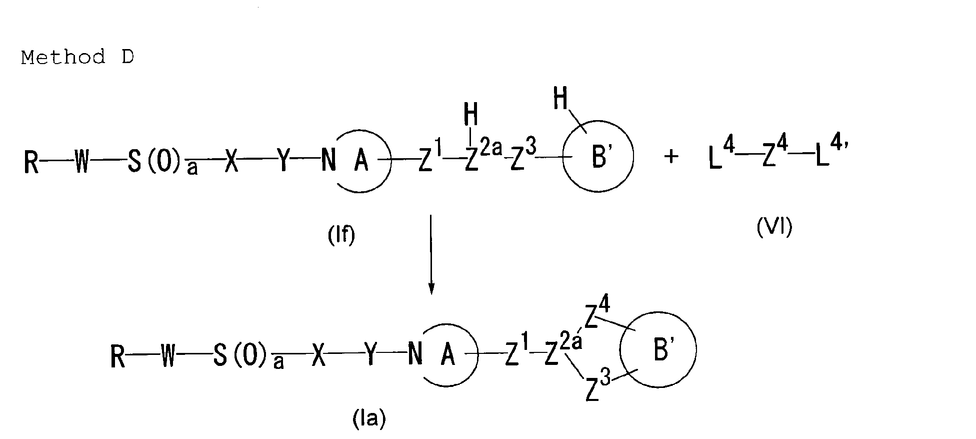

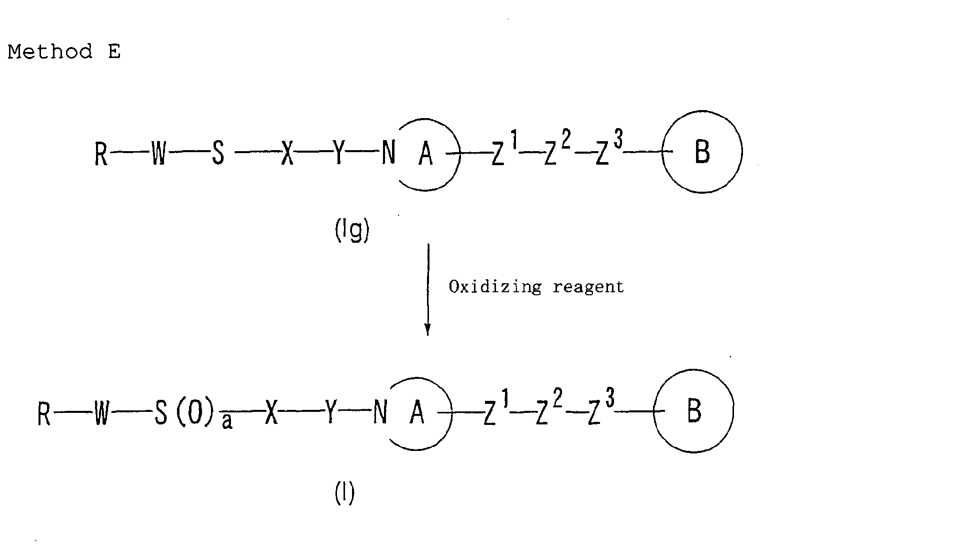

### Method A

Compound (I) can be prepared by reacting Compound (II) represented by the formula (II): 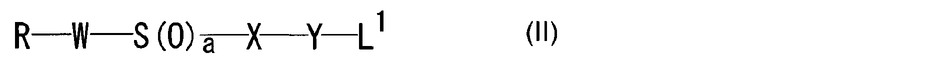 wherein L¹ represents a leaving group (e.g. a group forming free carboxylic acid, a salt thereof (inorganic salt, organic salt etc.) or a reactive derivative thereof (e.g. acid halide, ester, acid azide, acid anhydride, mixed acid anhydride, active amide, active ester, active thioester etc.), such as a halogen atom (e.g. fluorine, chlorine, bromine, iodine etc.), C₁₋₆ alkylsulfonyloxy group optionally substituted with 1 to 3 halogen atoms (e.g. methanesulfonyloxy, ethanesulfonyloxy, trifluoromethanesulfonyloxy etc.), an optionally substituted arylsulfonyloxy group (e.g. benzenesulfonyloxy, p-toluenesulfonyloxy, p-bromobenzenesulfonyloxy etc.) or a hydroxyl group), and other symbols are as defined above, (in particular, Compound (II) wherein L¹ is a hydroxyl group is referred to as free acid (II')), with Compound (III) represented by the formula (III); 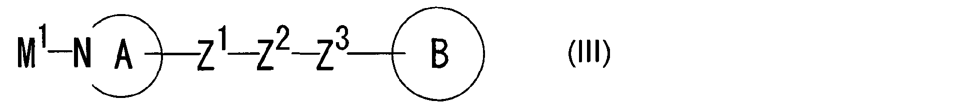 wherein M¹ represents a hydrogen atom, an alkaline metal (e.g. lithium, sodium, potassium, cesium etc.), an alkaline earth metal (e.g. magnesium, calcium etc.) or a leaving group (e.g. trimethylsilyl group), and other symbols are as defined above.

Alternatively, Compound (I) can be prepared by reacting Compound (III) or a salt thereof with free acid (II') or a salt thereof (inorganic salt, organic salt etc.) or a derivative thereof (e.g. acid halide, ester, acid azide, acid anhydride, mixed acid anhydride, active amide, active ester, active thioester etc.). A salt of Compound (III) includes acid addition salts with the above-mentioned acids which may form an acid addition salt with Compound (I) .

An inorganic salt of Compound (II) includes an alkaline metal salt (e.g. lithium salt, sodium salt, potassium salt, cesium salt etc.), and an alkaline earth metal salt (e.g. magnesium salt, calcium salt etc.). An organic salt of Compound (II) includes a trimethylamine salt, a triethylamine salt, a tert-butyldimethylamine salt, a dibenzylmethylamine salt, a benzyldimethylamine salt, a N,N-dimethylaniline salt, a pyridine salt, a quinoline salt and the like. Acid halide includes acid chloride, acid bromide and the like. Ester includes lower alkyl esters such as methyl, ethyl and the like. A mixed acid anhydride includes a mono C₁₋₄ alkylcarbonic acid mixed acid anhydride (e.g. a mixed acid anhydride of free acid (II') with monomethyl carbonate, monoethyl carbonate, monoisopropyl carbonate, monoisobutyl carbonate, mono-tert-butyl carbonate, monobenzyl carbonate, mono(p-nitrobenzyl) carbonate, monoallyl carbonate etc.), a C₁₋₆ aliphatic carboxylic acid mixed acid anhydride (e.g. a mixed acid anhydride of free acid (II) with acetic acid, cyanoacetic acid, propionic acid, butyric acid, isobutyric acid, valeric acid, isovaleric acid, pivalic acid, trifluoroacetic acid, trichloroacetic acid, acetoacetic acid etc.), a C₇₋₁₁ aromatic carboxylic acid mixed acid anhydride (e.g. a mixed acid anhydride of free acid (II') with benzoic acid, p-toluic acid, p-chlorobenzoic acid etc.), an organic sulfonic acid mixed acid anhydride (e.g. a mixed acid anhydride with methanesulfonic acid, ethanesulfonic acid, benzenesulfonic acid, p-toluenesulfonic acid etc.) and the like. Active amide includes amide with a nitrogen-containing heterocyclic compound (e.g. acid amide of free acid (II') with pyrazole, imidazole, benzotriazole etc., and these nitrogen-containing heterocyclic compounds may be substituted with C₁₋₆ alkyl (e.g. methyl, ethyl, propyl, isopropyl, butyl, isobutyl, sec-butyl, tert-butyl etc.), C₁₋₆ alkoxy (e.g. methoxy, ethoxy, propoxy, isopropoxy, butoxy, tert-butoxy etc.), a halogen atom (e.g. fluorine, chlorine, bromine etc.), oxo, thioxo, C₁₋₆ alkylthio (e.g. methylthio, ethylthio, propylthio, butylthio etc.), etc.) and the like.

Active ester includes, in addition to organic phosphoric acid ester (e.g. diethoxyphosphoric acid ester, diphenoxyphosphoric acid ester etc.), p-nitrophenyl ester, 2,4-dinitrophenyl ester, cyanomethyl ester, pentachlorophenyl ester, N-hydroxysuccinimide ester, N-hydroxyphthalimide ester, 1-hydroxybenzotriazole ester, 6-chloro-1-hydroxybenzotriazole ester, 1-hydroxy-1H-2-pyridone ester and the like. Active thioester includes esters with aromatic heterocyclic thiol compounds [these heterocyclic rings may be substituted with C₁₋₆ alkyl (e.g. methyl, ethyl, propyl, isopropyl, butyl, isobutyl, sec-butyl, tert-butyl etc.), C₁₋₆ alkoxy (e.g. methoxy, ethoxy, propoxy, isopropoxy, butoxy, tert-butoxy etc.), a halogen atom (e.g. fluorine, chlorine, bromine etc.), C₁₋₆ alkylthio (e.g. methylthio, ethylthio, propylthio, butylthio etc.) etc.] (e.g. 2-pyridylthiol ester, 2-benzothiazolylthiol ester) and the like.

This reaction is generally performed in a solvent and, if needed, in the presence of a base or a condensing agent (e.g. carbodiimides such as DCC, WSC, DIC etc.), phosphoric acid derivative (e.g. diethyl cyanophosphate, DPPA, DOP-Cl etc.), 4-(4,6-dimethoxy-1,3,5-triazin-2-yl)-4-methylmorpholinium chloride (DMTMM:Kunishima et al., Tetrahedron, 1999, 55, 13159) or the like.

As a solvent, a solvent which does not inhibit the reaction is appropriately selected and, for example, alcohols (e.g. methanol, ethanol, propanol, isopropanol, butanol, tert-butanol etc.), ethers (e.g. dioxane, tetrahydrofuran, diethyl ether, tert-butyl methyl ether, diisopropyl ether, ethylene glycol-dimethyl ether etc.), esters (e.g. ethyl formate, ethyl acetate, n-butyl acetate etc.), carboxylic acids (e.g. formic acid, acetic acid, propionic acid etc.), halogenated hydrocarbons (e.g. dichloromethane, chloroform, carbon tetrachloride, trichloroethylene, 1,2-dichloroethane, chlorobenzene etc.), hydrocarbons (e.g. n-hexane, benzene, toluene etc.), amides (e.g. formamide, N,N-dimethylformamide, N,N-dimethylacetamide etc.), ketones (e.g. acetone, methyl ethyl ketone, methyl isobutyl ketone etc.), nitriles (e.g. acetonitrile, propionitrile etc.) and, additionally, dimethyl sulfoxide, sulfolane, hexamethylphosphoramide, water and the like are used alone or as a mixed solvent.

A base includes inorganic bases such as lithium hydroxide, potassium hydroxide, sodium hydroxide, calcium hydroxide, sodium carbonate, potassium carbonate, sodium hydrogencarbonate, potassium hydrogencarbonate and the like; C₁₋₆ lower fatty acid alkaline metal salts such as sodium formate, sodium acetate, potassium acetate and the like; and tertiary amines such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine and the like.

In this reaction, 0.5 to 5 equivalents, preferably 0.8 to 2 equivalents of Compound (III) is used based on the amount of Compound (II).

The reaction temperature is -50 to 150°C, preferably -20 to 100°C.

The reaction time varies depending on the kind of Compound (II) or (III), the kinds of a solvent and a base, the reaction temperature and the like. It is usually 1 minute to about 100 hours, preferably about 15 minutes to about 48 hours.

### Method B

Compound (I) can be prepared by reacting Compound (IV) represented by the formula (IV): 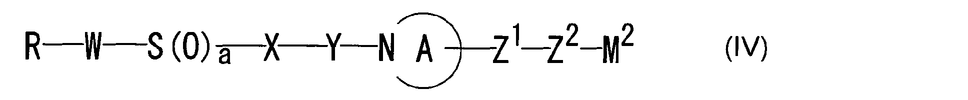 wherein M² represents a hydrogen atom, an alkaline metal (e.g. lithium, sodium, potassium, cesium etc.), an alkaline earth metal (e.g. magnesium, calcium etc.) or a leaving group (e.g. trimethylsilyl group etc.), and other symbols are as defined above, or a salt thereof with Compound (V) represented by the formula (V): 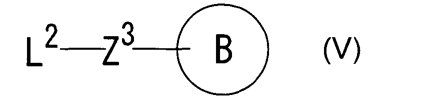 wherein L² represents a leaving group (e.g. a halogen atom (e.g. fluorine, chlorine, bromine, iodine etc.), a C₁₋₆ alkylsulfonyloxy group optionally substituted with 1 to 3 halogen atoms (e.g. methanesulfonyloxy, ethanesulfonyloxy, trifluoromethanesulfonyloxy etc.), an optionally substituted arylsulfonyloxy group (e.g. benzenesulfonyloxy, p-toluenesulfonyloxy, p-bromobenzenesulfonyloxy etc.) or a formyl group, and other symbols are as defined above, or a salt thereof. A salt of Compound (IV) or (V) includes acid addition salts with the aforementioned acids which may form acid addition salts with Compound (I).

This reaction is generally performed in a solvent, and a solvent which does not inhibit the reaction is appropriately selected. A solvent and a base used in this reaction are the same as those described for Method A.

When L² is a formyl group, the reaction is performed in the presence of a reducing agent (e.g. sodium cyanoborohydride, sodium triacetoxyborohydride, sodium borohydride, diborane, diborane-tetrahydrofuran complex, diborane-dimethyl sulfide complex etc.). Such a reducing agent is used in an amount of 0.5 to 10 equivalents, preferably 0.8 to 5 equivalents based on the amount of Compound (V).

In this reaction, 0.5 to 5 equivalents, preferably 0.8 to 2 equivalents of Compound (IV) is used based on the amount of Compound (V).

The reaction temperature is -20 to 200°C, preferably -5 to 170°C.

The reaction time varies depending on the kind of Compound (IV) or (V), the kind of a solvent, the reaction temperature and the like. It is usually about 1 minute to about 72 hours, preferably about 15 minutes to about 24 hours.

### Method C

Compound (Ia) can be prepared by reacting Compound (Ie) represented by the formula (Ie): 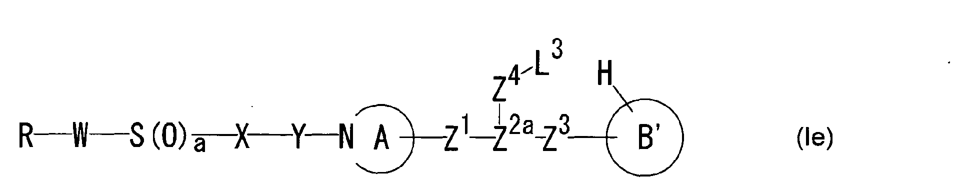 wherein L³ represents a leaving group and other symbols are as defined above, or a salt thereof with a base.

The leaving group represented by L³ includes the same group as that represented by L¹.

A salt of Compound (Ie) includes acid addition salts with the aforementioned acids which may form acid addition salts with Compound (I).

A base used in this reaction includes the same base as that described for Method A.

This reaction is generally performed in a solvent, if needed, in the presence of a condensing agent. A solvent and a condensing agent are the same as those described for Method A, respectively.

In this reaction, 0.5 to 5 equivalents, preferably 0.8 to 2 equivalents of a base is used based on the amount of Compound (Ie).

The reaction temperature is -50 to 150°C, preferably -20 to 100°C.

The reaction time varies depending on the kind of Compound (Ie) or a base, the kind of a solvent, the reaction temperature. It is usually about 1 minute to about 100 hours, preferably about 15 minutes to about 48 hours.

### Method D

Compound (Ia) or a salt thereof can be prepared by reacting Compound (If) represented by the formula (If): 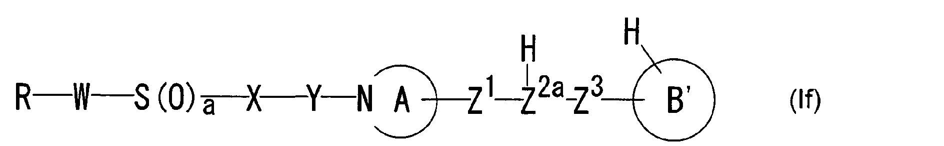 wherein symbols are as defined above, or a salt thereof with a compound represented by the formula (VI):

L⁴-Z⁴-L⁴, (VI)

wherein L⁴ and L⁴' represent a leaving group, and other symbols are as defined above.

This method can be performed by reacting Compound (If) or a salt thereof (inorganic salt, organic salt etc.) with Compound (VI).

A salt of Compound (If) includes acid addition salts with the aforementioned acids which may form acid addition salts with Compound (I).

In Compound (VI), the leaving group represented by L⁴ and L^{4'} includes the same group as that represented by L¹.

When Z⁴ is -CO-, as Compound (VI), a carbonylating reagent is used. A carbonylating reagent includes carbonyldiimidazole, phosgene, diphosgene, triphosgene, dialkyl carbonate (e.g. methyl carbonate, diethyl carbonate etc.) and the like.

The reaction of this method is generally performed in a solvent, and a solvent which does not inhibit the reaction is appropriately selected. As such the solvent, alcohols (e.g. methanol, ethanol, propanol, isopropanol, butanol, tert-butanol etc.), ethers (e.g. dioxane, tetrahydrofuran, diethyl ether, tert-butyl methyl ether, diisopropyl ether, ethylene glycol-dimethyl ether etc.), esters (e.g. ethyl formate, ethyl acetate, n-butyl acetate etc.), halogenated hydrocarbons (e.g. dichloromethane, chloroform, carbon tetrachloride, trichlene, 1,2-dichloroethane etc.), hydrocarbons (e.g. n-hexane, benzene, toluene etc.), amides such as formamide, N,N-dimethylformamide, N,N-dimethylacetamide and the like, nitriles such as acetonitrile, propionitrile and the like and, additionally, dimethyl sulfoxide, sulfolane, hexamethylphosphoramide, water and the like are used alone or as a mixed solvent.

This reaction may be also performed in the presence of a base. Such a base includes inorganic bases such as lithium hydroxide, potassium hydroxide, sodium hydroxide, sodium carbonate, potassium carbonate, sodium hydrogencarbonate and the like, and tertiary amines such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, diazabicycloundecane, diazabicycloundecene and the like.

In this reaction, 0.5 to 10 equivalents, preferably 0.8 to 3 equivalents of Compound (VI) is used based on the amount of Compound (If).

The reaction temperature is -30 to 250°C, preferably -10 to 100°C.

The reaction time varies depending on the kinds of Compound (If) and (VI), the kind of a solvent, the reaction temperature and the like. It is usually about 1 minute to about 72 hours, preferably about 15 minutes to about 24 hours.

### Method E

Compound (I) can be prepared by oxidizing Compound (Ig) represented by the formula (Ig): 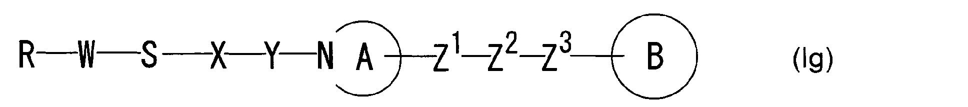 wherein symbols are as defined above, or a salt thereof.

This oxidization reaction is performed in the presence of an oxidizing agent. An oxidizing agent includes oxygen, hydrogen peroxide, organic peracids such as perbenzoic acid, m-chloroperbenzoic acid, peracetic acid, perchlorates such as lithium perchlorate, silver perchlorate, tetrabutylammonium perchlorate and the like, periodates such as sodium periodate, periodic acid, manganese dioxide, lead tetraacetate, permanganates such as potassium permanganate, halogen such as iodine, bromine, chlorine and the like, N-bromosuccinimide, N-chlorosuccinimide, sulfuryl chloride, chloramine T and the like.

This reaction is generally performed in a solvent, and a solvent which does not inhibit the reaction is appropriately selected. As such the solvent, alcohols (e.g. methanol, ethanol, propanol, isopropanol, butanol, tert-butanol etc.), ethers (e.g. dioxane, tetrahydrofuran, diethyl ether, tert-butyl methyl ether, diisopropyl ether, ethylene glycol-dimethyl ether etc.), esters (e.g. ethyl formate, ethyl acetate, n-butyl acetate etc.), carboxylic acids (e.g. formic acid, acetic acid, propionic acid etc.), halogenated hydrocarbons (e.g. dichloromethane, chloroform, carbon tetrachloride, trichloroethylene, 1,2-dichloroethane, chlorobenzene etc.), hydrocarbons (e.g. n-hexane, benzene, toluene etc.), amides (e.g. formamide, N,N-dimethylformamide, N,N-dimethylacetamide etc.), ketones (e.g. acetone, methyl ethyl ketone, methyl isobutyl ketone etc.), nitriles (e.g. acetonitrile, propionitrile etc.) and, additionally, sulfolane, hexamethyl phosphoramide, water and the like are used alone or as a mixed solvent.

This reaction can be also performed in the presence of a base. A base includes inorganic bases, for example, alkaline metal hydroxides such as lithium hydroxide, sodium hydroxide and potassium hydroxide, alkaline earth metal hydroxides such as magnesium hydroxide and potassium hydroxide, alkaline metal carbonates such as sodium carbonate and potassium carbonate, and alkaline metal hydrogencarbonates such as sodium hydrogencarbonate and potassium hydrogencarbonate.

In this reaction, an oxidizing agent is used in an amount of 0.1 to 20 equivalents (preferably about 0.4 to 10 equivalents) and a base is used in an amount of 0.1 to 20 equivalents (preferably 0.4 to 10 equivalents), based on the amount of Compound (Ig).

In addition, this reaction may be performed in the presence of an acid as necessary. Such acid includes mineral acids such as hydrochloric acid, hydrobromic acid, sulfuric acid, phosphoric acid, perchloric acid and the like, sulfonic acids such as methanesulfonic acid, ethanesulfonic acid, benzenesulfonic acid, toluenesulfonic acid, camphorsulfonic acid and the like, and organic acids such as formic acid, acetic acid, propionic acid, trifluoroacetic acid and the like. Such acid is used in an amount of 0.1 to 20 equivalents, preferably 0.5 to 10 equivalents based on the amount of Compound (Ig).

The reaction temperature is about -10°C to about 250°C, preferably about -5°C to about 150°C.

The reaction temperature varies depending on the kinds of Compound (Ig), a base and a solvent, the reaction temperature and the like. It is usually about 1 minute to about 50 hours, preferably about 5 minutes to about 24 hours.

Starting materials and intermediates used in the above respective reactions are prepared by applying or adapting a known method, for example, methods described in Examples or apparently chemically equivalent methods thereof, or by a method of the present invention.

The Compound (I) thus obtained can be isolated and purified from the reaction mixture by a known means per se, for example, means such as extraction, concentration, neutralization, filtration, recrystallization, column chromatography, thin layer chromatography and the like.

A salt of Compound (I) can be prepared by a known means per se, for example, by addition of an inorganic or organic acid to Compound (I).

When optical isomers of Compound (I) may be present, individual optical isomers and a mixture thereof are included in the scope of the present invention and, if desired, these isomers may be optically resolved according to a known means per se, or may be prepared individually.

In addition, Compound (I) or a salt thereof may be a hydrate, and both of a hydrate and a non-hydrate are included in the scope of the present invention.

Since Compound (I) or a salt thereof of the present invention is low toxic and safe (more excellent as a drug from a viewpoint of acute toxicity, chronic toxicity, hereditary toxicity, reproductive toxicity, cardiac toxicity, drug interaction, and carcinogenecity), inhibits FXa and has anti-coagulation activity, it is useful for preventing or treating various arterial and venous thrombosis, for example, myocardial infarction, cerebral infarction, deep venous thrombosis, pulmonary thromboembolism, arterioscleroticobliterans, economy class syndrome, thromboembolism during or after an operation, and the following diseases in animals especially in mammals (e.g., human, monkey, cat, swine, horse, bovine, mouse, rat, guinea pig, dog, rabbit and the like), and among them, it is preferably used for preventing or treating ischemic cerebral infarction (e.g. cardiogenic cerebral embolism due to atrial fibrillation, and ischemic cerebral infarction caused by progression of arteriosclerosis or activation of blood coagulation system), deep venous thrombosis, pulmonary thromboembolism and the like.

### Brain:

Prevention or treatment of cerebral infarction, ischemic cerebrovascular disorder, cerebral embolism caused by atrial fibrillation, heart failure and valvular disease, acute ischemic cerebral apoplexy, acute stage cerebral thrombosis, cerebrovascular contraction after subarachnoid hemorrhage, Alzheimer's disease, transient ischemic attack (TIA), mixed dementia, cerebrovascular dementia, asymptomatic/multiple cerebral infarction, lacunar infarction and the like, prognosis improvement or secondary onset prevention of cerebral infarction, prevention or treatment of thrombus after an extracranial and intracranial arterial bypass operation, combination use or supplemental use with a thrombolytic agent against cerebral infarction (among them, ischemic cerebrovascular disorder), combination therapy with an anti-platelet drug such as aspirin in preventing onset of cerebral infarction.

### Heart:

Prevention or treatment of acute coronary disease such as acute myocardial infarction, myocardial infarction, ischemic coronary disease, unstable angina, myocardiopathy, acute heart failure, congestive chronic heart failure, valvular disease and the like, prognosis improvement or secondary onset prevention of acute coronary disease such as angina, prevention or treatment of thrombus after artificial valve or artificial heart replacement, prevention or treatment of vascular reocclusion and restenosis after coronary intervention such as stent indwelling or PTCA (percutaneous transluminal coronary angioplasty) or atherectomy, prevention or treatment of vascular reocclusion and restenosis after coronary bypass operation, combination use or supplemental use with a thrombolytic agent against acute coronary disease, combination therapy with an anti-platelet drug such as aspirin in preventing onset of myocardial infarction.

### Periphery:

Prevention or treatment of deep venous thrombosis, chronic arterial obliterans, arteriosclerotic obliterans, peripheral circulation failure such as Buerger's disease, peripheral circulation failure after frostbite, aneurysm, varix, adult respiratory distress syndrome, acute renal failure, chronic renal disease (e.g. diabetic nephropathy, chronic glumerular nephritis, IgA nephropathy etc.), diabetic circulation disorder, pain, nerve disorder, diabetic complication such as diabetic retinopathy and the like, prognosis improvement or secondary onset prevention of deep venous thrombosis, prevention or treatment of deep venous thrombosis or pulmonary thromboembolism after a joint operation including total hip arthroplasty (THA) or total knee arthroplasty (TKA), prevention or treatment of deep venous thrombosis or pulmonary thromboembolism after an orthopedic, plastic surgical or general surgical operation including a spine operation, prevention or treatment of thrombus after a peripheral vascular bypass operation or artificial vessel or vena cava filter indwelling, prevention or treatment of reocclusion and restenosis after stent indwelling or PTA (percutaneous transluminal coronary angioplasty) or peripheral vascular intervention such as atherectomy, prevention or treatment of deep venous thrombosis or pulmonary thromboembolism accompanied with acute internal disease, combination use or supplemental therapy with a thrombolytic agent against deep venous thrombosis and pulmonary thromboembolism, combination therapy with an anti-platelet drug such as aspirin in therapy of peripheral circulation failure such as arteriosclerotic obliterans.

### Others:

Prevention or treatment of pulmonary embolism, acute pulmonary embolism, economy class syndrome, thrombocytopenia or activation of blood coagulation system or complement activation caused by dialysis, thrombocytopenia on a major operation, thrombocytopenic purpura, disseminated intravascular coagulation syndrome (DIC) developed in a patient suffering from progression of arteriosclerosis or cancer metastasis or systemic inflammatory reaction syndrome (SIRS) or pancreatitis or cancer or leukemia or a major operation or sepsis or the like, various organ disorders such as liver function disorder caused by oligemia or ischemia or retention of blood, various organ failures caused by progression of shock or DIC (e.g. lung failure, liver failure, kidney failure, heart failure etc.), systemic lupus erythematosus, collagen disease, hyperthyroidism, puerperal palsy and the like, inhibition of rejective response on transplantation, organ protection or function improvement on transplantation, prevention of perfusion blood coagulation during blood extracorporeal circulation, substitute therapeutic use against development of thrombocytopenia caused by heparin administration, promotion of bedsore or wound healing, inhibition of activation of blood excessive coagulation reaction on various hormone supplement therapy, substitute therapeutic use for a patient resistant or contraindicative to a coumarin drug including warfarin, inhibition of activation of excessive coagulation reaction on administration of a blood preparation or a blood coagulation factor-containing preparation, and the like.

Compound (I) of the present invention or a salt thereof can be orally or parenterally administered as it is or as a composition comprising a pharmacologically acceptable carrier.

An oral dosage form of a pharmaceutical composition containing Compound (I) of the present invention or a salt thereof includes a tablet (including a sugar-coated tablet, a film coating tablet), a pill, a granule, powder, a capsule (including a soft capsule, a microcapsule), syrup, emulsion and suspension. A parenteral dosage form of a pharmaceutical composition containing Compound (I) of the present invention or a salt thereof includes an injection, an infusion, a drip and a suppository. It is also advantageous that Compound (I) of the present invention or a salt thereof in combination with an appropriate base (e.g. a polymer of butyric acid, a polymer of glycolic acid, a copolymer of butyric acid-glycolic acid, a mixture of a polymer of butyric acid and a polymer of glycolic acid, polyglycerol fatty acid ester etc.) is formulated into a sustained release form.

The content of Compound (I) or a salt thereof in a pharmaceutical composition of the present invention varies depending on the form of a composition, and is usually 2 to 85% by weight, preferably 5 to 70% by weight of the total composition.

Compound (I) or a salt thereof may be formulated into the aforementioned dosage forms by known methods used generally in the art. When Compound (I) or a salt thereof is formulated into the aforementioned dosage forms, if necessary, appropriate amounts of an excipient, a binder, a disintegrant, a lubricant, a sweetener, a surfactant, a suspending agent, an emulsifying agent and the like which are conventionally used in pharmaceutical field may be added.

For example, when Compound (I) or a salt thereof is formulated into a tablet, an excipient, a binder, a disintegrant, a lubricant and the like are added. When Compound (I) or a salt thereof is formulated into a pill or a granule, an excipient, a binder, a disintegrant and the like are added. When Compound (I) or a salt thereof is formulated into powder or a capsule, an excipient and the like are added. When Compound (I) or a salt thereof is formulated into syrup, a sweetener and the like are added. When Compound (I) or a salt thereof is formulated into an emulsion or a suspension, a suspending agent, a surfactant, an emulsifying agent and the like are added.

An Excipient includes lactose, white sugar, glucose, starch, sucrose, microcrystalline cellulose, licorice powder, mannitol, sodium hydrogencarbonate, calcium phosphate, calcium sulfate and the like.

A binder includes 5 to 10% by weight starch paste, a 10 to 20% by weight gum arabic solution or gelatin solution, a 1 to 5% by weight tragacanth solution, a carboxymethylcellulose solution, a sodium alginate solution, glycerin and the like.

A disintegrant includes starch, calcium carbonate and the like.

A lubricant includes magnesium stearate, stearic acid, calcium stearate, purified talc and the like.

A sweetener includes glucose, fructose, invert sugar, sorbitol, xylitol, glycerin, simple syrup and the like.

A surfactant includes sodium lauryl sulfate, Polysorbate 80, sorbitan monofatty acid ester, polyoxyl stearate 40 and the like.

A suspending agent includes gum arabic, sodium alginate, sodium carboxymethylcellulose, methylcellulose, bentonite and the like.

An emulsifying agent includes gum arabic, tragacanth, gelatin, Polysorbate 80 and the like.

Further, when Compound (I) or a salt thereof is formulated into the aforementioned dosage forms, if desired, appropriate amounts of a coloring agent, a preservative, a flavor, a corrigent, a stabilizer, a thickener and the like which are conventionally used in pharmaceutical field may be added.

A pharmaceutical composition of the present invention containing Compound (I) or a salt thereof is safe and low toxic and can be used safely. A daily dose of a pharmaceutical composition of the present invention varies depending on the condition and body weight of a patient, the kind of a compound, an administration route and the like. For example, when it is administered orally to an adult patient (body weight about 60 kg) with thrombosis, the daily dose is about 1 to 1000 mg, preferably about 3 to 500 mg, more preferably about 10 to 350 mg of an active ingredient (Compound (I) or a salt thereof), which may be administered once or in two or three divided portions.

When Compound (I) or a salt thereof of the present invention is administered parenterally, it may be usually administered as a form of a solution (e.g. an injection). A dose per time varies depending on a subject to be administered, an organ to be targeted, symptom, an administration method and the like. For example, conveniently, about 0.01 mg to about 100 mg, preferably about 0.01 to about 50 mg, more preferably about 0.01 to about 20 mg per kg body weight of Compound (I) or a salt thereof is administered intravenously in a dosage form of an injection. An injection includes, in addition to intravenous injection, subcutaneous injection, intradermal injection, intramuscular injection, drip injection and the like. A long-acting preparation includes an iontophoresis transdermal agent. Such an injection is prepared by a known method per se, that is, by dissolving, suspending or emulsifying Compound (I) or a salt thereof of the present invention in a sterile aqueous or oily liquid. An aqueous liquid for injection includes physiological saline, and an isotonic solution containing glucose and other supplemental agent (e.g. D-sorbitol, D-mannitol, sodium chloride etc.) and may be used in combination with a suitable solubilizer, for example, alcohol (e.g. ethanol), polyalcohol (e.g. propylene glycol, polyethylene glycol) or a nonionic surfactant (e.g. Polysorbate 80, HCO-50). An oily liquid for injection includes sesame oil and soybean oil and may be used in combination with a solubilizer such as benzyl benzoate or benzyl alcohol. In addition, a buffer (e.g. phosphate buffer, sodium acetate buffer), a soothing agent (e.g. benzalkonium chloride, procaine hydrochloride etc.), a stabilizer (e.g. human serum albumin, polyethylene glycol etc.), a preservative (e.g. benzyl alcohol, phenol etc.) and the like may be added. The injection thus obtained is usually filled in an ampule.

The pharmaceutical composition of the present invention can be appropriately used in combination with a drug (hereinafter, abbreviated as a concomitant drug) such as a thrombolytic agent (e.g. TPA, urokinase etc.), an Alzheimer's disease treating drug (e.g. Avan, Calan etc.), a cholesterol treating drug (e.g. an HMG-CoA reductase inhibitor such as simvastatin, pravastatin etc.), a TG lowering drug (e.g. clofibrate etc.), an AII antagonist (e.g. candesartan cilexetil, losartan etc.), an anti-platelet drug (e.g. clopidogrel, abciximab, aspirin etc.), a Ca antagonist (e.g. Calslot, amlodipine etc.), an ACE inhibitor (e.g. enalapril, captopril etc.), a β blocker (e.g. metoprolol, carvedilol etc.) or an antiarrhythmic drug (e.g. procaine amide etc.). The concomitant drug may be a low-molecular compound, a high-molecular protein, a polypeptide, an antibody, or a vaccine. An administration mode of the compound of the present invention and a concomitant drug is not limited particularly, as long as the compound of the present invention and the concomitant drug are combined upon administration. For example, such an administration mode includes (1) administration of a single preparation obtained by formulating the compound of the present invention and a concomitant drug simultaneously, (2) simultaneous administration of two kinds of preparations obtained by formulating the compound of the present invention and a concomitant drug separately, via a single administration route, (3) separate administration at an interval of two kinds of preparations obtained by formulating the compound of the present invention and a concomitant drug separately, via a single administration route, (4) simultaneous administration of two kinds of preparations obtained by formulating the compound of the present invention and a concomitant drug separately, via different administration routes, and (5) separate administration at an interval of two kinds of preparations obtained by formulating the compound of the present invention and a concomitant drug separately, via different administration routes (e.g. administration of the compound of the present invention followed by the concomitant drug, or administration in the reverse order). The dose of a concomitant drug can be selected appropriately on the basis of a dose clinically used. In addition, a combination ratio of the compound of the present invention and a concomitant drug can be selected appropriately depending on a subject to be administered, an administration route, a disease to be treated, symptom, and a combination thereof. For example, when a subject to be administered is a human, 0.01 to 100 parts by weight of a concomitant drug may be used based on 1 part by weight of the compound of the present invention.

The present invention is further illustrated by the following Examples, Formulation Examples and Experimental Examples which are merely examples and do not limit the present invention, and various changes may be made without departing from the scope of the present invention.

In Examples, elution of column chromatography was confirmed under observation with TLC (Thin Layer Chromatography). For TLC observation, 60F₂₅₄ manufactured by Merck or NH manufactured by Fuji Silysia Chemical Ltd. as a TLC plate, a solvent used as an eluting solvent in column chromatography as a developing solvent, and a UV detector as a detection method were used. As a silica gel for a column, Kiesel Gel 60 (70 to 230 mesh) or Kiesel Gel 60 (230 to 400 mesh) manufactured by Merck was used. As a basic silica gel for a column, basic silica NH-DM1020 (100 to 200 mesh) manufactured by Fuji Silysia Chemical Ltd. was used. NMR spectrum was measured with a Varian Gemini 200-type or 300-type spectrometer using tetramethylsilane as an internal or external standard, and chemical shift was expressed as δ value and a coupling constant was expressed as Hz. IR spectrum was measured with Shimadzu FTZR-8200-type spectrometer. A numerical value shown within ( ) for a mixed solvent is a mixing ratio by volume of each solvent. In addition, % for a solution shows the amount (gram) of a solute in 100 ml of a solution. In addition, symbols used in Examples have the following meanings.
- s:: singlet
- d:: doublet
- t:: triplet
- q:: quartet
- dd:: double doublet
- m:: multiplet
- br:: broad
- brs:: broad singlet
- J:: coupling constant
- WSC:: water-soluble carbodiimide
- THF:: tetrahydrofuran
- DMF:: N,N'-dimethylformamide
- DMSO:: dimethyl sulfoxide
- HOBt:: 1-hydroxybenztriazole

### Example 1

### 1-{3-[(6-Chloro-2-naphthyl)sulfonyl]propanoyl}-4-(1H-imidazol-1-yl)piperidine

Tert-butyl 4-(1H-imidazol-1-yl)piperidine-1-carboxylic acid (JP-A 7-501556) (0.28 g) was dissolved in 40% hydrogen chloride ethanol (4 mL) and ethanol (5 mL) and stirred at room temperature for 3 hours. The reaction mixture was concentrated under reduced pressure and then subjected to azeotropic distillation with ethanol. The residue was washed with diisopropyl ether to obtain a solid, which was dissolved in acetonitrile (15 mL) together with DBU (0.34 g) and triethylamine (0.34 g). This solution was added to a suspension of 3-[(6-chrolo-2-naphthyl)sulfonyl]propionic acid (0.33 g), HOBt(0.26 g) and WSC (0.32 g) in acetonitrile (15 mL), and the mixture was stirred at room temperature for 15 hours. The reaction solution was concentrated under reduced pressure and diluted with ethyl acetate and an aqueous potassium carbonate solution. An organic layer was separated, dried over anhydrous sodium sulfate and concentrated under reduced pressure. The residue was purified with a basic silica gel column (ethyl acetate) to obtain the title compound (0.40 g, 83%) as pale yellow gum.
NMR (300 MHz, CDCl₃) δ: 1.70-1.92 (2H, m), 2.09-2.22 (2H, m), 2.64-2.72 (1H, m), 2.90-2.97 (2H, m), 3.17-3.25 (1H, m), 3.54-3.61 (2H, m), 4.00-4.09 (1H, m), 4.08-4.21 (1H, m), 4.69-4.73 (1H, m), 6.93 (1H, t, J = 1.2), 7.08 (1H, d, J = 1.2), 7.54 (1H, s), 7.60 (1H, dd, J = 8.7 and 2.1), 7.93-7.97 (4H, m), 8.49 (1H, d, J = 1.2).
Elemental analysis for C₂₁H₂₂ClN₃0₃S·0.5H₂O
Calculated (%): C, 57.20; H, 5.26; N, 9.53
Found (%): C, 57.42; H, 5.46; N, 9.47

### Example 2

### 1-{3-[(6-Bromo-2-naphthyl)sulfonyl]propanoyl}-4-(1H-imidazol-1-yl)piperidine

From 3-[(6-bromo-2-naphthyl)sulfonyl]propionic acid (0.38 g), the title compound (0.27 g, 51%) was obtained as colorless powder in a similar manner to Example 1.
NMR (300 MHz, CDCl₃) δ: 1.71-1.90 (2H, m), 2.08-2.22 (2H, m), 2.64-2.72 (1H, m), 2.90-2.97 (2H, m), 3.17-3.25 (1H, m), 3.54-3.61 (2H, m), 4.00-4.04 (1H, m), 4.09-4.21 (2H, m), 4.68-4.73 (1H, m), 6.93 (1H, d, J = 1.2), 7.09 (1H, s), 7.54 (1H, s), 7.73 (1H, dd, J = 8.8 and 2.0), 7.86-7.97 (3H, m), 8.14 (1H, d, J = 1.8), 8.48 (1H, s).
Elemental analysis for C₂₁H₂₂BrN₃O₃S·0.7H₂O
Calculated (%): C, 51.58; H, 4.82; N, 8.59
Found (%): C, 51.47; H, 4.85; N, 8.56

### Example 3

### 1-{3-[(6-Chloro-2-naphthyl)sulfonyl]propanoyl}-4-(2-methyl-1H-imidazol-1-yl)piperidine

From tert-butyl 4-(2-methyl-1H-imidazol-1-yl)piperidine-1-carboxylic acid (JP-A 7-501556)(0.27 g), the title compound (0.37 g, 83%) was obtained as colorless powder in a similar manner to Example 1.
NMR (300 MHz, CDCl₃) δ: 1.68-1.84 (2H, m), 1.97-2.09 (2H, m), 2.42 (3H, s), 2.61-2.69 (1H, m), 2.91-2.98(2H, m), 3.15-3.24 (1H, m), 3.54-3.62 (2H, m), 4.02-4.13 (2H, m), 4.73-4.77 (1H, m), 6.81 (1H, d, J = 1.5), 6.94 (1H, d, J = 1.5), 7.60 (1H, dd, J = 8.9 and 2.0), 7.91-7.97 (4H, m), 8.50 (1H, s).
Elemental analysis for C₂₂H₂₄ClN₃O₃S·0.9H₂O
Calculated (%): C, 57.17; H, 5.63; N, 9.09
Found (%): C, 57.28; H, 5.71; N, 9.16

### Example 4

### 1-{3-[(6-Bromo-2-naphthyl)sulfonyl]propanoyl}-4-(2-methyl-1H-imidazole-1-yl)piperidine

From tert-butyl 4-(2-methyl-1H-imidazol-1-yl)piperidine-1-carboxylate (0.27 g) and 3-[(6-bromo-2-naphthyl)sulfonyl]propionic acid (0.34 g), the title compound (0.47 g, 96%) was obtained as colorless powder in a similar manner to Example 1.
NMR (300 MHz, CDCl₃) δ: 1.69-1.85 (2H, m), 1.97-2.08 (2H, m), 2.42 (3H, s), 2.65 (1H, t, J = 12.1), 2.91-2.98 (2H, m), 3.16-3.25 (1H, m), 3.54-3.62 (2H, m), 4.02-4.11 (2H, m), 4.74-4.77 (1H, m), 6.81 (1H, d, J = 1.5), 6.94 (1H, d, J = 1.5), 7.73 (1H, dd, J = 8.9 and 1.9), 7.87-7.95 (3H, m), 8.14 (1H, s), 8.48 (1H, s).
Elemental analysis for C₂₂H₂₄BrN₃O₃S·0.7H₂O
Calculated (%): C, 52.53; H, 5.09; N, 8.35
Found (%): C, 52.34; H, 5.30; N, 8.19

### Example 5

### 1-{3-[(6-Chloro-2-naphthyl)sulfonyl]propanoyl}-4-(4-methyl-1H-imidazol-1-yl)piperidine

From tert-butyl 4-(4-methyl-1H-imidazol-1-yl)piperidine-1-carboxylate (0.39 g), the title compound (0.41 g, 70%) was obtained as colorless powder in a similar manner to Example 1.
NMR (200 MHz, CDCl₃) δ: 1.74-1.95 (2H, m), 2.12-2.27 (2H, m), 2.21 (3H, d, J = 0.8), 2.60-2.72 (1H, m), 2.89-2.97 (2H, m), 3.12-3.24 (1H, m), 3.53-3.62 (2H, m), 3.96-4.14 (2H, m), 4.65-4.72 (1H, m), 6.63 (1H, s), 7.41 (1H, d, J = 1.0), 7.60 (1H, dd, J = 8.8 and 1.8), 7.93-7.97 (4H, m), 8.49 (1H, s).
Elemental analysis for C₂₂H₂₄ClN₃O₃S·0.9H₂O
Calculated (%): C, 58.08; H, 5.54; N, 9.24
Found (%) : C, 57.81; H, 5.79; N, 9.53

### Example 6

### 1-{3-[(6-Bromo-2-naphthyl)sulfonyl]propanoyl}-4-(4-methyl-1H-imidazol-1-yl)piperidine

From tert-butyl 4-(4-methyl-1H-imidazol-1-y)piperidine-1-carboxylate (0.39 g) and 3-[(6-bromo-2-naphthyl)sulfonyl]propionic acid (0.45 g), the title compound (0.49 g, 75%) was obtained as colorless powder in a similar manner to Example 1.
NMR (200 MHz, CDCl₃) δ: 1.68-1.86 (2H, m), 2.12-2.27 (2H, m), 2.21 (3H, d, J = 0.6), 2.60-2.73 (1H, m), 2.89-2.96 (2H, m), 3.10-3.24 (1H, m), 3.53-3.61 (2H, m), 3.96-4.10 (2H, m), 4.65-4.71 (1H, m), 6.63 (1H, s), 7.41 (1H, d, J = 1.4), 7.73 (1H, dd, J = 8.8 and 2.0), 7.84-7.93 (3H, m), 8.13 (1H, d, J = 1.6), 8.48 (1H, s).
Elemental analysis for C₂₂H₂₄BrN₃O₃S·H₂O
Calculated (%): C, 51.97; H, 5.15; N, 8.26
Found (%): C, 52.03; H, 4.99; N, 8.39

### Example 7

### 1-{3-[(6-Chloro-2-naphthyl)sulfonyl]propanoyl}-4-(2,4-dimethyl-1H-imidazol-1-yl)piperidine

### 7a) Tert-butyl 4-(2,4-dimethyl-1H-imidazol-1-yl)piperidine-1-carboxylate

A suspension of 2,4-dimethylimidazole (5.16 g), tert-butyl 4-[(methylsulfonyl)oxy]piperidine-1-carboxylate (10 g) and potassium carbonate (4.95 g) in DMF (80 mL) was stirred at 100°C for 72 hours. The reaction mixture was concentrated under reduced pressure. The residue was diluted with water and ethyl acetate. An organic layer was separated, washed with saturated sodium hydrogencarbonate, and then dried over anhydrous sodium sulfate. The solvent was concentrated under reduced pressure. The residue was purified with a silica gel column (dichloromethane/methanol/aqueous ammonia = 100/3.5/0.5 to 100/10/1) to obtain the title compound (0.58 g, 6%) as a yellow oil.
NMR (300 MHz, CDCl₃) δ: 1.43 (9H, s), 1.66-1.92 (3H, m), 2.16 (3H, s), 2.37 (3H, s), 2.77-2.85 (2H, m), 3.79-3.91 (2H, m), 4.26 (2H, m), 6.55 (1H, s).

### 7b) 1-{3-[(6-Chloro-2-naphthyl)sulfonyl]propanoyl}-4-(2,4-dimethyl-1H-imidazol-1-yl)piperidine

From tert-butyl 4-(2,4-dimethyl-1H-imidazol-1-yl)piperidine-1-carboxylate (0.28 g) obtained in Example 7a), the title compound (0.21 g, 46%) was obtained as colorless powder in a similar manner to Example 1.
NMR (300 MHz, CDCl₃) δ: 1.65-1.79 (2H, m), 1.93-2.05 (2H, m), 2.16 (3H, d, J = 0.9), 2.37 (3H, s), 2.58-2.67 (1H, m), 2.90-2.97 (2H, m), 3.13-3:22 (1H, m), 3.54-3.61 (2H, m), 3.95-4.04 (2H, m), 4.71-4.75 (1H, m), 6.50 (1H, d, J = 1.2), 7.60 (1H, dd, J = 8.7 and 1.8), 7.91-7.97 (4H, m), 8.49 (1H, s) .
Elemental analysis for C₂₃H₂₆ClN₃O₃S·H₂O
Calculated (%): C, 57.79; H, 5.90; N, 8.79
Found (%): C, 57.84; H, 5.90; N, 8.68

### Example 8

### 1-{3-[(6-Bromo-2-naphthyl)sulfonyl]propanoyl}-4-(2,4-dimethyl-1H-imidazol-1-yl)piperidine

From tert-butyl 4-(2,4-dimethyl-1H-imidazol-1-yl)piperidine-1-carboxylate (0.29 g) obtained in Example 7a) and 3-[(6-bromo-2-naphthyl)sulfonyl]propionic acid (0.36 g), the title compound (70 mg, 14%) was obtained as colorless powder in a similar manner to Example 1.
NMR (300 MHz, CDCl₃) δ: 1.65-1.79 (2H, m), 1.94-2.00 (2H, m), 2.16 (3H, d, J = 0.6), 2.37 (3H, s), 2.59-2.68 (1H, m), 2.90-2.97 (2H, m), 3.13-3.22 (1H, m), 3.54-3.61 (2H, m), 3.95-4.03 (2H, m), 4.71-4.76 (1H, m), 6.50 (1H, s), 7.74 (1H, dd, J = 8.8 and 2.0), 7.85-7.98 (3H, m), 8.14 (1H, d, J = 2.1), 8.48 (1H, s).
Elemental analysis for C₂₃H₂₆BrN₃O₃S·H₂O
Calculated (%): C, 52.87; H, 5.40; N, 8.04
Found (%): C, 52.66; H, 5.23; N, 8.03

### Example 9

### 1-{3-[(6-Chloro-2-naphthyl)sulfonyl]propanoyl}-4-(2-ethyl-1H-imidazol-1-yl)piperidine

### 9a) Tert-butyl 4-(2-ethyl-1H-imidazol-1-yl)piperidine-1-carboxylate

From 2-ethylimidazole (4.13 g), the title compound (1.10 g, 11%) was obtained as a yellow oil in a similar manner to Example 7a).
NMR (300 MHz, CDCl₃) δ: 1.31-1.39 (3H, m), 1.49 (9H, s), 1.70-1.95 (4H, m), 2.67-2.87 (4H, m), 3.96-4.01 (1H, m), 4.29-4.33 (2H, m), 6.86 (1H, d, J = 1.5), 6.97 (1H, d, J = 1.5)

### 9b) 1-{3-[(6-Chloro-2-naphthyl)sulfonyl]propanoyl}-4-(2-ethyl-1H-imidazol-1-yl)piperidine

From tert-butyl 4-(2-ethyl-1H-imidazol-1-yl)piperidine-1-carboxylate (0.28 g) obtained in Example 9a), the title compound (0.45 g, 98%) was obtained as colorless powder in a similar manner to Example 1.
NMR (300 MHz, CDCl₃) δ: 1.36 (3H, t, J = 7.4), 1.65-1.86 (2H, m), 1.96-2.05 (2H, m), 2.61-2.68 (1H, m), 2.71 (2H, q, J = 7.5), 2.91-2.98 (2H, m), 3.16-3.24 (1H, m), 3.54-3.62 (2H, m), 4.01-4.13 (2H, m), 4.73-4.77 (1H, m), 6.81 (1H, d, J = 1.8), 6.98 (1H, d, J = 1.5), 7.60 (1H, dd, J = 8.7 and 1.8), 7.94-7.97 (4H, m), 8.49 (1H, d, J = 0.6).
Elemental analysis for C₂₃H₂₆ClN₃O₃S·0.5H₂O
Calculated (%): C, 58.90; H, 5.80; N, 8.96
Found (%): C, 58.72; H, 6.05; N, 9.08

### Example 10

### 1-{3-[(6-Chloro-2-naphthyl)sulfonyl]propanoyl}-4-(2-isopropyl-1H-imidazol-1-yl)piperidine

### 10a) Tert-butyl 4-(2-isopropyl-1H-imidazol-1-yl)piperidine-1-carboxylate

From 2-isopropylimidazole (4.73 g), the tile compound (0.40 g, 4%) was obtained as a yellow oil in a similar manner to Example 7a).
NMR (300 MHz, CDCl₃) δ: 1.32 (3H, s), 1.36 (3H, s), 1.49 (9H, s), 1.76-1.95 (4H, m), 2.78-2.88 (2H, m), 2.95-3.08 (1H, m), 4.06-4.14 (1H, m), 4.29-4.34 (2H, m), 6.84 (1H, d, J = 1.6), 6.98 (1H, d, J = 1.2).

### 10b) 1-{3-[(6-Chloro-2-naphthyl)sulfonyl]propanoyl}-4-(2-isopropyl-1H-imidazol-1-yl)piperidine

From tert-butyl 4-(2-isopropyl-1H-imidazol-1-yl)piperidine-1-carboxylate (0.29 g) obtained in Example 10a), the title compound (0.36 g, 76%) was obtained as colorless powder in a similar manner to Example 1.
NMR (300 MHz, CDCl₃) δ: 1.32-1.36 (6H, m), 1.70-1.86 (2H, m), 1.96-2.05 (2H, m), 2.61-2.69 (1H, m), 2.91-3.05 (3H, m), 3.16-3.25 (1H, m), 3.55-3.62 (2H, m), 4.01-4.18 (2H, m), 4.73-4.78 (1H, m), 6.78 (1H, d, J = 1.5), 6.98 (1H, d, J = 1.2), 7.60 (1H, dd, J = 8.9 and 2.0), 7.94-7.97 (4H, m), 8.49 (1H, s).
Elemental analysis for C₂₄H₂₈ClN₃O₃S·0.5H₂O
Calculated (%): C, 59.68; H, 6.05; N, 8.70
Found (%): C, 59.51; H, 6.22; N, 8.53

### Example 11

### 1-{3-[(6-Chloro-2-naphthyl)sulfonyl]propanoyl}-4-(2-propyl-1H-imidazol-1-yl)piperidine

### 11a) Tert-butyl 4-(2-propyl-1H-imidazol-1-yl)piperidine-1-carboxylate

From 2-propylimidazole (4.73 g, 43 mmol), the title compound (0.28 g, 3%) was obtained as a yellow oil in a similar manner to Example 7a).
NMR (300 MHz, CDCl₃) δ: 0.98-1.04 (3H, m), 1.49 (9H, s), 1.71-1.94 (6H, m), 2.59-2.69 (2H, m), 2.79-2.87 (2H, m), 3.96-4.04 (1H, m), 4.28-4.33 (2H, m), 6.85 (1H, d, J = 1.2), 6.97 (1H, d, J = 1.5).

### 11b) 1-{3-[(6-Chloro-2-naphthyl)sulfonyl]propanoyl}-4-(2-propyl-1H-imidazol-1-yl)piperidine

From tert-butyl 4-(2-propyl-1H-imidazol-1-yl)piperidine-1-carboxylate (0.28 g) obtained in Example 11a), the title compound (0.30 g, 66%) was obtained as colorless powder in a similar manner to Example 1.
NMR (200 MHz, CDCl₃) δ: 1.02 (3H, t, J = 7.5), 1.68-2.05 (6H, m), 2.62-2.69 (3H, m), 2.90-2.99 (2H, m), 3.14-3.26 (1H, m), 3.54-3.63 (2H, m), 4.01-4.14 (2H, m), 4.71-4.78 (1H, m), 6.80 (1H, d, J = 1.4), 6.97 (1H, d, J = 1.0), 7.60 (1H, dd, J = 8.8 and 1.8), 7.89-7.98 (4H, m), 8.49 (1H, s). Elemental analysis for C₂₉H₂₈ClN₃O₃S·0.5H₂O
Calculated (%): C, 59.68; H, 6.05; N, 8.70
Found (%): C, 59.74; H, 6.30; N, 8.62

### Example 12

### 1-{3-[(6-Chloro-2-naphthyl)sulfonyl]propanoyl}-4-(2-butyl-1H-imidazol-1-yl)piperidine

### 12a) Tert-butyl 4-(2-butyl-1H-imidazol-1-yl)piperidine-1-carboxylate

From 2-butylimidazole (6.66 g), the title compound (0.33 g, 3%) was obtained as a yellow oil in a similar manner to Example 7a).
NMR (300 MHz, CDCl₃) δ: 0.98-1.94 (11H, s), 1.49 (9H, s), 2.59-2.69 (2H, m), 2.79-2.87 (2H, m), 3.96-4.04 (1H, m), 4.28-4.33 (2H, m), 6.85 (1H, d, J = 1.2), 6.97 (1H, d, J = 1.5).

### 12b) 1-{3-[(6-Chloro-2-naphthyl)sulfonyl]propanoyl}-4-(2-butyl-1H-imidazol-1-yl)piperidine

From tert-butyl 4-(2-butyl-1H-imidazol-1-yl)piperidine-1-carboxylate (0.31 g) obtained in Example 12a), the title compound (0.41 g, 85%) was obtained as colorless powder in a similar manner to Example 1.
NMR (200 MHz, CDCl₃) δ: 0.96 (3H, t, J = 7.3), 1.33-1.52 (2H, m), 1.67-2.05 (6H, m), 2.50-2.90 (3H, m), 2.90-2.99 (2H, m), 3.08-3.27 (1H, m), 3.52-3.63 (2H, m), 4.01-4.14 (2H, m), 4.71-4.79 (1H, m), 6.80 (1H, d, J = 1.4), 6.97 (1H, d, J = 1.2), 7.58-7.63 (1H, m), 7.89-7.98 (4H, m), 8.49 (1H, s) .
Elemental analysis for C₂₅H₃₀ClN₃O₃S·0.5H₂O
Calculated (%): C, 60.41; H, 6.29; N, 8.45
Found (%): C, 60.33; H, 6.33; N, 8.40

### Example 13

### [1-(1-{3-[(6-Chloro-2-naphthyl)sulfonyl]propanoyl}-4-piperidinyl)-1H-imidazol-2-yl]methanol

### 13a) Tert-butyl 4-(2-formyl-1H-imidazol-1-yl)piperidine-1-carboxylate

From 2-formylimidazole (2.06 g), the title compound (1.88 g, 38%) was obtained as colorless powder in a similar manner to Example 7a).
NMR (200 MHz, CDCl₃+CD₃OD) δ: 1.48 (9H, s), 1.71-1.79 (2H, m), 2.10 (2H, m), 2.84-2.97 (2H, m), 4.26-4.32 (2H, m), 5.10-5.30 (1H, m), 7.32-7.36 (2H, m), 9.82 (1H, d, J = 0.8).

### 13b) Tert-butyl 4-(2-hydroxymethyl-1H-imidazol-1-yl)piperidine-1-carboxylate

Tert-butyl 4-(2-formyl-1H-imidazol-1-yl)piperidine-1-carboxylate (0.48 g) obtained in Example 13a) was dissolved in methanol (20 mL), and sodium borohydride (0.14 g) was added thereto. The mixture was stirred at room temperature for 3 hours. The reaction mixture was concentrated under reduced pressure. The residue was diluted with an aqueous saturated sodium bicarbonate solution and ethyl acetate. An organic layer was separated, washed with an aqueous saturated sodium bicarbonate solution, dried over anhydrous sodium sulfate and then concentrated under reduced pressure to obtain the title compound (0.40 g, 83%) as colorless powder.
NMR (200 MHz, CDCl₃) δ: 1.49 (9H, s), 1.68-1.85 (2H, m), 2.00-2.06 (2H, m), 2.79-2.92 (2H, m), 4.26-4.40 (3H, m), 4.68 (2H, s), 6.91 (2H, s).

### 13c) [1-(1-{3-[(6-Chloro-2-naphthyl)sulfonyl]propanoyl}-4-piperidinyl)-1H-imidazol-2-yl]methanol

From tert-butyl 4-(2-hydroxymethyl-1H-imidazol-1-yl)piperidine-1-carboxylate (0.38 g) obtained in Example 13b), the title compound (0.26 g, 42%) was obtained as colorless powder in a similar manner to Example 1.
NMR (200 MHz, CDCl₃+CD₃OD) δ: 1.69-1.83 (2H, m), 2.05-2.18 (2H, m), 2.37-2.58 (2H, m), 2.63-2.75 (1H, m), 2.87-2.99 (2H, m), 3.18-3.30 (1H, m), 3.53-3.64 (2H, m), 3.98-4.05 (1H, m), 4.40-4.52 (1H, m), 4.68-4.76 (1H, m), 6.92 (2H, d, J = 4.8), 7.61 (1H, dd, J = 8.8 and 1.8), 7.90-8.00 (4H, m), 8.50 (1H, s).
Elemental analysis for C₂₂H₂₄ClN₃O₄S·0.4H₂O
Calculated (%): C, 56.32; H, 5.33; N, 8.96
Found (%): C, 56.49; H, 5.08; N, 8.68

### Example 14

### 1-[1-(1-{3-[(6-Chloro-2-naphthyl)sulfonyl]propanoyl}-4-piperidinyl)-1H-imidazol-2-yl]ethanol

Tert-butyl 4-(2-formyl-1-imidazol-1-yl)piperidine-1-carbopxylate (0.48 g) obtained in Example 13a) was dissolved in THF (10 mL), and methylmagnesium bromide (3M diethyl ether solution; 1.0 mL) was added while cooling to 0°C thereto. The mixture was stirred at room temperature for 1 hour. The reaction mixture was diluted with an aqueous saturated ammonium chloride solution. An organic layer was separated, dried over anhydrous sodium sulfate and then concentrated under reduced pressure. The resulting residue was dissolved in a 4N solution of hydrogen chloride in ethyl acetate (5 mL). The mixture was stirred at room temperature for 3 hours. The reaction mixture was concentrated under reduced pressure and then subjected to azeotropic distillation with ethanol to remove water. The residue was dissolved in acetonitrile (15 mL) together with DBU (0.26 g) and triethylamine (0.26 g). This solution was added to a suspension of 3-[(6-chloro-2-naphthyl)sulfonyl]propionic acid (0.25 g), HOBt (0.20 g) and WSC (0.24 g) in acetonitrile (15 mL), and the mixture was stirred at room temperature for 15 hours. The reaction solution was concentrated under reduced pressure and diluted with ethyl acetate and an aqueous potassium carbonate solution. An organic layer was separated, dried over anhydrous sodium sulfate and concentrated under reduced pressure. The residue was purified with a basic silica gel column (ethyl acetate) to obtain the title compound (50 mg, 10%) as pale yellow powder.
NMR (200 MHz, CDCl₃) δ: 1.67-2.20 (7H, m), 2.59-2.72 (1H, m), 2.88-2.99 (2H, m), 3.14-3.27 (1H, m), 3.40-3.70 (2H, m), 3.98-4.07 (1H, m), 4.38-4.60 (1H, m), 4.72-4.78 (1H, m), 4.88-4.98 (1H, m), 6.88 (1H, s), 6.98 (1H, d, J = 0.8), 7.60 (1H, dd, J = 8.8 and 1.8), 7.89-7.98 (4H, m), 8.49 (1H, s).
Elemental analysis for C₂₃H₂₆ClN₃O₄S·0.5H₂O
Calculated (%): C, 56.96; H, 5.61; N, 8.66
Found (%): C, 57.18; H, 5.76; N, 8.47

### Example 15

### 1-{3-[(6-Chloro-2-naphthyl)sulfonyl]propanoyl}-4-(4,5-dimethy-1H-imidazol-1-yl)piperidine

### 15a) Tert-butyl 4-(4,5-dimethyl-1H-imidazol-1-yl)piperidine-1-carboxylate

From 4,5-dimethylimidazole (JP-A 60-56961) (5.00 g), the title compound (0.27 g, 3%) was obtained as a yellow oil in a similar manner to Example 7a).
NMR (200 MHz, CDCl₃) δ: 1.48 (9H, s), 1.74-1.89 (2H, m), 1.96-2.02 (2H, m), 2.14 (3H, s), 2.15 (3H, s), 2.76-2.89 (2H, m), 3.75-3.89 (1H, m), 4.26-4.33 (2H, m), 7.38 (1H, s).

### 15b) 1-{3-[(6-Chloro-2-naphthyl)sulfonyl]propanoyl}-4-(4,5-dimethyl-1H-imidazol-1-yl)piperidine

From tert-butyl 4-(4,5-dimethyl-1H-imidazol-1-yl)piperidine-1-carboxylate (0.27 g) obtained in Example 15a), the tile compound (0.14 g, 30%) was obtained as colorless powder in a similar manner to Example 1.
NMR (200 MHz, CDCl₃) δ: 1.67-1.85 (2H, m), 2.00-2.19 (2H, m), 2.15 (6H, s), 2.58-2.70 (1H, m), 2.90-2.98 (2H, m), 3.13-3.25 (1H, m), 3.53-3.61 (2H, m), 3.86-4.07 (2H, m), 4.71-4.76 (1H, m), 7.35 (1H, s), 7.61 (1H, dd, J = 1.8 and 8.8), 7.93-7.98 (4H, m), 8.49 (1H, s).
Elemental analysis for C₂₃H₂₆ClN₃O₃S·H₂O
Calculated (%): C, 57.79; H, 5.90; N, 8.79
Found (%): C, 57.95; H, 5.77; N, 8.72

### Example 16

### 1-(1-{3-[(6-Chloro-2-naphthyl)sulfonyl]propanoyl}-4-piperidinyl)-2-methyl-1H-benzimidazole

### 16a) Tert-butyl 4-(2-methyl-1H-benzimidazol-1-yl)piperidine-1-carboxylate

Sodium hydride (1.50 g) was added to a solution of 2-methylbenzimidazole (5.20 g) in DMF (80 mL) at 0°C, and the mixture was stirred at 0°C for 30 minutes. Tert-butyl 4-[(methylsulfonyl)oxy]piperidine-1-carboxylate (10 g) was added to the mixture, which was stirred at 100°C for 48 hours. The reaction mixture was concentrated under reduced pressure, and the residue was diluted with an aqueous sodium hydrogencarbonate solution and ethyl acetate. An organic layer was separated, washed with an aqueous saturated sodium hydrogencarbonate solution and dried over anhydrous sodium sulfate. The solvent was concentrated under reduced pressure. The residue was purified with a silica gel column (ethyl acetate/hexane = 1/1 to 3/1) to obtain the title compound (0.77 g, 7%) as a colorless solid. NMR (300 MHz, CDCl₃) δ: 1.53 (9H, s), 1.87-1.91 (2H, m), 2.37-2.50 (2H, m), 2.65 (3H, s), 2.84-2.94 (2H, m), 4.27-4.40 (3H, m), 7.18-7.22 (2H, m), 7.42-7.45 (1H, m), 7.68-7.71 (1H, m).

### 16b) 1-(1-{3-[(6-Chloro-2-naphthyl)sulfonyl]propanoyl}-4-piperidinyl)-2-methyl-lH-benzimidazole

From tert-butyl 4-(2-methyl-1H-benzimidazol-1-yl)piperidine-1-carboxylate (0.18 g, 0.6 mmol) obtained in Example 16a), the title compound (0.24 g, 48%) was obtained as colorless powder in a similar manner to Example 1.
NMR (200 MHz, CDCl₃) δ: 1.90-2.05 (2H, m), 2.38-2.52 (2H, m), 2.65 (3H, s), 2.65-2.77 (1H, m), 2.95-3.06 (2H, m), 3.20-3.32 (1H, m), 3.57-3.67 (2H, m), 4.07-4.18 (1H, m), 4.34-4.46 (1H, m), 4.83-4.89 (1H, m), 7.17-7.23 (2H, m), 7.39-7.43 (1H, m), 7.61 (1H, dd, J = 8.9 and 1.9), 7.67-7.71 (1H, m), 7.95-7.99 (4H, m), 8.52 (1H, s).
Elemental analysis for C₂₆H₂₆ClN₃O₃S·H₂O
Calculated (%): C, 60.75; H, 5.49; N, 8.17
Found (%): C, 60.88; H, 5.64; N, 7.99

### Example 17

### 1-{3-[(6-Chloro-2-naphthyl)sulfonyl]propanoyl}-4-(1H-imidazol-4-yl)-4-piperidinol

From tert-butyl 4-hydroxy-4-(1H-imidazol-4-yl)piperidine-1-carboxylate (Tetrahedron, 51, 13447 (1995))(0.27 g), the title compound(0.08 g, 18%) was obtained as colorless powder in a similar manner to Example 1.
NMR (200 MHz, CDCl₃) δ: 1.81-1.96 (4H, m), 2.86-2.93 (3H, m), 3.12-3.26 (2H, m), 3.53-3.63 (4H, m), 4.21-4.28 (1H, m), 6.87 (1H, d, J = 1.2), 7.56-7.61 (2H, m), 7.88-7.97 (4H, m), 8.48 (1H, s).
Elemental analysis for C₂₁H₂₂ClN₃O₄S·0.5H₂O
Calculated (%): C, 55.20; H, 5.07; N, 9.20
Found (%): C, 55.45; H, 5.11; N, 9.30

### Example 18

### 1-{3-[(6-Bromo-2-naphthyl)sulfonyl]propanoyl}-4-(1H-imidazol-4-yl)-4-piperidinol

From tert-butyl 4-hydroxy-4-(1H-imidazol-4-yl)piperidine-1-carboxylate (0.27 g) and 3-[(6-bromo-2-naphthyl)sulfonyl]propionic acid (0.34 g), the title compound (0.14 g, 28%) was obtained as colorless powder in a similar manner to Example 1.
NMR (200 MHz, CDCl₃) δ: 1.81-1.95 (4H, m), 2.86-2.93 (3H, m), 3.10-3.23 (2H, m), 3.47-3.63 (4H, m), 4.22-4.29 (1H, m), 6.87 (1H, d, J = 1.2), 7.61 (1H, d, J = 1.0), 7.71 (1H, dd, J = 2.0 and 8.6), 7.83-7.93 (3H, m), 8.12 (1H, s), 8.47 (1H, s).
Elemental analysis for C₂₁H₂₂BrN₃O₄S·0.5H₂O
Calculated (%): C, 50.30; H, 4.62; N, 8.38
Found (%): C, 50.46; H, 4.86; N, 8.54

### Example 19

### 1-(3-[(6-Chloro-2-naphthyl)sulfonyl]propanoyl}-4-(1H-imidazol-4-yl)-1,2,3,6-tetrahydropyridine

4-(1H-imidazol-4-yl)-1,2,3,4-tetrahydropyridine dihydrochloride (Tetrahedron, 51, 13447 (1995)) (0.13 g), DBU (0.15 g) and triethylamine (0.15 g) in acetonitrile (5 mL) were added to a suspension of 3-[(6-chloro-2-naphthyl)sulfonyl]propionic acid (0.15 g), HOBt (0.12 g) and WSC (0.14 g) in acetonitrile (10 mL), and the mixture was stirred at room temperature for 15 hours. The reaction solution was concentrated under reduced pressure and diluted with ethyl acetate and an aqueous potassium carbonate solution. An organic layer was separated, dried over anhydrous sodium sulfate and concentrated under reduced pressure. The residue was purified with a basic silica gel column (ethyl acetate to ethyl acetate/methanol = 20/1) to obtain the title compound (0.11 g, 49%) as colorless powder.
NMR (300 MHz, CDCl₃) δ: 2.34 (1H, m), 2.53 (1H, m), 2.87 (1H, t, J = 7.7), 2.94 (1H, t, J = 7.8), 3.58-3.71 (4H, m), 4.11-4.13 (2H, m), 6.24 (1H, d, J = 16.5), 6.96 (1H, d, J = 6.9), 7.54-7.58 (1H, m), 7.63 (1H, s), 7.91-7.94 (4H, m), 8.47 (1H, s).
Elemental analysis for C₂₁H₂₀ClN₃O₃S·0.6H₂O
Calculated (%): C, 57.23; H, 4.85; N, 9.53
Found (%): C, 57.04; H, 4.77; N, 9.35

### Example 20

### 1-{3-[(6-Chloro-2-naphthyl)sulfonyl]propanoyl}-4-(1H-imidazol-4-yl)piperidine

From 4-(1H-imidazol-4-yl)piperidine dihydrochloride (Tetrahedron, 51, 13447 (1995)) (0.46 g), the title compound (0.43 g, 50%) was obtained as colorless powder in a similar manner to Example 1.
NMR (300 MHz, CDCl₃+CD₃OD) δ: 1.40-1.60 (2H, m), 1.95-2.11 (2H, m), 2.66-2.91 (5H, m), 3.13-3.20 (1H, m), 3.54-3.61 (2H, m), 3.86-3.90 (1H, m), 4.47-4.51 (1H, m), 6.70 (1H, s), 7.53 (1H, d, J = 1.2), 7.59 (1H, dd, J = 8.4 and 2.4), 7.93-7.98 (4H, m), 8.48 (1H, s).
Elemental analysis for C₂₁H₂₂ClN₃O₃S·0.5H₂O
Calculated (%): C, 57.20; H, 5.26; N, 9.53
Found (%): C, 57.48; H, 5.05; N, 9.44

### Example 21

### 1-{3-[(6-Chloro-2-naphthyl)sulfonyl]propanoyl}-4-(1-methyl-1H-imidazol-5-yl)piperidine

### 21a) 1-{3-[(6-Chloro-2-naphthyl)sulfonyl]propanoyl}-4-(1-trityl-1H-imidazol-4-yl)piperidine

1-{3-[(6-chloro-2-naphthyl)sulfonyl]propanoyl}-4-(1H-imidazol-4-yl)piperidine (0.35 g) obtained in Example 20 and triethylamine (0.10 g) were dissolved in DMF (10 mL), triphenylchloromethane (0.25 g) was added at 0°C thereto. The mixture was stirred at 0°C for 1 hour and then at room temperature for 15 hours. The reaction mixture was concentrated under reduced pressure, and the residue was diluted with an aqueous saturated sodium bicarbonate solution and ethyl acetate. An organic layer was separated, washed with an aqueous saturated sodium chloride solution, dried over anhydrous sodium sulfate and concentrated under reduced pressure. The residue was purified with a basic silica gel column (ethyl acetate) to obtain the title compound (0.54 g, quantitative) as colorless powder.
NMR (200 MHz, CDCl₃) δ: 1.40-1.50 (2H, m), 1.92-2.10 (2H, m), 2.58-2.87 (4H, m), 3.04-3.17 (1H, m), 3.51-3.59 (2H, m), 3.78-3.84 (1H, m), 4.41-4.47 (1H, m), 6.49 (1H, s), 7.09-7.35 (16H, m), 7.57 (1H, dd, J = 8.8 and 1.8), 7.92-8.02 (4H, m), 8.46 (1H, s).

### 21b) 1-{3-[(6-Chloro-2-naphthyl)sulfonyl]propanoyl}-4-(1-methyl-1H-imidazol-5-yl)piperidine

1-{3-[(6-Chloro-2-naphtyl)sulfonyl]propanoyl}-4-(1-trityl-1H-imidazol-4-yl)piperidine (0.54 g) obtained in Example 21a) and methyl iodide (0.10 mL) were dissolved in DMF (5 mL), and the mixture was stirred at room temperature for 15 hours. The reaction mixture was concentrated under reduced pressure, the residue was dissolved in acetic acid (5 mL), water (5 mL) and methanol (2 mL). The solution was stirred at 95°C for 2 hours. The reaction mixture was concentrated under reduced pressure. After the residue was basified by addition of an aqueous saturated sodium bicarbonate solution, ethyl acetate was added thereto. An organic layer was separated, washed with an aqueous saturated bicarbonate solution, dried over anhydrous sodium sulfate and concentrated under reduced pressure. The residue was purified with a basic silica gel column (ethyl acetate to ethyl acetate/methanol = 10/1) to obtain the title compound (0.27 g, 76%) as colorless powder.
NMR (200 MHz, CDCl₃) δ: 1.43-1.70 (2H, m), 1.88-2.04 (2H, m), 2.60-2.78 (2H, m), 2.87-2.95 (2H, m), 3.09-3.22 (1H, m), 3.53-3.61 (2H, m), 3.60 (3H, s), 3.91-3.98 (1H, m), 4.55-4.62 (1H, m), 6.76 (1H, s), 7.38 (1H, s), 7.59 (1H, dd, J = 8.8 and 1.8), 7.93-7.97 (4H, m), 8.48 (1H, s).
Elemental analysis for C₂₂H₂₄ClN₃O₃S.0.5H₂O
Calculated (%): C, 58.08; H, 5.54; N, 9.24
Found (%): C, 57.79; H, 5.74; N, 9.26

### Example 22

### 1-{3-[(6-Chloro-2-naphthyl)sulfonyl]propanoyl}-4-(2-methyl-1H-imidazol-4-yl)piperidine

From 4-(2-methyl-1H-imidazol-4-yl)piperidine dihydrochloride (Farmaco, 47, 1343 (1992))(1.00 g), the title compound (1.70 g, 91%) was obtained as colorless powder in a similar manner to Example 19.
NMR (200 MHz, CDCl₃) δ: 1.41-1.60 (2H, m), 1.93-2.10 (2H, m), 2.39 (3H, s), 2.59-2.71 (2H, m), 2.83-2.91 (2H, m), 3.07-3.21 (1H, m), 3.53-3.60 (2H, m), 3.83-3.90 (1H, m), 4.47-4.54 (1H, m), 6.56 (1H, s), 7.58 (1H, dd, J = 9.0 and 2.0), 7.88-7.97 (4H, m), 8.48 (1H, br).
Elemental analysis for C₂₂H₂₄ClN₃O₃S·0.2H₂O
Calculated (%): C, 58.78; H, 5.47; N, 9.35
Found (%): C, 58.68; H, 5.25; N, 9.29

### Example 23

### 1-{3-[(6-Chloro-2-naphthyl)sulufonyl]propanoyl}-4-(2-methyl-1-trityl-1H-imidazol-4-yl)piperidine

From 1-{3-[(6-chloro-2-naphthyl)sulfonyl]propanoyl}-4-(2-methyl-1H-imidazol-4-yl)piperidine (1.30 g) obtained in Example 22, the title compound (1.20 g, 60%) was obtained as colorless powder in a similar manner to Example 21a). NMR (300 MHz, CDCl₃) δ: 1.33-1.59 (2H, m), 1.90-2.06 (2H, m), 2.57-2.74 (2H, m), 2.82-2.89 (2H, m), 3.05-3.13 (1H, m), 3.52-3.57 (2H, m), 3.80-3.84 (1H, m), 4.45-4.49 (1H, m), 6.34 (1H, s), 7.09-7.12 (5H, m), 7.30-7.35 (10H, m), 7.58 (1H, dd, J = 8.7 and 1.8), 7.91-7.96 (4H, m), 8.47 (1H, s).

### Example 24

### 1-{3-[(6-Chloro-2-naphthyl)sulufonyl]propanoyl}-4-(1,2-dimethyl-1H-imidazol-5-yl)piperidine

From 1-{3-[(6-chloro-2-naphthyl)sulufonyl]propanoyl}-4-(2-methyl-1-trityl-1H-imidazol-4-yl)piperidine (0.28 g) obtained in Example 23, the title compound (0.09 g, 48%) was obtained as colorless powder in a similar manner to Example 21b).
NMR (200 MHz, CDCl₃) δ: 1.40-1.56 (2H, m), 1.87-2.00 (2H, m), 2.36 (3H, s), 2.60-2.71 (2H, m), 2.87-2.95 (2H, m), 3.09-3.21 (1H, m), 3.45 (3H, s), 3.53-3.61 (2H, m), 3.90-3.96 (1H, m), 4.54-4.60 (1H, m), 6.60 (1H, s), 7.60 (1H, dd, J = 8.8 and 1.8), 7.93-7.97 (4H, m), 8.49 (1H, s).
Elemental analysis for C₂₃H₂₆ClN₃O₃S·H₂O
Calculated (%): C, 57.79; H, 5.90; N, 8.79
Found (%): C, 57.75; H, 5.87; N, 8.50

### Example 25

### 2-[5-(1-{3-[(6-Chloro-2-naphthyl)sulufonyl]propanoyl}-4-piperidinyl)-2-methyl-1H-imidazol-1-yl]acetamide

From 1-{3-[(6-chloro-2-naphthyl)sulufonyl]propanoyl}-4-(2-methyl-1-trityl-1H-imidazol-4-yl)piperidine (0.69 g) obtained in Example 23 and iodoacetamide (0.28 g), the title compound (0.10 g, 20%) was obtained as colorless powder in a similar manner to Example 21b).
NMR (300 MHz, CDCl₃) δ: 1.38-1.58 (2H, m), 1.94-2.10 (2H, m), 2.35 (3H, s), 2.60-2.77 (2H, m), 2.85-2.91 (2H, m), 3.08-3.18 (1H, m), 3.54-3.59 (2H, m), 3.86-3.90 (1H, m), 4.50 (2H, s), 4.50-4.56 (1H, m), 5.37 (1H, br), 5.55 (1H, br), 6.54 (1H, s), 7.59 (1H, dd, J = 9.0 and 2.1), 7.90-7.97 (4H, m), 8.48 (1H, d, J = 0.9).
Elemental analysis for C₂₄H₂₇ClN₄O₄S·0.8H₂O
Calculated (%): C, 55.71; H, 5.57; N, 10.83
Found (%): C, 55.87; H, 5.57; N, 10.90

### Example 26

### 1-{3-[(6-Chloro-2-naphthyl)sulfonyl]propanoyl}-4-(2-ethyl-1H-imidazol-4-yl)piperidine

### 26a) 4-(2-Ethyl-1H-imidazol-4-yl)pyridine

2,2-Diethoxy-2-(4-pyridinyl)ethylamine (Org. Synth., 64, 19 (1985)) (1.24 g) and ethyl propaneimidate (1.38 g) were dissolved in ethanol (30 mL), and the mixture was heated to reflux for 24 hours. The reaction mixture was concentrated under reduced pressure. After the residue was basified by addition of an aqueous potassium carbonate solution, ethyl acetate was added thereto. An organic layer was separated, dried over anhydrous sodium sulfate and concentrated under reduced pressure. The residue was purified with a basic silica gel column (ethyl acetate to ethyl acetate/methanol = 10/1) to obtain the title compound (0.65 g, 38%) as a yellow solid.
NMR (300 MHz, CDCl₃) δ: 1.36 (3H, t, J = 7.7), 2.83 (2H, q, J = 7.7), 7.42 (1H, s), 7.63 (2H, d, J = 5.1), 8.54 (2H, dd, J = 4.7 and 1.7), 10.50 (1H, br).

### 26b) 4-(2-Ethyl-1H-imidazol-4-yl)piperidine dihydrochloride

4-(2-Ethyl-1H-imidazol-4-yl)pyridine (0.60 g, 3.2 mmol) obtained in Example 26a) and 5% rhodium carbon (50% hydrous, 0.10 g) were added to 1N hydrochloric acid (40 mL), and the mixture was stirred at room temperature for 6 hours under 5 atm hydrogen atmosphere. The reaction mixture was filtered, and the filtrate was concentrated under reduced pressure to obtain the title compound (0.70 g, 81%) as colorless powder.
NMR (200 MHz, DMSO-d₆) δ: 1.29 (3H, t, J = 7.6), 1.69-1.86 (2H, m), 2.14-2.20 (2H, m), 2.90 (2H, q, J = 7.6), 2.90-3.55 (5H, m), 7.37 (1H, d, J = 0.8), 9.06 (2H, br).

### 26c) 1-{3-[(6-Chloro-2-naphthyl)sulufonyl]propanoyl}-4-(2-ehtyl-1H-imidazol-4-yl)piperidine

From 4-(2-ethyl-1H-imidazol-4-yl)piperidine dihydrochloride (0.65 g) obtained in Example 26b), the title compound (0.30 g, 25%) was obtained as colorless powder in a similar manner to Example 19.
NMR (300 MHz, CDCl₃) δ: 1.30 (3H, t, J = 7.8), 1.40-1.55 (2H, m), 1.95-2.10 (2H, m), 2.61-2.70 (2H, m), 2.73 (2H, q, J = 7.8), 2.85-2.90 (2H, m), 3.08-3.18 (1H, m), 3.54-3.60 (2H, m), 3.84-3.88 (1H, m), 4.49-4.53 (1H, m), 6.58 (1H, s), 7.58 (1H, dd, J = 8.7 and 1.8), 7.92-7.96 (4H, m), 8.48 (1H, s), 8.62 (1H, br).
Elemental analysis for C₂₃H₂₆ClN₃O₃S·0.2H₂O
Calculated (%): C, 59.59; H, 5.74; N, 9.06
Found (%): C, 59.50; H, 5.50; N, 8.98

### Example 27

### 1-{3-[(6-Chloro-2-naphthyl)sulfonyl]propanoyl}-4-(2-ethyl-1-trityl-1H-imidazol-4-yl)piperidine

From 1-{3-[(6-chloro-2-naphthyl)sulfonyl]propanoyl}-4-(2-ethyl-lH-imdazol-4-yl)piperidine (0.25 g) obtained in Example 26c), the title compound (0.30 g, 79%) was obtained as colorless powder in a similar manner to Example 21a). NMR (200 MHz, CDCl₃) δ: 0.71 (3H, t, J = 7.5), 1.30-1.46 (2H, m), 1.91 (2H, q, J = 7.6), 1.93-2.10 (2H, m), 2.53-2.89 (4H, m), 3.01-3.18 (1H, m), 3.50-3.58 (2H, m), 3.78-3.84 (1H, m), 4.43-4.49 (1H, m), 6.28 (1H, s), 7.08-7.13 (5H, m), 7.27-7.34 (10H, m), 7.58 (1H, dd, J = 8.8 and 1.8), 7.92-8.02 (4H, m), 8.47 (1H, s).

### Example 28

### 1-{3-[(6-Chloro-2-naphthyl)sulfonyl]propanoyl}-4-(2-ethyl-1-methyl-1H-imidazol-5-yl)piperidine

From 1-{3-[(6-chloro-2-naphthyl)sulfonyl]propanoyl}-4-(2-ethyl-1-trityl-1H-imidazol-4-yl)piperidine (0.30 g) obtained in Example 27, the title compound (0.09 g, 45%) was obtained as colorless powder in a similar manner to Example 21b).
NMR (200 MHz, CDCl₃) δ: 1.33 (3H, t, J = 7.0), 1.48-1.63 (2H, m), 1.88-2.05 (2H, m), 2.61-2.73 (4H, m), 2.87-2.95 (2H, m), 3.08-3.22 (1H, m), 3.46 (3H, s), 3.53-3.61 (2H, m), 3.90-3.96 (1H, m), 4.54-4.61 (1H, m), 6.64 (1H, s), 7.60 (1H, dd, J = 9.0 and 2.0), 7.93-7.97 (4H, m), 8.48 (1H, d, J = 1.2).
Elemental analysis for C₂₉H₂₈ClN₃O₃S·H₂O
Calculated (%): C, 58.59; H, 6.15; N, 8.54
Found (%): C, 58.72; H, 6.19; N, 8.38

### Example 29

### 1-{3-[(6-Chloro-2-naphthyl)sulfonyl]propanoyl}-4-(2,4-dimethyl-1H-imidazol-5-yl)piperidine

### 29a) 4-(2,4-Dimethyl-1H-imidazol-5-yl)pyridine

Sodium hydride (60%; 0.67 g) was added to a solution of 2,4-dimethyl-5-iodoimidazole (Tetrahedron, 54, 3235 (1998)) (3.40 g) in DMF (20 mL) at 0°C and the mixture was stirred at 0°C for 30 minutes. Then, benzyl bromide (2.0 mL) was added to the mixture and stirred at room temperature for 15 hours. The reaction mixture was concentrated under reduced pressure, and the residue was diluted with water and ethyl acetate. An organic layer was separated, washed with an aqueous saturated sodium chloride solution, dried over anhydrous sodium sulfate and concentrated under reduced pressure. The residue was purified with a silica gel column (ethyl acetate/hexane = 1/1 to 3/1) to obtain a benzyl compound (4.30 g, 90%) as a colorless oil.

The resulting benzyl compound (1.60 g) together with 4-pyridinylboronic acid (0.63 g), tetrakistriphenylphosphine palladium (0.59 g) and potassium t-butoxide (4.60 g) was added to a dimethoxyethane (70 mL)-water (25 mL) mixture, and the mixture was heated to reflux for 40 hours. The reaction mixture was diluted with an aqueous saturated sodium bicarbonate solution and ethyl acetate. An organic layer was separated, dried over anhydrous sodium sulfate and concentrated under reduced pressure. The residue was purified with a basic silica gel column (ethyl acetate/hexane = 2/1 to ethyl acetate) to obtain a pyridine compound (0.64 g, 47%) as a yellow oil.

To a solution of the pyridine compound (0.60 g) in methanol (100 mL) were added 10% Palladium carbon (50%; 0.60 g) and then ammonium formate (3.00 g), and the mixture was heated to reflux for 2 hours. The reaction solution was cooled to room temperature, ammonium formate (4.00 g) was added, and the mixture was further heated to reflux for 15 hours. The reaction mixture was filtered, and the filtrate was concentrated under reduced pressure. The residue was purified with a basic silica gel column (ethyl acetate to ethyl acetate/methanol = 10/1) to obtain the title compound (0.10 g, 25%) as colorless powder.
NMR (300 MHz, CDCl₃) δ: 2.45 (3H, s), 2.48 (3H, s), 7.57 (2H, br), 8.57 (2H, dd, J = 6.3 and 1.7), 8.88 (1H, br). 29b) 4-(2,4-Dimethyl-1H-imidazol-5-yl)piperidine dihydrochloride

From 4-(2,4-dimethyl-1H-imidazol-5-yl)pyridine (0.10 g) obtained in Example 29a), the title compound (0.15 g, quantitative) was obtained as colorless powder in a similar manner to Example 26b).
NMR (200 MHz, CD₃OD) δ: 2.00-2.08 (4H, m), 2.29 (3H, s), 2.57 (3H, s), 3.08-3.23 (3H, m), 3.49-3.55 (2H, m).

### 29c) 1-{3-[(6-chloro-2-naphthyl)sulfonyl]propanoyl}-4-(2,4-dimethyl-1H-imidazol-5-yl)piperidine

From 4-(2,4-Dimethyl-1H-imidazol-5-yl)piperidine dihydrochloride (0.15 g) obtained in Example 29b), the title compound (0.16 g, 60%) was obtained as colorless powder in a similar manner to Example 19.
NMR (200 MHz, CDCl₃) δ: 1.62-1.80 (4H, m), 2.15 (3H, s), 2.32 (3H, s), 2.52-2.80 (2H, m), 2.84-2.92 (2H, m), 3.03-3.18 (1H, m), 3.53-3.60 (2H, m), 3.89-3.95 (1H, m), 4.57-4.63 (1H, m), 7.59 (1H, dd, J = 9.0 and 2.0), 7.94-7.98 (4H, m), 8.49 (1H, s).
Elemental analysis for C₂₃H₂₆ClN₃O₃S
Calculated (%): C, 60.05; H, 5.70; N, 9.14
Found (%): C, 59.82; H, 5.73; N, 9.27

### Example 30

### 1-{3-[(5-Chloro-1H-indol-2-yl)sulfonyl]propanoyl}-4-(2-methyl-1H-imidazol-4-yl)piperidine hydrochloride

### 30a) Tert-butyl 5-chloro-2-({3-[4-(2-methyl-1H-imidazol-4-yl)-1-piperidinyl]-3-oxopropyl}sulfonyl)-1H-indole-1-carboxylate

From 4-(2-methyl-1H-imidazol-4-yl)piperidine dihydrochloride (0.71 g) and 3-{[1-(tert-butoxycarbonyl)-5-chloro-1H-indol-2-yl]sulfonyl}propionic acid (1.16 g), the title compound (0.70 g, 44%) was obtained as colorless powder in a similar manner to Example 19.
NMR (200 MHz, CDCl₃) δ: 1.46-2.10 (4H, m), 1.73 (9H, s), 2.39 (3H, s), 2.61-2.80 (2H, m), 2.88-2.96 (2H, m), 3.05-3.20 (1H, m), 3.84-3.91 (1H, m), 4.00-4.10 (2H, m), 4.48-4.56 (1H, m), 6.57 (1H, s), 7.43 (1H, dd, J = 9.4 and 2.2), 7.63 (1H, d, J = 2.0), 8.01 (1H, d, J = 9.2).

### 30b) 1-{3-[(5-chloro-1H-indol-2-yl)sulfonyl]propanoyl}-4-(2-methyl-1H-imidazol-4-yl)piperidine hydrochloride

Tert-butyl 5-chloro-2-({3-[4-(2-methyl-1H-imidazol-4-yl)-1-piperidinyl]-3-oxopropyl}sulfonyl)-1H-indole-1-carboxylate (0.26 g) obtained in Example 30a) was dissolved in concentrated hydrochloric acid (1.5 mL), and the solution was stirred at room temperature for 30 minutes. The reaction solution was concentrated under reduced pressure, and the residue was washed with isopropyl alcohol to obtain the title compound (0.20 g, 87%) as brown powder. NMR (200 MHz, CD₃OD) δ: 1.22-1.63 (2H, m), 1.87-2.06 (2H, m), 2.59 (3H, s), 2.60-2.97 (4H, m), 3.11-3.29 (1H, m), 3.61-3.72 (2H, m), 3.95-4.01 (1H, m), 4.35-4.42 (1H, m), 7.30 (1H, dd, J = 8.8 and 2.2), 7.47 (1H, d, J = 9.0), 7.67 (1H, d, J = 2.2).
Elemental analysis for C₂₀H₂₃ClN₄O₃S·HCl·0.8C₃H₈O·H₂O Calculated (%): C, 50.06; H, 6.08; N, 10.42
Found (%): C, 49.75; H, 5.97; N, 10.13

### Example 31

### 1-{3-[(5-Chloro-1H-indol-2-yl)sulfonyl]propanoyl}-4-(2,4-dimethyl-1H-imidazol-5-yl)piperidine

### 31a) Tert-butyl 5-chloro-2-({3-[4-(2,4-dimethyl-1H-imidazol-5-yl)-1-piperidinyl]-3-oxopropyl}sulfonyl)-1H-indole-1-carboxylate

From 4-(2,4-dimethyl-1H-imidazol-5-yl)piperidine dihydrochloride (0.21 g) obtained in Example 29b) and 3-{[1-(tert-butoxycarbonyl)-5-chloro-1H-indol-2-yl]sulfonyl}propionic acid (0.32 g), the title compound (0.32 g, 70%) was obtained as colorless powder in a similar manner to Example 19.
NMR (200 MHz, CDCl₃) δ: 1.46-1.80 (4H, m), 1.73 (9H, s), 2.17 (3H, s), 2.38 (3H, s), 2.52-3.10 (5H, m), 3.83-3.90 (1H, m), 4.00-4.07 (2H, m), 4.57-4.64 (1H, m), 7.43 (1H, dd, J = 9.2 and 2.2), 7.50 (1H, s), 7.64 (1H, d, J = 2.0), 7.70 (1H, br), 7.99 (1H, d, J = 8.8).

### 31b) 1-{3-[(5-Chloro-1H-indol-2-yl)sulfonyl]propanoyl}-4-(2,4-dimethyl-1H-imidazol-5-yl)piperidine

Tert-butyl 5-chloro-2-({3-[4-(2,4-dimethyl-1H-imidazol-5-yl)-1-piperidinyl]-3-oxopropyl}sulfonyl)-1H-indole-1-carboxylate (0.32 g) obtained in Example 31a) was dissolved in concentrated hydrochloric acid (3 mL), and the solution was stirred at room temperature for 30 minutes. The reaction solution was neutralized by addition of triethylamine and then concentrated under reduced pressure. The residue was purified with a silica gel column (chloroform/methanol = 20/1 to 5/1) to obtain the title compound (0.11 g, 42%) as yellow powder.
NMR (200 MHz, CDCl₃) 5: 1.58-1.91 (4H, m), 2.19 (3H, s), 2.33-2.99 (5H, m), 2.46 (3H, s), 3.50-3.96 (3H, m), 4.56-4.63 (1H, m), 7.13 (1H, d, J = 0.8), 7.30 (1H, dd, J = 8.8 and 2.2), 7.46 (1H, d, J = 8.8), 7.68 (1H, d, J = 1.8). Elemental analysis for C₂₁H₂₅ClN₄O₃S·1.H₂O
Calculated (%): C, 53.80; H, 5.85; N, 11.95
Found (%): C, 53.62; H, 5.51; N, 11.78

### Example 32

### 1-{3-[(6-Chloro-2-naphthyl)sulfonyl]propanoyl}-4-(1H-imidazol-2-yl)-4-piperidinol

From tert-butyl 4-hydroxy-4-(lH-imidazol-2-yl)piperidine-1-carboxylate (JP-A 7-501556) (0.55 g), the title compound (0.11 g, 12%) was obtained as pale brown powder in a similar manner to Example 1.
NMR (300 MHz, CDCl₃+CD₃OD) δ: 1.79-2.11(4H, m), 2.87-2.96 (3H, m), 3.13-3.21 (1H, m), 3.50-3.61 (3H, m), 3.66-3.73 (1H, m), 4.11-4.19 (1H, m), 6.95 (1H, d, J = 1.5), 7.59 (1H, dd, J = 2.1 and 8.7), 7.90-7.99 (4H, m), 8.49 (1H, d, J = 1.5).
Elemental analysis for C₂₁H₂₂ClN₃O₄S
Calculated (%): C, 56.31; H, 4.95; N, 9.38
Found (%): C, 56.16; H, 4.86; N, 9.43

### Example 33

### 1-{3-[(6-Chloro-2-naphthyl)sulfonyl]propanoyl}-4-(1H-imidazol-2-yl)piperidine

From tert-butyl 4-(1H-imidazol-2-yl)piperidine-1-carboxylate (JP-A 7-501556) (0.26 g), the title compound (0.24 g, 54%) was obtained as colorless powder in a similar manner to Example 1.
NMR (300 MHz, CDCl₃) δ: 1.60-1.85 (2H, m), 1.98-2.14 (2H, m), 2.72-3.04 (4H, m), 3.15-3.24 (1H, m), 3.49-3.61 (2H, m), 3.90-3.95 (1H, m), 4.46-4.50 (1H, m), 6.96 (1H, br), 7.00 (1H, br), 7.58 (1H, dd, J = 2.1 and 9.0), 7.90-7.97 (4H, m), 8.48 (1H, d, J = 0.9), 8.88 (1H, br).
Elemental analysis for C₂₁H₂₂BrN₃O₃S
Calculated (%): C, 58.39; H, 5.13; N, 9.73
Found (%): C, 58.14; H, 5.13; N, 9.73

### Example 34

### 1-{3-[(6-Chloro-2-naphthyl)sulfonyl)propanoyl}-4-(1-methyl-1H-imidazol-2-yl)piperidine

### 34a) Tert-butyl 4-(1-methyl-1H-imidazol-2-yl)piperidine-1-carboxylate

Sodium hydride (60%; 40 mg) was added to a solution of tert-butyl 4-(1H-imidazol-2-yl)piperidine-1-carboxylate (0.25 g) in DMF (3 ml) at 0°C, and the mixture was stirred at 0°C for 30 minutes. Then, methyl iodide (0.06 mL) was added and the mixture was stirred at 0°C for 2 hours. The reaction mixture was concentrated under reduced pressure, and the residue was diluted with an aqueous sodium hydrogencarbonate solution and ethyl acetate. An organic layer was separated and dried over anhydrous sodium sulfate, and the solvent was concentrated under reduced pressure. The residue was purified with a silica gel column (chloroform/methanol = 10/1) to obtain the title compound (0.27 g, quantitative) as a pale yellow oil.
NMR (200 MHz, CDCl₃) δ: 1.46 (9H, s), 1.80-1.91 (4H, m), 2.70-2.96 (3H, m), 3.61 (3H, s), 4.18-4.25 (2H, m), 6.79 (1H, d, J = 1.0), 6.94 (1H, d, J = 1.0).

### 34b) 1-{3-[(6-Chloro-2-naphtyl)sulfonyl]propanoyl}-4-(1-methyl-1H-imidazol-2-yl)piperidine

From tert-butyl 4-(1-methyl-1H-imidazol-2-yl)piperidine-1-carboxylate (0.27 g) obtained in Example 34a), the title compound (0.31 g, 70%) was obtained as colorless powder in a similar manner to Example 1.
NMR (200 MHz, CDCl₃) δ: 1.60-2.00 (4H, m), 2.70-2.94 (4H, m), 3.11-3.26 (1H, m), 3.52-3.61 (2H, m), 3.61 (3H, s), 3.93-4.00 (1H, m), 4.45-4.52 (1H, m), 6.79 (1H, d, J = 1.2), 6.92 (1H, d, J = 1.4), 7.58 (1H, dd, J = 2.0 and 8.8), 7.89-7.98 (4H, m), 8.48 (1H, s), 8.88 (1H, br).
Elemental analysis for C₂₂H₂₄ClN₃O₃S·0.5H₂O
Calculated (%): C, 58.08; H, 5.54; N, 9.24
Found (%): C, 57.98; H, 5.64; N, 9.20

### Example 35

### 1-{3-[(6-Chloro-2-naphthyl)sulfonyl]propanoyl}-4-(4,5-dimethyl-1H-imidazol-2-yl)piperidine

### 35a) Tert-butyl 4-hydroxy-4-(4,5-dimethyl-1H-imidazol-2-yl)piperidine-1-carboxylate

A mixture of 4,5-dimethylimidazole (3.00 g), p-toluenesulfonic acid monohydrate (1.50 g) and triethyl orthoformate (60 mL) was stirred at 130°C for 6 hours. Sodium carbonate (1.50 g) was added, the reaction mixture was concentrated under reduced pressure, and the residue was dissolved in THF (50 mL). To the solution was added n-butyllithium (a 1.6 M solution in hexane, 17 mL, 27 mmol) while cooling to -40°C or lower, and then added dropwise was a solution of Boc-piperidone (2.66 g) in THF (20 mL). After completion of addition, the mixture was stirred at - 40°C or lower for 2 hours. After the reaction solution was warmed to room temperature, 0.1N hydrochloric acid (40 mL) was added and the mixture was stirred for 15 minutes. Then, ethyl acetate (50 mL) was added and the mixture was stirred for 5 minutes. An organic layer was separated, washed with an aqueous saturated sodium bicarbonate solution and an aqueous saturated sodium chloride solution, and then dried over anhydrous sodium sulfate. The solvent was concentrated under reduced pressure. The residue was purified with a silica gel column (chloroform/methanol = 20/1 to 10/1) to obtain the title compound (2.93 g, 21%) as a pale yellow oil.
NMR (200 MHz, CDCl₃) δ: 1.45 (9H, s), 1.71-1.78 (4H, m), 1.98-2.24 (2H, m), 2.13 (6H, s), 3.17-3.33 (2H, m), 3.89-3.96 (2H, m).

### 35b) Tert-butyl 4-(4,5-dimethyl-1H-imidazol-2-yl)-1,2,3,6-tetrahydropyridine-1-carboxylate

Tert-butyl 4-hydroxy-4-(4,5-dimethyl-1H-imidazol-2-yl)piperidine-1-carboxylate (1.96 g) obtained in Example 35a) and diisopropylethylamine (1.68 g) were dissolved in DMF (100 ml). While cooling to 0°C methanesulfonyl chloride (1.52 g) was added, and the mixture was stirred at 0°C for 2 hours. Additional diisopropylethylamine (1.68 g) and methanesulfonyl chloride (1.52 g) were added, and the mixture was stirred at room temperature for 16 hours. The mixture was diluted with water, adjusted to pH 9 by addition of a 1N aqueous sodium hydroxide solution, and then extracted with ethyl acetate. The extract was dried over anhydrous sodium sulfate and the solvent was concentrated under reduced pressure. The residue was purified with a silica gel column (chloroform/methanol = 20/1 to 10/1) to obtain the title compound (1.3 g, 70%) as a pale yellow oil.
NMR (200 MHz, CDCl₃) δ: 1.48 (9H, s), 2.16 (6H, s), 2.59 (2H, br), 3.58 (2H, t, J = 5.7), 4.02-4.06 (2H, m), 6.13 (1H, br).

### 35c) Tert-butyl 4-(4,5-dimethyl-1H-imidazol-2-yl)piperidine -1-carboxylate

Tert-butyl 4-(4,5-dimethyl-1H-imidazol-2-yl)-1,2,3,6-tetrahydropyridine-1-carboxylate (1.30 g) obtained in Example 35b) and 10% palladium carbon (50% hydrous, 0.20 g) were added to methanol (30 mL), and the mixture was stirred at room temperature for 10 hours under 5 atm hydrogen atmosphere. The reaction mixture was filtered and the filtrate was concentrated under reduced pressure to obtain the title compound (1.18 g, 90%) as colorless powder.
NMR (200 MHz, CDCl₃) δ: 1.45 (9H, s), 1.53-1.74 (2H, m), 1.93-1.98 (2H, m), 2.13 (6H, m), 2.75-2.89 (3H, m), 4.14-4.20 (2H, m).
35d) 1-{3-[(6-Chloro-2-naphthyl)sulfonyl]propanoyl}-4-(4,5-dimethyl-1H-imidazol-2-yl)piperidine

From tert-butyl 4-(4,5-dimethyl-1H-imidazol-2-yl)piperidine-1-carboxylate (0.24 g) obtained in Example 35c), the title compound (53 mg, 13%) was obtained as colorless powder in a similar manner to Example 1.
NMR (200 MHz, CDCl₃) δ: 1.52-2.10 (4H, m), 2.12 (6H, s), 2.63-2.74 (1H, m), 2.83-2.94 (3H, m), 3.08-3.20 (1H, m), 3.52-3.61 (2H, m), 3.87-3.94 (1H, m), 4.48-4.54 (1H, m), 7.58 (1H, dd, J = 2.0 and 8.8), 7.88-7.97 (4H, m), 8.48 (1H, s) .
Elemental analysis for C₂₃H₂₆ClN₃O₃S
Calculated (%): C, 60.05; H, 5.70; N, 9.14
Found (%): C, 59.82; H, 5.67; N, 9.08

### Example 36

### 1-{3-[(6-Chloro-2-naphthyl)sulfonyl]propanoyl}-4-(1,4,5-trimethyl-lH-imidazol-2-yl)piperidine

### 36a) Tert-butyl 4-(1,4,5-trimethyl-1H-imidazol-2-yl)piperidine-1-carboxylate

From tert-butyl 4-(4,5-dimethyl-1H-imidazol-2-yl)piperidine-1-carboxylate (0.24 g) obtained in Example 35c), the title compound (0.26 g, quantitative) was obtained as a brown oil in a similar manner to Example 34a). NMR (200 MHz, CDCl₃) δ: 1.46 (9H, s), 1.76-1.82 (4H, m), 2.09 (3H, s), 2.13 (3H, s), 2.70-2.90 (3H, m), 3.42 (3H, s), 4.18-4.25 (2H, m).

### 36b) 1-{3-[(6-Chloro-2-naphthyl)sulfonyl]propanoyl}-4-(1,4,5-trimethyl-1H-imidazol-2-yl)piperidine

From tert-butyl 4-(1,4,5-trimethyl-1H-imidazol-2-yl)piperidine-1-carboxylate (0.25 g) obtained in Example 36a), the title compound (0.16 g, 38%) was obtained as colorless powder in a similar manner to Example 1.
NMR (200 MHz, CDCl₃) δ: 1.60-2.05(4H, m), 2.09 (3H, s), 2.10 (3H, s), 2.30-3.35 (5H, m), 3.41 (3H, s), 3.51-3.61 (2H, m), 3.90-3.97 (1H, m), 4.52-4.58 (1H, m), 7.58 (1H, dd, J = 1.8 and 8.8), 7.89-7.99 (4H, m), 8.49 (1H, s).
Elemental analysis for C₂₄H₂₈ClN₃O₃S·0.7H₂O
Calculated (%): C, 59.24; H, 6.09; N, 8.63
Found (%): C, 59.33; H, 6.13; N, 8.34

### Example 37

### 1-{3-[(6-Chloro-2-naphthyl)sulfonyl]propanoyl}-4-(1-ethyl-4,5-dimethy-1H-imidazol-2-yl)piperidine

### 37a) Tert-butyl 4-(1-ethyl-4,5-dimethyl-1H-imidazol-2-yl)piperidine-1-carboxylate

From tert-butyl 4-(4,5-dimethyl-1H-imidazol-2-yl)piperidine-1-carboxylate (0.24 g) obtained in Example 35c) and ethyl iodide (134 mg), the title compound (0.27 g, quantitative) was obtained as a brown oil as in a similar manner to Example 34a).
NMR (200 MHz, CDCl₃) δ: 1.26 (3H, t, J = 3.6), 1.45 (9H, s), 1.70-2.22 (4H, m), 2.11 (3H, s), 2.12 (3H, s), 2.70-2.90 (3H, m), 3.81 (2H, q, J = 7.0), 4.18-4.25 (2H, m).

### 37b) 1-{3-[(6-Chloro-2-naphthyl)sulfonyl]propanoyl}-4-(1-ethyl-4,5-dimethyl-1H-imidazol-2-yl)piperidine

From tert-butyl 4-(1-ethyl-4,5-dimethyl-1H-imidazol-2-yl)piperidine-1-carboxylate (0.26 g) obtained in Example 37a), the title compound (0.15 g, 36%) was obtained as colorless powder in a similar manner to Example 1.
NMR (200 MHz, CDCl₃) δ: 1.27 (3H, t, J = 7.2), 1.76-3.19(9H, m), 2.10 (6H, s), 3.15-3.61 (2H, m), 3.85 (2H, q, J = 7.2), 3.51-3.61 (2H, m), 3.91-3.99 (1H, m), 4.54-4.61 (1H, m), 7.58 (1H, dd, J = 2.0 and 8.8), 7.94-7.99 (4H, m), 8.49 (1H, s).
Elemental analysis for C₂₅H₃ClN₃O₃S·0.9H₂O·0.2C₄H₈O₂
Calculated (%): C, 59.38; H, 6.45; N, 8.05
Found (%): C, 59.71; H, 6.72; N, 7.83

### Example 38

### 7-(1-{3-[(6-Chloro-2-naphthyl)sulfonyl]propanoyl}-4-piperidinyl)-3-methyl-6,7-dihydroimidazo[1,5-a]pyrazin-8(5H)-one

### 38a) Tert-butyl 4-[(2-hydroxyethyl)amino]piperidine-1-carboxylate

A solution of tert-butyl 4-oxopiperidine-1-carboxylate (15.0 g), 2-aminoethanol (14.0 mL) and acetic acid (6.6 mL) in 1,2-dichloroethane (300 mL) was stirred at room temperature for 1 hour. Sodium triacetoxyborohydride (49.2 g) was added thereto, and the mixture was stirred at room temperature for 15 hours. The reaction solution was adjusted to pH 12 with a 1N aqueous sodium hydroxide solution and then extracted with chloroform (100 mL). An organic layer was dried over anhydrous magnesium sulfate and the solvent was then distilled off under reduced pressure to obtain the title compound (18.0 g, 89%) as a colorless oil.
NMR (200 MHz, CDCl₃) δ: 1.17-1.37 (2H, m), 1.46 (9H, s), 1.88 (2H, t), 2.57-2.85 (5H, m), 3.66 (2H, t), 4.06 (2H, d).

### 38b) Tert-butyl 4-{(2-hydroxyethyl)[(2-methyl-1H-imidazol-4-yl)carbonyl]amino}piperidine-1-carboxylate

HOBt (3.7 g) and WSC (4.6 g) were successively added to a suspension of 2-methylimidazole-4-carboxylic acid (2.0 g) in acetonitrile (150 mL), and the mixture was stirred at room temperature for 20 minutes. To this reaction solution was added a solution of tert-butyl 4-[(2-hydroxyethyl)amino]piperidine-1-carboxylate (4.7 g) obtained in Example 38a) and triethylamine (8.0 mL) in acetonitrile (50 mL), and the mixture was stirred at room temperature for 15 hours. After acetonitrile was distilled off under reduced pressure, chloroform (100 mL) and water (100 mL) were added to the residue. An organic layer was separated and dried over anhydrous magnesium sulfate, and the solvent was distilled off under reduced pressure. The residue was purified with a basic silica gel column (ethyl acetate: ethanol = 5:1) to obtain the title compound (1.0 g, 18%) as a colorless oil.
NMR (200 MHz, CDCl₃) δ: 1.47 (9H, s), 1.84 (4H, bs), 2.37 (2H, bs), 2.78 (2H, bs), 3.82 (4H, bs), 4.27 (3H, bs), 7.31 (1H, bs).

### 38c) Tert-butyl 4-(3-methyl-8-oxo-5,6-dihydroimidazo[1,5-a]pyrazin-7(8H)-yl)piperidine-1-carboxylate

Under ice-cooling, methanesulfonic acid chloride (240 µL) was added dropwise to a solution of tert-butyl 4-{(2-hydroxyethyl)[(2-methyl-1H-imidazol-5-yl)carbonyl]amino}piperidine-1-carboxylate (940 mg) obtained in Example 38b) and triethylamine (720 µL) in THF (30 mL), and the mixture was stirred at room temperature for 3 hours. To the reaction solution were added chloroform (50 mL) and water (50 mL). An organic layer was separated and dried over anhydrous magnesium sulfate, and the solvent was distilled off under reduced pressure. The residue was purified with a basic silica gel column (ethyl acetate: ethanol = 5:1) to obtain the title compound (390 mg, 44%) as a white solid.
NMR (300 MHz, CDCl₃) δ: 1.47 (9H, s), 1.52-1.72 (4H, s), 2.41 (3H, s), 2.85 (2H, t), 3.57 (2H, t), 4.04 (2H, t), 4.23 (2H, bs), 4.73-4.81 (1H, m), 7.62 (1H, s).

### 38d) 7-(1-{3-[(6-chloro-2-naphthyl)sulfonyl]propanoyl}-4-piperidinyl)-3-methyl-6,7-dihydroimidazo[1,5-a]pyrazin-8(5H)-one

Concentrated hydrochloric acid (5 mL) was added to tert-butyl 4-(3-methyl-8-oxo-5,6-dihydroimidazo[1,5-a]pyrazin-7(8H)-yl)piperidine-1-carboxylate (420 mg) obtained in Example 38c) to dissolve it. To this solution was added ethanol (50 mL) and the solvent was distilled off under reduced pressure. To the residue was added ethanol again and the solvent was distilled off under reduced pressure. To the residue was added isopropyl alcohol and a precipitate was filtered. The precipitate was washed successively with isopropyl alcohol and diethyl ether, and dried under reduced pressure to obtain 3-methyl-7-(4-piperidinyl)-6,7-dihydroimidazo[1,5-a]pyrazin-8(5H)-one dihydrochloride as a white solid.

3-[(6-Chloro-2-naphthyl)sulfonyl]propionic acid (400 mg) was suspended in acetonitrile (10 mL) and to the suspension, HOBt(310 mg) and WSC(380 mg) were added successively. The mixture was stirred at room temperature for 20 minutes. To this reaction solution was added a solution of 3-methyl-7-(4-piperidinyl)-6,7-dihydroimidazo[1,5-a]pyrazin-8(5H)-one dihydrochloride obtained in Example 38c), DBU (330 mL) and triethylamine (460 mL) in acetonitrile (10 mL), and the mixture was stirred at room temperature for 15 hours. After acetonitrile was distilled off under reduced pressure, chloroform (50 mL) and water (50 mL) were added to the residue. An organic layer was separated and dried over anhydrous magnesium sulfate, and the solvent was distilled off under reduced pressure. The residue was purified with a basic silica gel column (ethyl acetate: ethanol = 5:1) and then recrystallized from ethyl acetate:ethanol to obtain the title compound (320 mg, 48%) as a white crystal. NMR (300 MHz, CDCl₃) δ: 1.54-1.67 (11H, m), 1.72-1.85 (2H, m), 2.41 (3H, s), 2.61-2.79 (1H, m), 2.80-2.87 (1H, m), 2.94-3.05 (1H, m), 3.17-3.25 (1H, m), 3.45-3.55 (1H, m), 3.60-3.70 (3H, m), 4.67-4.72 (1H, m), 4.83-4.90 (1H, m), 7.58-7.63 (2H, m), 7.89-7.96 (4H, m), 8.48 (1H, d). Elemental analysis for C₂₅H₂₇ClN₄O₄S
Calculated (%): C, 58.30; H, 5.28; N, 10.88
Found (%): C, 58.13; H, 5.15; N, 10.67

### Example 39

### 7-(1-{3-[(6-Chloro-2-naphthyl)sulfonyl]propanoyl}-4-piperidinyl)-1-methyl-6,7-dihydoroimidazo[1,5-a]pyrazin-8(5H)-one

### 39a) Tert-butyl 4-{(2-hydroxyethyl)[(4-methyl-1H-imidazol-5-yl)carbonyl]amino}piperidine-1-carboxylate

HOBt (2.8 g) and WSC (3.5 g) were added successively to a suspension of 4-methylimidazole-5-carboxylic acid hydrochloride (G. Wellmann et al. Synthesis, 356 (1984))(1.6 g) in acetonitrile (100 mL), and the mixture was stirred at room temperature for 20 minutes (reaction solution A). In another flask, a solution of tert-butyl 4-[(2-hydroxyethyl)amino]piperidine-1-carboxylate (3.0 g), N-trimethylsilylacetamide (8.1 g) and triethylamine (5.0 mL) in acetonitrile (50 mL) was stirred at room temperature for 20 minutes (reaction solution B). The reaction solution B was added to the reaction solution A, and the mixture was stirred at room temperature for 15 hours. After acetonitrile was distilled off under reduced pressure, chloroform (100 mL) and water (100 mL) were added to the residue. An organic layer was separated and dried over anhydrous magnesium sulfate, and the solvent was distilled off under reduced pressure. The residue was purified with a basic silica gel column (ethyl acetate: ethanol = 5:1) to obtain the title compound (2.0 g, 59%) as a colorless oil. NMR (300 MHz, CDCl₃) δ: 1.46 (9H, s), 1.84 (4H, bs), 2.27 (3H, s), 2.77 (2H, bs), 3.68 (2H, bs), 3.79-3.82 (2H, m), 4.20-4.33 (3H, m), 7.32 (1H, s).

### 39b) Tert-butyl 4-(1-methyl-8-oxo-5,6-dihydroimidazo[1,5-a]pyrazin-7(8H)-yl)piperidine-1-carboxylate

From tert-butyl 4-{(2-hydroxyethyl)[(4-ethyl-1H-imidazol-5-yl)carbonyl]amino}piperidine-1-carboxylate obtained in Example 39a), the title compound (560 mg, 30%) was obtained as a white solid in a similar manner to Example 38c).
NMR (300 MHz, CDCl₃) δ: 1.47 (9H, s), 1.55-1.71 (4H, m), 2.54 (3H, s), 2.84 (2H, t), 3.55 (2H, t), 4.13 (2H, t), 4.22 (2H, bs), 4.74-4.83 (1H, m), 7.39 (1H, s).

### 39c) 7-(1-{3-[(6-chloro-2-naphthyl)sulfonyl]propanoyl}-4-piperidinyl)-1-methyl-6,7-dihydroimidazo[1,5-a]pyrazin-8(5H)-one

From tert-butyl 4-(1-methyl-8-oxo-5,6-dihydroimidazo[1,5-a]pyrazin-7(8H)-yl)piperidine-1-carboxylate obtained in Example 39b), the title compound (475 mg, 46%) was obtained as a white crystal (ethanol/ethyl acetate) in a similar manner to Example 38d). NMR (300 MHz, CDCl₃) δ: 1.52-1.80 (6H, m), 2.54 (3H, s), 2.57-3.26 (3H, m), 3.43-3.68 (3H, m), 3.97 (1H, d), 4.14 (1H, t), 4.69 (1H, d), 4.81-4.93 (1H, m), 7.41 (1H, s), 7.61 (1H, dd), 7.89-7.98 (4H, m), 8.49 (1H, s).
Elemental analysis for C₂₅H₂₇ClN₄O₄S·0.2EtOAc
Calculated (%): C, 58.18; H, 5.41; N, 10.52
Found (%): C, 58.01; H, 5.19; N, 10.39

### Example 40

### 7-(1-{3-[(6-Chloro-2-naphthyl)sulforiyl]propanoyl}-4-piperidinyl)-1-ethyl-6,7-dihydoroimidazo[1,5-a]pyrazin-8(5H)-one

### 40a) Tert-butyl 4-{(2-hydroxyethyl)[(4-ethyl-1H-imidazol-5-yl)carbonyl]amino}piperidine-1-carboxylate

Ethyl 4-ethylimidazole-5-carboxylate (2.3 g) was dissolved in 8N hydrochloric acid (50 mL), and the solution was heated at 100°C for 15 hours. The solvent was distilled off under reduced pressure to obtain 4-ethylimidazole-5-carboxylic acid hydrochloride as a brown solid. Using this brown solid, the title compound (2.5 g, 46%) was obtained as a brown oil in a similar manner to Example 39a).
NMR (200 MHz, CDCl₃) δ: 1.14-1.29 (3H, m), 1.46 (9H, s), 1.83 (4H, bs), 2.73-3.02 (4H, m), 3.60-3.81 (4H, m), 4.08-4.39 (3H, m), 7.32 (1H, s).

### 40b) Tert-butyl 4-(1-ethyl-8-oxo-5,6-dihydroimidazo[1,5-a]pyrazin-7(8H)-yl)piperidine-1-carboxylate

From tert-butyl 4-{(2-hydroxyethyl)[(4-ethyl-1H-imidazol-5-yl)carbonyl]amino}piperidine-1-carboxylate obtained in Example 40a), the title compound (1.5 g, 62%) was obtained as a brown oil in a similar manner to Example 38c).
NMR (200 MHz, CDCl₃) δ: 1.28 (3H, t), 1.46 (9H, s), 1.53-1.75 (4H, m), 2.84 (2H, t), 2.97 (2H, q), 3.52-3.58 (2H, m), 4.11-4.29 (4H, m), 4.73-4.85 (1H, m), 7.42 (1H, s).

### 40c) 7-(1-{3-[(6-chloro-2-naphthyl)sulfonyl]propanoyl}-4-piperidinyl)-1-ethyl-6,7-dihydroimidazo[1,5-a]pyrazin-8(5H)-one

Form tert-butyl 4-(1-ethyl-8-oxo-5,6-dihydroimidazo[1,5-a]pyrazin-7(8H)-yl)piperidine-1-carboxylate obtained in Example 40b), the title compound (1.3 g, 57%) was obtained as a white crystal (ethyl acetate/diethyl ether) in a similar manner to Example 38d). NMR (300 MHz, CDCl₃) δ: 1.28 (3H, t), 1.49-1.66 (2H, m), 1.69-1.85 (2H, m), 2.59-2.68 (1H, m), 2.77-2.92 (1H, m), 2.93-3.03 (3H, m), 3.14-3.24 (1H, m), 3.45-3.55 (3H, m), 3.59-3.69 (1H, m), 3.97 (1H, d), 4.10-4.15 (2H, m), 4.74 (1H, d), 4.80-4.90 (1H, m), 7.41 (1H, s), 7.59 (1H, dd), 7.89-7.93 (4H, m), 8.47 (1H, s).
Elemental analysis for C₂₆H₂₉ClN₄O₄S·0.5H₂O·0.1EtOAc Calculated (%): C, 57.98; H, 5.68; N, 10.25
Found (%): C, 58.25; H, 5.64; N, 9.98

### Example 41

### 7-(1-{3-[(6-Chloro-2-naphthyl)sulfonyl]propanoyl}-4-piperidinyl)-1-ethyl-3-methyl-6,7-dihydroimidazo[1,5-a]pyrazin-8(5H)-one

### 41a) 4-Ethyl-2-methyl-1H-imidazole-5-carboxylic acid

4-Ethyl-2-methyl-1H-imidazole-5-carbaldehyde (10 g) and sodium dihydrogenphosphate (26 g) were suspended in a tert-butanol:water:2-methyl-2-butene = 5:4:1 mixture (200 mL) and to the suspension, sodium chlorite (35 g) was added slowly. The mixture was then stirred at room temperature for 5 hours. After tert-butanol was distilled off under reduced pressure, the residue was adjusted to pH 3 with 1N hydrochloric acid to obtain a precipitate. The precipitate was filtered, washed with diethyl ether and dried under reduced pressure to obtain the title compound (2.8 g, 25%) as a pale yellow solid.
NMR (300 MHz, DMSO-d₆) 5: 1.17 (3H, t), 2.32 (3H, s), 2.56 (2H, q).

### 41b) Tert-butyl 4-{[(4-ethyl-2-methyl-1H-imidazol-5-yl)carbonyl](2-hydroxyethyl)amino}piperidine-1-carboxylate

From 4-ethyl-2-methyl-1H-imidazole-5-carboxylic acid obtained in Example 41a), the title compound (3.3 g, 57%) was obtained as a yellow oil in a similar manner to Example 39a).
NMR (300 MHz, CDCl₃) δ: 1.24 (3H, t), 1.46 (9H, s), 1.67-1.87 (4H, m), 2.24 (3H, m), 2.61 (2H, q), 2.76-2.83 (4H, m), 3.64 (2H, t), 3.79-3.81 (2H, m).

### 41c) Tert-butyl 4-(1-ethyl-3-methyl-8-oxo-5,6-dihydroimidazo[1,5-a]pyrazin-7(8H)-yl)piperidine-1-carboxylate

From tert-butyl 4-{[(4-ethyl-2-methyl-1H-imidazol-5-yl)carbonyl](2-hydroxyethyl)amino}piperidine-1-carboxylate obtained in Example 41b), the title compound (1.1 g, 35%) was obtained as a yellow oil in a similar manner to Example 38c).
NMR (200 MHz, CDCl₃) δ: 1.31 (3H, t), 1.47 (9H, s), 1.67-1.75 (4H, m), 2.52 (3H, s), 2.67 (2H, q), 2.84 (2H, t), 3.50-3.56 (2H, m), 3.91-4.02 (2H, m), 4.21-4.27 (2H, m), 4.72-4.84 (1H, m).

### 41d) 7-(1-{3-[(6-Chloro-2-naphthyl)sulfonyl]propanoyl}-4-piperidinyl)-1-ethyl-3-methyl-6,7-dihydroimidazo[1,5-a]pyrazin-8(5H)-one

From tert-butyl 4-(1-ethyl-3-methyl-8-oxo-5,6-dihydroimidazo[1,5-a]pyrazin-7(8H)-yl)piperidine-1-carboxylate obtained in Example 41c), the title compound (740 mg, 45%) was obtained as a white crystal (ethyl acetate/ethanol) in a similar manner to Example 38d).
NMR (300 MHz, CDCl₃) δ: 1.31 (3H, t), 1.50-1.84 (4H, m), 2.51 (3H, s), 2.60-2.70 (3H, m), 2.77-2.87 (1H, m), 2.93-3.04 (1H, m), 3.19 (1H, t), 3.45-3.55 (3H, m), 3.59-3.69 (1H, m), 3.96-4.00 (3H, m), 4.69 (1H, d), 4.85 (1H, tt), 7.59 (1H, dd), 7.89-7.96 (4H, m), 8.48 (1H, d).
Elemental analysis for C₂₇H₃₁ClN₄O₄S·0.1EtOAc
Calculated (%): C, 59.63; H, 5.81; N, 10.15
Found (%): C, 59.34; H, 5.61; N, 10.16

### Example 42

### 2-(1-{3-[(6-Chloro-2-naphthyl)sulfonyl]propanoyl}-4-piperidinyl)-5-ethyl-1,2-dihydro-3H-imidazo[1,5-c]imidazol-3-one

### 42a) Tert-butyl 4-{[(2-ethyl-1H-imidazol-4-yl)methyl]amino}piperidine-1-carboxylate

After a solution of 2-ethylimidazole-4-carbaldehyde (5.0 g), tert-butyl 4-aminopiperidine-1-carboxylate (8.9 g) and acetic acid (1.0 mL) in 1,2-dichloroethane (100 mL) was stirred at room temperature for 1 hour, sodium triacetoxyborohydride (17 g) was added and the mixture was stirred at room temperature for 15 hours. An aqueous saturated sodium hydrogencarbonate solution (100 mL) was added to the reaction solution. An organic layer was separated and dried over anhydrous magnesium sulfate, and the solvent was distilled off under reduced pressure. The residue was purified with a basic silica gel column (ethyl acetate: ethanol = 10:1) to obtain the title compound (10 g, 81%) as a yellow oil.
NMR (300 MHz, CDCl₃) δ: 1.19-1.31 (5H, m), 1.46 (9H, s), 1.46-1.84 (2H, m), 2.67-2.81 (4H, m), 3.75 (2H, s), 4.04-4.09 (3H, m), 6.73 (1H, s).

### 42b) Tert-butyl 4-(5-ethyl-3-oxo-1H-imidazo[1,5-c]imidazol-2(3H)-yl)piperidine-1-carboxylate

N,N'-carbonyldiimidazole (5.8 g) was added to a solution of tert-butyl 4-{[(2-ethyl-1H-imidazol-4-yl)methyl]amino}piperidine-1-carboxylate (10 g) obtained in Example 42a) and DBU (5.8 mL) in dichloromethane (100 mL), and the mixture was stirred at room temperature for 5 hours. Water (100 mL) was added to the reaction solution, and an organic layer was separated and dried over anhydrous magnesium sulfate. The solvent was distilled off under reduced pressure. The residue was purified with a silica gel column (ethyl acetate:ethanol = 5:1) to obtain the title compound (4.8 g, 44%) as a colorless oil.
NMR (300 MHz, CDCl₃) δ: 1.35 (3H, t), 1.40-1.49 (11H, m), 1.64 (2H, qd), 1.85 (2H, d), 2.83 (2H, t), 3.02 (2H, t), 4.04-4.15 (1H, m), 4.30 (2H, m), 6.72 (1H, t).

### 42c) 2-(1-{3-[(6-Chloro-2-naphthyl)sulfonyl]propanoyl}-4-piperidinyl)-5-ethyl-1,2-dihydro-3H-imidazo[1,5-c]imidazol-3-one

From tert-butyl 4-(5-ethyl-3-oxo-1H-imidazo[1,5-c]imidazol-2(3H)-yl)piperidine-1-carboxylate obtained in Example 42b), the title compound (1.7 g, 47%) was obtained as a white crystal (ethanol/ethyl acetate) in a similar manner to Example 38d).
NMR (300 MHz, CDCl₃) δ: 1.35 (3H, t), 1.58-1.75 (2H, m), 1.87-1.99 (2H, m), 2.63 (1H, t), 2.82-2.93 (2H, m), 3.00 (2H, q), 3.19 (1H, t), 3.47-3.65 (1H, m), 3.99 (1H, d), 4.08-4.22 (1H, m), 4.25 (2H, s), 4.72 (1H, d), 6.72 (1H, t), 7.59 (1H, dd), 7.89-7.26 (4H, m), 8.48 (1H, s).
Elemental analysis for C₂₅H₂₇ClN₄O₄S·0.5EtOAc
Calculated (%): C, 58.00; H, 5.59; N, 10.02
Found (%): C, 57.83; H, 5.32; N, 10.25

### Example 43

### 2-(1-{3-[(6-Chloro-2-naphthyl)sulfonyl]propanoyl}-4-methyl-4-piperidinyl)-5-methyl-1,2-dihydro-3H-imidazo[1,5-c]imidazol-3-one hydrochloride

### 43a) Tert-butyl 4-methyl-4-{[(2-methyl-1H-imidazol-4-yl)methyl]amino}piperidine-1-carboxylate

From 2-methyl-4-formylimidazole (670 mg) and tert-butyl 4-amino-4-methylpiperidine-1-carboxylate (WO 01/40217) (1.3 g), the title compound (230 mg, 12%) was obtained as a colorless oil in a similar manner to Example 42a).
NMR (200 MHz, CDCl₃) δ: 1.18 (3H, s), 1.45 (9H, s), 1.48-1.57 (4H, m), 2.39 (3H, s), 3.36-3.49 (4H, m), 3.66 (2H, s), 6.75 (1H, s).

### 43b) Tert-butyl 4-methyl-4-(5-methyl-3-oxo-1H-imidazo[1,5-c]imidazol-2(3H)-yl)piperidine-1-carboxylate

From tert-butyl 4-methyl-4-{[(2-methyl-1H-imidazol-4-yl)methyl]amino}piperidine-1-carboxylate obtained in Example 43a), the title compound (170 mg, 68%) was obtained as a yellow oil in a similar manner to Example 42b).
NMR (300 MHz, CDCl₃) δ: 1.44 (3H, s), 1.46 (9H, s), 1.67-1.80 (2H, m), 2.04 (2H, bs), 2.59 (3H, s), 3.32-3.41 (2H, m), 3.55-3.60 (2H, m), 4.34 (2H, s), 6.66 (1H, s).

### 43c) 2-(1-{3-[(6-Chloro-2-naphthyl)sulfonyl]propanoyl}-4-methyl-4-piperidinyl)-5-methyl-1,2-dihydro-3H-imidazo[1,5-c]imidazol-3-one hydrochloride

From tert-butyl 4-methyl-4-(5-methyl-3-oxo-1H-imidazo[1,5-c]imidazol-2(3H)-yl)piperidine-1-carboxylate obtained in Example 43b), 2-(1-13-[6-chloro-2-naphthyl)sulfonyl]propanoyl}-4-methyl-4-piperidinyl)-5-methyl-1,2-dihydro-3H-imidazo[1,5-c]imidazol-3-one was obtained as a colorless oil in a similar manner to Example 38d). This oil was dissolved in ethyl acetate (2 mL) and thereto a 4N solution of hydrogen chloride in ethyl acetate (200 µL) was added. The mixture was stirred at room temperature for 10 minutes. A Precipitate was filtered, washed successively with ethyl acetate and diethyl ether, and dried under reduced pressure to obtain the title compound (153 mg, 51%) as a white solid.
NMR (200 MHz, CDCl₃) δ: 1.54 (3H, s), 1.75-2.02 (2H, m), 2.17-2.25 (1H, m), 2.47-2.55 (1H, m), 2.85-2.92 (2H, m), 2.96 (3H, s), 3.46-3.63 (6H, m), 4.65 (2H, s), 7.22 (1H, s), 7.57-7.62 (1H, m), 7.88-7.98 (4H, m), 8.48 (1H, s). Elemental analysis for C₂₅H₂₇ClN₄O₄S·HCl
Calculated (%): C, 51.90; H, 5.40; N, 9.68
Found (%): C, 52.16; H, 5.55; N, 9.81

### Example 44

### 2-(1-{3-[(4-Bromophenyl)sulfonyl]propanoyl}-4-piperidinyl)-5-methyl-1,2-dihydro-3H-imidazo[1,5-climidazol-3-one

From 3-[(4-bromophenyl)sulfonyl]propionic acid (WO 98/05635) (1.1 g) and 5-methyl-2-(4-piperidinyl)-1,2-dihydro-3H-imidazo[1,5-c]imidazole (1.0 g) obtained in Example 69b), the title compound (720 mg, 39%) was obtained as a white crystal (chloroform/diethyl ether) in a similar manner to Example 38d).
NMR (300 MHz, CDCl₃) δ: 1.56-1.77 (2H, m), 1.88-2.05 (2H, m), 2.61 (3H, s), 2.66-2.70 (1H, m), 2.76-2.99 (2H, m), 3.15-3.25 (1H, m), 3.38-3.59 (2H, m), 3.98 (1H, d), 4.18 (1H, m), 4.27 (2H, s), 4.73 (1H, d), 6.71 (1H, t), 7.71-7.80 (4H, m).
Elemental analysis for C₂₀H₂₃BrN₄O₄S·H₂O
Calculated (%): C, 46.79; H, 4.91; N, 10.91
Found (%): C, 47.09; H, 4.77; N, 11.03

### Example 45

### 6-(4-{3-[(6-Chloro-2-naphthyl)sulfonyl]propanoyl}-1-piperidinyl)-2-methyl-6,7-dihydro-5H-imidazo[1,5-a]imidazol-5-one

### 45a) 4-Methylimidazole-2-carbaldehyde

According to the article (N. J. Curtis et al. J. Org. Chem., 45, 4038 (1980)), the title compound was synthesized from 4-methylimidazole (99%).
NMR (300 MHz, DMSO-d₆) δ: 2.23 (3H, s), 6.81 (1H, s), 9.51 (1H, s).

### 45b) Tert-butyl 4-{[(4-methyl-1H-imidazol-2-yl)methyl]amino}piperidine-1-carboxylate

From 4-methylimidazole-2-carbaldehyde (3.4 g) obtained in Example 45a) and tert-butyl 4-aminopiperidine-1-carboxylate (6.3 g), the title compound (6.3 g, 68%) was obtained as a yellow oil in a similar manner to Example 42a).
NMR (300 MHz, CDCl₃) δ: 1.16-1.29 (2H, m), 1.45 (9H, s), 1.84 (2H, d), 2.22 (3H, s), 2.59-2.66 (1H, m), 2.76 (2H, t), 3.89 (2H, s), 4.00 (2H, bs), 6.63 (1H, s).

### 45c) Tert-butyl 4-(2-methyl-5-oxo-5H-imidazo[1,5-a]imidazol-6(7H)-yl)piperidine-1-carboxylate

From tert-butyl 4-{[(4-methyl-1H-imidazol-2-yl)methyl]amino}piperidine-1-carboxylate obtained in Example 45b), the title compound (1.2 g, 46%) was obtained as a yellow oil in a similar manner to Example 42b).
NMR (300 MHz, CDCl₃) δ: 1.47 (9H, s), 1.56-1.70 (2H, m), 1.85 (2H, d), 2.28 (3H, s), 2.82 (2H, t), 4.10-4.24 (3H, m), 4.27 (2H, s), 7.00 (1H, s).

### 45d) 6-(4-{3-[(6-Chloro-2-naphthyl)sulfonyl]propanoyl}-1-piperidinyl)-2-methyl-6,7-dihydro-5H-imidazo[1,5-a]imidazol-5-one

From tert-butyl 4-(2-methyl-5-oxo-5H-imidazo[1,5-a]imidazol-6(7H)-yl)piperidine-1-carboxylate obtained in Example 45c), the title compound (950 mg 51%) was obtained as a white crystal (ethyl acetate/ethanol) in a similar manner to Example 38b).
NMR (300 MHz, CDCl₃) δ: 1.53-1.75 (2H, m), 1.88-2.01 (2H, m), 2.28 (3H, s), 2.62 (1H, t), 2.81-3.02 (2H, m), 3.18 (1H, t), 3.47-3.64 (2H, m), 3.99 (1H, d), 4.18-4.27 (3H, m), 4.71 (1H, d), 7.01 (1H, d), 7.59 (1H, dd), 7.89-7.96 (4H, m), 8.47 (1H, d).
Elemental analysis for C₂₄H₂₅N₄O₄SCl·0.1EtOAc
Calculated (%): C, 57.48; H, 5.10; N, 10.99
Found (%): C, 57.19; H, 5.17; N, 11.01

### Example 46

### 6-(4-{3-[(6-Chloro-2-naphthyl)sulfonyl]propanoyl}-1-piperidinyl)-2-methyl-6,7-dihydro-5H-imidazo[1,5-a]imidazol-5-one hydrochloride

### 46a) Tert-butyl 4-hydroxy-4-(7-oxo-6,7-dihydro-5H-pyrrolo[1,2-c]imidazol-6-yl)piperidine-1-carboxylate

Under argon atmosphere, a solution of bis(trimethylsilyl)amidolithium in hexane (1.0 M, 13 mL) was diluted with THF(10 mL) and then cooled to -60°C. To this solution was added dropwise a solution of 5,6-dihydro-7H-pyrrolo[1,2-c]imidazol-7-one(C. Christine et al. Tetrahedron, 56, 1837 (2000))(1.0 g) in THF (50 mL) at -60°C. The mixture was stirred at that temperature for 2 hours. To the mixture were added a suspension of anhydrous cerium chloride (3.0 g) in THF (20 mL) at -60°C and then a solution of tert-butyl 4-oxopiperidine-1-carboxylate (1.4 g) in THF (20 mL) dropwise, and the mixture was stirred at the same temperature for 3 hours. After addition of an aqueous saturated ammonium chloride solution (50 mL) at -60°C, the mixture was warmed to room temperature and then extracted with chloroform (50 mL). An organic layer was separated and dried over anhydrous magnesium sulfate, and the solvent was distilled off. The residue was purified with a basic silica gel column (ethyl acetate) to obtain the title compound (1.2 g, 52%) as a white solid.
NMR (300 MHz, CDCl₃) δ: 1.45 (9H, s), 1.48-1.71 (2H, m), 3.15 (2H, bs), 3.37 (1H, dd), 3.69-3.92 (4H, m), 4.24 (1H, dd), 4.48 (1H, dd), 7.60 (1H, s), 7.74 (1H, s).

### 46b) 6-(1-{3-[(6-Chloro-2-naphthyl)sulfonyl]propanoyl}-4-piperidinyl)-5,6-dihydro-7H-pyrrolo[1,2-c]imidazol-7-one hydrochloride

Tert-butyl 4-hydroxy-4-(7-oxo-6,7-dihydro-5H-pyrrolo[1,2-c]imidazol-6-yl)piperidine-1-carboxylate (480 mg) obtained in Example 46a) was dissolved in concentrated hydrochloric acid (10 mL), and the solution was heated at 100°C for 15 hours. After the solution was cooled to room temperature, the solvent was distilled off under reduced pressure. The residue was dissolved in a mixed solvent (10 mL) of methanol:water = 1:1 and to the solution 10% Pd/C (50 mg) was added. The mixture was stirred for 3 hours under hydrogen atmosphere. The reaction solution was filtered using Celite and the filtrate was concentrated under reduced pressure to obtain 6-(4-piperidinyl)-5,6-dihydro-7H-pyrrolo[1,2-c]imidazol-7-one dihydrochloride as a brown solid. From this compound, the title compound (300 mg, 34%) was obtained as a white solid in a similar manner to Example 43c).
NMR (300 MHz, CDCl₃) δ: 1.17-1.55 (3H, m), 1.78-1.97 (1H, m), 2.29-2.55 (2H, m), 2.73-3.03 (3H, m), 3.41-3.57 (3H, m), 3.88 (1H, bs), 4.53 (1H, bs), 4.71 (1H, bs), 4.95 (1H, bs), 7.58 (1H, dd), 7.86-7.99 (5H, m), 8.47 (1H, s), 9.66 (1H, s).
Elemental analysis for C₂₄H₂₄N₃O₄SCl·HCl·0.5H₂O
Calculated (%): C, 54.24; H, 4.93; N, 7.91
Found (%): C, 54.37; H, 4.93; N, 7.86

### Example 47

### 6-(1-{3-[(6-Chloro-2-naphthyl)sulfonyl]propanoyl}-4-hydroxy-4-piperidinyl)-1-methyl-5,6-dihydro-7H-pyrrolo[1,2-c]imidazol-7-one

### 47a) 1-Methyl-5,6-dihydro-7H-pyrrolo[1,2-c]imidazol-7-one

The title compound was synthesized from 4-methyl-1H-imidazole-5-carbaldehyde according to a method described in the article (C. Christine et al. Tetrahedron, 56, 1837 (2000)) (yield 8.5%).
NMR (300 MHz, CDCl₃) δ: 2.44 (3H, s), 3.19 (2H, t), 4.31 (2H, t), 7.58 (1H, s).

### 47b) Tert-butyl 4-hydroxy-4-(1-methyl-7-oxo-6,7-dihydoro-5H-pyrrolo[1,2-c]imdazol-6-yl)piperidine-1-carboxylate

From 1-methyl-5,6-dihydro-7H-pyrrolo[1,2-c]imidazol-7-one (500 mg) obtained in Example 47a), the title compound (790 mg, 64%) was obtained as a white solid in a similar manner to Example 46a).
NMR (300 MHz, CDCl₃) 5: 1.45 (9H, s), 1.48-1.76 (4H, m), 3.15-3.21 (2H, m), 3.82-3.95 (2H, m), 4.09 (1H, dd), 4.39 (1H, dd), 7.60 (1H, s).

### 47c) 6-(1-{3-[(6-Chloro-2-naphthyl)sulfonyl]propanoyl}-4-hydroxy-4-piperidinyl)-1-methyl-5,6-dihydro-7H-pyrrolo[1,2-c]imidazol-7-one

From tert-butyl 4-hydroxy-4-(1-methyl-7-oxo-6,7-dihydoro-5H-pyrrolo[1,2-c]iimdazol-6-yl)piperidine-1-carboxylate obtained in Example 47b), the title compound (170 mg, 50%) was obtained as a white crystal (ethyl acetate/ethanol/diethyl ether) in a similar manner to Example 38d).
NMR (300 MHz, CDCl₃) δ: 1.46-1.89 (2H, m), 2.44 (3H, s), 2.83-3.06 (3H, m), 3.32-3.73 (6H, m), 3.97-4.12 (2H, m), 4.39 (2H, dd), 7.58-7.61 (2H, m), 7.88-7.98 (4H, m), 8.48 (1H, m).
Elemental analysis for C₂₅H₂₆N₃O₅SCl·0.6H₂O
Calculated (%): C, 57.00; H, 5.20; N, 7.98
Found (%): C, 56.79; H, 5.28; N, 7.83

### Example 48

### 6-(1-{3-[(6-Chloro-2-naphthyl)sulfonyl]propanoyl}-4-piperidinyl)-1-methyl-5,6-dihydro-7H-pyrrolo[1,2-c]imidazol-7-one hydrochloride

From tert-butyl 4-hydroxy-4-(1-methyl-7-oxo-6,7-dihydoro-5H-pyrrolo[1,2-c]iimdazol-6-yl)piperidine-1-carboxylate obtained in Example 47b), the title compound (411 mg, 38%) was obtained as a white solid in a similar manner to Example 46b).
NMR (300 MHz, CDCl₃) δ: 1.31-1.40 (1H, m), 1.51-1.56 (1H, m), 1.80-1.98 (1H, m), 2.28 (1H, bs), 2.50 (1H, t), 2.62 (3H, s), 2.75-3.09 (3H, m), 3.25-3.31 (1H, m), 3.44-3.60 (2H, m), 3.88-3.89 (1H, m), 4.52-4.57 (2H, m), 4.78-4.87 (1H, m), 7.59 (1H, d), 7.88-7.97 (4H, m), 8.47 (1H, s), 9.54-9.58 (1H, m).
Elemental analysis for C₂₅H₂₇N₃O₄SCl₂·1.5H₂O
Calculated (%): C, 53.29; H, 5.37; N, 7.46
Found (%): C, 53.08; H, 5.31; N, 7.65

### Example 49

### 6-(1-{3-[(6-Chloro-2-naphthyl)sulfonyl]propanoyl}-4-piperidinyl)-6,7-dihydro-5H-pyrrolo[1,2-c]imidazole-7-ol

Acetic acid (2 mL) and sodium cyanoborohydride (390 mg) were added successively to a solution of 6-(1-{3-[(6-chloro-2-naphthyl)sulfonyl]propanoyl}-4-piperidinyl)-5,6-dihydro-7H-pyrrolo[1,2-c]imidazol-7-one hydrochloride (200 mg) obtained in Example 46b) in dichloroethane (3 mL), and the mixture was stirred at room temperature for 2 hours. An aqueous saturated sodium hydrogencarbonate solution (30 mL) was added to the reaction solution, and extracted with ethyl acetate (30 mL). An organic layer was separated and dried over anhydrous magnesium sulfate, and the solvent was distilled off under reduced pressure. The residue was purified with a basic silica gel column (ethyl acetate:ethanol = 5:1) and recrystallized from ethyl acetate/ethanol to obtain the title compound (45 mg, 24%) as a white crystal.
NMR (300 MHz, CDCl₃) δ: 1.12-1.38 (2H, m), 1.57-2.20 (5H, m), 2.48-2.60 (1H, m), 2.66-2.68 (1H, m), 2.83-2.89 (1H, m), 2.98-3.18 (1H, m), 3.54-3.64 (2H, m), 3.84-3.85 (1H, m), 4.06-4.24 (1H, m), 4.53-4.57 (1H, m), 4.98-4.99 (1H, m), 6.82 (1H, bs), 7.38 (1H, bs), 7.56-7.60 (1H, m), 7.88-7.95 (4H, m), 8.46 (1H, s).
Elemental analysis for C₂₄H₂₆N₃O₄SCl·0.5H₂O·0.1EtOAc Calculated (%): C, 57.94; H, 5.54; N, 8.31
Found (%): C, 58.04; H, 5.53; N, 8.04

### Example 50

### N-(1-{3-[(6-Chloro-2-naphthyl)sulfonyl]propanoyl}-4-piperidinyl)-lH-imidazol-4-carboxamide

### 50a) 1-{3-[(6-chloro-2-naphthyl)sulfonyl]propanoyl}piperidine-4-amine

From piperidine-4-amine (26 g) and benzaldehyde (27 mL), N-phenylmethylidenepiperidinyl-4-amine (49 g, quantitative) was synthesized according to a method described in the article (Synthetic Communications, 22, 1357-2360(1992)), and was used in the next reaction without purification.

WSC (1.7 g) was added to a solution of 3-[(6-chloro-2-naphthyl)sulfonyl]propionic acid (2.4 g), N-phenylmethylidenepiperidinyl-4-amine (1.5 g) and HOBt (1.3 g) in dichloromethane (50 mL), and the mixture was stirred at room temperature for 16 hours. 1 N HCl (30 mL) was added to the reaction solution, and the mixture was stirred at room temperature for 8 hours. The reaction solution was made alkaline with an aqueous potassium carbonate solution and then extracted with chloroform. The extract was dried over anhydrous magnesium sulfate and the solvent was distilled off. The residue was purified with a basic silica gel column to obtain the title compound (2.0 g, 59%) as an oil.
NMR (200 MHz, CDCl₃) δ: 1.59-1.70 (4H, m), 1.77-1.92 (2H, m), 2.62-2.75 (1H, m), 2.84-3.02 (2H, m), 3.06-3.25 (2H, m), 3.48-3.60 (2H, m), 3.76-3.84 (1H, m), 4.32-4.40 (1H, m), 7.59 (1H, dd, J = 8.8, 1.8), 7.93-7.98 (4H, m), 8.48 (1H, s).

### 50b) N-(1-{3-[(6-Chloro-2-naphthyl)sulfonyl]propanoyl}-4-piperidinyl)-1H-imidazole-4-carboxamide

WSC (0.16 g) was added to a solution of 1-{3-[(6-chloro-2-naphthyl)sulfonyl]propanoyl}piperidine-4-amine (0.29 g) obtained in Example 50a), imidazole-4-carboxylic acid (0.08 g) and HOBt (0.13 g) in dichloromethane (30 mL), and the mixture was stirred at room temperature for 16 hours. The reaction solution was made alkaline with an aqueous potassium carbonate solution and then extracted with chloroform. The extract was dried over anhydrous magnesium sulfate and the solvent was distilled off. The residue was purified with a basic silica gel column to obtain the title compound (47 mg, 13%) as white powder.
NMR (300 MHz, CDCl₃) δ: 1.13-1.45 (2H, m), 1.92-2.08 (2H, m), 2.74 (1H, t, J = 11.7), 2.82-2.89 (2H, m), 3.15 (1H, t, J = 11.7), 3.52-3.57 (2H, m), 3.78-3.82 (1H, m), 4.10-4.13 (1H, m), 4.36-4.41 (1H, m), 7.17-7.20 (1H, br), 7.56-7.59 (3H, m), 7.86-7.94 (4H, m), 8.45 (1H, s).

### Example 51

### 1-{3-[(6-Chloro-2-naphthyl)sulfonyl]propanoyl}-N-[(1H-imidazol-4-yl)methyl]piperidine-4-amine

Sodium triacetoxyborohydride (1.3 g) was added to a solution of 1-{3-[(6-chloro-2-naphthyl)sulfonyl]propanoyl}piperidine-4-amine (2.0 g) obtained in Example 50a), 1-tritylimidazole-4-carbaldehyde (1.8 g) and acetic acid (0.3 mL) in 1,2-dichloroethane (30 mL), and the mixture was stirred at room temperature for 3 hours. The reaction solution was made alkaline with an aqueous potassium carbonate solution and extracted with chloroform. The extract was dried over anhydrous magnesium sulfate. After the solvent was distilled off, the residue was purified with a basic silica gel column to obtain 1-{3-[(6-chloro-2-naphthyl)sulfonyl]propanoyl}-N-[(1-trithylimidazol-4-yl)methyl]piperidine-4-amine (3.0 g, 84%). This intermediate (0.5 g) was dissolved in methanol (10 mL) and 1N hydrochloric acid (5 mL), and then stirred at 90°C for 3 hours. The reaction solution was made alkaline with an aqueous potassium carbonate solution and extracted with chloroform. The extract was dried over anhydrous magnesium sulfate. After the solvent was distilled off, the residue was purified with a basic silica gel column to obtain the title compound (62 mg, 19%) as white powder.
NMR (300 MHz, CDCl₃) δ: 1.15-1.29 (2H, m), 1.79-1.93 (2H, m), 2.62-2.86 (4H, m), 2.97-3.09 (1H, m), 3.48-3.56 (2H, m), 3.63-3.75 (3H, m), 4.24-4.31 (1H, m), 6.85 (1H, s), 7.52-7.57 (2H, m), 7.87-7.93 (4H, m), 8.43(1H, s).

### Example 52

### 1-{3-[(6-Chloro-2-naphthyl)sulfonyl]propanoyl}-N-[(2-methyl-1H-imidazol-4-yl)methyl]piperidine-4-amine

### 52a) 1-{3-[(6-chloro-2-naphthyl)sulfonyl]propanoyl}-N-[(2-methyl-1-trithylimidazol-4-yl)methyl]piperidine-4-amine

From 1-{3-[(6-chloro-2-naphthyl)sulfonyl]propanoyl}-4-piperidineamine (4.0 g) obtained in Example 50a) and 2-methyl-1-trithylimidazole-4-carbaldehyde (3.7 g), the title compound (1.9 g, 24%) was prepared as white powder by reductive amination according to a similar manner to Example 51.
NMR (200 MHz, CDCl₃) δ: 1.21-1.28(2H, m), 1.61(3H, s), 1.81-2.03(2H, m), 2.61-2.88(4H, m), 2.98-3.11(1H, m), 3.47-3.57(2H, m), 3.63(2H, s), 3.75-3.81(1H, m), 4.28-4.34(1H, m), 6.57(1H, s), 7.10-7.31(15H, m), 7.33-7.60(1H, m), 7.91-7.96(4H, m), 8.46(1H, s).

### 52b) 1-{3-[(6-Chloro-2-naphthyl)sulfonyl]propanoyl}-N-[(2-methyl-1H-imidazol-4-yl)methyl]piperidine-4-amine

From 1-{3-[(6-chloro-2-naphthyl)sulfonyl]propanoyl}-N-[(2-methyl-1-tritylimidazol-4-yl)methyl]piperidine-4-amine (0.4 g) obtained in Example 52a), the title compound (0.23 g, 87%) was prepared as white powder by detritylation according to a similar manner to Example 51.
NMR (200 MHz, CDCl₃) δ: 1.19-1.31 (2H, m), 1.83-1.96 (2H, m), 2.37 (3H, s), 2.68-2.91 (4H, m), 3.00-3.13 (1H, m), 3.51-3.60 (2H, m), 3.72-3.82 (3H, m), 4.28-4.34 (1H, m), 6.74 (1H, s), 7.61 (1H, dd, J = 8.2, 1.8), 7.87-7.97(4H, m), 8.47(1H, m).

### Example 53

### 1-{3-[(6-Chloro-2-naphthyl)sulfonyl]propanoyl}-N-[(4-methyl-1H-imidazol-5-yl)methyl]piperidine-4-amine

### 53a) 1-{3-[(6-Chloro-2-naphthyl)sulfonyl]propanoyl}-N-[(4-methyl-1-tritylimidazol-5-yl)methyl]piperidine-4-amine

From 1-{3-[(6-chloro-2-naphthyl)sulfonyl]propanoyl}piperidine-4-amine (2.0 g) obtained in Example 50a) and 4-methyl-1-tritylimidazole-5-carbaldehyde (1.9 g), the title compound (2.2 g, 59%) was prepared as white powder by reductive amination according to a similar manner to Example 51.
NMR (200 MHz, CDCl₃) δ: 1.21-1.29(2H, m), 1.39(3H, s), 1.78-1.98(2H, m), 2.66-2.89(4H, m), 2.97-3.14(1H, m), 3.49-3.57(2H, m), 3.65(2H, s), 3.71-3.80(1H, m), 4.26-4.32(1H, m), 7.11-7.32(16H, m), 7.54-7.59(1H, m), 7.91-7.95(4H, m), 8.46(1H, s).

### 53b) 1-{3-[(6-Chloro-2-naphthyl)sulfonyl]propanoyl}-N-[(4-methyl-1H-imidazol-5-yl)methyl]piperidine-4-amine

From 1-{3-[(6-chloro-2-naphthyl)sulfonyl]propanoyl}-N-[(4-methyl-1-tritylimidazol-5-yl)methyl]piperidine-4-amine (0.7 g) obtained in Example 53a), the title compound (0.20 g, 76%) was prepared as white powder by detritylation according to a similar manner to Example 51.
NMR (200 MHz, CDCl₃) δ: 1.20-1.30 (2H, m), 1.78-1.98 (2H, m), 2.15 (3H, s), 2.65-2.87 (4H, m), 2.95-3.12 (1H, m), 3.45-3.58 (2H, m), 3.67-3.78 (3H, m), 4.23-4.30 (1H, m), 5.01-5.68 (1H, br), 7.36-7.41 (1H, m), 7.54 (1H, dd), 7.88-7.93 (4H, m), 8.44(1H, s).

### Example 54

### 1-{3-[(6-Chloro-2-naphthyl)sulfonyl]propanoyl}-N-[(1H-imidazol-2-yl)methyl]piperidine-4-amine

From 1-{3-[(6-chloro-2-naphthyl)sulfonyl]propanoyl}piperidine-4-amine (0.5 g) obtained in Example 50a) and imidazole-2-carbaldehyde (0.13 g), the title compound (0.54 g, 90%) was prepared as white powder by reductive amination according to a similar manner to Example 51.
NMR (200 MHz, CDCl₃) δ: 1.02-1.48 (2H, m), 1.80-2.03 (2H, m), 2.69-3.06 (5H, m), 3.53-3.60 (2H, m), 3.75-3.96 (3H, m), 4.29-4.43 (1H, m), 7.02 (1H, s), 7.04 (1H, s), 7.51 (1H, dd), 7.93-7.98 (4H, m), 8.49(1H, s).

### Example 55

### N-(1-{3-[(6-Chloro-2-naphthyl)sulfonyl]propanoyl}-4-piperidinyl)-N-(1H-imidazol-4-yl)methylacetamide

Acetyl chloride (0.068 mL) was added to a solution of 1-{3-[(6-chloro-2-naphthyl)sulfonyl]propanoyl}-N-[(1-tritylimidazol-4-yl)methyl]piperidine-4-amine (0.6 g) obtained in Example 51 and triethylamine (0.24 mL) in dichloromethane (30 mL) under ice-cooling, and the mixture was stirred at room temperature for 16 hours. After the solvent was distilled off, trifluoroacetic acid (5 mL) was added to the residue and the reaction solution was stirred at room temperature for 1 hour. After the solvent was distilled off, the residue was made alkaline with an aqueous potassium carbonate solution and then extracted with chloroform. The extract was dried over anhydrous magnesium sulfate and the solvent was distilled off. The residue was purified with a basic silica gel column to obtain the title compound (68 mg, 16%) as pale yellow powder.
NMR (200 MHz, CDCl₃) δ: 1.52-1.84 (4H, m), 2.15 (3H, d), 2.44-2.60 (1H, m), 2.79-3.10 (3H, m), 3.49-3.56 (2H, m), 3.58-4.07 (2H, m), 4.32-4.37 (2H, m), 4.49-4.63 (1H, m), 6.79-6.89 (1H, m), 7.47-7.59 (2H, m), 7.89-7.95 (4H, m), 8.43-8.46 (1H, m).

### Example 56

### N-(1-{3-[(6-Chloro-2-naphthyl)sulfonyl]propanoyl}-4-piperidinyl)-N-[(1H-imidazol-4-yl)methyl]methanesulfonamide

Methanesulfonyl chloride (0.074 mL) was added to a solution of 1-{3-[(6-chloro-2-naphthyl)sulfonyl]propanoyl}-N-[(1-tritylimidazol-4-yl)methyl]piperidine-4-amine (0.6 g) obtained in Example 51 and triethylamine (0.24 mL) in dichloromethane (30 mL) under ice-cooling, and the mixture was stirred at room temperature for 16 hours. After the solvent was distilled off, trifluoroacetic acid (5 mL) was added to the residue and the reaction solution was stirred at room temperature for 1 hour. After the solvent was distilled off, the residue was made alkaline with an aqueous potassium carbonate solution and then extracted with chloroform. The extract was dried over anhydrous magnesium sulfate and the solvent was distilled off. The residue was purified with a basic silica gel column to obtain the title compound (90 mg, 19%) to obtain pale yellow powder.
NMR (200 MHz, CDCl₃) δ: 1.58-1.87 (4H, m), 2.17 (3H, s), 2.30-2.48 (1H, m), 2.64-3.09 (3H, m), 3.47-3.63 (2H, m), 3.72-3.94 (2H, m), 4.21-4.39 (2H, m), 4.51-4.57 (1H, m), 6.97 (1H, s), 7.51-7.55 (2H, m), 7.82-7.92 (4H, m), 8.42 (1H, s).

### Example 57

### 1-{3-[(6-Chloro-2-naphthyl)sulfonyl]propanoyl}-N-ethyl-N-[(2-methyl-1H-imidazol-4-yl)methyl]piperidine-4-amine

From 1-{3-[(6-chloro-2-naphthyl)sulfonyl]propanoyl}-N-[(2-methyl-1-tritylimidazol-4-yl)methyl]piperidine-4-amine (0.5 g) obtained in Example 52a) and acetoaldehyde (0.05 mL), the title compound (0.23 g, 65%) was obtained as pale yellow powder in a similar manner to Example 51.
NMR (200 MHz, CDCl₃) δ: 1.03 (3H, t), 1.32-1.46 (2H, m), 1.72-1.86 (2H, m), 2.38 (3H, s), 2.46-2.58 (3H, m), 2.69-3.02 (4H, m), 3.52-3.60 (4H, m), 3.81-3.88 (1H, m), 4.49-4.56 (1H, m), 6.72 (1H, s), 7.29(1H, d), 7.58 (1H, dd), 7.92-7.97 (4H, m), 8.47 (1H, s).

### Example 58

### N-(1-{3-[(6-Chloro-2-naphthyl)sulfonyl]propanoyl}-4-piperidinyl)-N-[(2-methyl-1H-imidazol-4-yl)methyl]acetaimde

From 1-{3-[(6-chloro-2-naphthyl)sulfonyl]propanoyl}-N-[(2-methyl-1-tritylimidazol-4-yl)methyl]piperidine-4-amine (0.5 g) obtained in Example 52a) and acetyl chloride (0.06 mL), the title compound (0.11 g, 31%) was obtained as pale yellow powder in a similar manner to Example 55.
NMR (200 MHz, CDCl₃) δ: 1.25-1.77 (4H, m), 2.16 (3H, s), 2.33 (3H, s), 2.41-2.48 (1H, m), 2.58-3.05 (3H, m), 3.49-3.58 (2H, m), 3.79-4.03 (1H, m), 4.27-4.32 (2H, m), 4.56-4.67 (2H, m), 6.68(1H, d), 7.54-7.60 (1H, m), 7.89-7.97 (4H, m), 8.44-8.48 (1H, m).

### Example 59

### 7-(1-{3-[(6-Chloro-2-naphthyl)sulfonyl]propanoyl}-4-piperidinyl)-3-methyl-7,8-dihydroimidazo[1,5-a]pyrazin-6(5H)-one

From 1-{3-[(6-chloro-2-naphthyl)sulfonyl]propanoyl}-N-[(2-methyl-1-tritylimidazol-4-yl)methyl]piperidine-4-amine (0.5 g) obtained in Example 52a) and chloroacetyl chloride (0.1 mL), the title compound (0.08 g, 24%) was obtained as white crystals in a similar manner to Example 55.
NMR (200 MHz, CDCl₃) δ: 1.60-1.80 (4H, m), 2.38(3H, s), 2.63-2.72 (1H, m), 2.86-2.99 (2H, m), 3.10-3.29 (1H, m), 3.50-3.64 (2H, m), 3.97-4.03 (1H, m), 4.42 (2H, s), 4.55 (2H, s), 4.69-4.82 (2H, m), 6.80 (1H, s), 7.62 (1H, dd), 7.90-7.99 (4H, m), 8.50 (1H, s).

### Example 60

### N-(1-{3-[(6-Chloro-2-naphthyl)sulfonyl]propanoyl}-4-piperidinyl)-N-[(4-methyl-1H-imidazol-5-yl)methyl]acetamide

From 1-{3-[(6-chloro-2-naphthyl)sulfonyl]propanoyl}-N-[(4-methyl-1-tritylimidazol-5-yl)methyl]piperidine-4-amine (0.5 g) obtained in Example 53a) and acetyl chloride (0.06 mL), the title compound (0.10 g, 28%) was obtained as pale yellow powder in a similar manner to Example 55.
NMR (200 MHz, CDCl₃) δ: 1.60-1.78 (4H, m), 2.18 (3H, s), 2.19 (3H, s), 2.49-2.55 (1H, m), 2.78-3.08 (3H, m), 3.48-3.62 (2H, m), 3.65-4.05 (1H, m), 4.24-4.39 (2H, m), 4.48-4.65 (2H, m), 7.34-7.43 (1H, m), 7.57 (1H, dd), 7.91-7.97 (4H, m), 8.45-8.47 (1H, m).

### Example 61

### 1-{3-[(6-Chloro-2-naphthyl)sulfonyl]propanoyl}-N-ethyl-N-[(4-methyl-1H-imidazol-5-yl)methyl]piperidine-4-amine

From 1-{3-[(6-chloro-2-naphthyl)sulfonyl]propanoyl}-N-[(4-methyl-1-tritylimidazol-5-yl)methyl]piperidine-4-amine (0.5 g) obtained in Example 53a) and acetoaldehyde (0.05 mL), the title compound (0.14 g, 40%) was obtained as pale yellow powder in a similar manner to Example 51.
NMR (200 MHz, CDCl₃) δ: 0.99 (3H, t), 1.32-1.47 (2H, m), 1.71-1.84 (2H, m), 2.18 (3H, s), 2.38-2.55 (3H, m), 2.70-2.94 (4H, m), 3.52-3.58 (4H, m), 3.82-3.88 (1H, m), 4.49-4.56 (1H, m), 7.55 (1H, s), 7.57 (1H, dd), 7.92-7.96 (4H, m), 8.47 (1H, s).

### Example 62

### 7-(1-{3-[(6-Chloro-2-naphthyl)sulfonyl]propanoyl}-4-piperidinyl)-7,8-dihydroimidazo[1,2-a]pyrazin-6(5H)-one

From 1-(3{[(6-chloro-2-naphthyl)sulfonyl]propanoyl}-N-[(1H-imidazol-2-yl)methyl]piperidine-4-amine (0.5 g) obtained in Example 54 and chloroacetyl chloride (0.1 mL), the title compound(0.03 g, 5%) was obtained as colorless powder in a similar manner to Example 55.
NMR (200 MHz, CDCl₃) δ: 1.61-1.81 (4H, m), 2.56-2.72 (1H, m), 2.87-2.97 (2H, m), 3.11-3.28 (1H, m), 3.54-3.62 (2H, m), 3.97-4.09 (1H, m), 4.49 (2H, s), 4.69-4.78 (4H, m), 6.89 (1H, s), 7.11 (1H, s), 7.60 (1H, dd), 7.95-7.99 (4H, m), 8.49 (1H, s).

### Example 63

### 7-(1-{3-[(6-Chloro-2-naphthyl)sulfonyl]propanoyl}-4-piperidinyl)-3-methyl-5,6,7,8-tetrahydroimidazo[1,5-a]pyrazine

From tert-butyl 4-aminopiperidine-1-carboxylate (2.0 g) and 2-methyl-1-tritylimidazole-4-carbaldehyde (3.5 g), tert-butyl 4-[(2-methyl-1-tritylimidazol-4-yl)methyl]aminopiperidine-1-carboxylate (4.0 g, 75%) was obtained in a similar manner to Example 51. Then, from this compound and chloroacetyl chloride (1.1 mL), tert-butyl 4-(3-methyl-6-oxo-5,6-dihydroimidazo[1,5-a]pyrazin-7(8H)-yl)piperidine-l-carboxylate (2.0 g, 80%) was obtained in a similar manner to Example 59. This compound was dissolved in THF (20 mL) and a 1 M solution of borane/THF complex in THF (18 mL) was added. The mixture was heated to reflux overnight. After 1N hydrochloric acid (20 mL) was added, the mixture was stirred and then concentrated. The residue was dissolved in trifluoroacetic acid (15 mL), de-tert-butoxycarbonylated, and then condensed with 3-[(6-chloro-2-naphthyl)sulfonyl]propionic acid (1.7 g) to obtain the title compound(0.04 g, 1.4%) as pale yellow powder in a similar manner to Example 50b).
NMR (200 MHz, CDCl₃) δ: 1.17-1.48 (2H, m), 1.80-1.94 (2H, m), 2.27 (3H, s), 2.58-2.63 (2H, m), 2.81-2.89 (4H, m), 2.98-3.12 (1H, m), 3.42-3.56 (2H, m), 3.70-3.81 (5H, m), 4.43-4.50 (1H, m), 6.59 (1H, s), 7.54 (1H, dd), 7.89-7.93 (4H, m), 8.44 (1H, s).

### Example 64

### 7-(1-{3-[(6-Chloro-2-naphthyl)sulfonyl]propanoyl}-4-piperidinyl)-1,5-dimethyl-7,8-dihydroimidazo[1,5-a]pyrazin-6(5H)-one

From 1-{3-[(6-chloro-2-naphthyl)sulfonyl]propanoyl}-N-[(4-methyl-1-tritylimidazol-5-yl)methyl]piperidine-4-amine (1.5 g) obtained in Example 53a) and 2-chloropropionyl chloride (0.24 mL), the title compound (0.66 g, 62%) was obtained as white crystals in a similar manner to Example 59.
NMR (200 MHz, CDCl₃) δ: 1.16 (3H, d), 1.69-1.76 (4H, m), 2.20 (3H, s), 2.48-2.78 (1H, m), 2.93-3.02 (2H, m), 3.11-3.30 (1H, m), 3.53-3.73 (2H, m), 3.98-4.05 (1H, m), 4.33 (2H, s), 4.72-4.78 (3H, m), 7.46 (1H, s), 7.62 (1H, dd), 7.96-7.99 (4H, m), 8.51 (1H, s).

### Example 65

### 2-(1-{3-[(6-Chloro-2-naphthyl)sulfonyl]propanoyl}-4-piperidinyl)-7-methyl-1,2-dihydro-3H-imidazo[1,5-c]imidazol-3-one

1-{3-[(6-chloro-2-naphthyl)sulfonyl]propanoyl}-N-[(4-methyl-1-tritylimidazol-5-yl)methyl]piperidine-4-amine (1.5 g) obtained in Example 53a) was dissolved in 1N hydrochloric acid (20 mL), and the solution was stirred at 70°C for 4 hours. After cooled, the reaction solution was made alkaline with an aqueous potassium carbonate solution and then extracted with chloroform. The extract was dried over anhydrous magnesium sulfate and the solvent was distilled off. The residue was dissolved in dichloromethane (20 mL), and DBU (0.16 mL) and carbonyldiimidazole (0.19 g) were added. The reaction solution was stirred overnight and poured into an aqueous potassium carbonate solution. The reaction solution was extracted with chloroform and the extract was dried over anhydrous magnesium sulfate. After the solvent was distilled off, the residue was purified with a silica gel column to obtain the title compound (83 mg, 7%) as a white crystal.
NMR (200 MHz, CDCl₃) δ: 1.64-1.88 (2H, m), 1.96-2.04 (2H, m), 2.24 (3H, s), 2.60-2.73 (1H, m), 2.87-3.01 (2H, m), 3.15-3.27 (1H, m), 3.46-3.73 (2H, m), 3.99-4.33 (2H, m), 4.28 (2H, s), 4.71-4.78 (1H, m), 7.62 (1H, dd), 7.87-8.00 (4H, m), 8.51 (1H, s).

### Example 66

### 7-(1-{3-[(6-Chloro-2-naphthyl)sulfonyl]propanoyl}-4-piperidinyl)-1-methyl-7,8-dihydroimidazo[1,5-a]pyrazin-6(5H)-one

From 1-{3-[(6-chloro-2-naphthyl)sulfonyl]propanoyl}-N-[(4-methyl-1-tritylimidazol-5-yl)methyl]piperidine-4-amine (0.5 g) obtained in Example 53a) and acetyl chloride (0.1 mL), the title compound (0.11 g, 30%) was obtained as white crystals in a similar manner to Example 59.
NMR (200 MHz, CDCl₃) δ: 1.67-1.81 (4H, m), 2.19 (3H, s), 2.59-2.72 (1H, m), 2.87-3.02 (2H, m), 3.08-3.32 (1H, m), 3.50-3.65 (2H, m), 3.99-4.08 (1H, m), 4.19 (2H, s), 4.67 (2H, s), 4.71-4.82 (2H, m), 7.44 (1H, s), 7.62 (1H, dd), 7.90-7.99 (4H, m), 8.50 (1H, s).

### Example 67

### 7-(1-{3-[(6-Chloro-2-naphthyl)sulfonyl]propanoyl}-4-piperidinyl)-1-methyl-5,6,7,8-tetrahydroimidazo[1,5-a]pyrazine

From tert-butyl 4-aminopiperidine-1-carboxylate (2.0 g) and 4-methyl-1-tritylimidazole-5-carbaldehyde (3.5 g), tert-butyl 4-[(4-methyl-1-tritylimidazol-5-yl)methyl]aminopiperidine-1-carboxylate (4.5 g, 84%) was obtained in a similar manner to Example 51. Then, from this compound (3.0 g) and bromoacetyl chloride (1.4 mL), tert-butyl 4-(1-methyl-6-oxo-5,6-dihydroimidazo[1,5-a]pyrazin-7(8H)-yl)piperidine-1-carboxylate (2.0 g, 67%) was obtained in a similar manner to Example 59. This compound was dissolved in THF (20 mL) and a 1 M solution of borane/THF complex in THF (18 mL) was added. The mixture was heated to reflux overnight. After 1N hydrochloric acid (20 mL) was added, the reaction solution was stirred and then concentrated. The residue was dissolved in trifluoroacetic acid (15 mL), de-tert-butoxycarbonylated, and then condensed with 3-[(6-chloro-2-naphthyl)sulfonyl]propionic acid (1.7 g) to obtain the title compound (0.12 g, 4%) as pale yellow powder in a similar manner to Example 50b).
NMR (200 MHz, CDCl₃) δ: 1.39-1.57 (2H, m), 1.85-1.99 (2H, m), 2.11 (3H, s), 2.56-2.94 (6H, m), 3.02-3.14 (1H, m), 3.53-3.60 (2H, m), 3.69 (2H, s), 3.71-3.82 (1H, m), 3.89-4.00 (2H, m), 4.50-4.57 (1H, m), 7.30 (1H, d), 7.58 (1H, dd), 7. 93-7. 97 (4H, m), 8.48 (1H, s).

### Example 68

### 2-(1-{3-[(6-Chloro-2-naphthyl)sulfonyl]propanoyl}-4-piperidinyl)-5-methyl-1,2-dihydro-3H-imidazo[1,5-c]imidazol-3-one

WSC (1.9 g) was added to a solution of 5-methyl-2-(4-piperidinyl)-1,2-dihydro-3H-imidazo[1,5-c]imidazol-3-one (2.0 g) obtained in Example 69b), 3-[(6-chloro-2-naphthyl)sulfonyl]propionic acid (2.7 g), HOBt (1.5 g) and triethylamine (1.27 mL) in dichloromethane (50 mL) at 0°C, and the mixture was stirred at room temperature overnight. The reaction solution was washed with an aqueous potassium carbonate solution and dried over anhydrous magnesium sulfate. The solvent was distilled off and the residue was purified with a silica gel column. The product was recrystallized from ethanol to obtain the title compound (2.36 g, 52%) as a white crystal (melting point 195°C).
NMR (200 MHz, CDC1₃) δ: 1.58-1.72 (2H, m), 1.86-1.99 (2H, m), 2.61 (3H, s), 2.63-2.68 (1H, m), 2.81-3.01 (2H, m), 3.14-3.23 (1H, m), 3.46-3.65 (2H, m), 3.97-4.02 (1H, m), 4.13-4.22 (1H, m), 4.25 (2H, s), 4.69-4.74 (1H, m), 6.70 (1H, d), 7.59 (1H, dd), 7.89-7.96 (4H, m), 8.47 (1H, d). Elemental analysis for C₂₄H₂₅N₄O₄SCl
Calculated (%): C, 57.54; H, 5.03; N, 11.18
Found (%): C, 57.28; H, 5.03; N, 10.91

### Example 69

### 2-(1-{3-[(6-Chloro-2-naphthyl)sulfonyl]propanoyl}-4-piperidinyl)-5-methyl-1,2-dihydro-3H-imidazo[1,5-c]imidazol-3-one

### 69a) 2-(1-Benzyl-4-piperidinyl)-5-methyl-1,2-dihydro-3H-imidazo[1,5-c]imidazol-3-one

Under ice-cooling, sodium triacetoxyborohydride (31.8 g) was added to a solution of 2-methylimidazole-4-carbaldehyde (11.0 g), 1-benzylpiperidine-4-amine (19.0 g) and acetic acid (6.7 mL) in 1,2-dichloroethane (200 mL), and the mixture was stirred at room temperature overnight. The reaction solution was washed with an aqueous potassium carbonate solution and dried over anhydrous magnesium sulfate. After the solvent was distilled off, the residue was dissolved in THF (200 mL), and N, N'-carbonylimidazole (17.8 g) and DBU (16.7 g) were added. The mixture was stirred at room temperature for 15 hours. The reaction solution was concentrated and water was added. After extraction with ethyl acetate, the extract was dried over anhydrous magnesium sulfate and the solvent was distilled off. The residue was purified with a silica gel column to obtain the title compound (18.5 g, 60%).
NMR (200 MHz, CDCl₃) δ: 1.74-1.85 (4H, m), 2.07-2.20 (2H, m), 2.61 (3H, s, Me), 2.97-3.03 (2H, m), 3.53 (2H, s), 3.89-4.06 (1H, m), 4.30 (2H, s), 6.70 (1H, s), 7.32 (5H, m).

### 69b) 5-Methyl-2-(4-piperidinyl)-1,2-dihydro-3H-imidazo[1,5-c]imidazol-3-one

2-(1-Benzyl-4-piperidinyl)-5-methyl-1,2-dihydro-3H-imidazo[1,5-c]imidazol-3-one (18.2 g) obtained in Example 69a) and 10% Pd/C (50% hydrous :1.5 g) were added to methanol (300 mL), and the mixture was stirred for 2.5 days under hydrogen atmosphere. The catalyst was filtered off and the filtrate was concentrated. The residue was recrystallized from ethyl acetate-hexane to obtain the title compound (10.7 g, 83%).
NMR (200 MHz, CDCl₃) δ: 1.56-1.89 (4H, m), 2.62 (3H, s, Me), 2.75 (2H, dt), 3.17-3.23 (2H, m), 3.97-4.13 (1H, m), 4.32 (2H, s), 6.71 (1H, s).

### 69c) 2-(1-{3-[(6-Chloro-2-naphthyl)sulfonyl]propanoyl}-4-piperidinyl)-5-methyl-1,2-dihydro-3H-imidazo[1,5-c]imidazol-3-one

Under ice-cooling, a solution of 3-[(6-chloro-2-naphthyl)sulfonyl]propanoyl chloride (14.7 g) obtained in Example 71d) in THF (100 mL) was added dropwise to a solution of 5-methyl-2-(4-piperidinyl)-1,2-dihydro-3H-imidazo[1,5-c]imidazol-3-one (8.8 g) obtained in Example 69b) and triethylamine (6.7 mL) in THF (150 mL). The reaction solution was stirred at 0°C for 5 hours and the solvent was then distilled off. The residue was diluted with water and extracted with ethyl acetate-THF. The extract was washed with water and then dried over anhydrous magnesium sulfate. After the solvent was distilled off, the residue was purified with a silica gel column and the product was recrystallized from ethyl acetate-methanol to obtain the title compound (11.2 g, 51%) as colorless crystals.
Elemental analysis for C₂₄H₂₅N₄O₄SCl
Calculated (%): C, 57.54; H, 5.03; N, 11.18
Found (%): C, 57.42; H, 5.13; N, 10.99

### Example 70

### 2-(1-{3-[(6-Chloro-2-naphthyl)sulfonyl]propanoyl}-4-piperidinyl)-5-methyl-1,2-dihydro-3H-imidazo[1,5-c]imidazol-3-one hydrochloride

A 1N solution of hydrogen chloride in ether (4 mL) was added to a solution of 2-(1-{3-[(6-chloro-2-naphthyl)sulfonyl]propanoyl}-4-piperidinyl)-5-methyl-1,2-dihydro-3H-imidazo[1,5-c]imidazol-3-one (0.50 g) obtained in Example 69c) in methanol (20 mL) and the solvent was then distilled off. Acetone and ether were added to the residue. A precipitated solid was filtered to obtain the title compound (0.51 g, 86%) as colorless powder.
NMR (200 MHz, DMSO-d₆) δ: 1.40-1.88 (4H, m), 2.54-2.65 (1H, m), 2.73-2.79(5H, m), 3.05-3.16 (1H, m), 3.63 (2H, t), 3.88-4.15 (2H, m), 4.23-4.39 (1H, m), 4.55 (2H, s), 7.49 (1H, s), 7.74 (1H, dd), 8.00 (1H, dd), 8.17-8.31 (3H, m), 8.66 (1H, s).
Elemental analysis for C₂₄H₂₅N₄O₄SCl·HCl·1.5H₂O·0.4Et₂O Calculated (%): C, 51.75; H, 5.60; N, 9.43
Found (%): C, 51.95; H, 5.56; N, 9.30

### Example 71

### 2-(1-{3-[(6-Chloro-2-naphthyl)sulfonyl]propanoyl}-4-piperidinyl)-1,7-dimethyl-1,2-dihydro-3H-imidazo[1,5-c]imidazol-3-one

### 71a) Benzyl 4-[(4-methylimidazol-5-yl)methylamino]piperidine-1-carboxylate

Benzyl 4-aminopiperidine-1-carboxylate (10 g), 4-methylimidazole-5-carbaldehyde (4.7 g) and acetic acid (3.0 mL) were dissolved in 1,2-dichloroethane (100 mL), sodium triacetoxyborohydride (13.6 g) was added under ice-cooling, and the mixture was stirred at room temperature overnight. The reaction solution was poured into an aqueous potassium carbonate solution and extracted with chloroform. The extract was dried over anhydrous magnesium sulfate. After the solvent was distilled off, the residue was purified with a silica gel column to obtain the title compound (8.0 g, 57%) as an oil.
NMR (200 MHz, CDCl₃) δ: 1.23-1.38 (2H, m), 1.85-1.95 (2H, m), 2.19 (3H, s), 2.64-2.74 (1H, m), 2.83-2.95 (2H, m), 3.72 (2H, s), 4.07-4.18 (2H, m), 5.12 (2H, s), 7.28-7.37 (5H, m), 7.44 (1H, s).

### 71b) Benzyl 4-(7-methyl-3-oxo-1,2-dihydro-3H-imidazo[1,5-c]imidazol-2-yl)piperidine-1-carboxylate

Benzyl 4-[(4-methylimidazol-5-yl)methylamino]piperidine-1-carboxylate (7.0 g) obtained in Example 71a) was dissolved in dichloromethane (70 mL), and DBU (3.4 mL) and N,N'-carbonyldiimidazole (3.5 g) were added. The reaction solution was stirred overnight, poured into an aqueous potassium carbonate solution and extracted with chloroform. The extract was dried over anhydrous magnesium sulfate and the solvent was distilled off. The residue was purified with a silica gel column to obtain the title compound (5.1 g, 69%) as white crystals.
NMR (200 MHz, CDCl₃) δ: 1.63-1.76 (2H, m), 1.84-1.90 (2H, m), 2.22 (3H, s), 2.85-2.98 (2H, m), 4.09-4.36 (3H, m), 4.27 (2H, s), 5.15 (2H, s), 7.28-7.37 (5H, m), 7.85(1H, s).

### 71c) Benzyl 4-(1,7-dimethyl-3-oxo-1,2-dihydro-3H-imidazo[1,5-c]imidazol-2-yl)piperidin-1-carboxylate

Benzyl 4-(7-methyl-3-oxo-1,2-dihydro-3H-imidazo[1,5-c]imidazol-2-yl)piperidin-1-carboxylate (1.5 g) obtained in Example 71b) was dissolved in THF (40 mL) and a 1.1 M solution of lithium hexamethyldisilazane in THF (4.2 mL) was added dropwise at -78°C. The mixture was stirred at -78°C for 30 minutes. Then, methyl iodide (0.31 mL) was added at -78°C and the mixture was stirred for 30 minutes. An aqueous ammonium chloride solution was added to the reaction solution, which was then extracted with ethyl acetate. The extract was dried over anhydrous magnesium sulfate and the solvent was distilled off. The residue was purified with a silica gel column to obtain the title compound (1.1 g, 71%) as an oil.
NMR (200 MHz, CDCl) δ: 1.54 (3H, t), 1.58-2.08 (4H, m), 2.22 (3H, s), 2.77-2.98 (2H, m), 3.75-3.92 (1H, m), 4.26-4.46 (2H, m), 4.65 (1H, q), 5.15 (2H, s), 7.37-7.40 (5H, m), 7.76 (1H, s).

### 71d) 3-[(6-Chloro-2-naphthyl)sulfonyl]propanoyl chloride

3-[(6-Chloro-2-naphthyl)sulfonyl]propionic acid (14.9 g), thionyl chloride (4.4 mL) and DMF (2 drops) were suspended in toluene (100 mL), and the suspension was heated to reflux for 1.5 hours. The solvent was distilled off and the residue was washed with ether and hexane to obtain the title compound (15.5 g, 98%) as a brown solid. NMR (200 MHz, CDCl₃) δ: 3.35-3.44 (2H, m), 3.49-3.57 (2H, m), 7.62 (1H, dd), 7.87-8.00 (4H, m), 8.48 (1H, s).

### 71e) 2-(1-{3-[(6-chloro-2-naphthyl)sulfonyl]propanoyl}-4-piperidinyl)-1,7-dimethyl-1,2-dihydro-3H-imidazo[1,5-c]imidazol-3-one

Benzyl 4-(1,7-dimethyl-3-oxo-1,2-dihydro-3H-imidazo[1,5-c]imidazol-2-yl)piperidin-1-carboxylate (3.3 g) obtained in Example 71c) was dissolved in ethanol (50 mL) and after addition of 10%Pd/C (50% hydrous: 1.6 g), subjected to catalytic reduction overnight under hydrogen atmosphere. The reaction solution was filtered and the filtrate was concentrated. The residue was dissolved in a mixture of an aqueous sodium hydrogencarbonate solution and chloroform and 3-[(6-chloro-2-naphthyl)sulfonyl]propanoyl chloride (0.6 g) obtained in Example 71d) was added under ice-cooling. The reaction solution was stirred at room temperature for 2 hours and then extracted with chloroform. The extract was dried over anhydrous magnesium sulfate and the solvent was distilled off. The residue was purified with a silica gel column to obtain the title compound (1.2 g, 28%) as pale yellow powder.
NMR (200 MHz, CDCl₃) δ: 1.54 (3H, t), 1.88-2.05 (4H, m), 2.23 (3H, s), 2.53-2.64 (1H, m), 2.87-2.95 (2H, m), 3.08-3.20 (1H, m), 3.54-3.62 (2H, m), 3.81-3.94 (2H, m), 4.62-4.72 (2H, m), 7.60 (1H, dd), 7.79 (1H, s), 7.85-7.98 (4H, m), 8.49 (1H, s).

### Example 72

### 2-(1-{3-[(6-Chloro-2-naphthyl)sulfonyl]propanoyl}-4-piperidinyl)-5,7-dimethyl-1,2-dihydro-3H-imidazo[1,5-c]imidazol-3-one

### 72a) Tert-butyl 4-[(2,4-methylimidazol-5-yl)methylamino]piperidin-1-carboxylate

Tert-butyl 4-aminopiperidine-1-carboxylate (4.8 g), 2,4-dimethylimidazole-5-carbaldehyde (3.0 g) and acetic acid (1.7 ml) were dissolved in 1,2-dichloroethane (50 mL), and sodium triacetoxyborohydride (7.7 g) was added under ice-cooling. The mixture was stirred at room temperature overnight. The reaction solution was poured into an aqueous potassium carbonate solution and extracted with chloroform. The extract was dried over anhydrous magnesium sulfate and the solvent was distilled off. The residue was purified with a silica gel column to obtain the title compound (8.0 g, quantitative) as a pale yellow oil.
NMR (200 MHz, CDCl₃) δ: 1.27-1.40 (2H, m), 1.45 (9H, s), 1.85-1.90 (2H, m), 2.15 (3H, s), 2.31 (3H, s), 2.66-2.80 (3H, m), 3.71 (2H, s), 4.00-4.18 (2H, m), 6.06 (2H, brs).

### 72b) Tert-butyl 4-(5,7-dimethyl-3-oxo-1,2-dihydro-3H-imidazo[1,5-c]imidazol-2-yl)piperidin-1-carboxylate

Tert-butyl 4-[(2,4-dimethylimidazol-5-yl)methylamino]piperidin-1-carboxylate (8.0 g) obtained in Example 72a) was dissolved in dichloromethane (100 mL), and DBU (3.6 mL) and N,N'-carbonyldiimidazole (3.9 g) were added. The reaction solution was stirred overnight, poured into an aqueous potassium carbonate solution, and then extracted with chloroform. The extract was dried over anhydrous magnesium sulfate and the solvent was distilled off. The residue was purified with a silica gel column to obtain the title compound (7.8 g, 97%) as a pale yellow oil. NMR (200 MHz, CDCl₃) δ: 1.47 (9H, s), 1.57-1.72 (2H, m), 1.77-1.92 (2H, m), 2.15 (3H, s), 2.57 (3H, s), 2.77-2.88 (2H, m), 4.03-4.15 (2H, m), 4.20 (2H, s), 4.29 (1H, brs).

### 72c) 2-(1-{3-[(6-Chloro-2-naphthyl)sulfonyl]propanoyl}-4-piperidinyl)-5,7-dimethyl-1,2-dihydro-3H-imidazo[1,5-c]imidazol-3-one

Tert-butyl 4-(5,7-dimethyl-3-oxo-1,2-dihydro-3H-imidazo[1,5-c]imidazol-2-yl)piperidin-1-carboxylate (5.0 g) obtained in Example 72b) was dissolved in concentrated hydrochloric acid (10 ml), and the solution was stirred at room temperature for 30 minutes. The reaction solution was dissolved in chloroform (150 ml) and an aqueous saturated sodium hydrogencarbonate solution (150 ml), and 3-[(6-chloro-2-naphthyl)sulfonyl]propanoyl chloride (4.7 g) was added. The reaction solution was stirred at room temperature for 2 hours and then extracted with chloroform. The extract was dried over anhydrous magnesium sulfate and the solvent was distilled off. The residue was purified with a silica gel column to obtain the title compound (3.8 g, 50%) as a white crystal.
NMR (200 MHz, CDCl₃) δ: 1.62-1.79 (2H, m), 1.83-1.99 (2H, m), 2.15 (3H, s), 2.57 (3H, s), 2.64-2.71 (1H, m), 2.86-2.99 (2H, m), 3.31-3.26 (1H, m), 3.49-3.63 (2H, m), 3.97-4.18 (4H, m), 4.75-4.96 (1H, m), 7.61 (1H, dd), 7.89-7.99 (4H, m), 8.49 (1H, s).

### Example 73

### 1-{3-[(6-Chloro-2-naphthyl)sulfonyl]propanoyl}-4-[2-(1H-imidazol-4-yl)ethyl]piperidine

### 73a) 2-{1-[3-(6-Chloro-2-naphthyl)sulfonyl]propanoyl}-4-piperidinyl)ethanol

3-[(6-Chloro-2-naphthyl)sulfonyl]propanoyl chloride (7.57 g) obtained in Example 71d) was added in portions to a mixture of 2-(4-piperidinyl)ethanol (3.70 g) and sodium hydrogencarbonate (2.03 g) in water (50 mL)-THF (50 mL). The reaction solution was stirred at 0°C for 1 hour and the organic solvent was then distilled off. The residue was extracted with ethyl acetate. The extract was washed with water and dried over anhydrous magnesium sulfate. After the solvent was distilled off, the residue was purified with a silica gel column to obtain the title compound (6.18 g, 63%) as a brown oil.
NMR (200 MHz, CDCl₃) δ: 1.01-1.13 (2H, m), 1.45-1.56 (2H, m), 1.67-1.81 (2H, m), 2.45-2.57 (1H, m), 2.80-2.90 (2H, m), 2.93-3.06 (1H, m), 3.52-3.60 (2H, m), 3.66-3.83 (3H, m), 4.44-4.50 (1H, m), 7.59 (1H, dd), 7.93-7.97 (4H, m), 8.47 (1H, s).

### 73b) 2-(1-{3-[(6-Chloro-2-naphthyl)sulfonyl]propanoyl}-4-piperidinyl)ethyl iodide

Under ice-cooling, methanesulfonyl chloride (1.4 mL) was added to a solution of 2-{1-[3-(6-chloro-2-naphthyl)sulfonyl]propanoyl}-4-piperidinyl)ethanol (6.18 g) obtained in Example 73a) in ethyl acetate (100 mL), and the mixture was stirred for 1.5 hours. The reaction solution was washed with water and then dried over anhydrous magnesium sulfate. After the solvent was distilled off, the residue was dissolved in acetonitrile (100 mL) and sodium iodide (11.3 g) was added. The mixture was stirred at room temperature for 24 hours. The solvent was distilled off and the residue was diluted with water and extracted with ethyl acetate. The extract was washed with water and dried over anhydrous magnesium sulfate. The solvent was distilled off to obtain the residue, which was purified with a silica gel column to obtain the title compound (5.58 g, 71%).
NMR (200 MHz, CDCl₃) δ: 0.90-1.23 (2H, m), 1.60-1.84 (5H, m), 2.46-2.58 (1H, m), 2.82-2.90 (2H, m), 2.95-3.08 (1H, m), 3.20 (2H, t, J = 6.7), 3.51-3.60 (2H, m), 3.79-3.86 (1H, m), 4.46-4.53 (1H, m), 7.60 (1H, dd), 7.92-7.97 (4H, m), 8.48 (1H, s) .

### 73c) 1-{3-[(6-Chloro-2-naphthyl)sulfonyl]propanoyl}-4-[2-(1H-imidazol-4-yl)ethyl]piperidine

Imidazole (0.1 g) and potassium carbonate (0.4 g) were dissolved in DMF (30 mL), and 2-(1-{3-[(6-chloro-2-naphthyl)sulfonyl]propanoyl}-4-piperidinyl)ethyl iodide (0.8 g) obtained in Example 73b) was added under ice-cooling. The reaction solution was stirred at 80°C for 4 hours and the solvent was then concentrated. The residue was poured into water and extracted with chloroform. The extract was dried over anhydrous magnesium sulfate. After the solvent was distilled off, the residue was purified with a silica gel column to obtain the title compound (0.13 g, 19%) as pale yellow powder.
NMR (200 MHz, CDCl₃) δ: 1.01-1.18 (2H, m), 1.29-1.57 (1H, m), 1.64-1.77 (4H, m), 2.39-2.51 (1H, m), 2.80-3.01 (3H, m), 3.51-3.60 (3H, m), 3.77-3.84 (1H, m), 3.94-4.01 (2H, m), 4.44-4.51 (1H, m), 6.90 (1H, s), 7.07 (1H, s), 7.49 (1H, brs), 7.57 (1H, dd), 7.91-7.96 (4H, m), 8.47 (1H, s).

### Example 74

### 5-Chloro-2-{3-oxo-3-[4-(5,6,7,8-tetrahydroimidazo[1,2-a]pyridin-3-yl)-1-piperidinyl]propyl}sulfonyl-1H-benzimidazole

### 74a) Allyl 3-(5-chloro-1H-benzimidazol)thiopropionate

3-(5-Chloro-1H-benzimidazole)thiopropionic acid (Indian J. Chem., 11(11), 1119-21 (1973)) (5.0 g) was dissolved in allyl alcohol (50 mL) and thionyl chloride (1.6 mL) was added. The reaction solution was refluxed for 2 hours and then concentrated. The residue was made alkaline with an aqueous potassium carbonate solution and then extracted with chloroform. The extract was dried over anhydrous magnesium sulfate and the solvent was distilled off to obtain the title compound (5.1 g, 90%) as a yellow oil.
NMR (200 MHz, CDCl₃) δ: 1.72 (9H, s), 2.97 (2H, t), 3.54 (2H, t), 4.63 (2H, dd), 5.22-5.38 (2H, m), 5.86-6.04 (1H, m), 7.17-7.28 (2H, m), 7.47-7.58 (1H, m), 7.72-7.86 (1H, m)

### 74b) Tert-butyl 5-chloro-2-{3-oxo-3-[4-(5,6,7,8-tetrahydroimidazo[1,2-a]pyridin-3-yl)-1-piperidinyl]propyl}thiobenzimidazole-1-carboxylate

Allyl 3-(5-chloro-1H-benzimidazole)thiopropionate (4.0 g) obtained in Example 74a) and 4-(dimethylamino)pyridine (0.1 g) were dissolved in THF (40 mL) and di-tert-butyl dicarbonate (3.4 g) was added. The mixture was stirred at room temperature for 1 hour and then concentrated. A portion (1.5 g) of the residue was dissolved in THF (40 mL) and Meldrum's acid (0.81 g) and tetrakistriphenylphosphine palladium (0.2 g) were added. The mixture was stirred at room temperature overnight. The reaction solution was concentrated and then dissolved in a dichloromethane (30 mL). To the solution were added 3-(4-piperidinyl)-5,6,7,8-tetrahydroimidazo[1,2-a]pyridine dihydrochloride (0.52 g) obtained in Example 209b), triethylamine (0.53 mL), HOBt (0.32 g) and WSC (0.40 g). The mixture was stirred at room temperature for 16 hours. The reaction solution was made alkaline with an aqueous potassium carbonate solution and then extracted with chloroform. The extract was dried over anhydrous magnesium sulfate and the solvent was distilled off. The residue was purified with a silica gel column to obtain the title compound (1.4 g, 68%) as pale yellow crystals.
NMR (300 MHz, CDCl₃) δ: 1.47 (9H, s), 1.51-1.77 (2H, m), 1.92-1.98 (6H, m), 2.63-2.75 (1H, m), 2.83-3.00 (5H, m), 3.03-3.23 (1H, m), 3.50-3.60 (2H, m), 3.79-3.85 (2H, m), 4.06-4.23 (1H, m), 4.69-4.76 (1H, m), 6.68 (1H, s), 7.17-7.25 (1H, m), 7.45-7.54 (1H, m), 7.74-7.87 (1H, m).

### 74c) 5-Chloro-2-{3-oxo-3-[4-(5,6,7,8-tetrahydroimidazo[1,2-a]pyridin-3-yl)-1-piperidinyl]propyl}sulfonyl-1H-benzimidazole

Tert-butyl 5-chloro-2-{3-oxo-3-[4-(5,6,7,8-tetrahydroimidazo[1,2-a]pyridin-3-yl)-1-piperidinyl]propyl}thiobenzimidazole-1-carboxylate (0.4 g) obtained in Example 74b) was dissolved in trifluoroacetic acid (5 mL) and stirred at room temperature for 0.5 hours. After the solvent was concentrated, the residue was made alkaline with an aqueous potassium carbonate solution and extracted with chloroform. The extract was dried over anhydrous magnesium sulfate and the solvent was distilled off. The residue was dissolved in chloroform (30 mL) and m-chloroperbenzoic acid (0.74 g) was added at room temperature. The reaction solution was stirred at room temperature for 3 hours and the solvent was then distilled off. The residue was purified with a silica gel column to obtain the title compound (56 mg, 16%) as pale yellow powder.
NMR (300 MHz, CDCl₃) δ: 1.14-1.68 (2H, m), 1.86-2.12 (6H, m), 2.57-2.78 (1H, m), 2.85-2.99 (5H, m), 3.10-3.23 (1H, m), 3.75-3.81 (2H, m), 3.94-3.99 (3H, m), 4.36-4.42 (1H, m), 6.87 (1H, s), 7.28-7.34 (1H, m), 7.63-7.69 (2H, m), 7.89-7.94 (1H, m).

### Example 75

### 2-(1-{3-[(6-Chloro-2-naphthyl)sulfonyl]propanoyl}-4-piperidinyl)-1-(1H-imidazol-4-yl)ethanone

### 75a) Tert-butyl 4-[2-(1H-imidazol-4-yl)-2-oxoethyl]piperazin-1-carboxylate

A 1 M solution (22 mL) of ethylmagnesium bromide in THF was added to a solution of 4-iodo-1H-imidazole (2.1 g) and tetramethylethylenediamine (1.7 mL) in THF (15 mL) at 25°C or lower. After the reaction solution was stirred at 60°C for 1 hour, a solution of tert-butyl 4-[(N-methoxy-N-methylcarbamoyl)methyl]piperazine-1-carboxylate (2.0 g) in THF (15 mL) was added at room temperature and stirred at room temperature for 16 hours. After an aqueous ammonium chloride solution was added, the reaction solution was extracted with ethyl acetate and the extract was dried over anhydrous magnesium sulfate. After the solvent was distilled off, the residue was purified with a silica gel column to obtain the title compound (41.0 g, 47%) as an oil. NMR (200 MHz, CDCl₃) δ: 1.20-1.27 (2H, m), 1.47 (9H, s), 1.71-1.78 (2H, m), 2.75-2.85 (4H, m), 3.31-3.39 (1H, m), 4.07-4.13 (2H, m), 7.19 (1H, s), 7.65 (1H, s).

### 75b) 2-(1-{3-[(6-Chloro-2-naphthyl)sulfonyl]propanoyl}-4-piperidinyl)-1-(1H-imidazol-4-yl)ethanone

Tert-butyl 4-[2-(1H-imidazol-4-yl)-2-oxoethyl]piperazine-1-carboxylate (1.0 g) obtained in Example 75a) was dissolved in trifluoroacetic acid (10 mL), de-tert-butoxycarbonylated, and then condensed with 3-[(6-chloro-2-naphthyl)sulfonyl]propionic acid (1.0 g) to obtain the title compound (0.04 g, 1.4%) as pale yellow powder in a similar manner to Example 50b).
NMR (200 MHz, CDCl₃) δ: 1.08-1.26 (2H, m), 1.71-1.87 (2H, m), 2.10-2.32 (1H, m), 2.50-2.62 (1H, m), 2.78-2.91 (4H, m), 2.99-3.11 (1H, m), 3.54-3.61 (2H, m), 3.78-3.85 (1H, m), 4.45-4.51 (1H, m), 7.59 (1H, dd), 7.75-7.97 (4H, m), 8.47 (1H, s).

### Example 76

### 2- (1-(3- [(6-Chloro-2-naphthyl) sulfonyl]propanoyl)-4-piperidinyl)-1-(1H-imidazol-4-yl)ethanol

2-(1-{3-[(6-Chloro-2-naphthyl)sulfonyl]propanoyl}-4-piperidinyl)-1-(1H-imidazol-4-yl)ethanone (0.1 g) obtained in Example 75b) was dissolved in methanol (10 mL) and sodium borohydride (0.1 g) was added under ice-cooling. The mixture was stirred at room temperature for 2 hours. After 1N hydrochloric acid was added to the reaction solution, the solvent was concentrated. The residue was poured into an aqueous potassium carbonate solution and then extracted with chloroform. The extract was dried over anhydrous magnesium sulfate and the solvent was distilled off. The residue was purified with a silica gel column to obtain the title compound (18 mg, 17%) as pale yellow powder.
NMR (200 MHz, CDCl₃) δ: 0.92-1.20 (2H, m), 1.27 (1H, s), 1.71-1.79 (5H, m), 2.41-2.58 (1H, m), 2.84-3.07 (3H, m), 3.52-3.58 (2H, m), 3.74-3.81 (1H, m), 4.38-4.45 (1H, m), 4.80-4.98 (1H, m), 7.57 (1H, dd), 7.92-7.97 (4H, m), 8.47 (1H, s).

### Example 77

### 1-{3-[(6-Chloro-2-napthyl)sulfonyl]propanoyl}-4-(4-methyl-1H-imidazol-5-yl)piperidine

WSC (0.45 g) was added to a solution of 3-[(6-chloro-2-naphthyl)sulfonyl]propionic acid (0.5 g), 4-(4-methyl-1H-imidazol-5-yl)piperidine dihydrochloride (Farmaco, 47(11), 1343-65(1992)) (0.63 g) and HOBt (0.36 g) in dichloromethane (30 mL) and stirred at room temperature for 16 hours. The reaction solution was made alkaline with an aqueous potassium carbonate solution and then extracted with chloroform. The extract was dried over anhydrous magnesium sulfate and the solvent was distilled off. The residue was purified with a basic silica gel column to obtain the title compound (32 mg, 4%) as pale yellow powder. NMR (200 MHz, CDCl₃) δ: 1.63-1.83 (4H, m), 2.26 (3H, s), 2.54-2.66 (1H, m), 2.74-2.90 (3H, m), 3.07-3.17 (1H, m), 3.53-3.59 (2H, m), 3.89-3.93 (1H, m), 4.56-4.61 (1H, m), 6.76 (1H, s), 7.53 (1H, s), 7.58 (1H, dd), 7.89-7.96 (4H, m), 8.47 (1H, s).

### Example 78

### 2-(1-{3-[(6-Chloro-2-naphthyl)sulfonyl]propanoyl}-4-piperidinyl)-5-ethyl-7-methyl-1,2-dihydro-3H-imidazo[1,5-c]imidazol-3-one

### 78a) 1-{3-[(6-Chloro-2-naphthyl)sulfonyl]propanoyl}-N-[(2-ethyl-4-methyl-1H-imidazol-5-yl)methyl]piperidine-4-amine

From 1-{3-[(6-chloro-2-naphthyl)sulfonyl]propanoyl}piperidine-4-amine (3.0 g) obtained in Example 50a) and 2-ethyl-4-methylimidazole-5-carbaldehyde (1.1 g), the title compound (2.0 g, 50%) was prepared as pale yellow powder by reductive amination according to a similar manner to Example 51.
NMR (200 MHz, CDCl₃) δ: 1.33 (3H, t), 1.48-1.72 (2H, m), 1.84-1.97 (2H, m), 2.16 (3H, s), 2.45-2.66 (1H, m), 2.82-2.97 (5H, m), 3.11-3.22 (1H, m), 3.52-3.75 (4H, m), 3.81-4.01 (1H, m), 4.11-4.45 (1H, m), 7.39 (1H, s), 7.58 (1H, dd), 7.89-7.97 (4H, m), 8.47 (1H, s).

### 78b) 2-(1-{3-[(6-Chloro-2-naphthyl)sulfonyl]propanoyl}-4-piperidinyl)-5-ethyl-7-methyl-1,2-dihydro-3H-imidazo[1,5-c]imidazol-3-one

From 1-{3-[(6-chloro-2-naphthyl)sulfonyl]propanoyl}-N-[(2-ethyl-4-methyl-1H-imidazol-5-yl)methyl]piperidine-4-amine (2.0 g) obtained in Example 78a), DBU (0.6 mL) and N,N'-carbonyldiimidazole (0.7 g), the title compound (71 mg, 4%) was obtained as white crystals in a similar manner to Example 65.
NMR (200 MHz, CDCl₃) δ: 1.33 (3H, t), 1.56-1.71 (2H, m), 1.84-1.93 (2H, m), 2.17 (3H, s), 2.58-2.66 (1H, m), 2.86-2.98 (4H, m), 3.13-3.22 (1H, m), 3.52-3.62 (2H, m), 3.97-4.01 (1H, m), 4.11-4.15 (1H, m), 4.18 (2H, s), 4.68-4.72 (1H, m), 7.58 (1H, dd), 7.89-7.96 (4H, m), 8.47 (1H, s).

### Example 79

### 6-(1-{3-[(6-Chloro-2-naphthyl)sulfonyl]propanoyl}-4-piperidinyl)-6,7-dihydro-5H-imidazo[1,5-a]imidazol-5-one

From 1-{3-[(6-chloro-2-napthyl)sulfonyl]propanoyl}-N-[(2-imidazolyl)methyl]piperidine-4-amine (1.5 g) obtained in Example 54, DBU (0.16 mL) and N,N'-carbonyldiimidazole (0.19 g), the title compound (0.62 g, 19%) was obtained as pale yellow powder in a similar manner to Example 65.
NMR (200 MHz, CDCL₃) δ: 1.60-1.75 (2H, m), 1.90-2.05 (2H, m), 2.64 (3H,t), 2.85-3.15 (2H, m), 3.19 (3H, t), 3.49-3.70 (2H, m), 3.99-4.04 (1H, m), 4.22-4.30 (1H, m), 4.31 (2H, s), 4.70-4.75 (1H, m), 7.18 (1H, d), 7.31 (1H, d), 7.59 (1H, dd), 7.89-7.96 (4H, m), 8.48 (1H, s).

### Example 80

### N-(1-{3-[(6-Chloro-2-naphthyl)sulfonyl]propanoyl}-4-piperidinyl)-N-(4-methyl-1H-imidazol-5-yl)methylacrylamide

From 1-{3-[(6-chloro-2-naphthyl)sulfonyl]propanoyl}-N-[(4-methyl-1-tritylimidazol-5-yl)methyl]piperidine-4-amine (1.5 g) obtained in Example 53a) and 3-bromopropionyl chloride (0.24 mL), the title compound (0.34 g, 33%) was obtained as pale yellow powder in a similar manner to Example 55.
NMR (200 MHz, CDCl₃) δ: 1.61-1.89 (4H, m), 2.23 (3H, s), 2.48-2.67 (1H, m), 2.82-3.17 (3H, m), 3.57-3.64 (2H, m), 3.80-4.07 (2H, m), 4.39 (2H, s), 4.60-4.82 (1H, m), 5.78-5.82 (1H, m), 6.38 (1H, dd), 6.50-6.78 (1H, m), 7.45 (1H, s), 7.61 (1H, dd), 7.91-7.99 (4H, m), 8.50 (1H, s).

### Example 81

### 3-(1-{3-[(6-Chloro-2-naphthyl)sulfonyl]propanoyl}-4-piperidinyl)-8-methylimidazo[1,2-a]pyridine hydrochloride

### 81a) Tert-butyl 4-(8-methylimidazo[1,2-a]pyridin-3-yl)piperidine-1-carboxylate

A solution of tert-butyl 4-(1-bromo-2-oxoethyl)piperidine-1-carboxylate (1.0 g) and 3-methyl-2-aminopyridine (0.28 g) in ethanol (10 mL) was refluxed for 4 hours. After the reaction solution was concentrated under reduced pressure, the residue was dissolved in ethyl acetate and 1N hydrochloric acid and an aqueous layer was separated. The aqueous layer was made alkaline with 6N sodium hydroxide and extracted with ethyl acetate. The extract was washed with an aqueous saturated sodium chloride solution and dried over anhydrous sodium sulfate. The solvent was distilled off to obtain the title compound (0.65 g, 79%) as a colorless oil.
NMR (300 MHz, CDCl₃) δ: 1.49 (9H, s), 1.66-1.78 (2H, m), 1.90 (1H, br), 2.09 (1H, br), 2.61 (3H, s), 2.78-2.97 (3H, m), 3.24 (1H, m), 4.25 (1H, m), 6.73 (1H, q, J = 6.9), 6.95 (1H, m), 7.39 (1H, d, J = 2.4), 7.84 (1H, dd, J = 3.0, 6.3). 81b) 3-(1-{3-[(6-Chloro-2-naphthyl)sulfonyl]propanoyl}-4-piperidinyl)-8-methylimidazo[1,2-a]pyridine hydrochloride

Concentrated hydrochloric acid (2 mL) was added to tert-butyl 4-(8-methylimidazo[1,2-a]pyridin-3-yl)piperidine-1-carboxylate (0.47 g) obtained in Example 81a), diluted with ethanol, and then concentrated under reduced pressure. The residue was suspended in acetonitrile (10 mL), and triethylamine (0.63 mL) and DBU (0.45 mL) were added. This solution was added to a suspension of 3-[(6-chloro-2-naphthyl)sulfonyl]propionic acid (0.45 g), WSC (0.43 g) and HOBt (0.35 g) in acetonitrile (10 mL) and stirred for 12 hours. The reaction solution was concentrated under reduced pressure and the residue was dissolved in chloroform and an aqueous saturated sodium bicarbonate solution to separate a chloroform layer. The chloroform solution was dried over anhydrous sodium sulfate and the solvent was distilled off. The residue was purified with a silica gel column (ethyl acetate/methanol=10/1). The resulting white powder was treated with a 4N solution of hydrogen chloride in ethyl acetate and a precipitated solid was filtered. The resulting solid was dried under reduced pressure to obtain the title compound (0.28 g, 38%) as a white solid.
NMR (200 MHz, CDCl₃) δ: 1.53-1.84 (2H, m), 2.05-2.20 (2H, m), 2.60 (3H, s), 2.77 (1H, m), 2.90-2.96 (2H, m), 3.05 (1H, m), 3.26 (1H, m), 3.55-3.63 (2H, m), 4.00 (1H, d, J = 13.8), 4.62 (1H, d, J = 13.8), 6.76 (1H, t, J = 7.0), 6.97 (1H, d, J = 7.0), 7.36 (1H, s), 7.56-7.61 (1H, m), 7.82 (1H, d, J = 7.0), 7.90-7.97 (4H, m), 8.49 (1H, s).
Elementary analysis for C₂₆H₂₆N₃O₃SCl·HCl·H₂O
Calculated (%): C, 56.73; H, 5.31; N, 7.63.
Found (%): C, 57.09; H, 5.50, N, 7.32.

### Example 82

### 3-(1-{3-[(6-Chloro-2-naphthyl)sulfonyl]propanoyl}-4-piperidinyl)-7-methylimidazo[1,2-a]pyridine hydrochloride

### 82a) Tert-butyl 4-(7-methylimidazo[1,2-a]pyridin-3-yl)piperidin-1-carboxylate

From tert-butyl 4-(1-bromo-2-oxoethyl)piperidine-1-carboxylate (1.0 g) and 4-methyl-2-aminopyridine (0.28 g), the title compound (0.65 g, 79%) was obtained in a similar manner to Example 81a).
NMR (200 MHz, CDC1₃) 6: 1. 49 (9H, s), 1. 62-1. 78 8 (2H, m), 2.04-2.08 (2H, m), 2.39 (3H, s), 2.85-2.98 (3H, m), 4.25 (2H, d, J = 14.6), 6.65 (1H, d, J = 7.4), 7.32 (1H, s), 7.37 (1H, s), 7.83 (1H, d, J = 7.4).

### 82b) 3-(1-{3-[(6-Chloro-2-naphthyl)sulfonyl]propanoyl}-4-piperidinyl)-7-methylimidazo[1,2-a]pyridine hydrochloride

From tert-butyl 4-(7-methylimidazo[1,2-a]pyridin-3-yl)piperidin-1-carboxylate (0.47 g) obtained in Example 82a) and 3-[6-chloro-2-naphthyl)sulfonyl]propionic acid (0.45 g), the title compound (0.30 g, 39%) was obtained in a similar manner to Example 81b).
NMR (300 MHz, CDCl₃) δ: 1.56-1.79 (2H, m), 2.06-2.18 (2H, m), 2.36 (3H, s), 2.73-2.82 (1H, m), 2.91-2.96 (2H, m), 3.00-3.09 (1H, m), 3.20-3.30 (1H, m), 3.58-3.62 (2H, m), 4.00 (1H, d, J = 14.4), 4.62 (1H, d, J = 14.4), 7.02 (1H, d, J = 7.2), 7.32 (1H, s), 7.50-7.60 (2H, m), 7.68 (1H, s), 7.86-7.96 (4H, m), 8.48 (1H, s).
Elemental analysis for C₂₆H₂₆N₃O₃SCl·HCl·H₂O
Calculated (%): C, 56.73; H, 5.31; N, 7.63.
Found (%): C, 56.95; H, 5.42, N, 7.50.

### Example 83

### 3-(1-{3-[(6-Chloro-2-naphthyl)sulfonyl]propanoyl}-4-piperidinyl)-6-methylimidazo[1,2-a]pyridine hydrochloride

### 83a) Tert-butyl 4-(6-methylimidazo[1,2-a]pyridin-3-yl)piperidine-1-carboxylate

From tert-butyl 4-(1-bromo-2-oxoethyl)piperidine-1-carboxylate (1.0 g) and 5-methyl-2-aminopyridine (0.28 g), the title compound (0.67 g, 81%) was obtained in a similar manner to Example 81a).
NMR (200 MHz, CDCl₃) δ: 1.49 (9H, s), 1.65-1.80 (2H, m), 1.95-2.09 (2H, m), 2.35 (3H, s), 2.88-3.00 (3H, m), 4.27 (2H, d, J = 13.2), 7.00 (1H, dd, J = 1.8, 9.4), 7.35 (1H, s), 7.52 (1H, d, J = 9.4), 7.70 (1H, d, J = 1.8).

### 83b) 3-(1-{3-[(6-Chloro-2-naphthyl)sulfonyl]propanoyl}-4-piperidinyl)-6-methyl[1,2-a]pyridine hydrochloride

From tert-butyl 4-(6-methylimidazo[1,2-a]pyridin-3-yl)piperidine-1-carboxylate (0.47 g) obtained in Example 83a) and 3-[(6-chloro-2-naphthyl)sulfonyl]propionic acid (0.45 g), the title compound (0.36 g, 45%) was obtained in a similar manner to Example 81b).
NMR (200 Hz, CDCl₃) δ: 1.56-1.76 (2H, m), 2.11-2.18 (2H, m), 2.40 (3H, s), 2.73-2.82 (1H, m), 2.91-2.96 (2H, m), 3.00-3.09 (1H, m), 3.20-3.30 (1H, m), 3.56-3.62 (2H, m), 3.97 (1H, d, J = 14.4), 4.61 (1H, d, J = 14.4), 6.66 (1H, d, J = 7.2), 7.28 (1H, d, J = 2.7), 7.37 (1H, s), 7.57-7.60 (1H, m), 7.81 (1H, d, J = 7.2), 7.89-7.96 (4H, m), 8.48 (1H, s). Elemental analysis for C₂₆H₂₆N₃O₃SCl·HCl·H₂O
Calculated (%) C, 56.73; H, 5.31; N, 7.63.
Found (%) C, 56.54; H, 5.44, N, 7.54.

### Example 84

### 3-(1-{3-[(6-Chloro-2-naphthyl)sulfonyl]propanoyl}-4-piperidinyl)-5-methylimidazo[1,2-a]pyridine hydrochloride

### 84a) Tert-butyl 4-(5-methylimidazo[1,2-a]pyridinyl)piperidin-1-caroboxylate

From tert-butyl 4-(1-bromo-2-oxoethyl)piperidine-1-carboxylate (1.0 g) and 6-methyl-2-aminopyridine (0.28 g), the title compound (0.65 g, 79%) was obtained in a similar manner to Example 81a).
NMR (200 MHz, CDCl₃) δ: 1.45 (9H, m), 1.66-1.78 (2H, m), 1.88-2.05 (2H, m), 2.84 (3H, s), 2.78-2.91 (2H, m), 3.41 (1H, m), 4.27 (2H, d, J = 13.2), 6.50 (1H, d, J= 7.0), 7.02 (1H, dd, J = 7.0, 9.2), 7.45 (1H, s), 7.47 (1H, d, J = 9.2). 84b) 3-(1-{3-[(6-Chloro-2-naphthyl)sulfonyl]propanoyl}-4-piperidinyl)-5-methylimidazo[1,2-a]pyridine hydrochloride

From tert-butyl 4-(5-methylimidazo[1,2-a]pyridin-3-yl)piperidin-1-caroboxylate (0.47 g) obtained in Example 84a) and 3-[(6-chloro-2-naphthyl)sulfonyl]propionic acid (0.45 g), the title compound (0.33 g, 41%) was obtained in a similar manner to Example 81b).
NMR (300 MHz, CDCl₃) δ: 1.53-1.77 (2H, m), 2.05-2.17 (2H, m), 2.65 (1H, m), 2.82 (3H, s), 2.90-2.95 (2H, m), 3.20 (1H, m), 3.44-3.62 (3H, m), 3.97 (1H, d, J = 13.5), 4.66 (1H, d, J = 13.5), 6.51 (1H, d, J = 6.9), 7.03 (1H, dd, J = 6.9, 9.0), 7.40 (1H, s), 7.47 (1H, d, J = 9.0), 7.57 (1H, dd, J = 2.1, 9.0), 7.88-7.95 (4H, m), 8.48 (1H, s).
Elemental analysis for C₂₆H₂₆N₃O₃SC·HCl·H₂O
Calculated (%) C, 56.73; H, 5.31; N, 7.63.
Found (%) C, 56.59; H, 5.31, N, 7.30.

### Example 85

### 3-(1-{3-[(6-Chloro-2-naphthyl)sulfonyl]propanoyl}-4-piperidinyl)imidazo[1,2-a]pyrazine

### 85a) Tert-butyl 4-(imidazo[1,2-a]pyrazin-3-yl)piperidine-1-carboxylate

From tert-butyl 4-(1-bromo-2-oxoethyl)piperidine-1-carboxylate (1.0 g) and 2-aminopyrazine (0.28 g), the title compound (0.44 g, 56%) was obtained in a similar manner to Example 81a).
NMR (200 MHz, CDCl₃) δ: 1.49 (9H, s), 1.64-1.85 (4H, m), 2.78-3.23 (3H, m), 4.30 (d, J = 13.2), 7.61 (1H, d, J = 2.2), 7.87-7.94 (2H, m), 9.09 (1H, d, J = 1.0)

### 85b) 3-(1-{3-[(6-Chloro-2-naphthyl)sulfonyl]propanoyl}-4-piperidinyl)imidazo[1,2-a]pyrazine

Concentrated hydrochloric acid (2 mL) was added to tert-butyl 4-(imidazo[1,2-a]pyrazin-3-yl)piperidine-1-carboxylate (0.38 g) obtained in Example 85a), diluted with ethanol, and then concentrated under reduced pressure. The residue was suspended in acetonitrile (10 mL), and triethylamine (0.63 mL) and DBU(0.45 mL) were added. This solution was added to a suspension of 3-[(6-chloro-2-naphthyl)sulfonyl]propionic acid (0.45 g), WSC (0.43 g) and HOBt (0.35 g) in acetonitrile (10 mL), and stirred for 12 hours. The reaction solution was concentrated under reduced pressure and the residue was dissolved in chloroform and an aqueous saturated sodium bicarbonate solution to separate a chloroform layer. The chloroform solution was dried over anhydrous sodium sulfate and the solvent was distilled off. The residue was purified with a silica gel column (8/1 ethyl acetate-methanol) to obtain the title compound as a colorless powdery solid (0.25 g, 36%).
NMR (200 MHz, CDCl₃) δ: 1.63-1.82 (2H, m), 2.05-2.21 (2H, m), 2.78 (1H, m), 2.95 (2H, t, J = 7.0), 3.09-3.35 (2H, m), 3.59 (2H, t, J = 7.0), 4.03 (1H, d, J = 14.0), 4.68 (1H, d, J = 14.0), 7.58-7.63 (2H, m), 7.91-7.96 (6H, m), 8.49 (1H, s), 9.10 (1H, s).
Elemental analysis for C₂₄H₂₃N₄O₃SCl·0.5H₂O·0.5AcOEt Calculated (%) C, 58.26; H, 5.26; N, 10.45.
Found (%) C, 58.13; H, 5.29, N, 10.75.

### Example 86

### 3-(1-{3-[(6-Chloro-2-naphthyl)sulfonyl]propanoyl}-4-piperidinyl)imidazo[1,2-a]pyrimidine

### 86a) Tert-butyl 4-(imidazo[1,2-a]pyrimidin-3-yl)piperidin-1-carboxylate

From tert-butyl 4-(1-bromo-2-oxoethyl)piperidine-1-carboxylate (1.0 g) and 2-aminopyrimidine (0.25 g), the title compound (0.43 g, 55%) was obtained in a similar manner to Example 81a).
NMR (200 MHz, CDCl₃) δ: 1.49 (9H, s), 1.65-2.07 (4H, m), 2.87-3.02 (3H, m), 4.27 (2H, d, J = 14.0), 6.90 (1H, dd, J = 4.0, 7.0), 7.61 (1H, s), 8.30 (1H, dd, J = 2.0, 7.0), 8.55 (1H, dd, J = 2.0, 4.0)

### 86b) 3-(1-{3-[(6-chloro-2-naphthyl)sulfonyl]propanoyl}-4-piperidinyl)imidazo[1,2-a]pyrimidine

From tert-butyl 4-(imidazo[1,2-a]pyrimidin-3-yl)piperidin-1-carboxylate (0.35 g) obtained in Example 86a) and 3-[(6-chloro-2-naphthyl)sulfonyl]propionic acid (0.38 g), the title compound (0.12 g, 20%) was obtained in a similar manner to Example 85b).
NMR (200 MHz, CDCl₃) δ: 1.62-1.88(2H, m), 2.00-2.18 (2H, m), 2.71-2.84 (1H, m), 2.94 (2H, t, J = 7.0), 3.06-3.20 (1H, m), 3.21-3.33 (1H, m), 3.59 (2H, t, J = 7.0), 4.03 (1H, d, J = 13.4), 4.65 (1H, d, J = 13.4), 6.91 (1H, dd, J = 4.2, 7.0), 7.57-7.62 (2H, m), 7.89-7.97 (4H, m), 8.33 (1H, dd, J = 1.8, 7.0), 8.49 (1H, s), 8.55 (1H, dd, J = 1.8, 4.2)
Elemental analysis for C₂₄H₂₃N₄O₃SCl·1.5H₂O
Calculated (%) C, 56.52; H, 5.14; N, 10.99.
Found (%) C, 56.77; H, 4.82; N, 10.99.

### Example 87

### 3-(1-{3-[(7-Chloro-2H-chromen-3-yl)sulfonyl]propanoyl}-4-piperidinyl)-5,6,7,8-tetrahydroimidazo[1,2-a]pyridine hydrochloride

### 87a) 3-[(7-Chloro-2H-chromen-3-yl)sulfonyl]propionic acid

3-(7-Chloro-2H-chromen-3-yl)sulfonyl chloride (5.0 g) was added to a solution of sodium hydrogencarbonate (3.2 g) and sodium sulfite (2.6 g) in water (100 mL) and stirred at 70°C for 90 minutes. Then, sodium hydroxide (1.9 g) and bromosuccinic acid (9.3 g) were added and the mixture was stirred at 110°C for 8 hours. The reaction solution was allowed to be cooled. A precipitate was filtered, washed with water, and then concentrated under reduced pressure to obtain the title compound (4.1 g, 71%) as a light brown solid.
NMR (300 MHz, DMSO-d₆) δ: 2.63 (2H, t, J = 7.2), 3.50 (2H, t, J = 7.2), 5.62 (2H, s), 7.04-7.11 (2H, m), 7.46-7.49 (2H, m) .

### 87b) 3-(1-{3-[(7-Chloro-2H-chromen-3-yl)sulfonyl]propanoyl}-4-piperidinyl)-5,6,7,8-tetrahydroimidazo[1,2-a]pyridine hydrochloride

A solution of 3-(4-piperidinyl)-5,6,7,8-tetrahydroimidazo[1,2-a]pyridine dihydrochloride (0.92 g) obtained in Example 209b), triethylamine (1.3 mL) and DBU (0.99 mL) in acetonitrile (10 mL) was added to a suspension of 3-[(7-chloro-2H-chromen-3-yl)sulfonyl]propionic acid (0.91 g) obtained in Example 87a), WSC (0.86 g) and HOBt (0.64 g) in acetonitrile (20 mL), and stirred for 12 hours. The reaction solution was concentrated under reduced pressure and the residue was dissolved in chloroform and an aqueous saturated sodium bicarbonate solution to separate a chloroform layer. The chloroform solution was dried over anhydrous sodium sulfate and the solvent was distilled off. The residue was purified with a silica gel column and the resulting pale yellow viscous substance was treated with a saturated solution of hydrogen chloride in ethanol to obtain the title compound (1.36 g, 86%).
NMR (200 MHz, CDCl₃) δ: 1.39-1.72 (2H, m), 1.84-2.03 (6H, m), 2.62-2.68 (2H, m), 2.83-2.89 (4H, m), 3.10-3.24 (1H, m), 3.44-3.52 (2H, m), 3.81 (2H, t, J = 6.0), 3.92 (1H, d, J = 13.8), 4.60 (1H, d, J = 13.8), 5.04 (2H, s), 6.64 (1H, s), 6.92-6.98 (2H, m), 7.10 (1H, d, J = 8.4), 7.32 (1H, s)

### Example 88

### 3-(1-{3-[(7-Bromo-2H-chromen-3-yl)sulfonyl]propanoyl}-4-piperidinyl)-5,6,7,8-tetrahydroimidazo[1,2-a]pyridine hydrochloride

From 3-(4-piperidinyl)-5,6,7,8-tetrahydroimidazo[1,2-a]pyridine dihydrochloride (0.46 g) obtained in Example 209b) and 3-[(7-bromo-2H-chromen-3-yl)sulfonyl]propionic acid (0.52 g) obtained in Example 87a), the title compound (0.51 g, 58%) was obtained as a colorless solid in a similar manner to Example 87b).
NMR (300 MHz, CDCl₃) δ: 1.35-1.63 (2H, m), 1.85-2.02 (6H, m), 2.62-2.71 (2H, m), 2.84-2.89 (4H, m), 3.10-3.22 (1H, m), 3.45-3.51 (2H, m), 3.81 (2H, t, J = 5.7), 3.92 (1H, t, J = 14.1), 4.60 (1H, d, J = 14.1), 5.03 (2H, s), 6.64 (1H, s), 7.02-7.14 (3H, m), 7.32 (1H, s)

### Example 89

### 3-[1-(3-{ [(E)-2-(4-chlorophenyl)vinyl]sulfonyl}propanoyl)-4-piperidinyl]-5,6,7,8-tetrahydroimidazo[1,2-a]pyridine

### 89a) 3-[(E)-2-(4-chlorophenyl)vinyl]sulfonylpropionic acid

From (E)-2-(4-chlorophenyl)ethylenesulfonyl chloride (30. g), the title compound (0.86 g, 25%) was obtained in a similar manner to Example 87a).
NMR (200 MHz, DMSO-d₆) δ: 2.66 (2H, t, J = 7.4), 3.41 (2H, t, J = 7.4), 7.48-7.57 (4H, m), 7.76-7.81 (2H, m).

### 89b) 3-[1-(3-{[(E)-2-(4-chlorophenyl)vinyl]sufonyl}propanoyl-4-piperidinyl]-5,6,7,8-tetrahydroimidazo[1,2-a]pyridine

A solution of 3-(4-piperidinyl)-5,6,7,8-tetrahydroimidazo[1,2-a]pyridine dihydrochloride (0.46 g) obtained in Example 209b), triethylamine (0.65 mL) and DBU (0.49 mL) in acetonitrile (10 mL) was added to a suspension of 3-[(E)-2-(4-chlorophenyl)vinyl]sulfonylpropionic acid (0.41 g) obtained in Example 89a), WSC (0.43 g) and HOBt (0.32 g) in acetonitrile (20 mL) and stirred for 12 hours. The reaction solution was concentrated under reduced pressure and the residue was dissolved in chloroform and an aqueous saturated sodium bicarbonate solution to separate a chloroform layer. The chloroform solution was dried over anhydrous sodium sulfate and the solvent was distilled off. The residue was purified with a silica gel column to obtain the title compound (0.12 g, 18%) as a colorless solid.
NMR (300 MHz, CDCl₃) δ: 1.39-1.68 (2H, m), 1.83-2.01(6H, m), 2.62-2.74 (2H, m), 3.83-3.92 (4H, m), 3.11-3.23 (1H, m), 3.50 (2H, t, J = 7.2), 3.80 (2H, t, J = 5.7), 3.94 (1H, d, J = 14.1), 4.61 (1H, d, J = 14.1), 6.62 (1H, s), 6.85 (1H, d, J = 15.3), 7.39-7.48 (4H, m), 7.54 (1H, d, J = 15.3). Elemental analysis for C₂₃H₂₈N₃O₃SCl·1.5H₂O
Calculated (%) C, 56.49; H, 6.39; N, 8.59.
Found (%) C, 56.35; H, 6.12; N, 8.37.

### Example 90

### 3-[1-(3-{[(E)-2-(4-bromophenyl)vinyl]sulfonyl}propanoyl)-4-piperidinyl]-5,6,7,8-tetrahydroimidazo[1,2-a]pyridine

### 90a) 3-{[(E)-2-(4-bromophenyl)vinyl]sulfonyl}propionic acid

From (E)-2-(4-bromophenyl)ethylenesulfonyl chloride (1.0 g), the title compound (0.31 g, 27%) was obtained in a similar manner to Example 87a).
NMR (200 MHz, DMSO-d₆) δ: 2.65 (2H, t, J = 7.4), 3.39 (2H, t, J = 7.4), 7.48-7.57 (4H, m), 7.76-7.85 (2H, m).

### 90b) 3-[1-(3-{[(E)-2-(4-bromophenyl)vinyl]sulfonyl}propanoyl)-4-piperidinyl]-5,6,7,8-tetrahydroimidazo[1,2-a]pyridine

From 3-(4-piperidinyl)-5,6,7,8-tetrahydroimidazo[1,2-a]pyridine dihydrochloride (0.30 g) obtained in Example 209b) and 3-{[(E)-2-(4-bromophenyl)vinyl]sulfonyl}propionic acid (0.31 g) obtained in Example 90a), the title compound (0.17 g, 34%) was obtained in a similar manner to Example 89b).
NMR (300 MHz, CDCl₃) δ: 1.38-1.67 (2H, m), 1.88-2.00 (6H, m), 2.62-2.74 (2H, m), 2.83-2.89 (4H, m), 3.11-3.23 (1H, m), 3.50 (2H, t, J = 6.6), 3.80 (2H, t, J = 6.0), 3.93 (1, d, J = 12.6), 4.60 (1H, d, J = 12.6), 6.63 (1H, s), 6.87 (1H, d, J = 15.3), 7.37-7.59 (5H, m).

### Example 91

### N-(6-{3-oxo-3-[4-(5,6,7,8-tetrahydroimidazo[1,2-a]pyridin-3-yl)-1-piperidinyl]propyl}sulfonyl-2-naphthyl)acetamide

### 91a) Pyridinium 6-acetylaminonapthalene-2-sulfonate

A mixture of 6-aminonaphthalene-2-sulfonic acid (11.2 g), acetic acid (23.6 mL) and pyridine (12.1 mL) was stirred at room temperature for 16 hours. Diethyl ether was added to the reaction solution and a precipitate was filtered to obtain the title compound (16.4 g, 96%) as a colorless solid.
NMR (200 MHz, DMSO-d₆) 8: 2.11 (3H, s), 7.57 (1H, dd, J = 2.2, 8.8), 7.65 (1H, dd, J = 1.5, 8.8), 7.74 (1H, d, J = 8.8), 7.65 (1H, dd, J = 1.5, 8.8), 7.74 (1H, d, J = 8.8), 7.90 (1H, d, J= 8.8), 8.02-8.09 (3H, m), 8.27 (1H, d, J = 1.5), 8.54-8.63 (1H, m), 8.91-8.95 (2H, m).

### 91b) 6-Acetylaminonaphthalene-2-sulfonyl chloride

Under ice-cooling, thionyl chloride (3.5 mL) was added to a solution of pyridinium 6-acetylaminonapthalene-2-sulfonate (15.0 g) obtained in Example 91a) in DMF (20 mL). The reaction solution was stirred at room temperature for 90 minutes and then poured into a mixture of ethyl acetate and iced water to separate an ethyl acetate layer. The ethyl acetate solution was washed with 1N hydrochloric acid, dried over anhydrous magnesium sulfate, and concentrated. The residue was purified with a silica gel column to obtain the title compound (1.67 g, 14%) as a pale yellow solid. NMR (200 MHz, CDCl₃) δ: 2.29 (3H, s), 7.50 (1H, dd, J = 2.2, 8.8), 7.95-7.99 (3H, m), 8.44 (1H, s), 8.51 (1H, s).

### 91c) 3-[(6-Acetylamino-2-naphthyl)sulfonyl]propionic acid

Using 6-aminonaphthalene-2-sulfonyl chloride (1.69 g) obtained in Example 91b), the title compound (0.68 g, 36%) was obtained in a similar manner to Example 87a).
NMR (200 MHz, DMSO-d₆) δ: 2.24 (3H, s), 2.59 (2H, t, J = 7.4), 3.66 (2H, t, J = 7.4), 8.00 (1H, dd, J = 1.8, 8.4), 8.08 (1H, dd, J = 1.8, 8.4), 8.33 (1H, d, J = 8.8), 8.44 (1H, d, J = 8.8), 8.75 (2H, d, J = 9.0).

### 91d) N-(6-{3-oxo-3-[4-(5,6,7,8-tetrahydroimidazo[1,2-a]pyridin-3-yl)-1-piperidinyl]propyl}sulfonyl-2-naphthyl)acetamide

From 3-[(6-acetylamino-2-naphthyl)sulfonyl]propionic acid(0.68 g) obtained in Example 91c) and 3-(4-piperidinyl)-5,6,7,8-tetrahydroimidazo[1,2-a]pyridine dihydrochloride (0.71 g) obtained in Example 209b), the title compound (0.38 g, 35%) was obtained in a similar manner to Example 89b).
NMR (300 MHz, CDCl₃) δ: 1.14-1.43 (2H, m), 1.79-2.00(6H, m), 2.26 (3H, s), 2.54-2.65 (2H, m), 2.80-2.88 (4H, m), 3.07 (1H, t, J = 11.1), 3.47-3.57 (1H, m), 3.66-3.88 (4H, m), 4.47 (1H, d, J = 13.2), 6.51 (1H, s), 7.59 (1H, dd, J = 2.4, 9.0), 7.80-7.93 (3H, m), 8.36 (1H, s), 8.44 (1H, s), 8.55 (1H, br)
Elemental analysis for C₂₇H₃₂N₄O₄S·3.5H₂O
Calculated (%) C, 56.73; H, 6.88; N, 9.80.
Found (%) C, 56.75; H, 6.94, N, 9.80.

### Example 92

### 3-(1-{3-[(6-Chloro-2-naphthyl)sulfonyl]propanoyl}-4-piperidinyl)-2-methyl-5,6,7,8-tetrahydroimidazo[1,2-a]pyridine hydrochloride

### 92a) 2-Methyl-3-(4-pyridinyl)imidazo[1,2-a]pyridine

A solution of tributyl(4-pyridinyl)tin (5.0.g), 3-bromo-2-methyl-5,6,7,8-tetrahydroimidazo[1,2-a]pyridine (EP 556080 (1993)) (2.6 g), dichlorobis(triphenylphosphine)palladium(0.86 g) and lithium chloride (52 mg) in toluene (20 mL) was stirred at 100°C for 12 hours under argon atmosphere. Insoluble substances were filtered off and the filtrate was concentrated. The residue was purified with a silica gel column to obtain the title compound (0.83 g, 32%).
NMR (300 MHz, CDCl₃) δ: 2.54 (3H, s), 6.81 (1H, ddd, J = 2.4, 10.5, 12.3), 7.23 (1H, m), 7.42 (2H, dd, J = 2.7, 6.9), 7.61 (1H, ddd, J = 1.5, 1.5, 11.7), 8.22 (1H, ddd, J = 1.5, 1.5, 11.7), 8.77 (2H, dd, J = 2.7, 6.9).

### 92b) 2-Methyl-3-(4-piperidinyl)-5,6,7,8-tetrahydroimidazo[1,2-a]pyridine dihydrochloride

Palladium-carbon (0.10 g) was added to a solution of 2-methyl-3-(4-pyridinyl)imidazo[1,2-a]pyridine (0.82 g) obtained in Example 92a) in acetic acid (100 mL) and stirred at 100°C for 8 hours under hydrogen atmosphere (10 MPa). Insoluble substances were filtered off and the filtrate was concentrated. The residue was dissolved in 1N hydrochloric acid and ethanol and the solvent was distilled off to obtain the title compound (1.02 g, 89%) as a colorless solid.
NMR (200 MHz, D₂O) δ: 1.91-2.18 (8H, m), 2.33 (3H, s), 2.94 (2H, t, J = 6.6), 3.09-3.23 (3H, m), 3.58 (2H, d, J = 12.8), 4.04 (2H, t, J = 5.8).

### 92c) 3-(1-{3-[(6-Chloro-2-naphthyl)sulfonyl]propanoyl}-4-piperidinyl)-2-methyl-5,6,7,8-tetrahydroimidazo[1,2-a]pyridine hydrochloride

From 2-methyl-3-(4-piperidinyl)-5,6,7,8-tetrahydroimidazo[1,2-a]pyridine dihydrochloride (0.50 g) obtained in Example 92b) and 3-[(6-chloro-2-naphthyl)sulfonyl]propionic acid (0.51 g), the title compound (0.40 g, 43%) was obtained in a similar manner to Example 87b).
NMR (200 MHz, CDCl₃) δ: 1.70-2.00 (8H, m), 2.18 (3H, s), 2.54 (1H, m), 2.70-3.10 (6H, m), 3.57 (2H, t, J = 6.6), 3.77 (2H, t, J = 6.0), 3.96 (1H, d, J = 12.8), 4.57 (1H, d, J = 12.8), 7.60 (1H, dd, J = 1.8, 8.8), 7.93-7.97 (4H, m), 8.49 (1H, s)
Elemental analysis for C₂₆H₃₀N₃O₃SCl·HCl·H₂O
Calculated (%) C, 56.31; H, 6.00; N, 7.58.
Found (%) C, 56.35; H, 6.37, N, 7.21.

### Example 93

### 3-(1-{[3-(6-Chloro-2-naphthyl)sulfonyl]propanoyl}-4-piperidinyl)-6,7,8,9-tetrahydro-5H-imidazo[1,2-a]azepine hydrochloride

### 93a) 3-Bromo-6,7,8,9-tetrahydro-5H-imidazo[1,2-a]azepine

N-bromosuccinimide (1.3 g) was added to a solution of 6,7,8,9-tetrahydro-5H-imidazo[1,2-a]azepine (Hua, D. H. et al., J. Org. Chem., 59, 5084 (1994)) (1.0 g) in carbon tetrachloride (20 mL) and stirred for 1 hour. After an aqueous saturated sodium bicarbonate solution was added, the reaction solution was extracted with chloroform. The extract was dried over anhydrous sodium sulfate and then concentrated. The resulting residue was purified with a silica gel column to obtain the title compound (1.29 g, 81%) as a pale yellow solid.
NMR (300 MHz, CDCl₃) δ: 1.66-1.90 (6H, m), 2.89-2.93 (2H,m), 3.98 (2H, t, J = 4.8), 6.80 (1H, s).

### 93b) 3-(4-Pyridinyl)-6,7,8,9-tetrahydro-5H-imidazo[1,2-a]azepine

From 3-bromo-6,7,8,9-tetrahydro-5H-imidazo[1,2-a]azepine (1.29 g) obtained in Example 93a), the title compound (0.90 g, 71%) was obtained in a similar manner to Example 92a).
NMR (200 MHz, CDCl₃) δ: 1.76-1.93 (6H, m), 2.99 (2H, t, J = 4.8), 3.99 (2H, t, J = 4.8), 7.01 (1H, s), 7.21 (2H, m), 8.64 (2H, m).

### 93c) 3-(4-Piperidinyl)-6,7,8,9-tetrahydro-5H-imidazo[1,2-a]azepine dihydrochloride

From 3-(4-pyridinyl)-6,7,8,9-tetrahydro-5H-imidazo[1,2-a]azepine (0.90 g) obtained in Example 93b), the title compound (1.19 g, 96%) was obtained in a similar manner to Example 92b).
NMR (200 MHz, D₂O) δ: 176-1.95 (8H, m), 2.24 (2H, d, J = 10.2), 3.06-3.26 (5H, m), 3.57 (2H, d, J = 13.2), 4.21 (2H, t, J = 4.8), 7.11 (1H, s).

### 93d) 3-(1-{[3-(6-Chloro-2-naphthyl)sulfonyl]propanoyl}-4-piperidinyl)-6,7,8,9-tetrahydro-5H-imidazo[1,2-a]azepine hydrochloride

From 3-(4-piperidinyl)-6,7,8,9-tetrahydro-5H-imidazo[1,2-a]azepine dihydrochloride (0.50 g) obtained in Example 93c) and 3-[(6-chloro-2-naphthyl)sulfonyl]propionic acid (0.56 g), the title compound (0.47 g, 52%) was obtained in a similar manner to Example 87b).
NMR (200 MHz, CDCl₃) δ: 1.39-2.00 (10H, m), 2.61-2.69 (2H, m), 2.88-2.96 (4H, m), 3.11-3.23 (1H, m), 3.51-3.60 (2H, m), 3.81-3.84 (2H, m), 3.92 (1H, d, J = 14.4), 4.56 (1H, d, J = 14.4), 6.52 (1H, s), 7.59 (1H, m), 7.90-7.97 (4H, m), 8.48 (1H, s).
Elemental analysis for C₂₆H₃₀N₃O₃SCl·HCl·0.25H₂O
Calculated (%) C, 53.70; H, 6.24; N, 7.23.
Found (%) C, 53.96; H, 6.44, N, 7.11.

### Example 94

### 3-(1-{3-[(6-Vinyl-2-naphthyl)sulfonyl]propanoyl}-4-piperidinyl)-5,6,7,8-tetrahydroimidazo[1,2-a]pyridine hydrochloride

### 94a) Tert-butyl 3-[(6-bromo-2-naphthyl)sulfonyl]propionate

A solution of 6-bromonaphthalene-2-sulfonyl chloride (30.6 g) in THF (200 mL) was added dropwise to a solution of sodium borohydride (7.57 g) in THF (200 mL) at room temperature. The reaction solution was stirred at 40°C for 10 hours, and ice and 6N hydrochloric acid were added to acidify the reaction solution. After extraction with ethyl acetate, the extract was washed with an aqueous saturated sodium chloride solution, dried over anhydrous sodium sulfate, and concentrated. The residue was dissolved in ethyl acetate (200 mL), and triethylamine (20.8 mL) and tert-butyl acrylate (9.5 mL) were added. The mixture was refluxed for 24 hour, diluted with ethyl acetate, washed successively with 1N hydrochloric acid, an aqueous saturated sodium hydrogencarbonate solution and an aqueous saturated sodium chloride solution, and then dried over anhydrous sodium sulfate. The solvent was distilled off to obtain the title compound (28.3 g, 71%) as a pale yellow solid.
NMR (200 MHz, CDCl₃) δ: 1.36 (9H, s), 2.69 (2H, t, J = 8.0), 3.46 (2H, t, J = 8.0), 7.72 (1H, dd, J = 1.8, 8.8), 7.86-7.92 (3H, m), 8.12 (1H, brs), 8.46 (1H, s).

### 94b) Tert-butyl 3-[(6-vinyl-2-naphthyl)sulfonyl]propionate

A solution of tert-butyl 3-[(6-bromo-2-naphthyl)sulfonyl]propionate (2.0 g) obtained in Example 94a), tributyl(vinyl)tin (2.4 g), lithium chloride (1.5 g) and dichlorobis(triphenylphosphine)palladium (0.18 g) in toluene (40 ml) was stirred at 90°C for 3 hours under argon atmosphere. Insoluble substances were filtered off and the filtrate was diluted with ethyl acetate. A 10% solution of potassium fluoride in water was added and precipitated insoluble substances were filtered with Celite. An organic layer was separated from the filtrate, washed with an aqueous saturated sodium chloride solution, dried over anhydrous sodium sulfate, and concentrated. The residue was purified with a silica gel column to obtain the title compound (0.96 g, 55%) as a colorless solid.
NMR (300 MHz, CDCl₃) δ: 1.36 (9H, s), 2.69 (2H, t, J = 7.2), 3.47 (2H, t, J = 7.2), 5.46 (1H, d, J = 10.8), 5.97 (1H, d, J = 17.7), 6.90 (1H, dd, J = 10.8, 17.7), 7.76-7.87 (3H, m), 7.93-7.99 (2H, m), 8.43 (1H, d, J = 1.5).

### 94c) 3-(6-Vinyl-2-naphthyl)sulfonylpropionic acid

Trifluoroacetic acid (10 mL) was added to a solution of tert-butyl 3-[(6-vinyl-2-naphthyl)sulfonyl]propionate (0.96 g) obtained in Example 94b) in toluene (10 mL) and stirred at room temperature for 12 hours. The reaction solution was concentrated to obtain the title compound (0.67 g, 83%) as a brown solid.
NMR (200 MHz, DMSO-d₆) δ: 2.59 (2H, t, J = 7.4), 3.66 (2H, t, J = 7.4), 5.46 (1H, d, J = 10.8), 5.97 (1H, d, J = 17.7), 6.90 (1H, dd, J = 10.8, 17.7), 8.00 (1H, dd, J = 1.8, 8.4), 8.08 (1H, dd, J = 1.8, 8.4), 8.33 (1H, d, J = 8.8), 8.44 (1H, d, J = 8.8), 8.75 (2H, d, J = 9.0).

### 94d) 3-(1-{3-[(6-Vinyl-2-naphthyl)sulfonyl]propanoyl}-4-piperidinyl)-5,6,7,8-tetrahydroimidazo[1,2-a]pyridine hydrochloride

From 3-(6-vinyl-2-naphthyl)sulfonylpropionic acid (0.33 g) obtained in Example 94c) and 3-(4-piperidinyl)-5,6,7,8-tetrahydroimidazo[1,2-a]pyridine dihydrochloride (0.38 g) obtained in Example 209b), the title compound (0.36 g, 57%) was obtained as a white solid in a similar manner to Example 87b).
NMR (200 MHz, CDCl₃) δ: 1.36-1.75 (2H, m), 1.87-2.04 (6H, m), 2.57-2.70 (2H, m), 2.82-2.93 (4H, m), 3.11 (1H, m), 3.53-3.61 (2H, m), 3.80 (2H, t, J = 5.8), 3.91 (1H, d, J = 13.6), 4.57 (1H, d, J = 13.6), 5.47 (1H, d, J = 11.0), 5.96 (1H, d, J = 17.6), 6.65 (1H, s), 6.90 (1H, dd, J = 11.0, 17.6), 7.75-8.01 (5H, m), 8.45(1H, s).
Elemental analysis for C₂₇H₃₁N₃O₃S·HCl·1.8H₂O
Calculated (%) C, 59.34; H, 6.57; N, 7.69.
Found (%) C, 59.55; H, 6.96, N, 7.79.

### Example 95

### 3-(1-{3-[(6-Vinyl-2-naphthyl)sulfonyl]propanoyl}-4-piperidinyl)imidazo[1,2-a]pyridine hydrochloride

From 3-(6-vinyl-2-naphthyl)sulfonylpropionic acid (0.33 g) obtained in Example 94c) and 3-(4-piperidinyl)imidazo[1,2-a]pyridine dihydrochloride (0.37 g) obtained in Example 209b), the title compound (0.15 g, 26%) was obtained in a similar manner to Example 89b).
NMR (300 MHz, CDCl₃) δ: 1.54-1.80 (2H, m), 2.07-2.20 (2H, m), 2.77 (1H, m), 2.93 (2H, t, J = 7.5), 3.08 (1H, m), 3.26 (1H, m), 3.57-3.62 (2H, m), 3.99 (1H, d, J = 13.5), 4.62 (1H, d, J = 13.5), 5.47 (1H, d, J = 11.1), 5.97 (1H, d, J = 17.7), 6.82-6.96 (2H, m), 7.38 (1H, s), 7.61-7.65 (1H, d, J = 10.8), 7.77-8.01 (6H, m), 8.46 (1H, s).
Elemental analysis for C₂₇H₃₁N₃O₃S•HCl•2H₂O
Calculated (%) C, 59.39; H, 5.91; N, 7.69.
Found (%) C, 59.45; H, 5.94, N, 7.56.

### Example 96

### 6-{3-Oxo-3-[4-(5,6,7,8-tetrahydroimidazo[1,2-a]pyridin-3-yl)-1-piperidinyl]propyl}sulfonyl-2-naphthonitrile

### 96a) Tert-butyl 3-[(6-cyano-2-naphthyl)sulfonyl]propionate

A solution of tert-butyl 3-[(6-bromo-2-naphthyl)sulfonyl]propionate (3.9 g) obtained in Example 94a), zinc cyanate (0.69 g) and tetrakis(triphenylphosphine)palladium (0.56 g) in DMF (40 mL) was stirred at 80°C for 5 hours under argon atmosphere. After allowed to cool, the reaction solution was poured into a mixture of ethyl acetate and water to separate an ethyl acetate layer. The ethyl acetate solution was washed with 5% aqueous ammonia and an aqueous saturated sodium chloride solution, dried over anhydrous sodium sulfate, and concentrated. The residue was solidified with isopropyl ether to obtain the title compound (2.04 g, 60%) as a pale yellow solid.
NMR (200 MHz, CDCl₃) δ: 1.36 (9H, s), 2.71 (2H, t, J = 7.6), 3.50 (2H, t, J = 7.6), 7.79 (1H, dd, J = 1.6, 8.4), 7.99-8.15 (3H, m), 8.35 (1H, s), 8.55 (1H, s).

### 96b) 3-[(6-Cyano-2-naphthyl)sulfonyl]propionic acid

From tert-butyl 3-[(6-cyano-2-naphthyl)sulfonyl]propionate (0.85 g) obtained in Example 96a), the title compound (0.70 g, 98%) was obtained in a similar manner to Example 94c).
NMR (200 MHz, DMSO-d₆) δ: 2.59 (2H, t, J = 7.4), 3.66 (2H, t, J = 7.4), 8.00 (1H, dd, J = 1.8, 8.4), 8.08 (1H, dd, J = 1.8, 8.4), 8.33 (1H, d, J = 8.8), 8.44 (1H, d, J = 8.8), 8.75 (2H, d, J = 9.0).

### 96c) 6-{3-Oxo-3-[4-(5,6,7,8-tetrahydroimidazo[1,2-a]pyridin-3-yl)-1-piperidinyl]propyl}sulfonyl-2-naphthonitrle

From 3-[(6-cyano-2-naphthyl)sulfonyl]propionic acid (0.35 g) obtained in Example 96b) and 3-(4-piperidinyl)-5,6,7,8-tetrahydroimidazo[1,2-a]pyridine dihydrochloride (0.34 g) obtained in Example 209b), the title compound (0.42 g, 68%) was obtained in a similar manner to Example 89b).
NMR (300 MHz, CDCl₃) δ: 1.35-1.75 (2H, m), 1.80-2.00 (6H, m), 2.55-2.63 (2H, m), 2.83-2.91 (4H, m), 3.10 (1H, m), 3.56 (1H, m), 3.70 (1H, m), 3.78 (2H, t, J = 5.7), 3.86 (1H, d, J = 12.9), 4.47 (1H, d, J = 12.9), 6.50 (1H, s), 7.95-8.14 (4H, m), 8.43 (1H, s), 8.54 (1H, s).

### Example 97

### 6-{3-Oxo-3-[4-(5,6,7,8-tetrahydroimidazo[1,2-a]pyridin-3-yl)-1-piperidinyl]propyl}sulfonyl-2-naphthamide

### 97a) Tert-butyl 3-[(6-carbamoyl-2-naphthyl)sulfonyl]propionate

Hydrogen peroxide (4 mL) and potassium carbonate (0.7 g) were added to a solution of tert-butyl 3-[(6-cyano-2-naphthyl)sulfonyl]propionate (1.1 g) obtained in Example 96a) in DMSO (20 mL) and stirred at room temperature for 20 minutes. The reaction solution was diluted with ethyl acetate and water to separate an organic layer. The ethyl acetate solution was washed with an aqueous saturated sodium chloride solution and then dried over anhydrous sodium sulfate. The solvent was distilled off to obtain the title compound (0.88 g, 76%) as a colorless solid.
NMR (200 MHz, CDCl₃) δ: 1.36 (9H, s), 2.70 (2H, t, J = 7.0), 3.48 (2H, t, J = 7.0), 6.20 (1H, br), 6.55 (1H, br), 7.91 (1H, dd, J = 1.8, 8.8), 8.02-8.10 (3H, m), 8.42 (1H, s), 8.47 (1H, br).

### 97b) 3-[(6-Carbamoyl-2-naphthyl)sulfonyl]propionic acid

From tert-butyl 3-[(6-carbamoyl-2-naphthyl)sulfonyl]propionate (0.85 g) obtained in Example 97a), the title compound (0.73 g, quantitative) was obtained in a similar manner to Example 94c).
NMR (200 MHz, DMSO-d₆) δ: 2.59 (2H, t, J = 7.0), 3.63 (2H, t, J = 7.0), 7.64 (1H, br), 7.97 (1H, dd, J = 1.8, 8.4), 8.11 (1H, dd, J = 1.8, 8.4), 8.26-8.32 (3H, m), 8.63 (2H, br).

### 97c) 6-{3-Oxo-3-[4-(5,6,7,8-tetrahydroimidazo[1,2-a]pyridin-3-yl)-1-piperidinyl]propyl}sulfonyl-2-naphthamide

From 3-[(6-carbamoyl-2-naphthyl)sulfonyl]propionic acid (0.35 g) obtained in Example 97b) and 3-(4-piperidinyl)-5,6,7,8-tetrahydroimidazo[1,2-a]pyridine dihydrochloride (0.32 g) obtained in Example 209b), the title compound (0.41 g, 73%) was obtained in a similar manner to Example 89b).
NMR (300 MHz, CDCl₃) δ: 1.38-1.67 (2H, m), 1.88-2.05 (6H, m), 2.61-2.71 (2H, m), 2.84-2.97 (4H, m), 3.15 (1H, m), 3.57-3.62 (2H, m), 3.81 (2H, t, J = 5.7), 3.93 (1H, d, J = 14.1), 4.55 (1H, d, J = 14.1), 6.56 (1H, s), 7.79 (1H, dd, J = 1.2, 8.4), 8.02-8.13 (3H, m), 8.34 (1H, s), 8.56 (1H, s) .
Elemental analysis for C₂₆H₃₀N₄O₄S·H₂O·0.1i-Pr₂O
Calculated (%) C, 61.11; H, 6.44; N, 10.72.
Found (%) C, 61.33; H, 6.28, N, 10.56.

### Example 98

### 3-(1-{3-[(6-Ethyl-2-naphthyl)sulfonyl]propanoyl}-4-piperidinyl)-5,6,7,8-tetrahydroimidazo[1,2-a]pyridine hydrochloride

A solution of 3-(1-13-[(6-ethenyl-2-naphthyl)sulfonyl]propanoyl}-4-piperidinyl)-5,6,7,8-tetrahydroimidazo[1,2-a]pyridine hydrochloride (0.2 g) obtained in Example 94d) and palladium-carbon (0.04 g) in methanol (4 mL) was stirred for 1 hour under hydrogen atmosphere. Insoluble substances were filtered off and the filtrate was concentrated to obtain the title compound (0.2 g, 99%).
NMR (300 MHz, CDCl₃) δ: 1.35 (3H, t, J = 7.8), 1.42-1.63 (2H, m), 1.88-1.99 (6H, m), 2.60-2.69 (2H, m), 2.83-2.91 (6H, m), 3.12 (1H, m), 3.53-3.59 (2H, m), 3.80 (2H, t, J = 6.0), 3.91 (1H, d, J = 13.2), 4.56 (1H, d, J = 13.2), 6.65 (1H, s), 7.51 (1H, dd, J = 1.8, 8.7), 7.73 (1H, s), 7.83-7.97 (3H, m), 8.45 (1H, s).
Elemental analysis for C₂₇H₃₃N₃O₃SHCl·1.25H₂O
Calculated (%) C, 60.21; H, 6.83; N, 7.80.
Found (%) C, 60.29; H, 6.77, N, 7.53.

### Example 99

### 7-Acetyl-3-(1-{3-[(6-chloro-2-naphthyl)sulfonyl]propanoyl}-4-piperidinyl)-5,6,7,8-tetrahydroimidazo[1,2-a]pyrazine

### 99a) Tert-butyl 4-(7-acetyl-5,6,7,8-tetrahydroimidazo[1,2-a]pyrazin-3-yl)piperidine-1-carboxylate

A solution of tert-butyl 4-(imidazo[1,2-a]pyrazin-3-yl)piperidine-1-carboxylate (1.0 g) obtained in Example 85a), acetic anhydride (1.0 mL) and palladium-carbon (0.2 g) in ethyl acetate (5 mL) was stirred at room temperature for 3 hours under hydrogen atmosphere. Insoluble substances were filtered off and the filtrate was concentrated. The residue was purified with a silica gel column to obtain the title compound (0.47 g, 41%) as a pale yellow solid.
NMR (300 MHz, CDCl₃) δ: 1.48 (9H, s), 1.51-1.89 (4H, m), 2.19 (3H, s), 2.59 (1H, m), 2.76-2.85 (2H, m), 3.87 (2H, t, J = 5.4), 4.04 (2H, t, J = 5.4), 4.18-4.22 (2H, m), 4.74 (2H, s), 6.76 (1H, s).

### 99b) 7-Acetyl-3-(1-{3-[(6-chloro-2-naphthyl)sulfonyl]propanoyl}-4-piperidinyl)-5,6,7,8-tetrahydroimidazo[1,2-a]pyrazine

Trifluoroacetic acid (5 mL) was added to a solution (of tert-butyl 4-(7-acetyl-5,6,7,8-tetrahydroimidazo[1,2-a]pyrazin-3-yl)piperidine-1-carboxylate (0.45 g) obtained in Example 99a) in toluene 5 mL) and stirred at room temperature for 2 hours. The reaction solution was concentrated and the residue was dissolved in acetonitrile. After DBU (0.41 g) and triethylamine (0.56 mL) were added, this solution was added to a suspension of 3-[(6-chloro-2-naphthyl)sulfonyl]propionic acid (0.45 g), WSC (0.43 g) and HOBt (0.35 g) in acetonitrile (10 mL) and stirred for 12 hours. The reaction solution was concentrated under reduced pressure and the residue was dissolved in chloroform and an aqueous saturated sodium bicarbonate solution to separate a chloroform layer. The chloroform solution was dried over anhydrous sodium sulfate and the solvent was distilled off. The residue was purified with a silica gel column to obtain the title compound (0.39 g, 54%) as a white powdery solid.
NMR (300 MHz, CDCl₃) δ: 1.45-1.65 (2H, m), 1.88-2.00 (2H, m), 2.19 (3H, s), 2.61-2.78 (3H, m), 2.91 (2H, t, J = 8.4), 3.15 (1H, t, J = 14.4), 3.56 (2H, t, J = 8.4), 3.84-4.10 (4H, m), 4.58 (1H, d, J = 13.5), 4.75 (2H, s), 6.74 (1H, s), 7.59 (1H, dd, J = 1.2, 8.7), 7.90-7.96 (4H, m), 8.48 (1H, s) .

### Example 100

### (6-{3-Oxo-3-[4-(5,6,7,8-tetrahydroimidazo[1,2-a]pyridin-3-yl)-1-piperidinyl]propyl}sulfonyl-2-naphthyl)methylamine dihydrochloride

### 100a) Tert-butyl 3-[(6-formyl-2-naphthyl)sulfonyl]propionate

Osmium oxide (10% enmicrocapsulated, 1.1 g) was added to a solution of tert-butyl 3-[(6-vinyl-2-naphthyl)sulfonyl]propionate (5.5 g) obtained in Example 94b) and sodium periodate (13.8 g) in acetonirile-acetone-water (1:1:1, 300 mL) and stirred at room temperature for 2 days. Insoluble substances were filtered off and the organic solvent was distilled off from the filtrate under reduced pressure. The residue was extracted with ethyl acetate and the extract was washed with an aqueous saturated sodium hydrogencarbonate solution and an aqueous saturated sodium chloride solution, and then dried over anhydrous sodium sulfate. The solvent was distilled off and the residue was purified with a silica gel column to obtain the title compound (2.56 g, 46%) as a pale yellow solid.
NMR (300 MHz CDCl₃) δ: 0.36 (9H, s), 2.72 (2H, t, J = 8.0), 3.50 (2H, t, J = 8.0), 8.00 (1H, dd, J = 1.2, 8.8), 8.12-8.23 (3H, m), 8.45 (1H, s), 8.55 (1H, s), 10.24 (1H, s). 100b) Tert-butyl 3-[(6-hydroxymethyl-2-naphthyl)sulfonyl]propionate

Sodium borohydride (0.46 g) was added to a solution of tert-butyl 3-[(6-formyl-2-naphthyl)sulfonyl]propionate (2.0 g) obtained in Example 100a) in ethanol and stirred at room temperature for 30 minutes. The reaction solution was concentrated and after ethyl acetate was added, the residue was washed successively with 1N hydrochloric acid, an aqueous saturated sodium hydrogencarbonate solution and an aqueous saturated sodium chloride solution, and then dried over anhydrous sodium sulfate. The solvent was distilled off to obtain the title compound (2.0 g, 99%) as a colorless solid.
NMR (200 MHz, CDCl₃) δ: 1.36 (9H, s), 2.12 (1H, t, J = 5.8), 2.67 (2H, t, J =7.8), 3.45 (2H, t, J = 7.8), 4.92 (2H, d, J = 5.8), 7.61 (1H, dd, J = 1.8, 8.8), 7.83 (1H, dd, J = 1.8, 8.8), 7.91-7.98 (3H, m), 8.42 (1H, s).

### 100c) Tert-butyl 3-(6-azidomethyl-2-naphthyl)sulfonylpropionate

Methanesulfonyl chloride (0.15 mL) was added to a solution of tert-butyl 3-[(6-hydroxymethyl-2-naphthyl)sulfonyl]propionate (0.46 g) obtained in Example 100b) and pyridine (0.30 mL) in dichloromethane (10 mL) under ice-cooling. After stirring for 2 hours, ice was added to the reaction solution and the mixture was diluted with ethyl acetate and water. An organic layer was separated and washed successively with an aqueous saturated sodium hydrogencarbonate solution and an aqueous saturated sodium chloride solution, dried over anhydrous sodium sulfate, and then concentrated. The residue was dissolved in acetone (10 mL) and lithium bromide (1.0 g) was added. After stirring for 3 hours, the mixture was diluted with ethyl acetate, and water. An organic layer was separated, washed successively with an aqueous saturated sodium hydrogencarbonate solution and an aqueous sodium saturated chloride solution, dried over anhydrous sodium sulfate, and then concentrated. The residue was dissolved in DMF (10 mL) and sodium azide (0.19 g) was added. After stirring for 12 hours, the reaction solution was diluted with ethyl acetate and water. An organic layer was separated, washed successively with an aqueous saturated sodium hydrogencarbonate solution and an aqueous sodium saturated chloride solution and then dried over anhydrous sodium sulfate. The solvent was distilled off to obtain the title compound (0.32 g, 73%) as a colorless powdery solid.
NMR (200 MHz, CDCl₃) δ: 0.35 (9H, s), 2. 69 (2H, t, J = 7.8), 3.47 (2H, t, J = 7.8), 4.59 (2H, s), 7.61 (1H, d, J = 1.4), 7.90 (2H, dd, J = 1.4, 8.8), 8.01-8.06 (2H, m), 8.49 (1H, br) .

### 100d) 3-[(6-Azidomethyl-2-naphthyl)sulfonyl]propionic acid

A solution of tert-butyl 3-(6-azidomethyl-2-naphthyl)solfonylpropionate (0.24 g) obtained in Example 100c) in formic acid (5 mL) was stirred at room temperature for 2 hours. The reaction solution was concentrated to obtain the title compound (0.22 g, 99%) as a white solid. NMR (200 MHz, DMSO-d₆) δ: 2.57 (2H, t, J = 7.8), 3.61 (2H, t, J = 7.8), 4.73 (2H, s), 7.69 (1H, dd, J = 1.8, 8.4), 7.92 (1H, dd, J = 1.8, 8.8), 8.09 (1H, s), 8.19-8.29 (2H, m), 8.59 (1H, s).

### 100e) 3-(1-{3-[(6-Azidomethyl-2-naphthyl)sulfonyl]propanoyl}-4-piperidinyl)-5,6,7,8-tetrahydroimodazo[1,2-a]pyridine

From 3-[(6-azidomethyl-2-naphthyl)sulfonyl]propionic acid (0.22 g) obtained in Example100d) and 3-(4-piperidinyl)-5,6,7,8-tetrahydroimidazo[1,2-a]pyridine dihydrochloride (0.23 g) obtained in Example 209b), the title compound (0.26 g, 74%) was obtained in a similar manner to Example 89b).
NMR (200 MHz, CDCl₃) δ: 1.40-1.72 (2H, m), 1.82-2.05 (6H, m), 2.65 (1H, m), 2.83-2.94 (3H, m), 3.07-3.30 (3H, m), 3.53-3.61 (2H, m), 3.81 (2H, t, J = 5.8), 3.91 (1H, d, J = 13.4), 4.56 (1H, d, J = 13.4), 5.32 (2H, s), 6.65 (1H, s), 7.61 (1H, dd, J = 2.2, 8.8), 7.88-8.05 (4H, m), 8.50 (1H, s) .

### 100f) (6-{3-Oxo-3-[4-(5,6,7,8-tetrahydroimidazo[1,2-a]pyridin-3-yl)-1-piperidinyl]propyl}sulfonyl-2-naphthyl)methylamine dihydrochloride

A solution of 3-(1-{3-[(6-azidomethyl-2-naphthyl)sulfonyl]propanoyl}-4-piperidinyl)-5,6,7,8-tetrahydroimidazo[1,2-a]pyridine (0.25 g) obtained in Example 100e) and palladium-carbon (0.05 g) in methanol (5 mL) was stirred for 2 hours under hydrogen atmosphere. Insoluble substances were filtered off and the filtrate was concentrated. The residue was purified with a silica gel column and then treated with a saturated solution of hydrogen chloride in ethanol to obtain the title compound (0.27 g, quantitative).
NMR (200 MHz, DMSO-d₆) δ: 1.26 (1H, m), 1.44 (1H, m), 1.81-1.98 (6H, m), 2.58 (1H, m), 2.76 (2H, t, J = 6.9), 2.93-2.97 (3H, m), 3.08 (1H, t, J = 14.2), 3.88 (1H, d, J = 14.2), 4.03-4.07 (2H, m), 4.25-4.34 (3H, m), 7.33 (1H, s), 7.84 (1H, dd, J = 1.5, 8.7), 7.97 (1H, dd, J = 1.8, 8.7), 8.16-8.19 (2H, m), 8.27 (1H, d, J = 7.8), 8.63 (1H, s)

### Example 101

### 1-{3-[(6-Chloro-2-naphthyl)sulfonyl]propanoyl}-4-[(2-methyl-1H-imidazol-1-yl)methyl]piperidine hydrochloride

### 101a) Tert-butyl 4-[(2-methyl-1H-imidazol-1-yl)methyl]piperidine-1-carboxylate

Sodium hydride (60% in oil: 0.87 g) was added to a solution of 2-methylimidazole (1.8 g) and tert-butyl 4-(bromomethyl)piperidine-1-carboxylate (DeVita, Robert, J. et al., Bioorg. Med. Chem. Lett., 9, 261 (1999)) (8.5 g) in DMF (100 mL) and stirred at 80°C for 12 hours. The reaction solution was concentrated, and the residue was dissolved in chloroform, washed with an aqueous potassium carbonate solution, dried over anhydrous sodium sulfate, and then concentrated. The residue was purified with a silica gel column to obtain the title compound (0.43 g, 7%) as a light brown oil.
NMR (300 MHz, CDCl₃) δ: 1.13-1.28 (2H, m), 1.46 (9H, s), 1.57-1.63 (4H, m), 1.82 (1H, m), 2.37 (3H, s), 2.65 (2H, t, J = 12.9), 3.71 (2H, d, J = 7.5), 4.16 (2H, brs), 6.78 (1H, d, J = 1.5), 6.91 (1H, d, J = 1.5).

### 101b) 1-{3-[(6-chloro-2-naphthyl)sulfonyl]propanoyl}-4-[(2-methyl-1H-imidazol-1-yl)methyl]piperidine hydrochloride

From tert-butyl 4-[(2-methyl-1H-imidazol-1-yl)methyl]piperidine-1-carboxylate (0.42 g) obtained in Example101a) and 3-[(6-chloro-2-naphthyl)sulfonyl]propionic acid (0.45 g), the title compound (0.55 g, 74%)was obtained as a colorless solid in a similar manner to Example 81b). NMR (200 MHz, CDCl₃) δ: 1.00-1.24 (2H, m), 1.48 (1H, m),1.63-1.77 (4H, m), 2.37 (3H, s), 2.49 (1H, t, J = 13.2), 2.83-2.90 (2H, m), 2.98 (1H, t, J = 13.2), 3.47-3.60 (2H, m), 3.80-3.88 (3H, m), 4.49 (1H, d, J = 11.4), 6.79 (1H, s), 6.91 (1H, s), 7.59 (1H, dd, J = 1.8, 9.0), 7.90-7.96 (4H, m), 8.47 (1H, s)

### Example102

### 1-{3-[(6-Chloro-2-naphthyl)sulfonyl]propanoyl}-4-[2-(2-methyl-1H-imdazol-1-yl)ethyl]piperidine hydrochloride

### 102a) Tert-butyl 4-[2-(2-methyl-1H-imidazol-1-yl)ethyl]piperidine-1-carboxylate

From tert-butyl 4-(2-bromoethyl)piperidine-1-carboxylate (Dereck, B. et al., J. Med. Chem., 42, 4584 (1999)) (6.46 g) and 2-methylimidazole (0.93 g), the title compound (5.45 g, 80%) was obtained as a brown oil in a similar manner to Example 101a).
NMR (300 MHz, CDCl₃) δ: 1.13-1.28 (2H, m), 1.46 (9H, s), 1.57-1.63 (4H, m), 1.82 (1H, m), 2.37 (3H, s), 2.65 (2H, t, J = 12.9), 3.71 (2H, d, J = 7.5), 4.16 (2H, brs), 6.78 (1H, d, J = 1.5), 6.91 (1H, d, J = 1.5).

### 102b) 1-{3-[(6-chloro-2-naphthyl)sulfonyl]propanoyl}-4-[2-(2-methyl-1H-imidazol-1-yl)ethyl]piperidine hydrochloride

From tert-butyl 4-[2-(2-methyl-1H-imidazol-1-yl)ethyl]piperidine-1-carboxylate (0.43 g) obtained in Example 101a) and 3-[(6-chloro-2-naphthyl)sulfonyl]propionic acid (0.45 g), the title compound (0.52 g, 69%) was obtained as a colorless solid in a similar manner to Example 81b).
NMR (200 MHz, CDCl₃) δ: 1.00-1.24 (2H, m), 1.48 (1H, m), 1.63-1.77 (4H, m), 2.37 (3H, s), 2.49 (1H, t, J = 13.2), 2.83-2.90 (2H, m), 2.98 (1H, t, J = 13.2), 3.47-3.60 (2H, m), 3.80-3.88 (3H, m), 4.49 (1H, d, J = 11.4), 6.79 (1H, s), 6.91 (1H, s), 7.59 (1H, dd, J = 1.8, 9.0), 7.90-7.96 (4H, m), 8.47 (1H, s).
Elemental analysis for C₂₄H₂₈N₃O₃SCl·HCl·0.25H₂O
Calculated (%): C, 55.97; H, 5.77; N, 8.16.
Found (%): C, 55.98; H, 5.74; N, 8.16.

### Example 103

### 5-Chloro-2-(3-{4-[2-(2-methyl-1H-imidazol-1-yl)ethyl]-1-piperidinyl}-3-oxopropyl)sulfonyl-1H-indole

### 103a) Tert-butyl 5-chloro-2-(3-{4-[2-(2-methyl-1H-imidazol-1-yl)ethyl]-1-piperidinyl}-3-oxopropyl)sulfonyl-1H-indole-1-carboxylate

From tert-butyl 4-[(2-methyl-1H-imidazol-1-yl)ethyl]piperidine-1-carboxylate (0.59 g) obtained in Example 102a) and 3-[(1-tert-butoxycarbonyl-5-chloro-1H-indol-2-yl)sulfonyl]propionic acid (0.78 g) obtained in Example 211d), the title compound (0.64 g, 57%) was obtained in a similar manner to Example 85b).
NMR (200 MHz, CDCl₃) δ: 1.00-1.05 (2H, m), 1.53 (1H, m), 1.62-1.75 (4H, m), 1.73 (9H, m), 2.37 (3H, s), 2.50 (1H, t, J = 13.2), 2.87-3.04 (3H, m), 3.82-3.90 (3H, m), 4.00-4.08 (2H, m), 4.51 (1H, d, J = 12.8), 6.79 (1H, d, J = 1.0), 6.91 (1H, d, J = 1.0), 7.45 (1H, dd, J = 1.8, 8.8), 7.50 (1H, s), 7.65 (1H, d, J = 1.8) 7.99 (1H, d, J = 8.8).

### 103b) 5-chloro-2-(3-{4-[2-(2-methyl-1H-imidazol-1-yl)ethyl]-1-piperidinyl}-3-oxopropyl)sulfonyl-1H-indole

Trifluoroacetic acid (10 mL) was added to a solution of tert-butyl 5-chloro-2-(3-{4-[2-(2-methyl-1H-imidazol-1-yl)ethyl]-1-piperidinyl}-3-oxopropyl)sulfonyl-1H-indole-1-carboxylate (0.50 g) obtained in Example 103a) in dichloromethane (10 mL) and stirred at room temperature for 2 hours. The reaction solution was made alkaline with potassium carbonate and then extracted with chloroform. The extract was dried over anhydrous sodium sulfate and the solvent was distilled off to obtain the title compound (0.37 g, 90%).
NMR (200 MHz, DMSO-d₆) δ: 0.74-0.84 (2H, m), 1.38-1.75 (5H, m), 2.27 (3H, s), 2.40 (1H, t, J = 11.8), 2.70 (2H, t, J = 7.4), 2.89 (1H, t, J = 11.8), 3.57-3.70 (3H, m), 3.86 (2H, t, J = 7.4), 4.15 (1H, d, J = 11.8), 6.74 (1H, s,), 7.05 (1H, s), 7.14 (1H, s), 7.32 (1H, dd, J = 2.0, 9.0), 7.50 (1H, d, J = 9.0), 7.79 (1H, d, J = 2.0).

### Example 104

### 1-{3-[(6-Chloro-2-naphthyl)sulfonyl]propanoyl}-4-[3-(2-methyl-1H-imidazol-1-yl)propyl]piperidine hydrochloride

### 104a) Tert-butyl 4-[3-(2-methyl-1H-imidazol-1-yl)propyl]piperidine-1-carboxylate

From tert-butyl 4-(3-bromopropyl)piperidine-1-carboxylate (Siegel, M. G. et al., Tetrahedron, 55, 11619 (1999)) (2.0 g) and 2-methylimidazole (0.56 g), the title compound (2.05 g, quantitative) was obtained as a light brown oil in a similar manner to Example 101a).
NMR (200 MHz, CDCl₃) δ: 1.03-1.35 (4H, m), 1.45 (9H, s), 1.59-1.82 (5H, m), 2.37 (3H, s), 2.66 (2H, t, J = 12.4), 3.81 (2H, t, J = 7.0), 4.06 (2H, br), 6.80 (1H, d, J = 1.4), 6.90 (1H, d, J = 1.4).

### 104b) 1-{3-[(6-Chloro-2-naphthyl)sulfonyl]propanoyl}-4-[3-(2-methyl-1H-imidazol-1-yl)propyl]piperidine hydrochloride

From tert-butyl 4-[3-(2-methyl-1H-imidazol-1-yl)propyl]piperidine-1-carboxylate (0.61 g) obtained in Example 104a) and 3-[(6-chloro-2-naphthyl)sulfonyl]propionic acid (0.60 g) and 3-[(6-chloro-2-naphthyl)sulfonyl]propionic acid (0.60 g), the title compound (0.56 g, 53%) was obtained in a similar manner to Example 81b).
NMR (300 MHz, CDCl₃) δ: 1.00-1.31 (4H, m), 1.52 (1H, m), 1.65-1.88 (4H, m), 2.37 (3H, s), 2.49 (1H, t, J = 11.1), 2.83-2.90 (2H, m), 3.00 (1H, t, J = 11.1), 3.47-3.58 (2H, m), 3.84 (1H, d, J= 14.1), 4.02 (2H, t, J = 6.9), 4.51 (1H, d, J = 14.1), 7.05 (1H, s), 7.05 (1H, s), 7.28 (1H, s), 7.61 (1H, dd, J = 2.1, 9.0), 7.89-7.97 (4H, m), 8.47 (1H, s) .

### Example 105

### 1-{3-[(6-Chloro-2-naphthyl)sulfonyl]propanoyl}-4-[3-(2-ethyl-1H-imidazol-1-yl)propyl]piperidine hydrochloride

### 105a) Tert-butyl 4-[3-(2-ethyl-1H-imidazol-1-yl)propyl]piperidine-1-carboxylate

From tert-butyl 4-(3-bromopropyl)piperidine-1-carboxylate (2.0 g) and 2-ethylimidazole (0.66 g), the title compound (2.06 g, 98%) was obtained as a light brown oil in a similar manner to Example 101a).
NMR (200 MHz, CDCl₃) δ: 1.03-1.38 (6H, m), 1.45 (9H, s), 1.60-1.82 (3H, s), 2.61-2.72 (4H, m), 3.82 (2H, t, J = 7.0), 4.07 (2H, br), 6.80 (1H, d, J = 1.4), 6.94 (1H, d, J = 1.4).

### 105b) 1-{3-[(6-Chloro-2-naphthyl)sulfonyl]propanoyl}-4-[3-(2-ethyl-1H-imidazol-1-yl)propyl]piperidine hydrochloride

From tert-butyl 4-[3-(2-ethyl-1H-imidazol-1-yl)propyl]piperidine-1-carboxylate (0.61 g) obtained in Example 105a) and 3-[(6-chloro-2-naphthyl)sulfonyl]propionic acid (0.60 g), the title compound (0.50 g, 46%) was obtained in a similar manner to Example 81b).
NMR (300 MHz, CDCl₃) δ: 0.95-1.25 (2H, m), 1.20-1.27 (2H, m), 1.34 (3H, t, J = 7.5), 1.43 (1H, m), 1.64-1.76 (4H, m), 2.47 (1H, t, J = 13.2), 2.65 (2H, q, J = 7.5), 2.83-2.88 (2H, m), 2.97 (1H, t, J = 13.2), 3.52-3.58 (2H, m), 3.82 (2H, t, J = 7.2), 4.47 (1H, d, J = 13.5), 6.79 (1H, d, J = 1.0), 6.94 (1H, J = 1.0), 7.59 (1H, dd, J = 2.4, 8.7), 7.89-7.96 (4H, m), 8.47 (1H, s).

### Example 106

### 1-{3-[(6-Chloro-2-naphthyl)sulfonyl)propanoyl}-4-[2-(2-ethyl-1H-imidazol-1-yl)ethyl]piperidine hydrochloride

### 106a) Tert-butyl 4-[2-(2-ethyl-1H-imidazol-1-yl)ethyl]piperidine-1-carboxylate

From tert-butyl 4-(2-bromoethyl)piperidine-1-carboxylate (3.0 g) and 2-ethylimdazole (1.0 g), the title compound (1.55 g, 52%) was obtained as a light brown oil in a similar manner to Example 101a).
NMR (200 MHz, CDCl₃) δ: 1.18-1.38 (7H, m), 1.46 (9H, s), 1.63-1.74 (3H, m), 2.61-2.88 (4H, m), 3.87 (2H, t, J = 7.8), 4.08 (2H, d, J = 14.2), 6.80 (1H, d, J = 1.0), 6.94 (1H, d, J = 1.0).

### 106b) 1-{3-[(6-Chloro-2-naphthyl)sulfonyl]propanoyl}-4-[2-(2-ethyl-1H-imidazol-1-yl)ethyl]piperidine hydrochloride

From tert-butyl 4-[2-(2-ethyl-1H-imidazol-1-yl)ethyl]piperidine-1-carboxylate (0.58 g) obtained in Example 106a) and 3-[(6-chloro-2-naphthyl)sulfonyl]propionic acid (0.66 g), the title compound (0.29 g, 28%) was obtained in a similar manner to Example 81b).
NMR (300 MHz CDCl₃) δ: 1.02-1.27 (2H. m), 1.35 (3H, t, J = 7.2), 1.50 (1H, m), 1.64-1.84 (4H, m), 2.49 (1H, t, J = 10.8), 2.68 (2H, q, J = 7.2), 2.84-2.90 (2H, m), 2.99 (1H, t, J = 10.8), 3.50-3.59 (2H, m), 3.81-3.89 (3H, m), 4.50 (1H, J = 13.5), 6.79 (1H, d, J = 1.2), 6.95 (1H, d, J = 1.2), 7.59 (1H, dd, J = 1.5, 9.3), 7.89-7.94 (4H, m), 8.48 (1H, s).

### Example 107

### 5-Chloro-2-(3-{4-[2-(2-ethyl-1H-imidazol-1-yl)ethyl]-1-piperidinyl}-3-oxopropyl)sulfonyl-1H-indole

### 107a) Tert-butyl 5-chloro-2-(3-{4-[2-(2-ethyl-1H-imidazol-1-yl)ethyl]-1-piperidinyl}-3-oxopropyl)sulfonyl-1H-indole-1-carboxylate

From tert-butyl 4-(2-ethyl-1H-imidazol-1-yl)methylpiperidine-1-carboxylate (0.58 g) obtained in Example 106a) and 3-[(1-tert-butoxycarbonyl-5-chloro-1H-indol-2-yl)sulfonyl]propionic acid (0.85 g), the title compound (0.62 g, 54%) was obtained in a similar manner to Example 85b).
NMR (300 MHz, CDCl₃) δ: 1.05-1.21 (2H, m), 1.35 (3H, t, J = 7.5), 1.52 (1H, m), 1.53-1.73 (4H, m), 1.73 (9H, s), 2.49 (1H, t, J = 9.9), 2.65 (2H, q, J = 7.5), 2.88-3.03 (3H, m), 3.81-3.89 (3H, m), 4.02-4.08 (2H, m), 4.51 (1H, d, J = 13.2), 6.79 (1H, s), 6.95 (1H, s) 7.43-7.53 (2H, m), 7.63 (1H, m), 8.98 (1H, d, J = 9.0).

### 107b) 5-Chloro-2-(3-(4-[2-(2-ethyl-1H-imidazol-1-yl)ethyl]-1-piperidinyl}-3-oxopropyl)sulfonyl-1H-indole

From tert-butyl 5-chloro-2-(3-{4-[2-(2-ethyl-1H-imidazol-1-yl)ethyl]-1-piperidinyl}-3-oxopropyl)sulfonyl-1H-indole-1-carboxylate (0.60 g) obtained Example 107a), the title compound (0.45 g, 91%) was obtained in a similar manner to Example 103b).
NMR (200 MHz, DMSO-d₆) δ: 0.75-0.85 (2H, m), 1.19 (3H, t, J = 7.4), 1.49-1.62 (5H, m), 2.40 (1H, t, J = 11.8), 2.48-2.52 (2H, m), 2.57 (2H, q, J = 7.4), 2.70 (2H, t, J = 7.0), 2.89 (1H, t, J = 11.8), 3.57-3.76 (3H, m), 3.85 (2H, t, J = 7.0), 4.16 (1H, d, J = 11.8), 6.73 (1H, d, J = 1.4), 7.02 (1H, d, J = 1.4), 7.14 (1H, s), 7.32 (1H, dd, J = 1.8, 8.8), 7.51 (1H, d, J = 8.8), 7.90 (1H, d, J = 1.8).

### Example 108

### 1-{3-[(6-Chloro-2-naphthyl)sulfonyl]propanoyl}-4-[2-(4,5-dimethyl-1H-imidazol-1-yl)ethyl]piperidine

### 108a) Tert-butyl 4-[2-(4,5-dimethyl-1H-imidazol-1-yl)ethyl]piperidine-1-carboxylate

From 4,5-dimethylimidazole (JP-A 60-56961)(0.66 g) and tert-butyl 4-(2-bromoethyl)piperidine-1-carboxylate (2.0 g), the title compound (0.38 g, 18%) was obtained as a dark brown oil in a similar manner to Example 101a).
NMR (300 MHz, CDCl₃) δ: 1.07-1.91 (2H, m), 1.45 (9H, s), 1.59-1.70 (5H, m), 2.11 (3H, s), 2.15 (3H, s), 2.67 (2H, t, J = 11.4), 3.81 (2H, t, J = 7.0), 4.07-4.14 (2H, m), 7.30 (1H, s).

### 108b) 1-{3-[(6-Chloro-2-naphthyl)sulfonyl]propanoyl}-4-[2-(4,5-dimethyl-1H-imidazol-1-yl)ethyl]piperidine

From tert-butyl 4-[2-(4,5-dimethyl-1H-imidazol-1-yl)ethyl]piperidine-1-carboxylate (0.38 g) obtained in Example 108a) and 3-[(6-chloro-2-naphthyl)sulfonyl]propionic acid (0.37 g), the title compound (0.36 g,57%) was obtained in a similar manner to Example 85b).
NMR (300 MHz, CDCl₃) δ: 1.04-1.23 (2H, m), 1.48 (1H, m), 1.60-1.77 (4H, m), 2.11 (3H, s), 2.15 (3H, s), 2.49 (1H, t, J = 13.2), 2.83-2.90 (2H, m), 2.99 (1H, t, J = 13.2), 3.50-3.58 (2H, m), 3.79-3.84 (3H, m), 4.49 (1H, d, J = 13.2), 7.30 (1H, s), 7.59 (1H, dd, J = 2.4, 9.0), 7.89-7.94 (4H, m), 8.47 (1H, s).
Elemental analysis for C₂₅H₃₀N₃O₃SCl·0.5H₂O
Calculated (%) C, 60.41; H, 6.29; N, 8.45.
Found (%) C, 60.40; H, 6.42; N, 8.22

### Example 109

### 1-{3-[(6-Chloro-2-naphthyl)sulfonyl]propanoyl}-4-[2-(2,4,5-trimethyl-1H-imidazol-1-yl)ethyl]piperidine

### 109a) Tert-butyl 4-[2-(2,4,5-trimethyl-1H-imidazol-1-yl)ethyl]piperidine-1-carboxylate

From 2,4,5-trimethylimidazole (JP-A 60-56961)(1.8 g) and tert-butyl 4-(2-bromoethyl)piperidine-1-carboxylate (4.77 g), the title compound (1.18 g, 23%) was obtained as a dark brown oil in a similar manner to Example 101a).
NMR (300 MHz, CDCl₃) δ: 1.07-1.91 (2H, m), 1.46 (9H, s), 1.59-1.70 (5H, m), 2.08 (3H, s), 2.11 (3H, s), 2.15 (3H, s), 2.69 (2H, t, J = 11.4), 3.72 (2H, t, J = 7.0), 4.07-4.14 (2H, m).

### 109b) 1-{3-[(6-Chloro-2-naphthyl)sulfonyl]propanoyl}-4-[2-(2,4,5-trimethyl-1H-imidazol-1-yl)ethyl]piperidine

From tert-butyl 4-[2-(2,4,5-trimethyl-1H-imidazol-1-yl)ethyl]piperidine-1-carboxylate (0.73 g) obtained in Example 109a) and 3-[(6-chloro-2-naphthyl)sulfonyl]propionic acid (0.70 g), the title compound (0.39 g, 33%) was obtained in a similar manner to Example 85b).
NMR (300 MHz, CDCl₃) δ: 1.07-1.23 (2H, m), 1.51-1.55 (3H, m), 1.70-1.79 (2H, m), 2.07 (3H, s), 2.09 (3H, s), 2.31 (3H, s), 2.51 (1H, t, J = 12.6), 2.84-2.90 (2H, m), 2.97 (1H, t, J = 12.6), 3.50-3.57 (2H, m), 3.71 (2H, t, J = 7.8), 3.83 (1H, d, J = 13.2), 7.59 (1H, dd, J = 2.1, 9.0), 7.88-7.96 (4H, m), 8.47 (1H, s).

### Example 110

### 1-{3-((6-Chloro-2-naphthyl)sulfonyl]propanoyl}-4-((2-methyl-1H-imidazol-4-yl)methoxy]piperidine

### 110a) Tert-butyl 4-[(2-methyl-1-trityl-1H-imidazol-4-yl)methoxy]piperidine-1-carboxylate

Sodium hydride (60% in oil: 0.12 g) was added to a solution of tert-butyl 4-hydroxypiperidine-1-carboxylate (0.50 g) in DMF (20 mL) and stirred for 15 minutes. To the mixture, a solution of 4-chloromethyl-2-methyl-1-trityl-1H-imidazole (Cordi, A. A. et al., Eur. J. Med. Chem., 25, 557 (1990)) (0.93 g) in DMF (10 mL) was then added and was stirred for 12 hours. The reaction solution was then concentrated. The residue was dissolved in ethyl acetate, washed with an aqueous saturated sodium chloride solution, and then dried over anhydrous sulfate. The solvent was distilled off. The residue was purified with a silica gel column to obtain the title compound (0.58 g, 53%).
NMR (200 MHz, CDCl₃) δ: 1.45 (9H, s), 1.45-1.63 (2H, m), 1.72 (3H, s), 1.83-1.91 (2H, m), 2.92-3.05 (2H, m), 3.58 (1H, m), 3.79-3.90 (2H, m), 4.02 (2H, s), 6.68 (1H, s), 7.10-7.17 (6H, m), 7.26-7.39 (9H, m).

### 110b) 1-{3-[(6-chloro-2-naphthyl)sulfonyl]propanoyl}-4-[(2-methyl-1H-imidazol-4-yl)methoxy]piperidine

A solution of tert-butyl 4-[(2-methyl-1-trityl-1H-imidazol-4-yl)methoxy]piperidine-1-carboxylate (0.57 g) obtained in Example 110a) in methanol (10 mL)-1N hydrochloric acid (5 mL) was stirred at 80°C for 12 hours. The reaction solution was poured into a mixture of diethyl ether and water, and an aqueous layer was then separated and concentrated. To the residue were added DBU (0.32 mL) and triethylamine (0.44 mL), which was dissolved in acetonitrile (10 mL). This solution was added to a suspension of 3-[(6-chloro-2-naphthyl)sulfonyl]propionic acid (0.32 g), WSC (0.30 g) and HOBt (0.24 g) in acetonitrile (10 mL) and stirred for 12 hours. The reaction solution was concentrated under reduced pressure and the residue was dissolved in chloroform and an aqueous saturated sodium bicarbonate solution to separate a chloroform layer. The chloroform solution was dried over anhydrous sodium sulfate and the solvent was distilled off. The residue was purified with a silica gel column to obtain the title compound (0.27 g, 54%).

NMR (200 MHz, CDCl₃) δ: 1.65-1.84 (4H, m), 2.42 (3H, s), 2.86 (2H, t, J = 8.4), 3.18-3.26 (2H, m), 3.55 (2H, t, J = 8.4), 3.58-3.72 (2H, m), 3.83 (1H, m), 4.46 (2H, s), 6.87 (1H, s), 7.59 (1H, dd, J = 2.0, 9.0), 7.92-7.97 (4H, m), 8.47 (1H, s).

### Example 111

### 1-(3-[(6-Chloro-2-naphthyl)sulfonyl]propanoyl}-4-[2-(1-methyl-1H-imidazol-5-yl)ethyl]piperidine

### 111a) Tert-butyl 4-[(Z)-2-(1-trityl-1H-imidazol-4-yl)vinyl]piperidine-1-carboxylate

Potassium tert-butoxide (0.21 g) was added to a solution of 1-trityl-1H-imidazole-4-carbaldehyde (Kelly, J. L. et al., J. Med. Chem., 20, 721 (1977)) (0.63 g) and (1-tert-butoxycarbonyl-4-piperidinyl)methyl(triphenyl)phosphonium iodide (WO 99/24421)(1.0 g) in THF (35 mL). After stirred for 12 hours, the reaction solution was poured into a mixture of ethyl acetate and an aqueous saturated ammonium chloride solution to separate an organic layer. The ethyl acetate solution was dried over anhydrous sodium sulfate and the solvent was distilled off. The residue was purified with a silica gel column to obtain the title compound (0.45 g, 51%).
NMR (200 MHz, CDCl₃) δ: 1.20-1.36 (2H, m), 1.45 (9H, s), 1.64-1.69 (2H, m), 2.67 (2H, t, J = 12.2), 3.05 (1H, m), 4.01 (2H, d, J = 12.8), 5.32 (1H, dd, J = 9.0, 11.6), 6.16 (1H, d, J = 11.6), 6.69 (1H, s), 7.10-7.20 (6H, m), 7.30-7.36 (9H, m), 7.43 (1H, s).

### 111b) Tert-butyl 4-[2-(1-trityl-1H-imidazol-4-yl)ethyl]piperidine-1-carboxylate

A suspension of tert-butyl 4-[(Z)-2-(1-trityl-1H-imidazol-4-yl)vinyl]piperidine-1-carboxylate (0.43 g) obtained in Example 111a) and platinum oxide (0.08 g) in methanol (20 mL) was stirred at room temperature for 1 hour under hydrogen atmosphere. Insoluble substances were filtered off and the filtrate was concentrated to obtain the title compound (0.43 g, 99%).
NMR (200 MHz, CDCl₃) δ: 1.05-1.17 (2H, m), 1.45 (9H, s), 1.51-1.66 (5H, m), 2.52-2.68 (4H, m), 4.04 (2H, d, J = 13.5), 6.49 (1H, s), 7.11-7.18 (6H, s), 7.29-7.35 (10H, m).

### 111c) 1-{3-[(6-chloro-2-naphthyl)sulfonyl]propanoyl}-4-[2-(1-methyl-1H-imidazol-5-yl)ethyl]piperidine

Methyl iodide (1 mL) was added to a solution of tert-butyl 4-[2-(1-trityl-1H-imidazol-4-yl)ethyl]piperidine-1-carboxylate (0.43 g) obtained in Example 111b) in acetonitrile (10 mL) and stirred for 12 hours. The reaction solution was concentrated and the residue was dissolved in methanol (10 mL) and 1N hydrochloric acid (5 mL). After stirred at 80°C for 3 hours, the reaction solution was poured into a mixture of diethyl ether and water and an aqueous layer was then separated and concentrated. To the residue were added DBU (0.25 mL), triethylamine (0.35 mL) and acetonitrile (10 mL). This solution was added to a suspension of 3-[(6-chloro-2-naphthyl)sulfonyl]propionic acid (0.25 g), WSC (0.24 g) and HOBt (0.19 g) in acetonitrile (10 mL) and stirred for 12 hours. The reaction solution was concentrated under reduced pressure and the residue was dissolved in chloroform and an aqueous saturated sodium bicarbonate solution to separate a chloroform layer. The chloroform solution was dried over anhydrous sodium sulfate and the solvent was distilled off. The residue was purified with a silica gel column to obtain the title compound (0.29 g, 74%).
NMR (200 MHz, CDCl₃) δ: 1.05-1.28 (2H, m), 1.57-1.66 (3H, m), 1.73-1.86 (2H, m), 2.53-2.60 (2H, m), 2.85-3.07 (3H, m), 3.53-3.62 (2H, m), 3.56 (3H, s), 3.85 (1H, d, J = 13.8), 4.51 (1H, d, J = 13.8), 6.78 (1H, s), 7.40 (1H, s), 7.51 (1H, dd, J = 1.8, 8.8), 7.90-7.99 (4H, m), 8.50 (1H, s). Elemental analysis for C₂₄H₂₈N₃O₃SCl·0.5H₂O
Calculated (%) C, 59.68; H, 6.05; N, 8.70.
Found (%) C, 59.58; H, 6.14; N, 8.43.

### Example 112

### 1-{3-[(6-Chloro-2-naphthyl)sulfonyl]propanoyl}-4-[(Z)-2-(2-methyl-1H-imidazol-4-yl)ethenyl]piperidine

### 112a) Tert-butyl 4-[(Z)-2-(2-methyl-1-trityl-1H-imidazol-4-yl)ethenyl]piperidine-1-carboxylate

From 2-methyl-1-trityl-1H-imidazole-4-carbaldehyde (EP 451538 (1991)) (3.3 g) and (1-tert-butoxycarbonyl-4-piperidinyl)methyl(triphenyl)phosphonium iodide (5.0 g), the title compound (1.0 g, 22%) was obtained as colorless powder in a similar manner to Example 111a).
NMR (200 MHz, CDCl₃) δ: 1.20-1.31 (2H, m), 1.46 (3H, s), 1.56-1.63 (2H, m), 2.49-2.62 (3H, m), 3.97 (2H, d, J = 12.4), 5.28 (1H, dd, J = 8.8, 11.8), 6.18 (1H, d, J = 11.8), 6.61 (1H, s), 7.13-7.20 (6H, m), 7.30-7.37 (9H, m).

### 112b) 1-{3-[(6-chloro-2-naphthyl)sulfonyl]propanoyl}-4-[(Z)-2-(2-methyl-1H-imidazol-4-yl)ethenyl]piperidine

From tert-butyl 4-[(Z)-2-(2-methyl-1-trityl-1H-imidazol-4-yl)ethenyl]piperidine-1-carboxylate (0.39 g) obtained in Example 112a) and 3-[(6-chloro-2-naphthyl)sulfonyl]propionic acid (0.22 g), the title compound (0.25 g, 73%) was obtained as colorless powder in a similar manner to Example 85b).
NMR (300 MHz, CDCl₃) δ: 0.94-1.14 (2H, m), 1.19-1.26 (2H, m), 1.42 (1H, m), 1.64-1.76 (4H, m), 2.37 (3H, s), 2.47 (1H, m), 2.83-2.88 (2H, m), 2.96 (1H, m), 3.52-3.58 (2H, m), 3.79-3.84 (3H, m), 4.47 (1H, d, J = 12.8), 6.79 (1H, d, J = 1.2), 6.91 (1H, d, J = 1.2), 7.59 (1H, dd, J = 1.8, 8.7), 7.89-7.96 (4H, m), 8.47 (1H, s).

### Example 113

### 1-{3-[(6-Chloro-2-naphthyl)sulfonyl]propanoyl}-4-[(Z)-2-(1,2-dimethyl-1H-imidazol-5-yl)ethenyl)piperidine

From tert-butyl 4-[(Z)-2-(2-methyl-1-trityl-1H-imidazol-4-yl)ethenyl]piperidine-1-carboxylate (0.50 g) obtained in Example 112a), the title compound (0.16 g, 35%) was obtained as colorless powder in a similar manner to Example 111c).
NMR (200 MHz, CDCl₃) δ: 1.22-1.35 (2H, m), 1.65-1.82 (3H, m), 2.39 (3H, s), 2.59 (1H, t, J = 11.2), 2.82-2.92 (2H, m), 3.08 (1H, t, J = 11.2), 3.45 (3H, s), 3.52-3.62 (2H, m), 3.82 (1H, d, J = 11.2), 4.47 (1H, d, J= 11.8), 5.44 (1H, d, J = 9.6, 11.4), 6.05 (1H, d, J = 11.4), 6.88 (1H, s), 7.59 (1H, dd, J = 2.2, 11.2), 7.92-7.97 (4H, m), 8.48 (1H, s). Elemantal analysis for C₂₄H₂₆N₃O₃SCl·H₂O
Calculated (%) C, 58.83; H, 5.76; N, 8.58.
Found (%) C, 59.01; H, 5.46; N, 8.84.

### Example 114

### 1-{3-[(6-Chloro-2-naphthyl)sulfonyl]propanoyl}-4-[(1,2-dimethyl-1H-imidazol-5-yl)methoxy]piperidine

From tert-butyl 4-[(2-methyl-1-trityl-1H-imidazol-4-yl)methoxy]piperidine-1-carboxylate (0.50 g) obtained in Example 110a), the title compound (0.20 g, 44%) was obtained as colorless powder in a similar manner to Example 111c).
NMR (200 MHz, CDCl₃) δ: 1.42-1.61 (2H, m), 1.70-1.84 (2H, m), 2.37 (3H, s), 2.84-2.89 (2H, m), 3.18-3.28 (2H, m), 3.53 (3H, s), 3.51-3.67 (4H, m), 3.77-3.84 (1H, m), 4.46 (2H, s), 6.85 (1H, s), 7.58(1H, dd, J = 1.8, 8.7), 7.88-7.95 (4H, m), 8.46 (1H, s).
Elemental analysis for C₂₄H₂₈N₃0₄SCl·0.25H₂O
Calculated (%) C, 58.29; H, 5.81; N, 8.50.
Found (%) C, 58.29; H, 5.67; N, 8.56.

### Example 115

### 1-{3-[(6-Chloro-2-naphthyl)sulfonyl]propanoyl}-4-[2-(2-methyl-1H-imidazol-4-yl)ethyl]piperidine

A solution of 1-13-[(6-chloro-2-naphthyl)sulfonyl]propanoyl}-4-[(Z)-2-(2-methyl-1H-imidazol-4-yl)ethenyl]piperidine (0.1 g) obtained in Example 112b) and platinum oxide (0.01 g) in THF (2 mL) was stirred for 6 hours under hydrogen atmosphere. Insoluble substances were filtered off and the filtrate was concentrated. The residue was purified with a silica gel column to obtain the title compound (0.06 g, 60%) as colorless powder.
NMR (300 MHz, CDCl₃) δ: 0.98-1.15 (2H, m), 1.53-1.60 (3H, m), 1.69-1.81 (2H, m), 2.39 (3H, s), 2.41-2.57 (3H, m), 2.82-2.88 (2H, m), 3.01 (1H, dt, J = 2.7, 13.2), 3.52-3.58 (2H, m), 3.78 (1H, d, J = 13.5), 4.44 (1H, d, J = 13.5), 6.60 (1H, s), 7.60 (1H, dd, J = 1.8, 8.7), 7.88-7.96 (4H, m), 8.47 (1H, s).

### Example 116

### 1-{3-[(6-Chloro-2-naphthyl)sulfonyl]propanoyl}-4-[2-(1,2-dimethyl-1H-imidazol-5-yl)ethyl]piperidine

From 1-{3-[(6-chloro-2-naphthyl)sulfonyl]propanoyl}-4-[(Z)-2-(1,2-dimethyl-1H-imidazol-5-yl)ethenyl]piperidine (0.5 g) obtained in Example 113), the title compound (0.3 g, 60%) was obtained as colorless powder in a similar manner to Example 115).
NMR (300 MHz, CDCl₃) δ: 1.02-1.19 (2H, m), 1.53-1.57 (3H, m), 1.76 (2H, dd, J = 15.9, 19.2), 2.36 (3H, s), 2.45-2.53 (3H, m), 2.84-2.90 (2H, m), 2.98 (1H, m), 3.41 (3H, s), 3.53-3.59 (2H, m), 3.84 (1H, d, J = 13.5), 4.48 (1H, d, J = 13.5), 6.14 (1H, s), 7.59 (1H, dd, J = 2.4, 9.3), 7.89-7.96 (4H, m), 8.48 (1H, s).
Elemental analysis for C₂₅H₃₀N₃O₃SCl·0.5H₂O
Calculated (%) C, 60.41; H, 6.29; N, 8.45.
Found (%) C, 60.40; H, 6.12; N, 8.24.

### Example 117

### 1-[2-(1-{3-[(6-Chloro-2-naphthyl)sulfonyl]propanoyl}-4-piperidinyl)ethyl]-2-methyl-4,5,6,7-tetrahydro-1H-benzimidazole

### 117a) Tert-butyl 4-[2-(2-methyl-4,5,6,7-tetrahydro-1H-benzimidazol-1-yl)ethyl]piperidine-1-carboxylate

From 2-methyl-4,5,6,7-tetrahydro-1H-benzimidazole (JP-A 49-31666) (0.93 g) and tert-butyl 4-(2-bromoethyl)piperidine-1-carboxylate (2.0 g), the title compound (0.65 g, 27%) was obtained as a dark brown oil in a similar manner to Example 101a).
NMR (200 MHz, CDCl₃) δ: 1.13-1.19 (2H, m), 1.45 (9H, s), 1.50-1.80 (9H, m), 2.33 (3H, s), 2.43-2.60 (4H, m), 2.60-2.78 (2H, m), 3.71 (2H, t, J = 7.2), 4.06 (2H, d, J = 14.2).

### 117b) 1-[2-(1-{3-[(6-chloro-2-naphthyl)sulfonyl]propanoyl}-4-piperidine)ethyl]-2-methyl-4,5,6,7-tetrahydro-1H-benzimidazole

From tert-butyl 4-[2-(2-methyl-4,5,6,7-tetrahydro-1H-benzimidazol-1-yl)ethyl]piperidine-1-carboxylate (0.57 g) obtained in Example 117a) and 3-[(6-chloro-2-naphthyl-)sulfonyl]propionic acid (0.56 g), the title compound (0.60 g, 61%) was obtained in a similar manner to Example 85b). NMR (300 MHz, CDCl₃) δ: 10.7-1.19 (2H, m), 1.54-1.81 (10H, m), 2.34 (3H, s), 2.42-2.45 (2H, m), 2.52-2.56 (2H, m), 2.87 (2H, dt, J = 3.0, 7.2), 3.01 (1H, dt, J = 2.7, 13.5), 3.52-3.60 (2H, m), 3.72 (2H, t, J = 7.5), 3.84 (1H, d, J = 12.9), 4.50 (1H, d, J = 12.9), 7.59 (1H, dd, J = 2.4, 9.0), 7.89-7.96 (4H, m), 8.48 (1H, s).

### Example 118

### 1-(2-{1-{3-[(6-Chloro-2-naphthyl)sulfonyl]propanoyl}-4-piperidinyl}ethyl)-4,5,6,7-tetrahydro-1H-benzimidazole

### 118a) Tert-butyl 4-[2-(4,5,6,7-tetrahydro-1H-benzimidazol-1-yl)ethyl]piperidine-1-carboxylate

From 4,5,6,7-tetrahydro-1H-benzimidazole (WO 99/25710) (0.84 g) and tert-butyl 4-(2-bromoethyl)piperidine-1-carboxylate (2.0 g), the title compound (1.37 g, 60%) was obtained as a dark brown oil in a similar manner to Example 101a).
NMR (200 MHz, CDCl₃) δ: 1.13-1.19 (2H, m), 1.44 (9H, s), 1.50-1.80 (9H, m), 2.43-2.728 (6H, m), 3.80 (2H, t, J = 7.2), 4.06 (2H, d, J = 14.2), 7.29 (1H, s).

### 118b) 1-(2-{1-{3-[(6-chloro-2-naphthyl)sulfonyl]propanoyl}-4-piperidinyl}ethyl)-4,5,6,7-tetrahydro-1H-benzimidazole

From tert-butyl 4-[2-(4,5,6,7-tetrahydro-1H-benzimidazol-1-yl)ethyl]piperidine-1-carboxylate (0.54 g) obtained in Example 118a) and 3-[(6-chloro-2-naphthyl)sulfonyl]propionic acid (0.56 g), the title compound (0.46 g, 47%) was obtained in a similar manner to Example 85b).
NMR (200 MHz, CDCl₃) δ: 1.07-1.16 (2H, m), 1.48 (1H, m), 1.59-1.82 (9H, m), 2.45-2.48 (2H, m), 2.58-2.61 (2H, m), 2.83-2.90 (2H, m), 2.99 (1H, m), 3.13-3.25 (3H, m), 3.52-3.58 (2H, m), 3.81 (3H, m), 4.49 (1H, d, J = 13.2), 7.30 (1H, s), 7.59 (1H, dd, J = 2.1, 9.0), 7.89-7.96 (4H, m), 8.47 (1H, s).

### Example 119

### 1-{3-[(6-Chloro-2-naphthyl)sulfonyl]propanoyl}-4-[2-(1,4-dimethyl-1H-imidazol-5-yl)ethyl]piperidine

### 119a) Tert-butyl 4-[(Z)-2-(5-methyl-1-trityl-1H-imidazol-4-yl)ethenyl]piperidine-1-carboxylate

From 5-methyl-1-trityl-1H-imidazole-4-carbaldehyde (Yuan, W. et al., J. Med. Chem., 36, 211 (1993)) (3.3 g) and (1-tert-butoxycarbonyl-4-piperidinyl)methyl(triphenyl)phosphonium iodide (5.0 g), the title compound (2.55 g, 56%) was obtained as colorless powder in a similar manner to Example 111a).
NMR (300 MHz, CDCl₃) δ: 1.22-1.39 (2H, m), 1.45 (3H, s), 1.46 (9H, s), 1.77-1.82 (2H, m), 2.80-2.88 (2H, m) 3.66 (1H, m), 4.08 (2H, br), 5.29 (1H, dd, J = 9.3, 11.7), 6.04 (1H, d, J = 11.7), 7.13-7.17 (6H, m), 7.27-7.36 (9H, m).

### 119b) Tert-butyl 4-[2-(1-trityl-5-methyl-1H-imidazol-4-yl)ethyl]piperidine-1-carboxylate

From tert-butyl 4-[(Z)-2-(5-methyl-1-trityl-1H-imidazol-4-yl)ethenyl]piperidine-1-carboxylate (1.5 g) obtained in Example 119a), the title compound (1.5 g, 99%) was obtained as colorless powder in a similar manner to Example 111b).
NMR (300 MHz, CDCl₃) δ: 1.22-1.39 (2H, m), 1.45 (3H, s), 1.46 (9H, s), 1.77-1.82 (2H, m), 2.80-2.88 (2H, m), 3.66 (1H, m), 4.08 (2H, br), 5.29 (1H, dd, J = 9.3, 11.7), 6.04 (1H, d, J = 11.7), 7.13-7.17 (6H, m), 7.27-7.36 (9H, m).

### 119c) 1-{3-[(6-chloro-2-naphthyl)sulfonyl]propanoyl}-4-[2-(1,4-dimethyl-1H-imidazol-5-yl)ethyl]piperidine

From tert-butyl 4-[2-(1-trityl-5-methyl-1H-imidazol-4-yl)ethyl]piperidine-1-carboxylate (0.75 g) obtained in Example 119b), the title compound (0.22 g, 33%) was obtained as colorless powder in a similar manner to Example 111c) .
NMR (300 MHz, CDCl₃) δ: 0.99-1.12 (2H, m), 1.37-1.52 (3H, m), 1.72-1.81(4H, m), 2.15 (3H, s), 2.45-2.56 (3H, m), 2.84-2.90 (2H, dt, J = 4.2, 6.9), 2.99 (1H, dt, J = 3.0, 12.3), 3.51 (3H, s), 3.55 (2H, dt, J = 4.2, 6.9), 3.83 (1H, d, J = 13.2), 7.28 (1H, s), 7.59 (1H, dd, J = 2.1, 9.0), 7.89-7.94 (4H, m), 8.48 (1H, s).
Elemental analysis for C₂₅H₃₀N₃O₃SCl·0.75H₂O
Calculated (%) C, 59.87; H, 6.33; N, 8.38.
Found (%) C, 60.01; H, 6.33; N, 8.38.

### Example 120

### 1-{3-[(6-Chloro-2-naphthyl)sulfonyl]propanoyl}-4-[2-(5-methyl-1H-imidazol-4-yl)ethyl]piperidine

From tert-butyl 4-[2-(1-trityl-5-methyl-1H-imidazol-4-yl)ethyl]piperidine-1-carboxylate (0.66 g) obtained in Example 119b), the title compound (0.33 g, 57%) was obtained as colorless powder in a similar manner to Example 110b) .
NMR (300 MHz, CDCl₃) δ: 0.99-1.16 (2H, m), 1.51-1.59 (3H, m), 1.70-1.81 (2H, m), 2.18 (3H, s), 2.44-2.55 (3H, m), 2.83-2.88 (2H, m), 2.98 (1H, dt, J = 2.4, 12.6), 3.53-3.59 (2H, m), 3.79 (1H, d, J = 13.5), 4.45 (1H, d, J = 13.5), 7.44 (1H, s), 7.59 (1H, dd, J = 1.8, 8.7), 7.88-7.96 (4H, m), 8.47 (1H, s).
Elemental analysis for C₂₄H₂₈N₃O₃SCl·0.5H₂O
Calculated (%) C, 59.68; H, 6.05; N, 8.70.
Found (%) C, 60.05; H, 6.24; N, 8.90.

### Example 121

### 1-{3-[(6-Chloro-2-naphthyl)sulfonyl]propanoyl}-4-[(Z)-2-(1-methyl-1H-imidazol-2-yl)ethenyl]piperidine

### 121a) Tert-butyl 4-[(Z)-2-(1-methyl-1H-imidazol-2-yl)ethenyl]piperidine-1-carboxylate

From 1-methylimidazole-2-carbaldehyde (0.5 g) and (1-tert-butoxycarbonyl-4-piperidinyl)methyl(triphenyl)phosphonium iodide (4.0 g), the title compound (0.72 g, 54%) was obtained as colorless powder in a similar manner to Example 111a).
NMR (200 MHz, CDCl₃) δ: 1.22-1.34 (3H, m), 1.46 (9H, s), 1.72-1.79 (2H, m), 2.86 (2H, t, J = 12.8), 3.61 (3H, s), 4.03-4.14 (2H, m), 5.63 (1H, dd, J = 9.6, 11.8), 6.10 (1H, d, J = 11.8), 6.82 (1H, d, J = 1.0), 7.07 (1H, d, J = 1.0).

### 121b) 1-{3-[(6-chloro-2-naphthyl)sulfonyl]propanoyl)-4-[(Z)-2-(1-methyl-1H-imidazol-2-yl)ethenyl]piperidine

From tert-butyl 4-[(Z)-2-(1-methyl-1H-imidazol-2-yl)vinyl]piperidine-1-carboxylate (0.40 g) obtained in Example 121a) and 3-[(6-chloro-2-naphthyl)sulfonyl]propionic acid (0.45 g), the title compound (0.45 g, 71%) was obtained as colorless powder in a similar manner to Example 85b).
NMR (300 MHz, CDCl₃) δ: 1.14-1.34 (2H, m), 1.75-1.91 (3H, m), 2.67 (1H, dt, J = 3.0. 12.9), 2.83-2.90 (2H, m), 3.17 (1H, dt, J = 3.0, 12.9), 3.49-3.59 (2H, m), 3.62 (3H, s), 3.76 (1H, m), 4.24 (1H, d, J = 13.5), 5.55 (1H, dd, J = 9.3, 11.7), 6.11 (1H, d, J = 11.7), 6.83 (1H, d, J = 1.2), 7.06 (1H, d, J = 1.2), 7.59 (1H, dd, J = 1.8, 8.7), 7.89-7.96 (4H, m), 8.48 (1H, s).
Elemental analysis for C₂₄H₂₆N₃O₃SCl·•0.25H₂O
Calculated (%) C, 60.49; H, 5.61; N, 5.83.
Found (%) C, 60.67; H, 5.85; N, 8.59.

### Example 122

### 1-{3-[(6-Chloro-2-naphthyl)sulfonyl]propanoyl}-4-[2-(1-methyl-1H-imidazol-2-yl)ethyl]piperidine

From 1-{3-[(6-chloro-2-naphthyl)sulfonyl]propanoyl}-4-[(Z)-2-(1-methyl-1H-imidazol-2-yl)ethenyl]piperidine (0.20 g) obtained in Example 121b), the title compound (0.19 g, 95%) was obtained as colorless powder in a similar manner to Example 115).
NMR (300 MHz, CDCl₃) δ: 1.02-1.16 (2H, m), 1.58-1.86 (5H, m), 2.52 (1H, dt, J = 2.7, 12.9), 2.67 (2H, t, J = 8.1), 2.83-2.88 (2H, m), 3.00 (1H, dt, J = 2.7, 12.9), 3.53-3.59 (2H, m), 3.57 (3H, s), 3.81 (1H, d, J = 13.2), 4.46 (1H, d, J = 13.2), 6.79 (1H, d, J = 1.5), 6.91 (1H, d, J = 1.5), 7.59 (1H, dd, J = 1.8, 8.7), 7.89-7.96 (4H, m), 8.48 (1H, s) .
Elemental analysis for C₂₄H₂₈N₃O₃SCl·0.3H₂O
Calculated (%) C, 60.13; H, 6.01; N, 8.76.
Found (%) C, 60.21; H, 6.06; N, 8.46.

### Example 123

### 5-(1-{3-[(6-Chloro-2-naphthyl)sulfonyl]propanoyl}-4-piperidinyl)imidazo[2,1-b][1,3]thiazole

### 123a) Tert-butyl 4-(imidazo[2,1-b][1,3]thiazol-5-yl)piperidine-1-carboxylate

From tert-butyl 4-(1-bromo-2-oxoethyl)piperidine-1-carboxylate (2.0 g) and 2-aminothiazole (0.77 g), the title compound (0.55 g, 23%) was obtained in a similar manner to Example 81a).
NMR (200 MHz, CDCl₃) δ: 1.48 (9H, s), 1.55-1.76 (2H, m), 1.96-2.05 (2H, m), 2.82-2.95 (3H,m), 4.19-4.26 (2H, m), 6.85 (1H, d, J = 4.4), 7.03 (1H, s), 7.33 (1H, d, J = 4.4). 123b) 5-(1-{3-[(6-chloro-2-naphthyl)sulfonyl]propanoyl}-4-piperidinyl)imidazo[2,1-b][1,3]thiazole

From tert-butyl 4-(imidazo[2,1-b][1,3]thiazol-5-yl)piperidine-1-carboxylate (0.55 g) obtained in Example 123a), the title compound (0.48 g, 55%) was obtained as colorless powder in a similar manner to Example 85b).
NMR (300 MHz, CDCl₃) δ: 1.52-1.77 (2H, m), 2.00-2.12 (2H, m), 2.75 (1H, t, J = 15.6), 2.90-3.05 (3H, m), 3.22 (1H, t, J = 15.6), 3.53-3.62 (2H, m), 3.97 (1H, d, J = 13.8), 4.59 (1H, d, J = 13.8), 6.86 (1H, d, J = 4.5), 7.01 (1H, s), 7.34 (1H, d, J = 4.5), 7.59 (1H, dd, J = 2.1, 9.0), 7.90-7.97 (4H, m), 8.49 (1H, s).

### Example 124

### 1-{3-[(6-Chloro-2-naphthyl)sulfonyl]propanoyl}-4-[4-(2-methyl-1H-imidazol-1-yl)butyl]piperidine

### 124a) Tert-butyl 4-[4-(2-methyl-1H-imidazol-1-yl)butyl]piperidine-1-carboxylate

Tert-butyl 4-(4-bromobutyl)piperidine-1-carboxylate (Egbertson, M. S. et al., J. Med. Chem., 37, 2537 (1994)) (2.0 g) and 2-methylimidazole (0.56 g), the title compound (0.94 g, 47%) was obtained as a light brown oil in a similar manner to Example 101a).
NMR (200 MHz, CDCl₃) δ: 1.03-1.35 (4H, m), 1.45 (9H, s), 1.59-1.82 (7H, m), 2.37 (3H, s), 2.66 (2H, t, J = 12.4), 3.81 (2H, t, J = 7.0), 4.06 (2H, m), 6.80 (1H, d, J = 1.4), 6.90 (1H, d, J = 1.4).

### 124b) 1-{3-[(6-chloro-2-naphthyl)sulfonyl]propanoyl}-4-[4-(2-methyl-1H-imidazol-1-yl)butyl]piperidine

From tert-butyl 4-[4-(2-methyl-1H-imidazol-1-yl)butyl]piperidine-1-carboxylate (0.93 g) obtained in Example 124a), the title compound (0.1 g, 15%) was obtained as colorless powder in a similar manner to Example 85b). NMR (200 MHz, CDCl₃) δ: 0.91-1.08 (2H, m), 1.22-1.43 (5H, m), 1.61-1.74 (4H, m), 2.37 (3H, s), 2.40-2.54 (1H, m), 2.81-3.02 (3H, m), 3.52-3.60 (2H, m), 3.78-3.85 (3H, m), 4.26 (1H, d, J = 13.2), 6.79 (1H, d, J = 1.4), 6.90 (1H, d, J = 1.4), 7.58 (1H, dd, J = 2.0, 8.8), 7.92-7.97 (4H, m), 8.47 (1H, s).
Elemental analysis for C₂₆H₃₂N₃O₃SCl·0.5H₂O
Calculated (%) C, 61.10; H, 6.51; N, 8.22.
Found (%) C, 61.21; N, 6.57 ; N, 7.95.

### Example 125

### 2-(1-{3-[(6-Bromo-2-naphthyl)sulfonyl]propanoyl}-4-piperidinyl)-5-methyl-1,2-dihydro-3H-imidazo[1,5-c]imidazol-3-one

From 3-[(6-bromo-2-naphthyl)sulfonyl]propionic acid (0.17 g) and 5-methyl-2-(4-piperidinyl)-1,2-dihydro-3H-imidazo[1,5-c]imidazol-3-one (0.11 g) obtained in Example 69b), the title compound (0.11 g, 40%) was obtained as a white crystal in a similar manner to Example 89b).
NMR (300 MHz, CDCl₃) δ: 1.62-1.99 (4H, m), 2.61 (3H, s), 2.61-2.67 (1H, m), 2.82-3.01 (2H, m), 3.15-3.23 (1H, m), 3.46-3.65 (2H, m), 3.97-4.02 (1H, m), 4.08-4.21 (1H, m), 4.25 (2H, s), 4.69-4.74 (1H, m), 6.70 (1H, t, J = 1.5), 7.72 (1H, dd, J = 2.1, 8.7), 7.84-7.96 (3H, m), 8.13 (1H, d,
J = 1.8), 8.47 (1H, s).
Elemental analysis for C₂₄H₂₅BrN₄O₄S·0.3H₂O
Calculated (%): C, 52.33; H, 4.68; N, 10.17
Found (%): C, 52.57; H, 4.66; N, 9.88

### Example 126

### 2-(1-{3-[(6-Chloro-2-naphthyl)sulfonyl]propanoyl}-4-piperidinyl)-5-methyl-1H-imidazo[1,5-c]imidazol-1,3(2H)-dione

### 126a) Benzyl 4-{[(2-methyl-1H-imidazol-4-yl)carbonyl]amino}piperidin-1-carboxylate

WSC (4.0 g), HOBt (3.2 g) and triethylamine (3.8 mL) were added to a solution of benzyl 4-aminopiperidine-1-carboxylate (3.3 g) and 2-methyl-1H-imidazole-4-carboxylic acid (2.0 g) in acetonitrile (50 mL) and stirred for 12 hours. The reaction solution was concentrated and the residue was dissolved in chloroform and an aqueous saturated sodium bicarbonate solution. A chloroform layer was separated, dried over anhydrous sodium sulfate, and then concentrated under reduced pressure. The residue was purified with silica gel column chromatography to obtain the title compound (2.4 g, 49%) as colorless powder.
NMR (300 MHz, CDCl₃) δ: 1.41-1.56 (2H, m), 1.93-2.06 (3H, m), 2.40 (3H, s), 2.92-3.07 (2H, m), 4.06-4.21 (2H, m), 5.14 (2H, s), 7.07 (1H, d, J = 8.3), 7.30-7.42 (5H, m), 10.92 (1H, br)

### 126b) Benzyl 4-(5-methyl-1,3-dioxo-1H-imidazo[1,5-c]imidazol-2(3H)-yl)piperidine-1-carboxylate

N,N'-carbonyldiimidazole (1.65 g) and DBU (3.0 mL) were added to a solution of benzyl 4-{[(2-methyl-1H-imidazol-4-yl)carbonyl]amino}piperidine-1-carboxylate (1.16 g) obtained in Example 126a) in 1,2-dichloroethane (20 mL) and stirred for 3 hours under reflux. The reaction solution was diluted with chloroform, washed successively with water and an aqueous saturated sodium chloride solution, dried over anhydrous sodium sulfate, and then concentrated. The residue was purified with a silica gel column to obtain the title compound (0.43 g, 34%) as a colorless solid.
NMR (300 MHz, CDCl₃) δ: 1.69-1.81 (2H, m), 2.27-2.42 (2H, m), 2.67 (3H, s), 2.77-2.92 (2H, m), 4.14 (1H, m), 4.29-4.45 (2H, m), 5.15 (2H, s), 7.31-7.40 (6H, m)

### 126c) 2-(1-{3-[(6-Chloro-2-naphthyl)sulfonyl]propanoyl}-4-piperidinyl)-5-methyl-1H-imidazo[1,5-c]imidazol-1,3(2H)-dione

A 25% solution of hydrogen bromide in acetic acid (10 mL) was added to benzyl 4-(5-methyl-1,3-dioxo-1H-imidazo[1,5-c]imidazol-2(3H)-yl)piperidine-1-carboxylate (0.38 g) obtained in Example 126b) and stirred for 1 hour. The reaction solution was concentrated, and the residue was dissolved together with triethylamine (0.53.mL) in acetonitrile (20 mL). This solution was added to a suspension of 3-[(6-chloro-2-naphthyl)sulfonyl]propionic acid (0.38 g), WSC (0.37 g) and HOBt (0.29 g) in acetonitrile (20 mL) and stirred for 12 hours. The reaction solution was concentrated and the residue was dissolved in chloroform and an aqueous saturated sodium bicarbonate solution. A chloroform layer was separated, dried over anhydrous sodium sulfate, and then concentrated under reduced pressure. The residue was purified with a silica gel column to obtain the title compound (0.42 g, 65%) as a colorless solid.
NMR (300 MHz, CDCl₃) δ: 1.74-1.90 (2H, m), 2.22-2.33 (2H, m), 2.55 (1H, m), 2.67 (3H, s), 2.88-3.02 (2H, m), 3.11 (1H, m), 3.52-3.67 (2H, m), 4.00 (1H, m), 4.12 (1H, m), 4.69 (1H, m), 7.34 (1H, s), 7.60 (1H, dd, J = 2.1, 8.7), 7.90-8.01 (4H, m), 8.4.9 (1H, s).
Elemental analysis for C₂₄H₂₃N₄O₅SCl·0.1iPr₂O
Calculated (%): C, 56.26; H, 4.68; N, 11.67
Found (%): C, 56.41; H, 4.79; N, 11.03

### Example 127

### 2-(1-{3-[(7-Chloro-2H-chromen-3-yl)sulfonyl]propanoyl}-4-piperidinyl)-5-methyl-1,2-dihydro-3H-imidazo[1,5-c]imidazol-3-one

Triethylamine (0.56 mL), WSC (0.57 g) and HOBt (0.46 g) were added to a suspension of 5-methyl-2-(4-piperidinyl)-1,2-dihydro-3H-imidazo[1,5-c]imidazol-3-one dihydrochloride (0.44 g) and 3-[(7-chloro-2H-chromen-3-yl)sulfonyl]propionic acid (0.61 g) in acetonitrile (20 mL) and stirred for 12 hours. The reaction solution was concentrated and to the residue, chloroform and water were added to separate a chloroform layer. The chloroform solution was dried over anhydrous sodium sulfate and then concentrated under reduced pressure. After the residue was crystallized from ethyl acetate, the crude crystal was recrystallized from ethyl acetate-methanol to obtain the title compound (0.79 g, 78%) as colorless needles.
NMR (300 MHz, CDCl₃) δ: 1.50-1.72 (2H, m), 1.87-2.00 (2H, m), 2.61 (3H, s), 2.65 (1H, m), 2.81-2.93 (2H, m), 3.20 (1H, m), 3.38-3.57 (2H, m), 3.99 (1H, d, J = 11.1), 4.16 (1H, m), 4.22 (2H, s), 4.75 (1H, d, J = 11.1), 5.04 (1H, s), 6.72 (1H, s), 6.93 (1H, d, J = 1.5), 7.00 (1H, dd, J = 1.8, 7.8), 7.13 (1H, d, J = 7.8), 7.35 (1H, d, J = 1.8)
Elemental analysis for C₂₃H₂₅N₄O₅SCl
Calculated (%): C, 54.70; H, 4.99; N, 11.90
Found (%): C, 54.55; H, 4.81; N, 11.24

### Example 128

### 2-[1-(3-{[(E)-2-(4-chlorophenyl)vinyl]sulfonyl}propanoyl)-4-piperidinyl]-5-methyl-1,2-dihydro-3H-imidazo[1,5-c]imidazol-3-one

Triethylamine (0.56 mL), WSC (0.57 g) and HOBt (0.46 g) were added to a suspension of 5-methyl-2-(4-piperidinyl)-1,2-dihydro-3H-imidazo[1,5-c]imidazol-3-one dihydrochloride (0.44 g) and 3-{[(E)-2-(4-chlorophenyl)vinyl]sulfonyl}propionic acid (0.55 g) in acetonitrile (20 mL) and stirred for 12 hours. The reaction solution was concentrated and to the residue, chloroform and water were added to separate a chloroform layer. The chloroform solution was dried over an anhydrous sodium sulfate and then concentrated under reduced pressure. The residue was purified with a basic silica gel column to obtain the title compound (0.71 g, 74%) was obtained as a colorless solid.
NMR (300 MHz, CDCl₃) δ: 1.55-1.69 (2H, m), 1.88-1.98 (2H, m), 2.61 (3H, s), 2.65 (1H, m), 2.86-2.95 (2H, m), 3.20 (1H, m), 3.39-3.59 (2H, m), 4.00 (1H, d, J = 13.5), 4.15 (1H, m), 4.21 (2H, s), 4.75 (1H, d, J = 13.5), 6.71 (1H, s), 6.87 (1H, d, J = 15.3), 7.40-7.49 (4H, m), 7.56 (1H, d, J = 15.3)
Elemental analysis for C₂₂H₂₅N₄O₄SCl
Calculated (%): C, 55.40; H, 5.28; N, 11.75
Found (%): C, 55.40; H, 5.28; N, 11.79

### Example 129

### 7-(1-{3-[(6-Chloro-2-naphthyl)sulfonyl]propanoyl}-4-piperidinyl)-5-hydroxy-3-methyl-6,7-dihydroimidazo[1,5-a]pyrazin-8(5H)-one

### 129a) Tert-butyl 4-[(2,2-dimethoxyethyl)amino]piperidine-1-carboxylate

A suspension of tert-butyl 4-aminopiperidine-1-carboxylate (0.50 g), 2-bromo-1,1-dimethoxyethane (1.5 mL) and potassium carbonate (1.4 g) in acetonitrile (10 mL) was heated to reflux for 20 hours. The reaction solution was cooled to room temperature and then filtered using Celite. The filtrate was then concentrated under reduced pressure. The residue was purified with a silica gel column (ethyl acetate) to obtain the title compound (0.35 g, 49%) as a pale yellow oil.
NMR (200 MHz, CDCl₃) δ: 1.09-1.35 (2H, m), 1.45 (9H, s), 1.80-1.86 (2H, m), 2.52-2.67 (1H, m), 2.75-2.84 (4H, m), 3.39 (6H, s), 4.04 (2H, d, J = 13.2), 4.45 (1H, t, J = 5.5).

### 129b) Tert-butyl 4-{(2,2-dimethoxyethyl)[(2-methyl-1H-imidazol-4-yl)carbonyl]amino}piperidine-1-carboxylate

From 2-methyl-1H-imidazole-4-carboxylic acid (1.4 g) and tert-butyl 4-[(2,2-dimethoxyethyl)amino]piperidine-1-carboxylate (3.3 g) obtained in Example 129a), the title compound (2.4 g, 53%) was obtained as a pale yellow oil in a similar manner to Example 38b).
NMR (200 MHz, CDCl₃) δ: 1.47 (9H, s), 1.77-1.86 (4H, m), 2.39-2.45 (3H, m), 2.71-2.82 (2H, m), 3.43 (6H, s), 3.57 (2H, bs), 4.22-4.42 (3H, m), 4.63 (1H, t, J = 4.7), 7.32-7.45 (1H, m).

### 129c) 5-Hydoxy-3-methyl-7-(4-piperidinyl)-6,7-dihydroimidazo[1,5-a]pyrazin-8(5H)-one dihydrochloride

Tert-butyl 4-{(2,2-dimethoxyethyl)[(2-methyl-1H-imidazol-4-yl)carbonyl]amino}piperidine-1-carboxylate (1.4 g) obtained in Example 129b) was dissolved in concentrated hydrochloric acid (3 mL) and stirred at room temperature for 30 minutes. The reaction solution was subjected to azeotropic distillation with toluene under reduced pressure to remove water to obtain the title compound (1.1 g, 97%) as white powder.
NMR (300 MHz, DMSO-d₆) δ: 1.66-1.78 (2H, m), 2.00-2.17 (2H, m), 2.79 (3H, s), 3.03 (2H, d, J= 11.9), 3.33 (2H, d, J = 11.9), 3.63 (1H, d, J = 13.1), 3.79 (1H, d, J = 13.1), 4.68-4.76 (1H, m), 6.15 (1H, s), 8.20 (1H, s).

### 129d) 7-(1-{3-[(6-Chloro-2-naphthyl)sulfonyl]propanoyl}-4-piperidinyl)-5-hydroxy-3-methyl-6,7-dihydroimidazo[1,5-a]pyrazin-8(5H)-one

From 3-[(6-chloro-2-naphthyl)sulfonyl]propionic acid (0.56 g) and 5-hydoxy-3-methyl-7-(4-piperidinyl)-6,7-dihydroimidazo[1,5-a]pyrazin-8(5H)-one dihydrochloride (0.56 g) obtained in Example 129c), the title compound (0.48 g, 54%) was obtained as colorless crystals in a similar manner to Example 128.
NMR (300 MHz, CDCl₃) δ: 1.42-1.66 (2H, m), 1.66-1.82 (2H, m), 2.28-2.30 (m, 3H), 2.59 (1H, t, J = 3.0), 2.78-2.97 (2H, m), 3.15 (1H, t, J = 13.0), 3.44-3.66 (4H, m), 3.90-3.95 (1H, m), 4.62-4.74 (2H, m), 5.65 (1H, d, J = 1.5), 7.39 (1H, d, J = 3.6), 7.59 (1H, dd, J = 8.7, 2.1), 7.88-7.97 (4H, m), 8.47 (1H, s).
Elemental analysis for C₂₅H₂₇ClN₄O₅S·0.5H₂O·0.1EtOAc Calculated (%): C, 55.58; H, 5.29; N, 10.21
Found (%): C, 55.80; H, 5.48; N, 9.91

### Example 130

### 2-(1-{3-[(6-Chloro-2-naphthyl)sulfonyl]propanoyl}-4-piperidinyl)-1,2-dihydro-3H-imidazo[1,5-c]imidazol-3-one

### 130a) Tert-butyl 4-[(1H-imidazol-4-ylmethyl)amino]piperidine-1-carboxylate

From tert-butyl 4-aminopiperidine-1-carboxylate (2.1 g) and imidazole-4-carbaldehyde (1.0 g), the title compound (2.4 g, 82%) was obtained as a yellow oil in a similar manner to Example 42a).
NMR (200 MHz, CDCl₃) δ: 1.18-1.38 (2H, m), 1.45 (9H, s), 1.86 (2H, d, J = 12.3), 2.61-2.85 (3H, m), 3.82 (2H, m), 4.03 (2H, d, J = 12.3), 6.89 (1H, s), 7.57 (1H, s).

### 130b) Tert-butyl 4-(3-oxo-1H-imidazo[1,5-c]imidazol-2(3H)-yl)piperidine-1-carboxylate

From tert-butyl 4-[(1H-imidazol-4-ylmethyl)amino]piperidine-1-carboxylate (2.4 g) obtained in Example 130a), the title compound (1.8 g, 70%) was obtained as a white solid in a similar manner to Example 42b).
NMR (300 MHz, CDCl₃) δ: 1.48 (9H, s), 1.58-1.70 (2H, m), 1.71-1.88 (2H, m), 2.83 (2H, t, J = 12.6), 4.08-4.18 (1H, m), 4.27 (2H, d, J = 10.8), 4.36 (2H, s), 6.91 (1H, s), 7.94 (1H, s).

### 130c) 2-(1-{3-[(6-Chloro-2-naphthyl)sulfonyl]propanoyl}-4-piperidinyl)-1,2-dihydro-3H-imidazo[1,5-c]imidazol-3-one

From 3-[(6-chloro-2-naphthyl)sulfonyl]propionic acid (1.7 g) and tert-butyl 4-(3-oxo-1H-imidazo[1,5-c]imidazol-2(3H)-yl)piperidine-1-carboxylate (1.6 g) obtained in Example 130b), the title compound (2.0 g, 72%) was obtained as colorless crystals in a similar manner to Example 38d). NMR (300 MHz, CDCl₃) δ: 1.58-1.82 (2H, m), 1.88-2.04 (2H, m), 2.64 (1H, t, J = 12.6), 2.85-3.05 (2H, m), 3.20 (1H, t, J = 12.6), 3.43-3.64 (2H, m), 4.03 (1H, d, J = 14.1), 4.15-4.27 (1H, m), 4.33 (2H, s), 4.74 (1H, d, J = 14.1), 6.92 (1H, d J = 1.0), 7.60 (1H, dd, J = 8.8, 1.8), 7.89-7.98 (5H, m), 8.49 (1H, s).
Elemental analysis for C₂₃H₂₃ClN₄O₄S
Calculated (%): C, 56.73; H, 4.76; N, 11.51
Found (%): C, 56.36; H, 4.67; N, 11.37

### Example 131

### 6-(1-{3-[(6-Chloro-2-naphthyl)sulfonyl]propanoyl}-4-piperidinyl)-7,8-dihydroimidazo[1,5-c]pyrimidin-5(6H)-one

### 131a) Tert-butyl 4-(5-oxo-7,8-dihydroimidazo[1,5-c]pyrimidin-6(5H)-yl)piperidine-1-caroboxylate

Sodium triacetoxyborohydride (17.3 g) was added to a solution of tert-butyl 4-oxopiperidine-1-carboxylate (10.8 g) and histamine dihydrochloride (10.0 g) in dichloroethane (300 mL) and stirred at room temperature for 15 hours. The reaction solution was adjusted to about pH 12 with addition of a 1N solution of sodium hydroxide in water, and then extracted with chloroform (100 mL). An organic layer was dried over anhydrous magnesium sulfate and the solvent was then distilled off under reduced pressure. The resulting yellow oil was dissolved in dichloromethane (300 mL) and to the solution, DBU (13.4 mL) and N,N'-carbonyldimidazole (7.64 g) were added. After stirred at room temperature for 15 hours, the reaction mixture was diluted with water and chloroform and an organic layer was separated and then dried over anhydrous magnesium sulfate. The solvent was concentrated under reduced pressure. The residue was purified with a silica gel column (ethyl acetate:ethanol = 5:1) to obtain the title compound (13.4 g, 77%) as a colorless oil.
NMR (200 MHz, CDCl₃) δ: 1.49 (9H, s), 1.54-1.79 (4H, m), 2.78-2.99 (4H, c), 3.43 (2H, t, J = 6.4), 4.26 (2H, d, J = 12.4), 4.48-4.64 (1H, m), 6.81 (1H, d, J = 1.1), 8.14 (1H, d, J = 1.1).

### 131b) 6-(1-{3-[(6-Chloro-2-naphthyl)sulfonyl]propanoyl}-4-piperidinyl)-7,8-dihydroimidazo[1,5-c]pyrimidin-5(6H)-one

From 3-[(6-chloro-2-naphthyl)sulfonyl]propionic acid (0.26 g) and tert-butyl 4-(5-oxo-7,8-dihydromidazo[1,5-c]pyrimidin-6(5H)-yl)piperidine-1-carboxylate (0.25 g) obtained in Example 131a), the title compound (0.27 g, 64%) was obtained as colorless crystals in a similar manner to Example 38d).
NMR (200 MHz, CDCl₃) δ: 1.60-1.85 (5H, m), 2.59-2.70 (1H, m), 2.84-2.99 (3H, m), 3.13-3.26 (1H, m), 3.40 (2H, t, J = 6.4), 3.50-3.65 (2H, m), 4.02 (1H, d, J = 14.0), 4.58-4.74 (2H, m), 6.82 (1H, s), 7.61 (1H, dd, J = 8.8, 1.8), 7.94-7.98 (4H, m), 8.14 (1H, s), 8.49 (1H, s).
Elemental analysis for C₂₄H₂₅ClN₄O₄S
Calculated (%): C, 57.54; H, 5.03; N, 11.18
Found (%): C, 57.32; H, 5.05; N, 10.91

### Example132

### 2-(1-{3-[(5-Chloro-1H-indol-2-yl)sulfonyl]propanoyl}-4-piperidinyl)-5-methyl-1,2-dihydro-3H-imidazo[1,5-c]imidazol-3-one

### 132a) Tert-butyl 5-chloro-2-({3-[4-(5-methyl-3-oxo-1H-imidazo[1,5-c]imidazol-2(3H)-yl)-1-piperidinyl]-3-oxopropyl}sulfonyl)-1H-indole-1-carboxylate

From 3-{[1-(tert-butoxycarbonyl)-5-chloro-1H-indol-2-yl]sulfonyl}propionic acid (5.00 g) and 5-methyl-2-(4-piperidinyl)-1,2-dihydro-3H-imidazo[1,5-c]imidazol-3-one dihydrochloride (2.8 g) obtained in Example 69b), the title compound (2.9 g, 38%) was obtained as a white solid in a similar manner to Example 128).
NMR (300 MHz, CDCl₃) δ: 1.54-1.71 (2H, m), 1.74 (9H, s), 1.85-1.98 (2H, m), 2.59-2.67 (4H, m), 2.88-3.00 (2H, m), 3.18 (1H, t, J = 12.2), 3.98-4.20 (4H, m), 4.26 (2H, s), 4.72 (1H, d, J = 13.5), 6.71 (1H, t, J = 1.5), 7.44 (1H, dd, J = 9.0, 2.4), 7.51 (1H, s), 7.65 (1H, d, J = 2.4), 7.98 (1H, d, J = 9.0).

### 132b) 2-(1-{3-[(5-Chloro-1H-indol-2-yl)sulfonyl]propanoyl}-4-piperidinyl)-5-methyl-1,2-dihydro-3H-imidazo[1,5-c]imidazol-3-one

From tert-butyl 5-chloro-2-({3-[4-(5-methyl-3-oxo-1H-imidazo[1,5-c]imidazol-2(3H)-yl)-1-piperidinyl]-3-oxopropyl}sulfonyl)-1H-indole-1-carboxylate (2.9 g) obtained in Example 132a), the title compound (2.0 g, 83%) was obtained as colorless crystals in a similar manner to Example 103b).
NMR (300 MHz, CDCl₃) δ: 1.49-2.04 (2H, m), 2.61-2.70 (4H, m), 2.81-3.06 (2H, m), 3.20 (1H, t, J = 12.3), 3.56-3.65 (1H, m), 3.73-3.83 (1H, m), 3.98 (1H, d, J = 14.7), 4.08-4.17 (1H, m), 4.22 (2H, s), 4.72 (1H, d, J = 13.8), 6.71 (1H, s), 7.15 (1H, d, J = 1.8), 7.33 (1H, dd, J = 9.0, 1.8), 7.42 (1H, d, J = 9.0), 7.69 (1H, d, J = 1.8).
Elemental analysis for C₂₂H₂₄ClN₅O₄S·0.5H₂O
Calculated (%): C, 52.95; H, 5.05; N, 14.04
Found (%): C, 53.05; H, 4.89; N, 14.03

### Example 133

### 2-(1-{3-[(6-Chloro-2-naphthyl)sulfonyl]propanoyl}pyrrolidin-3-yl)-5-methyl-1,2-dihydro-3H-imidazo[1,5-c]imidazol-3-one

### 133a) 2-(1-Benzylpyrrolidin-3-yl)-5-methyl-1,2-dihydro-3H-imidazo[1,5-c]imidazol-3-one

From 1-benzyl-3-pyrrolidineamine (14.1 g) and imidazole-2-carbaldehyde (7.7 g), the title compound (2.7 g 12%) was obtained as a yellow oil in a similar manner to Example 69a).
NMR (300 MHz, CDCl₃) δ: 1.79-1.90 (1H, m), 2.24-2.41 (2H, m), 2.45-2.51 (1H, m), 2.80 (1H, d, J = 10.5), 3.02-3.09 (1H, m), 3.51 (1H, d, J = 12.6), 3.70 (1H, d, J = 12.6), 4.49 (2H, s), 4.79-4.86 (1H, m), 7.16 (1H, d, J = 1.5), 7.22-7.35 (6H, m).

### 133b) 6-(3-Pyrrolidinyl)-6,7-dihydro-5H-imidazo[1,5-a]imidazol-5-one

2-(1-Benzylpyrrolidin-3-yl)-5-methyl-1,2-dihydro-3H-imidazo[1,5-c]imidazol-3-one (2.7 g) obtained in Example 133a), ammonium formate (1.8 g) and 10% palladium carbon (0.54 g) were suspended in methanol (100 mL) and heated to reflux for 2 hours. After cooled to room temperature, a precipitate was filtered using Celite and the filtrate was concentrated under reduced pressure. A mixed solvent of ethyl acetate:chloroform = 5:1 was added to the residue. After a precipitate was filtered, the filtrate was concentrated again to obtain the title compound (1.4 g, 78%) as a pale yellow solid.
NMR (300 MHz, CDCl₃) δ: 1.85-1.96 (1H, m), 2.18-2.30 (1H, m), 2.96-3.07 (2H, m), 3.15-3.28 (2H, m), 4.42 (1H, d, J = 16.8), 4.49 (1H, d, J = 16.8), 4.65-4.74 (1H, m), 7.19 (1H, d, J = 1.2), 7.30 (1H, d, J = 1.2).

### 133c) 2-(1-{3-[(6-chloro-2-naphthyl)sulfonyl]propanoyl}pyrrolidin-3-yl)-5-methyl-1,2-dihydro-3H-imidazo[1,5-c]imidazol-3-one

From 3-[(6-chloro-2-naphthyl)sulfonyl]propionic acid (0.51 g) and 6-(3-pyrrolidinyl)-6,7-dihydro-5H-imidazo[1,5-a]imidazol-5-one (0.50 g) obtained in Example 133b), the title compound (0.47 g, 56%) was obtained as colorless crystals in a similar manner to Example 89b).
NMR (300 MHz, CDCl₃) δ: 2.07-2.39 (2H, m), 2.59-2.61 (3H, m), 2.72-2.94 (2H, m), 3.38-3.87 (6H, m), 4.30 (1H, m), 4.36 (1H, s), 4.60-4.76 (1H, m), 6.69-6.71 (1H, m), 7.57-7.60 (1H, m), 7.88-7.96 (4H, m), 8.47 (1H, s).
Elemental analysis for C₂₃H₂₃ClN₄O₄S·0.5H₂O·0.3EtOAc Calculated (%): C, 55.64; H, 5.09; N, 10.72
Found (%): C, 55.64; H, 5.00; N, 10.62

### Example134

### 2-(1-{3-[(6-Chloro-2-naphthyl)sulfonyl]-2-hydroxypropanoyl}-4-piperidinyl)-5-methyl-1,2-dihydro-3H-imidazo[1,5-c]imidazol-3-one

### 134a) Ethyl 3-[(6-chloro-2-naphthyl)thio]-2-hydroxypropionate and ethyl 2-[(6-chloro-2-naphthyl)thio]-3-hydroxypropionate

A solution of ethyl oxiran-2-carboxylate (W. D. Emmons et al. J. Am. Chem. Soc., 77, 89, (1955)) (0.60 g), 6-chloronaphthalene-2-thiol (1.0 g) and triethylamine (1.4 mL) in DMF (10 mL) was stirred at 60°C for 3 hours under argon atmosphere. After the reaction solution was cooled to room temperature, ethyl acetate (50 mL) and water (50 mL) were added. An organic layer was separated, washed with an aqueous saturated sodium chloride solution, dried over anhydrous magnesium sulfate, and then concentrated under reduced pressure. The residue was purified with a silica gel column (hexane:ethyl acetate = 1:1 to 1:2).
From a first fraction, the title compound (0.27 g, 17%) was obtained as a white solid.
NMR (300 MHz, CDCl₃) δ: 1.18 (3H, t, J = 7.1), 3.21 (1H, d, J = 6.2), 3.37 (1H, dd, J = 13.8, 5.4), 3.49 (1H, dd, J = 13.8, 4.2), 3.93-4.04 (1H, m), 4.06-4.17 (1H, m), 4.42-4.47 (1H, m), 7.40 (1H, dd, J = 8.7, 1.8), 7.50 (1H, dd, J = 8.7, 1.8), 7.65 (1H, d, J = 3.0), 7.67 (1H, d, J = 3.0), 7.75 (1H, s), 7.83 (1H, s).

From a second fraction, ethyl 2-[(6-chloro-2-naphthyl)thio]-3-hydroxypropionate (0.31 g, 19%) was obtained as a colorless oil.
NMR (300 MHz, CDCl₃) δ: 1.22 (3H, t, J = 7.2), 2.39-2.44 (1H, m), 3.87-4.01 (3H, m), 4.14-4.24 (2H, m), 7.44 (1H, dd, J = 8.7, 2.1), 7.54 (1H, dd, J = 8.7, 1.8), 7.69 (1H, d, J = 3.2), 7.72 (1H, d, J = 3.2), 7.79 (1H, d, J = 2.1), 7.95 (1H, d, J = 1.8).

### 134b) 2-(1-{3-[(6-Chloro-2-naphthyl)sulfonyl]-2-hydroxypropanoyl}-4-piperidinyl)-5-methyl-1,2-dihydro-3H-imidazo[1,5-c]imidazol-3-one hydrochloride

A 1N solution of sodium hydroxide in water (1.7 mL) was added to a solution of ethyl 3-[(6-chloro-2-naphthyl)thio]-2-hydroxypropionate (0.27 g) obtained in Example 134a) in ethanol (2 mL) and stirred at room temperature for 2 hours. The reaction solution was neutralized with 1N hydrochloric acid and then concentrated under reduced pressure. The residue was diluted with ethyl acetate and water, and an organic layer was separated and then washed with an aqueous saturated sodium chloride solution. The ethyl acetate solution was dried over anhydrous magnesium sulfate and concentrated under reduced pressure. The resulting residue, 5-methyl-2-(4-piperidinyl)-1,2-dihydro-3H-imidazo[1,5-c]imidazol-3-one dihydrochloride (0.29 g) obtained in Example 69b), HOBt (0.16 g), DBU (0.24 mL) and triethylamine (0.34 mL) were dissolved in acetonitrile (3 mL). To the solution was added WSC (0.20 g), and the mixture was stirred at room temperature for 5 hours. The reaction solution was concentrated and the residue was diluted with chloroform and water. An organic layer was separated and washed with an aqueous saturated sodium chloride solution. The chloroform solution was dried over anhydrous magnesium sulfate and concentrated under reduced pressure. The residue was dissolved in chloroform, and m-chloroperbenzoic acid (0.15 g) was added. The mixture was stirred at room temperature for 3 hours. The reaction solution was diluted with chloroform and an aqueous sodium thiosulfate solution. An organic layer was separated, washed successively with an aqueous saturated sodium hydrogencarbonate solution and an aqueous saturated sodium chloride solution, dried over anhydrous magnesium sulfate, and then concentrated under reduced pressure. The residue was purified by preparative HPLC, and treated with a 4N solution of hydrogen chloride in ethyl acetate (0.5 mL) to obtain the title compound (0.033 g, 11%) as a white solid.
NMR (300 MHz, CDCl₃) δ: 1.52-1.57 (1H, m), 1.71-1.87 (2H, m), 2.45-2.73 (4H, m), 3.14-3.67 (3H, m), 3.80 (1H, dd, J = 15.2, 4.1), 4.05-4.07 (2H, m), 4.33 (1H, d, J = 12.6), 4.54 (2H, d, J = 9.9), 4.79-4.81 (1H, m), 7.43 (1H, d, J = 12.9), 7.72 (1H, d, J = 9.0), 7.98 (1H, d, J = 8.7), 8.15 (1H, d, J = 9.0), 8.24-8.28 (2H, m), 8.62 (1H, s).
Elemental analysis for C₂₄H₂₅ClN₄O₅S·HCl·H₂O·0.2Et₂O Calculated (%): C, 50.98; H, 5.18; N, 9.59
Found (%): C, 51.16; H, 5.44; N, 9.49

### Example135

### 2-(1-{2-[(6-Chloro-2-naphthyl)sulfonyl]-3-hydroxypropanoyl}-4-piperidinyl)-5-methyl-1,2-dihydro-3H-imidazo[1,5-c]imidazol-3-one

From ethyl 3-[(6-chloro-2-naphthyl)thio]-3-hydroxypropionate (0.31 g) obtained in Example 134a), the title compound (0.030 g, 6%) was obtained as a white solid in a similar manner to Example 134b).
NMR (300 MHz, CDCl₃) δ: 1.62-2.02 (5H, m), 2.62-2.83 (4H, m), 3.30-3.46 (1H, m), 4.04-4.40 (5H, m), 4.62-4.67 (1H, m), 4.77-4.86 (1H, m), 6.74-6.75 (1H, m), 7.58-7.62 (1H, m), 7.80-8.01 (4H, m), 8.41-8.48 (1H, m).
Elemental analysis for C₂₄H₂₅ClN₄O₅S•0.5H₂O
Calculated (%): C, 54.80; H, 4.98; N, 10.65
Found (%): C, 54.89; H, 4.96; N, 10.48

### Example 136

### 2-(1-{(2S)-3-[(6-chloro-2-naphthyl)sulfonyl]-2-hydroxypropanoyl}-4-piperidinyl)-5-methyl-1,2-dihydro-3H-imidazo[1,5-c]imidazol-3-one

### 136a) Methyl (2S)-3-[(6-chloro-2-naphthyl)thio]-2-hydroxypropionate

Under argon atmosphere, a 3 M solution of ethylmagnesium bromide in diethyl ether was added dropwise to THF (25 mL) under ice-cooling. To this solution was added dropwise a solution of 6-chloronaphthalene-2-thiol (5.0 g) in THF (50 mL) at 0°C, and the mixture was stirred at room temperature for 30 minutes. To this solution was added dropwise a solution of methyl (2R)-oxiran-2-carboxylate (2.3 mL) in THF (15 mL), and the reaction solution was stirred at room temperature for 3 hours. After an aqueous ammonium chloride solution (50 mL) was added, the reaction solution was extracted with ethyl acetate (100 mL). The extract was washed with an aqueous saturated sodium chloride solution, dried over anhydrous magnesium sulfate, and then concentrated under reduced pressure. The residue was recrystallized from hexane/ethyl acetate (3:1) to obtain the title compound (5.9 g, 77%) as colorless needles.
NMR (300 MHz, CDCl₃) δ: 3.12 (1H, d, J = 6.0), 3.35 (1H, dd, J = 14.1, 5.7), 3.48 (1H, dd, J = 14.1, 4.2), 3.58 (3H, s), 4.43-4.48 (1H, m), 7.39-7.43 (1H, m), 7.49-7.52 (1H, m), 7.66-7.69 (2H, m), 7.76-7.77 (1H, m), 7.83-7.84 (1H, m).

### 136b) (2S)-3-[(6-chloro-2-naphthyl)thio]-2-hydroxypropionic acid

A 8N solution of sodium hydroxide in water (6.8 mL) was added to a suspension of methyl (2S)-3-[(6-chloro-2-naphthyl)thio]-2-hydroxypropionate (5.4 g) obtained in Example 136a) in ethanol (150 mL), and the mixture was stirred at room temperature for 3 hours. After ethanol was distilled off under reduced pressure, a precipitate was filtered. The solid was suspended in water (100 mL) and adjusted to about pH 3 with 1N hydrochloric acid. A precipitate was filtered to obtain the title compound (5.0 g, 97%) as a white solid.
NMR (300 MHz, CD₃OD) δ: 3.27 (1H, dd, J = 14.1, 6.9), 3.51 (1H, dd, J = 14.1, 4.2), 4.33 (1H, dd, J = 6.9, 4.2), 7.40-7.43 (1H, m), 7.51-7.54 (1H, m), 7.71-7.77 (2H, m), 7.82 (1H, s), 7.86 (1H, s).

### 136c) 2-(1-{(2S)-3-[(6-chloro-2-naphthyl)thio]-2-hydroxypropanoyl}-4-piperidinyl)-5-methyl-1,2-dihydro-3H-imidazo[1,5-c]imidazol-3-one

(2S)-3-[(6-chloro-2-naphthyl)thio]-2-hydroxypropionic acid (3.0 g) obtained in Example 136b), 5-methyl-2-(4-piperidinyl)-1,2-dihydro-3H-imidazo[1,5-c]imidazol-3-one dihydrochloride (3.3 g) obtained in Example 69b), HOBt (2.4 g) and triethylamine (5.2 mL) were suspended in a mixture of acetonitrile (300 mL) and dichloromethane (100 mL). To the suspension was added WSC (3.1 g), and the mixture was stirred at room temperature for 18 hours. The reaction solution was concentrated and the residue was diluted with chloroform and water. An organic layer was separated and washed with an aqueous saturated sodium chloride solution. The chloroform solution was dried over anhydrous magnesium sulfate and concentrated under reduced pressure. The residue was purified with a basic silica gel column (ethyl acetate to ethyl acetate : ethanol = 10 : 1) to obtain the title compound (3.6 g, 71%) as colorless powder.
NMR (300 MHz, CDCl₃) δ: 1.54-1.83 (4H, m), 2.39-2.98 (4H, m), 2.98-3.44 (4H, m), 3.76-4.20 (3H, m), 4.61-4.73 (2H, m), 6.67-6.68 (1H, m), 7.41-7.51 (2H, m), 7.64-7.70 (2H, m), 7.75-7.83 (2H, m).

### 136d) 2-(1-{(2S)-3-[(6-chloro-2-naphthyl)sulfonyl]-2-hydroxypropanoyl}-4-piperidinyl)-5-methyl-1,2-dihydro-3H-imidazo[1,5-c]imidazol-3-one

Under ice-cooling, m-chloroperbenzoic acid (1.7 g) was added to a solution of 2-(1-{(2S)-3-[(6-chloro-2-naphthyl)thio]-2-hydroxypropanoyl}-4-piperidinyl)-5-methyl-1,2-dihydro-3H-imidazo[1,5-c]imidazol-3-one (3.1 g) obtained in Example 136c) in dichloromethane (100 mL), and the mixture was stirred at room temperature for 5 hours. The reaction solution was diluted with chloroform and an aqueous sodium thiosulfate solution to separate an organic layer. The chloroform solution was washed successively with an aqueous saturated sodium hydrogencarbonate solution and an aqueous saturated sodium chloride solution, dried over anhydrous magnesium sulfate, and then concentrated under reduced pressure. The residue was purified with a basic silica gel column (ethyl acetate to ethyl acetate:ethanol = 10:1) to obtain the title compound (2.7 g, 81%) as colorless powder.
NMR (300 MHz, CDCl₃) δ: 1.65-1.88 (2H, m), 1.93-2.04 (2H, m), 2.61 (3H, s), 2.76-2.85 (1H, m), 3.18-3.31 (1H, m), 3.44-3.51 (2H, m), 3.75-3.88 (1H, m), 4.10-4.30 (3H, m), 4.68-4.72 (1H, m), 5.03-5.04 (1H, m), 6.71 (1H, s), 7.59 (1H, dd, J = 8.9, 2.0), 7.87-8.00 (4H, m), 8.50 (1H, s). Elemental analysis for C₂₄H₂₅ClN₄O₅S
Calculated (%): C, 55.76; H, 4.87; N, 10.84
Found (%): C, 55.60; H, 4.98; N, 10.85

### Example 137

### 2-(1-{3-[(6-Chloro-2-naphthyl)sulfonyl]propanoyl}-4-piperidinyl)-5-(hydroxymethyl)-1,2-dihydro-3H-imidazo[1,5-c]imidazol-3-one

### 137a) 1-Benzyl-N-(1H-imidazol-4-yl)methylpiperidine-4-amine

Sodium triacetoxyborohydride (4.7 g) was added to a solution of 1-benzyl-4-piperidineamine (3.4 g), 4-formylimidazole (1.4 g) and acetic acid (1.7 mL) in dichloroethane (100 mL), and the mixture was stirred at room temperature for 15 hours. The reaction solution was adjusted to about pH 12 with a 1N solution of sodium hydroxide in water, and then extracted with chloroform (100 mL). The extract was dried over anhydrous magnesium sulfate and the solvent was then distilled off under reduced pressure to obtain the title compound (3.4 g) as a yellow oil. This crude product was used in the next reaction without further purification.
NMR (300 MHz, CDCl₃) δ: 1.36-1.53 (2H, m), 1.75-2.08 (6H, m), 2.50-2.65 (1H, m), 2.66-2.86 (2H, m), 3.49 (2H, s), 4.31 (1H, bs), 6.86 (1H, s), 7.23-7.31 (5H, m), 7.51 (1H, s).

### 137b) Tert-butyl (1-benzyl-4-piperidinyl)[(1H-imidazol-4-yl)methyl]carbamate

Di-tert-butyl dicarbonate (6.3 mL) was added to a solution of 1-benzyl-N-(1H-imidazol-4-yl)methyl-4-piperidineamine (3.4 g) obtained in Example 137a) in ethanol (50 mL), and the mixture was stirred at room temperature for 5 hours. Hydrazine monohydrate (10 mL) was added, and the mixture was stirred at room temperature for 18 hours. After the solvent was distilled off under reduced pressure, to the residue were added ethyl acetate (100 mL) and water (100 mL) to separate an organic layer. The ethyl acetate solution was washed with an aqueous saturated sodium chloride solution (100 mL) and then dried over anhydrous magnesium sulfate. The solvent was distilled off under reduced pressure. The residue was purified with a basic silica gel column (ethyl acetate:ethanol = 10:1) to obtain the title compound (2.3 g, 42%) as a colorless oil.
NMR (200 MHz, CDCl₃) δ: 1.42-1.61 (12H, m), 1.66-1.84 (2H, m), 1.93-2.04 (2H, m), 2.92 (2H, d, J = 7.6), 3.47 (2H, s), 3.70 (1H, bs), 4.29 (2H, s), 6.86 (1H, s), 7.24-7.33 (5H, m), 7.49 (1H, s).

### 137c) Tert-butyl (1-benzyl-4-piperidinyl)[(1-([2-(trimethylsilyl)ethoxy]methyl}-1H-imidazol-4-yl)methyl]carbamate

Under ice-cooling, sodium hydride (0.22 g) was added to a solution of tert-butyl (1-benzyl-4-piperidinyl)[(1H-imidazol-4-yl)methyl]carbamate (1.3 g) obtained in Example 137b) in DMF (30 mL), and the mixture was stirred at room temperature for 1 hour. Subsequently, 2-(trimethylsilyl)ethoxymethyl chloride (1.3 mL) was added under ice-cooling, and the mixture was stirred at room temperature for 2 hours. To the reaction solution were added ethyl acetate (50 mL) and water (50 mL) to separate an organic layer. The ethyl acetate solution was washed with an aqueous saturated sodium chloride solution (50 mL), and dried over anhydrous magnesium sulfate. The solvent was distilled off. The residue was purified with a basic silica gel column (ethyl acetate:hexane = 1:1) to obtain the title compound (4.9 g, 24%) as a pale yellow oil.
NMR (300 MHz, CDCl₃) δ: 0.05 (9H, s), 0.91 (2H, t, J = 8.1), 1.47 (9H, s), 1.62-1.66 (2H, m), 1.84-1.90 (3H, m), 2.03-2.09 (2H, m), 2.91-2.95 (2H, m), 3.41-3.54 (4H, m), 4.35 (2H, s), 5.22 (2H, s), 6.90 (1H, bs), 7.24-7.43 (5H, m), 7.48 (1H, s).

### 137d) Tert-butyl (1-benzyl-4-piperidinyl)[(2-formyl-1-{[2-(trimethylsilyl)ethoxy]methyl}-1H-imidazol-4-yl)methyl]carbamate

A solution of n-butyllithium in hexane (1.5 M, 5.7 mL) was added dropwise to a solution of tert-butyl (1-benzyl-4-piperidinyl)[(1-{[2-(trimethylsilyl)ethoxy]methyl}-1H-imidazol-4-yl)methyl]carbamate (3.9 g) obtained in Example 137c) in THF (50 mL) at -40°C under argon atmosphere. After the reaction solution was stirred at -40°C for 15 minutes, DMF (0.7 mL) was added. The mixture was stirred at room temperature for 15 hours. After an aqueous saturated ammonium chloride solution (50 mL) was added, the reaction solution was extracted with ethyl acetate (50 mL). The extract was washed with an aqueous saturated sodium chloride solution (50 mL) and then dried over anhydrous magnesium sulfate. The solvent was distilled off under reduced pressure. The residue was purified with a silica gel column (ethyl acetate:ethanol = 10:1) to obtain the title compound (2.52 g, 61%) as a pale yellow oil.
NMR (300 MHz, CDCl₃) δ: -0.04 (9H, s), 0.90 (2H, t, J = 8.2), 1.45 (9H, s), 1.63-1.77 (5H, m), 1.99-2.05 (2H, m), 2.91 (2H, d, J = 11.1), 3.47 (2H, s), 3.53 (2H, t, J = 8.2), 4.37 (2H, s), 5.71 (2H, s), 7.20-7.35 (5H, m), 9.74 (1H, s).

### 137e) Tert-butyl (1-benzyl-4-piperidinyl)[(2-(hydroxymethyl)-1-{[2-(trimethylsilyl)ethoxy]methyl}-1H-imidazol-4-yl)methyl]carbamate

Under ice-cooling, sodium borohydride (95 mg) was added to a solution of tert-butyl (1-benzyl-4-piperidinyl)[(2-formyl-1-{[2-(trimethylsilyl)ethoxy]methyl}-1H-imidazol-4-yl)methyl]carbamate (1.3 g) obtained in Example 137d) in ethanol (10 mL), and the mixture was stirred at room temperature for 1 hour. After water (1 mL) was added, the solvent was distilled off and to the residue, ethyl acetate (50 mL) and water (50 mL) were added. An organic layer was separated, washed with an aqueous saturated sodium chloride solution (50 mL), and then dried over anhydrous magnesium sulfate. The solvent was distilled off under reduced pressure to obtain the title compound (1.3 g, 98%) as a colorless oil.
NMR (300 MHz, CDCl₃) δ: -0.05 (9H, s), 0.88 (2H, t, J = 8.4), 1.43 (9H, s), 1.57-1.60 (2H, m), 1.75-1.76 (1H, m), 1.98-1.99 (2H, m), 2.87 (2H, d, J = 12.0), 3.44-3.49 (4H, m), 4.28 (2H, s), 4.66 (2H, s), 5.26 (2H, s), 6.75 (1H, bs), 7.19-7.29 (5H, m).

### 137f) (4-{[(1-Benzyl-4-piperidinyl)amino]methyl}-1H-imidazol-2-yl)methanol

A solution of tert-butyl (1-benzyl-4-piperidinyl)[(2-(hydroxymethyl)-1-{[2-(trimethylsilyl)ethoxy]methyl}-1H-imidazol-4-yl)methyl]carbamate (480 mg) obtained in Example 137e) in trifluoroacetic acid (1 mL)-water (1 mL) was stirred at 80°C for 3 hours. After cooled to room temperature, the reaction solution was neutralized with an aqueous saturated sodium hydrogencarbonate solution, and then extracted with chloroform (50 mL). The extract was washed with an aqueous saturated sodium chloride solution (50 mL) and dried over anhydrous magnesium sulfate. The solvent was distilled off under reduced pressure to obtain the title compound (280 mg, quantitative) as a yellow oil. NMR (300 MHz, CDCl₃) δ: 1.48-1.55 (2H, m), 1.63-1.75 (2H, m), 1.87-2.02 (2H, m), 2.64-2.65 (1H, m), 2.84-2.88 (2H, m), 3.47 (2H, s), 3.74 (2H, s), 4.46 (2H, s), 5.42 (1H, bs), 6.76 (1H, s), 7.19-7.34 (5H, m).

### 137g) 1-Benzyl-N-{ [2-({ [tert-butyl(dimethyl)silyl]oxy}methyl)-1H-imidazol-4-yl]methyl}piperidine-4-amine

Tert-butyldimethylchlorosilane (170 mg) was added to a solution of (4-{[(1-benzyl-4-piperidinyl)amino]methyl}-1H-imidazol-2-yl)methanol (0.28 g) obtained in Example 137f) and triethylamine (0.26 mL) in dichloromethane (5 mL), and the mixture was stirred at room temperature for 3 hours. To the reaction solution were added chloroform (20 mL) and water (20 mL) to separate an organic layer. The chloroform solution was washed with an aqueous saturated sodium chloride solution (20 mL) and dried over anhydrous magnesium sulfate. The solvent was distilled off under reduced pressure and the residue was purified with a basic silica gel column (ethyl acetate:ethanol = 10:1) to obtain the title compound (0.20 g, 52%) as a pale yellow oil.
NMR (300 MHz, CDCl₃) δ: 0.10 (6H, s), 0.92 (9H, s), 1.41-1.44 (2H, m), 1.84-1.88 (2H, m), 1.97-2.04 (2H, m), 2.50-2.51 (1H, m), 2.82-2.86 (2H, m), 3.49 (2H, s), 3.75 (2H, s), 4.77 (2H, s), 6.81 (1H, s), 7.18-7.36 (5H, m).

### 137h) 2-(1-Benzyl-4-piperidinyl)-5-({[tert-butyl(dimethyl)silyl]oxy}methyl)-1,2-dihydro-3H-imidazo[1,5-c]imidazol-3-one

N,N'-carbonyldiimidazole (94 mg) was added to a solution of 1-benzyl-N-{[2-({[tert-butyl(dimethyl)silyl]oxy}methyl)-1H-imidazol-4-yl]methyl}piperidine-4-amine (0.20 g) obtained in Example 137 g) and DBU (0.14 mL) in dichloroethane (3 mL), and the mixture was stirred at 60°C for 30 minutes. After cooled to room temperature, to the reaction solution were added chloroform (20 mL) and water (20 mL) to separate an organic layer. The chloroform solution was washed with an aqueous saturated sodium chloride solution (20 mL) and dried over anhydrous magnesium sulfate. The solvent was distilled off under reduced pressure and the residue was purified with a silica gel column (ethyl acetate:ethanol = 5:1) to obtain the title compound (0.18 g, 85%) as a pale yellow oil.
NMR (300 MHz, CDCl₃) δ: 0.13 (6H, s), 0.91 (9H, s), 1.72-1.83 (4H, m), 2.08-2.17 (2H, m), 2.99 (2H, d, J = 11.4), 3.52 (2H, s), 3.95-4.02 (1H, m), 4.30-4.31 (2H, m), 4.92 (2H, s), 6.79-6.80 (1H, m), 7.24-7.37 (5H, m).

### 137i) 5-({[Tert-butyl(dimethyl)silyl]oxy}methyl)-2-(4-piperidinyl)-1,2-dihydro-3H-imidazo[1,5-c]imidazol-3-one

2-(1-Benzyl-4-piperidinyl)-5-({[tert-butyl(dimethyl)silyl]oxy}methyl)-1,2-dihydro-3H-imidazo[1,5-c]imidazol-3-one (0.18 g) obtained in Example 137h) and 10% palladium carbon (36 mg) were suspended in methanol (3 mL), and the mixture was stirred at room temperature for 18 hours under hydrogen atmosphere. The reaction solution was filtered using Celite, and the filtrated was concentrated under reduced pressure to obtain the title compound (0.16 g quantitative) as a white solid. NMR (300 MHz, CDCl₃) δ: 0.14 (6H, s), 0.92 (9H, s), 1.61-1.74 (2H, m), 1.85-1.88 (2H, m), 2.71-2.79 (2H, m), 3.20 (2H, d, J = 11.7), 4.02-4.10 (1H, m), 4.32-4.33 (2H, m), 4.93 (2H, s), 6.80-6.81 (1H, m).

### 137j) 5-({[Tert-butyl(dimethyl)silyl]oxy}methyl)-2-(1-{3-[(6-chloro-2-naphthyl)sulfonyl]propanoyl}-4-piperidinyl)-1,2-dihydro-3H-imidazo[1,5-c]imidazol-3-one

From 3-[(6-chloro-2-naphthyl)sulfonyl]propionic acid (0.18 g) and 5-({[tert-butyl(dimethyl)silyl]oxy}methyl)-2-(4-piperidinyl)-1,2-dihydro-3H-imidazo[1,5-c]imidazol-3-one (0.16 g) obtained in Example 137i), the title compound (0.22 g, 76%) was obtained as a colorless oil in a similar manner to Example 89b).
NMR (300 MHz, CDCl₃) δ: 0.14 (6H, s), 0.92 (9H, s), 1.57-1.74 (2H, m), 1.87-1.99 (2H, m), 2.59-2.66 (1H, m), 2.83-3.01 (2H, m), 3.14-3.22 (1H, m), 3.48-3.65 (2H, m), 4.00 (1H, d, J = 14.4), 4.08-4.27 (1H, m), 4.27 (2H, s), 4.72 (1H, d, J = 14.1), 4.91 (2H, s), 6.80-6.81 (1H, m), 7.54-7.61 (2H, m), 7.89-7.96 (3H, m), 8.47 (1H, s).

### 137k) 2-(1-{3-[(6-chloro-2-naphthyl)sulfonyl]propanoyl}-4-piperidinyl)-5-(hydroxymethyl)-1,2-dihydro-3H-imidazo[1,5-c]imidazol-3-one

A solution of 5-({[tert-butyl(dimethyl)silyl]oxy}methyl)-2-(1-{3-[(6-chloro-2-naphthyl)sulfonyl]propanoyl}-4-piperidinyl)-1,2-dihydro-3H-imidazo[1,5-c]imidazol-3-one (0.22 g) obtained in Example 137j) in acetic acid (4 mL)-THF (2 mL)-water (2 mL) was stirred at 60°C for 3 hours. After cooled to room temperature, the reaction solution was concentrated under reduced pressure, and to the residue were added chloroform and an aqueous saturated sodium hydrogencarbonate solution to separate an organic layer. The chloroform solution was washed with an aqueous saturated sodium chloride solution and dried over anhydrous magnesium sulfate. The solvent was distilled off under reduced pressure and the residue was purified with a basic silica gel column (ethyl acetate:ethanol = 10:1) to obtain the title compound (99 mg, 55%) as a white solid.
NMR (300 MHz, CDCl₃) δ: 1.56-1.80 (2H, m), 1.89-2.02 (2H, m), 2.80-2.91 (1H, m), 2.95-3.06 (1H, m), 3.17-3.25 (1H, m), 3.46-3.56 (1H, m), 3.59-3.69 (1H, m), 3.86 (1H, t, J = 6.6), 4.03 (1H, d, J = 15.3), 4.15-4.23 (1H, m), 4.36 (2H, s), 4.75 (1H, d, J = 11.7), 4.87 (2H, d, J = 6.6), 6.78 (1H, s), 7.61 (1H, dd, J = 9.0, 2.1), 7.90-7.98 (4H, m), 8.49 (1H, s).
Elemental analysis for C₂₄H₂₅ClN₄O₅S
Calculated (%): C, 55.76; H, 4.87; N, 10.84
Found (%): C, 55.52; H, 4.94; N, 10.74

### Example138

### [2-(1-{3-[(6-Chloro-2-naphthyl)sulfonyl]propanoyl}-4-piperidinyl)-3-oxo-2,3-dihydro-1H-imidazo[1,5-c]imidazol-5-yl]methyl acetate hydrochloride

A suspension of 2-(1-13-[(6-chloro-2-naphthyl)sulfonyl]propanoyl}-4-piperidinyl)-5-(hydroxymethyl)-1,2-dihydro-3H-imidazo[1,5-c]imidazol-3-one (0.17 g) obtained in Example 137k), acetic anhydride (0.5 mL) and pyridine (1 mL) in dichloromethane (2 mL) was stirred for 18 hours. To the reaction solution were added dichloromethane and water to separate an organic layer. The dichloromethane solution was washed with water three times and then dried over anhydrous magnesium sulfate. The solvent was distilled off under reduced pressure. The residue was purified with a silica gel column (ethyl acetate:ethanol = 10:1) and then treated with a 4N solution of hydrogen chloride in ethyl acetate (0.5 mL) to obtain the title compound (0.13 g, 65%) as white powder.
NMR (200 MHz, DMSO) δ: 1.44-1.77 (4H, m), 2.06 (3H, s), 2.50-2.61 (1H, m), 2.75 (2H, t, J = 7.2), 3.08 (1H, t, J = 11.5), 3.87-4.05 (4H, m), 4.35 (1H, d, J = 9.4), 4.48 (2H, s), 5.29 (2H, s), 7.10 (1H, s), 7.72-7.77 (1H, m), 7.97-8.03 (1H, m), 8.17-8.31 (3H, m), 8.65-8.66 (1H, m). Elemental analysis for C₂₆H₂₇ClN₄O₆S•HCl•H₂O
Calculated (%): C, 50.90; H, 4.93; N, 9.13
Found (%): C, 50.84; H, 5.26; N, 8.98

### Example 139

### 2-(1-{(2S)-3-[(6-chloro-2-naphthyl)sulfonyl]-2-hydroxypropanoyl}-4-piperidinyl)-5-(hydroxymethyl)-1,2-dihydro-3H-imidazo[1,5-c]imidazol-3-one

### 139a) 5-({[Tert-butyl(dimethyl)silyl]oxy}methyl)-2-(1-{(2S)-3-[(6-chloro-2-naphthyl)thio]-2-hydroxypropanoyl}-4-piperidinyl)-1,2-dihydro-3H-imidazo[1,5-c]imidazol-3-one

From (2S)-3-[(6-chloro-2-naphthyl)thio]-2-hydroxypropionic acid (0.44 g) obtained in Example 136b) and 5-({[tert-butyl(dimethyl)silyl]oxy}methyl)-2-(4-piperidinyl)-1,2-dihydro-3H-imidazo[1,5-c]imidazol-3-one (0.50 g) obtained in Example 137i), the title compound (0.69 g, 78%) was obtained as a colorless oil in a similar manner to Example 136c).
NMR (200 MHz, CDCl₃) δ: 0.13 (6H, s), 0.91 (9H, s), 1.51-1.62 (2H, m), 1.80-1.83 (2H, m), 2.35-2.42 (1H, m), 2.69-2.75 (1H, m), 2.95-3.44 (2H, m), 3.81-4.23 (4H, m), 4.58-4.61 (2H, m), 4.89 (2H, s), 6.79 (1H, s), 7.42-7.52 (2H, m), 7.68 (1H, s), 7.72 (1H, s), 7.79 (1H, s), 7.83 (1H, s).

### 139b) 5-({[Tert-butyl(dimethyl)silyl]oxy}methyl)-2-(1-{(2S)-3-[(6-chloro-2-naphthyl)sulfonyl]-2-hydroxypropanoyl}-4-piperidinyl)-1,2-dihydro-3H-imidazo[1,5-c]imidazol-3-one

From 5-({[tert-butyl(dimethyl)silyl]oxy}methyl)-2-(1-{(2S)-3-[(6-chloro-2-naphthyl)thio]-2-hydroxypropanoyl}-4-piperidinyl)-1,2-dihydro-3H-imidazo[1,5-c]imidazol-3-one (0.69 g) obtained in Example 139a), the title compound (0.60 g, 83%) was obtained as colorless amorphous powder in a similar manner to Example 136d).
NMR (300 MHz, CDCl₃) δ: 0.14 (6H, s), 0.92 (9H, s), 1.71-1.81 (2H, m), 1.85-2.07 (2H, m), 2.70-2.84 (1H, m), 3.03-3.16 (1H, m), 3.43-3.51 (2H, m), 4.10-4.32 (4H, m), 4.67-4.76 (1H, m), 4.92 (2H, s), 5.03-5.05 (1H, m), 6.81 (1H, s), 7.59 (1H, dd, J = 8.7, 2.1), 7.91-7.97 (4H, m), 8.51 (1H, s).

### 139c) 2-(1-{(2S)-3-[(6-chloro-2-naphthyl)sulfonyl]-2-hydroxypropanoyl}-4-piperidinyl)-5-(hydroxymethyl)-1,2-dihydro-3H-imidazo[1,5-c]imidazol-3-one

A solution of 5-({[tert-butyl(dimethyl)silyl]oxy}methyl)-2-(1-{(2S)-3-[(6-chloro-2-naphthyl)sulfonyl]-2-hydroxypropanoyl}-4-piperidinyl)-1,2-dihydro-3H-imidazo[1,5-c]imidazol-3-one (0.24 g) obtained in Example 139b), acetic acid (64 µL) and tetrabutylammonium fluoride (0.29 g) in THF (3 mL) was stirred at room temperature for 72 hours. To the reaction solution were added ethyl acetate and water to separate an organic layer. The ethyl acetate solution was washed with an aqueous saturated sodium chloride solution and dried over anhydrous magnesium sulfate. The solvent was distilled off under reduced pressure. The residue was purified with a basic silica gel column (ethyl acetate:ethanol = 5:1) to obtain the title compound (150 mg, 75%) as a white solid.
NMR (300 MHz, CDCl₃) δ: 1.72-1.88 (2H, m), 1.88-2.04 (2H, m), 2.72-2.85 (1H, m), 3.18-3.32 (1H, m), 3.44-3.52 (2H, m), 3.71 (1H, bs), 3.85 (1H, bs), 4.08-4.26 (2H, m), 4.37-4.41 (2H, m), 4.69-4.78 (1H, m), 4.87 (2H, s), 5.04-5.06 (1H, m), 6.78 (1H, s), 7.57-7.61 (1H, m), 7.90-7.96 (4H, m), 8.50 (1H, s).
Elemental analysis for C₂₉H₂₅ClN₄O₆S•0.5H₂O•0.2Et₂O
Calculated (%): C, 53.49; H, 5.07; N, 10.06
Found (%): C, 55.49; H, 5.09; N, 9.81

### Example 140

### [2-(1-((2S)-3-[(6-chloro-2-naphthyl)sulfonyl]-2-hydroxypropanoyl}-4-piperidinyl)-3-oxo-2,3-dihydo-1H-imidazo[1,5-c]imidazol-5-yl]methyl benzoate

From 2-(1-{(2S)-3-[(6-chloro-2-naphthyl)sulfonyl]-2-hydroxypropanoyl}-4-piperidinyl)-5-(hydroxymethyl)-1,2-dihydro-3H-imidazo[1,5-c]imidazol-3-one (0.14 g) obtained in Example 139c) and benzoyl chloride (31 µL), the title compound (58 mg, 34%) was obtained as white powder in a similar manner to Example 138.
NMR (300 MHz, CDCl₃) δ: 1.67-1.78 (2H, m), 1.90-2.05 (2H, m), 2.70-2.84 (1H, m), 3.17-3.31 (1H, m), 3.45-3.52 (2H, m), 3.80 (1H, bs), 4.11-4.38 (4H, m), 4.69-4.77 (1H, m), 5.04-5.05 (1H, m), 5.63 (2H, s), 6.91 (1H, s), 7.39-7.44 (2H, m), 7.53-7.62 (2H, m), 7.94-7.97 (4H, m), 8.05 (1H, s), 8.08 (1H, s), 8.51 (1H, s).
Elemental analysis for C₃₁H₂₉ClN₄O₇S•0.5H₂O
Calculated (%): C, 57.63; H, 4.68; N, 8.67
Found (%): C, 57.91; H, 4.90; N, 8.64

### Example 141

### 2-[1-(Trans-{2-[(6-chloro-2-naphthyl)sulfonyl]cyclopropyl}carbonyl)-4-piperidinyl]-5-methyl-1,2-dihydro-3H-imidazo[1,5-c]imidazol-3-one

### 141a) 2-chloro-6-(vinylsulfonyl)naphthalene

A suspension of 2-chloronaphthalene-6-sulfinic acid (4.3 g), 1,2-dibromoethane (3.3 mL) and potassium carbonate (4.0 g) in DMF (30 mL) was stirred at 70°C for 18 hours. After cooled to room temperature, ethyl acetate and water were added and an organic layer was separated. The ethyl acetate solution was washed with an aqueous saturated sodium chloride solution and dried over anhydrous magnesium sulfate. The solvent was distilled off under reduced pressure. The residue was purified with a silica gel column (ethyl acetate:hexane = 1:5) to obtain the title compound (1.9 g, 39%) as a pale yellow solid.
NMR (300 MHz, CDCl₃) δ: 6.09 (1H, d, J = 9.6), 6.53 (1H, d, J = 16.5), 6.72 (1H, dd, J = 16.5, 9.6), 7.55 (1H, dd, J = 8.6, 2.0), 7.82-7.92 (4H, m), 8.46 (1H, d, J = 0.6).

### 141b) Tert-butyl trans-2-[(6-chloro-2-naphthyl)sulfonyl]cyclopropanecarboxylate

A solution of 1,4-diazabicyclo[2.2.2]octane (0.89 g) and tert-butyl chloroacetate (1.1 mL) in acetonitrile (30 mL) was stirred at room temperature for 30 minutes. To the solution were added 2-chloro-6-(vinylsulfonyl)naphthalene (2.0 g) obtained in Example 141a) and sodium hydroxide (0.48 g) and the mixture was stirred at 80°C for 18 hours. After acetonitrile was distilled off under reduced pressure, ethyl acetate and water were added to separate an organic layer. The ethyl acetate solution was washed with an aqueous saturated sodium chloride solution, dried over anhydrous magnesium sulfate, and then concentrated under reduced pressure. The residue was purified with a silica gel column (ethyl acetate:hexane = 1:3) to obtain the title compound (0.44 g, 15%) as a yellow oil.
NMR (200 MHz, CDCl₃) δ: 1.41 (9H, s), 1.44-1.55 (1H, m), 1.64-1.74 (1H, m), 2.47-2.57 (1H, m), 2.93-3.02 (1H, m), 7.58 (1H, dd, J = 8.8, 1.8), 7.86-7.96 (4H, m), 8.45 (1H, s).

### 141c) Trans-2-[(6-chloro-2-naphthyl)sulfonyl]cyclopropanecarboxylic acid

A solution of tert-butyl 2-[(6-chloro-2-naphthyl)sulfonyl]cyclopropanecarboxylate (0.44 g) obtained in Example 141b) in trifluoroacetic acid (2 mL) was stirred at room temperature for 15 minutes. The reaction solution was concentrated under reduced pressure to obtain the title compound (0.35 g, 94%) as a pale brown solid.
NMR (200 MHz, CDCl₃) δ: 1.57-1.67 (1H, m), 1.81-1.91 (1H, m), 2.50-2.59 (1H, m), 3.02-3.11 (1H, m), 7.60 (1H, dd, J = 8.8, 1.8), 7.84-7.97 (4H, m), 8.45 (1H, s).

### 141d) 2-[1-(Trans-{2-[(6-chloro-2-naphthyl)sulfonyl]cyclopropyl}carbonyl)-4-piperidinyl]-5-methyl-1,2-dihydro-3H-imidazo[1,5-c]imidazol-3-one

From trans-2-[(6-chloro-2-naphthyl)sulfonyl]cyclopropanecarboxylic acid (0.20 g) obtained in Example 141c) and 5-methyl-2-(4-piperidinyl)-1,2-dihydro-3H-imidazo[1,5-c]imidazol-3-one dihydrochloride (0.23 g) obtained in Example 69b), the title compound (0.12 g, 36%) was obtained as white powder in a similar manner to Example 128.
NMR (300 MHz, CDCl₃) δ: 1.44-2.05 (6H, m), 2.62 (3H, s), 2.65-2.82 (2H, m), 3.03-3.09 (1H, m), 3.20-3.30 (1H, m), 4.06-4.37 (4H, m), 4.70 (1H, d, J = 13.2), 6.73 (1H, d, J = 7.5), 7.58-7.61 (1H, m), 7.87-7.97 (4H, m), 8.45 (1H, s). Elemental analysis for C₂₅H₂₅ClN₄O₄S•0.2H₂O
Calculated (%): C, 58.12; H, 4.96; N, 10.85
Found (%): C, 57.97; H, 5.08; N, 11.13

### Example 142

### 2-(1-{3-[(6-chloro-2-naphthyl)sulfonyl]-2-hydroxy-2-methylpropanoyl}-4-piperidinyl)-5-methyl-1,2-dihydro-3H-imidazo[1,5-c]imidazol-3-one hydrochloride

### 142a) Methyl 3-[(6-chloro-2-naphthyl)thio]-2-hydroxy-2-methylpropionate

From 6-chloronaphthalene-2-thiol (5.0 g) and methyl 2-methyloxiran-2-carboxylate (2.7 mL), the title compound (5.1 g, 63%) was obtained as colorless needles in a similar manner to Example 136a).
NMR (300 MHz, CDCl₃) δ: 1.52 (3H, s), 3.27 (1H, d, J = 13.8), 3.44 (3H, s), 3.48 (1H, d, J = 13.8), 3.53 (1H, s), 7.38-7.42 (1H, m), 7.48-7.51 (1H, m), 7.64-7.69 (2H, m), 7.75 (1H, d, J = 2.1), 7.82 (1H, s).

### 142b) 3-[(6-Chloro-2-naphthyl)thio]-2-hydroxy-2-methylpopionic acid

From methyl 3-[(6-chloro-2-naphthyl)thio]-2-hydroxy-2-methylpropionate (1.5 g) obtained in Example 142a), the title compound (1.3 g, 91%) was obtained as white powder in a similar manner to Example 136b).
NMR (300 MHz, CDCl₃) δ: 1.51 (3H, s), 3.38 (1H, d, J = 13.2), 3.48 (1H, d, J = 13.2), 7.41 (1H, dd, J = 8.7, 2.1), 7.53 (1H, dd, J = 8.7, 1.8), 7.69-7.77 (2H, m), 7.81 (1H, d, J = 2.1), 7.87 (1H, d, J = 1.8).

### 142c) 2-(1-{3-[(6-Chloro-2-naphthyl)thio]-2-hydroxy-2-methylpropanoyl}-4-piperidinyl)-5-methyl-1,2-dihydro-3H-imidazo[1,5-c]imidazol-3-one

From 3-[(6-chloro-2-naphthyl)thio]-2-hydroxy-2-methylpropionic acid (0.50 g) obtained in Example 142b) and 5-methyl-2-(4-piperidinyl)-1,2-dihydro-3H-imidazo[1,5-c]imidazol-3-one dihydrochloride (0.59 g) obtained in Example 69b), the title compound (0.16 g, 20%) was obtained as white powder in a similar manner to Example 136c).
NMR (300 MHz, CDCl₃) δ: 1.45-1.61 (6H, m), 1.84-1.88 (2H, m), 2.59 (3H, s), 2.80 (1H, bs), 3.33 (1H, d, J = 13.4), 3.70 (1H, d, J = 13.4), 4.07-4.15 (2H, m), 4.31 (1H, bs), 4.60 (1H, d, J = 12.2), 4.72 (1H, d, J = 12.2), 6.66 (1H, t, J = 1.5), 7.42 (1H, dd, J = 8.7, 2.1), 7.51 (1H, dd, J = 8.7, 2.1), 7.66 (1H, s), 7.69 (1H, s), 7.77-7.78 (1H, m), 7.83-7.84 (1H, m).

### 142d) 2-(1-{3-[(6-Chloro-2-naphthyl)sulfonyl]-2-hydroxy-2-methylpropanoyl}-4-piperidinyl)-5-methyl-1,2-dihydro-3H-imidazo[1,5-c]imidazol-3-one hydrochloride

From 2-(1-{3-[(6-chloro-2-naphthyl)thio]-2-hydroxy-2-methylpropanoyl}-4-piperidinyl)-5-methyl-1,2-dihydro-3H-imidazo[1,5-c]imidazol-3-one (0.16 g) obtained in Example 142c), a colorless oil was obtained in a similar manner to Example 136b). This oil was treated with a 4N solution of hydrogen chloride in ethyl acetate (0.5 mL) to obtain the title compound (20 mg, 11%) as white powder.
NMR (300 MHz, CDCl₃) δ: 1.55 (3H, s), 1.79-1.80 (2H, m), 2.76 (3H, s), 3.41-3.45 (4H, m), 3.71-3.80 (2H, m), 4.03-4.10 (2H, m), 4.58 (2H, s), 4.80-4.91 (1H, m), 5.92 (1H, bs), 7.45 (1H, s), 7.74 (1H, dd, J = 8.7, 2.1), 8.00 (1H, d, J = 8.7), 8.17 (1H, d, J = 8.7), 8.26-8.31 (2H, m), 8.60 (1H, s).
Elemental analysis for C₂₅H₂₇ClN₄O₄S•HCl•1.2H₂O•0.1Et₂O Calculated (%): C, 51.14; H, 5.31; N, 9.39
Found (%): C, 51.03; H, 5.55; N, 9.31

### Example 143

### Tert-butyl 1-{[(6-chloro-2-naphthyl)sulfonyl]methyl}-2-[4-(5-methyl-3-oxo-1H-imidazol[1,5-c]imidazol-2-yl)-1-piperidinyl]-2-oxoethylcarbamate

### 143a) Methyl 2-(tert-butoxycarbonylamino)-3-[(6-chloro-2-naphthyl)thio]propionate

Methanesuflonyl chloride (0.99 mL) was added dropwise to a solution of N-tert-butoxycarbonylserine methyl ester (2.82 g) and triethylamine (5.35 mL) in THF (50 mL). After the mixture was stirred at room temperature for 1 hour, 6-chloronaphthlane-2-thiol (2.50 g) was added. The reaction solution was stirred at room temperature for 20 hours and the solvent was distilled off under reduced pressure. Water was added to the residue, which was extracted with ethyl acetate. The extract was washed with water and dried over anhydrous magnesium sulfate. The solvent was distilled off under reduced pressure and the residue was then purified with a silica gel column to obtain the title compound (2.79 g, 55%) as a pale brown oil.
NMR (200 MHz, CDCl₃) δ: 1.37 (9H, s), 3.46-3.50 (5H, m), 4.60 (1H, m), 5.34 (1H, m), 7.42 (1H, dd, J = 1.4, 8.8), 7.49 (1H, dd, J = 1.8, 8.4), 7.66-7.71 (2H, m), 7.77 (1H, d, J = 2.2), 7.84 (1H, d, J = 1.4)

### 143b) 2-(Tert-butoxycarbonylamino)-3-[(6-chloro-2-naphthyl)thio]propionic acid

A solution of methyl 2-(tert-butoxycarbonylamino)-3-[(6-chloro-2-naphthyl)thio]propionate (2.79 g) obtained in Example 143a) and 1N sodium hydroxide (7.7 mL) in 2-propanol (70 mL) was stirred at room temperature for 3 hours. After the solvent was distilled off under reduced pressure, water was added to the residue and the mixture was acidified with 1N HCl and then extracted with ethyl acetate. The extract was washed with water and then dried over anhydrous magnesium sulfate. The solvent was distilled off under reduced pressure and the residue was purified with a silica gel column to obtain the title compound (1.1 g, 41%) as a solid.
NMR (200 MHz, CDCl₃) δ: 1.36 (9H, s), 3.38-3.58 (3H, m), 7.41 (1H, dd, J = 2.2, 8.8), 7.50 (1H, dd, J = 1.8, 8.8), 7.65-7.84 (4H, m).

### 143c) Tert-butyl 1-{[(6-chloro-2-naphthyl)thio]methyl}-2-[4-(5-methyl-3-oxo-1H-imidazol[1,5-c]imidazol-2-yl)-1-piperidinyl]-2-oxoethylcarbamate

From 2-(tert-butoxycarbonylamino)-3-[(6-chloro-2-naphthyl)thio]propionic acid (1.1 g) obtained in Example143b) and 5-methyl-2-(4-piperidinyl)-1,2-dihydro-3H-imidazo[1,5-c]imidazol-3-one dihydrochloride (0.85 g) obtained in Example 69b), the title compound (0.55 g, 33%) was obtained in a similar manner to Example 136c).
NMR (200 MHz, CDCl₃) δ: 1.45 (9H, s), 1.55-1.81 (4H, m), 2.59 (3H, s), 3.01-3.08 (1H, m), 3.30-3.40 (2H, m), 3.90 (2H, s), 3.98-4.19 (3H, m), 4.60-4.77 (1H, m), 4.91 (1H, m), 5.49-5.52 (1H,m), 6.68 (1H, s), 7.41-7.48 (2H, m), 7.67-7.78 (3H, m), 7.86-7.90 (1H, m).

### 143d) Tert-butyl 1-{[(6-chloro-2-naphthyl)sulfonyl]methyl}-2-[4-(5-methyl-3-oxo-1H-imidazol[1,5-c]imidazol-2-yl)-1-piperidinyl]-2-oxoethylcarbamate

From tert-butyl 1-{[(6-chloro-2-naphthyl)thio]methyl}-2-[4-(5-methyl-3-oxo-1H-imidazol[1,5-c]imidazol-2-yl)-1-piperidinyl]-2-oxoethylcarbamate (0.55 g) obtained in Example143c), the title compound (0.15 g, 26%) was obtained as colorless powder in a similar manner to Example 136d). NMR (200 MHz, CDCl₃) δ: 1.27-1.44 (11H, m), 1.82-2.02 (2H, m), 2.40-2.77 (4H, m), 3.21-3.89 (2H, m), 4.23-4.29 (4H, m), 4.56-4.85 (1H, m), 5.14-5.36 (2H, m), 6.66-6.71 (1H,m), 7.60 (1H, dd, J = 2.0, 9.0), 7.94-7.98 (4H, m), 8.50 (1H, s) .

### Example 144

### 2-(1-{2-Amino-3-[(6-chloro-2-naphthyl)sulfonyl]propanoyl}-4-piperidinyl)-5-methyl-1,2-dihydro-3H-imidazo[1,5-c]imidazol-3-one dihydrochloride

A saturated solution of hydrogen chloride in ethanol (1.5 mL) was added to a solution of tert-butyl 1-{[(6-chloro-2-naphthyl)sulfonyl]methyl}-2-[4-(5-methyl-3-oxo-1H-imidazol[1,5-c]imidazol-2-yl)-1-piperidinyl]-2-oxoethylcarbamate (0.10 g) obtained in Example 143d) in ethanol (0.5 mL) and stirred at room temperature for 30 minutes. Then the solvent was distilled off under reduced pressured and the residue was washed with ether to obtain the title compound (85 mg, 90%) as colorless powder.
NMR (200 MHz, DMSO-d₆) δ: 1.53-1.86 (4H, m), 2.07-2.42 (1H, m), 2.72 (3H, br s), 3.13-3.26 (1H, m), 3.71-4.37 (5H, m), 4.50-4.55 (2H, m), 4.86 (1H, m), 7.42 (1H, s), 7.77 (1H, d, J = 8.4), 8.02 (1H, dt, J = 1.8, 7.3), 8.20-8.34 (3H, m), 8.5-9.0 (3H, m).

### Example 145

### 2-(1-{3-[(6-Chloro-3,4-dihydroisoquinolin-2(1H)-yl)sulfonyl]propanoyl}-4-piperidinyl)-5-methyl-1,2-dihydro-3H-imidazo[1,5-c]imidazol-3-one

### 145a) Methyl 3-[(6-chloro-3,4-dihydroisoquinolin-2(1H)-yl)sulfonyl]propionate

A solution of 6-chloro-1,2,3,4-tetrahydroisoqiunoline hydrochloride (0.19 g) and triethylamine (0.26 mL) in acetonitrile (1 mL) was added to a solution of methyl 3-(chlorosulfonyl)propionate (0.19 g) in acetonitrile (1 mL) and the mixture was stirred at 0°C for 30 minutes. The reaction solution was concentrated under reduced pressure, and chloroform and water were added. An organic layer was separated, washed with an aqueous saturated sodium chloride solution and then dried over anhydrous magnesium sulfate. The solvent was distilled off under reduced pressure to obtain the title compound (0.30 g, quantitative) as a light brown solid.
NMR (300 MHz, CDCl₃) δ: 2.82 (2H, t, J = 7.2), 2.93 (2H, t, J = 6.0), 3.32 (2H, t, J = 7.5), 3.57 (2H, t, J = 6.0), 3.67 (3H, s), 4.44 (2H, s), 7.01-7.03 (1H, m), 7.16-7.19 (2H, m).

### 145b) 3-[(6-chloro-3,4-dihydroisoquinolin-2(1H)-yl)sulfonyl]propionic acid]

Methyl 3-[(6-chloro-3,4-dihydroisoquinolin-2(1H)-yl)sulfonyl]propionate (0.30 g) obtained in Example 145a) was added to a 1N aqueous sodium hydroxide solution (2.7 mL), and the mixture was heated at 100°C for 20 minutes. After cooled to 0°C, the mixture was acidified by addition of 1N hydrochloric acid and a precipitated solid was filtered to obtain the title compound (0.04 g, 16%) as light brown powder.
NMR (300 MHz, CDCl₃) δ: 2.86 (2H, t, J = 7.5), 2.93 (2H, t, J = 6.0), 3.31 (2H, t, J = 7.5), 3.57 (2H, t, J = 6.0), 4.44 (2H, s), 7.01 (1H, d, J = 8.2), 7.14-7.25 (2H, m).

### 145c) 2-(1-(3-((6-Chloro-3,4-dihydro-2(1H)-isoquinolinyl)sulfonyl)propanoyl)-4-piperidinyl)-5-methyl-1,2-dihydro-3H-imidazo[1,5-c]imidazol-3-one

Triethylamine (0.06 mL) was added to a solution of 3-[(6-chloro-3,4-dihydroisoquinolin-2(1H)-yl)sulfonyl]propionic acid (0.04 g) obtained in Example145b), WSC(0.05 g) and HOBt (0.04 g) in THF (3 mL) and the mixture was stirred at room temperature for 30 minutes. Then 5-methyl-2-(4-piperidinyl)-1,2-dihydro-3H-imidazo[1,5-c]imidazol-3-one dihydrochloride (0.03 g) obtained in Example 69b) was added and the mixture was stirred at room temperature for 3 hours. The solvent was distilled off under reduced pressure and the residue was dissolved in ethyl acetate. The ethyl acetate solution was washed with an aqueous saturated sodium hydrogencarbonate solution and then dried over anhydrous magnesium sulfate. The solvent was distilled off under reduced pressure and the residue was recrystallized from ethyl acetate-ether to obtain the title compound (0.01 g, 18%) as white powder. NMR (300 MHz, CDCl₃) δ: 1.19-1.28 (1H, m), 1.60-1.75 (2H, m), 1.89-1.99 (2H, m), 2.69 (3H, s), 2.77-2.97 (3H, m), 3.19-3.52 (3H, m), 3.58 (2H, t, J = 6.0), 3.97-4.02 (1H, m), 4.09-4.23 (2H, m), 4.30 (2H, s), 4.46 (2H, s), 4.75-4.79 (1H, m), 6.81 (1H, s), 7.03 (1H, d, J = 8.1), 7.17 (1H, s), 7.18 (1H, d, J = 8.1).
Elemental analysis for C₂₃H₂₈ClN₅O₄S•H₂O
Calculated (%): C, 52.72; H, 5.77; N, 13.36
Found (%): C, 52.65; H, 5.55; N, 13.12

### Example 146

### 2-[1-(4-{[(E)-2-(4-Chlorophenyl)vinyl]sulfonyl}butanoyl)-4-piperidinyl]-5-methyl-1,2-dihydro-3H-imidazo[1,5-c]imidazol-3-one

### 146a) Sodium [(E)-2-(4-chlorophenyl)vinyl]sulfinate

(E)-2-(4-chlorophenyl)ethylenesulfonyl chloride (Matier, W. L. et al., J. Med. Chem., 17, 549 (1974)) was added to a solution of sodium sulfite (3.8 g) and sodium hydrogencarbonate (2.5 g) in water (40 mL) and the mixture was stirred at 70°C for 2 hours. The reaction solution was cooled to room temperature. A precipitated crystal was filtered and then dried to obtain the title compound (1.7 g, 50%) as pale yellow powder.
NMR (200 MHz, DMSO-d₆) δ: 6.57 (1H, d, J = 16.0), 6.78 (1H, d, J = 16.0), 7.34-7.56 (4H, m).

### 146b) 4-{[(E)-2-(4-chlorophenyl)vinyl]sulfonyl}butanoic acid

A solution of sodium [(E)-2-(4-chlorophenyl)vinyl]sulfinate (0.23 g) obtained in Example 146a) and ethyl 4-bromobutanoate (0.16 mL) in DMF (1.0 mL) was stirred at 100°C for 1 hour. After the reaction mixture was cooled to room temperature, ethyl acetate was added. The mixture was washed with water and dried over anhydrous magnesium sulfate. The solvent was distilled off under reduced pressure to obtain ethyl 4-{[(E)-2-(4-chlorophenyl)vinyl]sulfonyl}butanoate (0.27 g) as a yellow oil. From this oil without purification, the title compound (0.05 g, 16%) was obtained as white powder in a similar manner to Example 145b).
NMR (200 MHz, CDCl₃) δ: 1.83-1.94 (2H, m), 2.36-2.43 (2H, m), 3.16-3.28 (2H, m), 7.42-7.56 (4H, m), 7.78-7.82 (2H, m).

### 146c) 2-[1-(4-{[(E)-2-(4-chlorophenyl)vinyl]sulfonyl}butanoyl)-4-piperidinyl]-5-methyl-1,2-dihydro-3H-imidazo[1,5-c]imidazol-3-one

From 4-{[(E)-2-(4-chlorophenyl)vinyl]sulfonyl}butanoic acid (0.04 g) obtained in Example146b), the title compound (0.02 g, 29%) was obtained as colorless powder in a similar manner to Example 145c).
NMR (300 MHz, CDCl₃) δ: 1.60-1.74 (2H, m), 1.83-1.97 (3H, m), 2.15-2.25 (2H, m), 2.55-2.69 (5H, m), 3.12-3.28 (3H, m), 3.96-4.00 (1H, m), 4.13-4.21 (1H, m), 4.27 (2H, s), 4.76-4.81 (1H, m), 6.71 (1H, s), 6.83 (1H, d, J = 15.6), 7.35-7.47 (4H, m), 7.54 (1H, d, J = 15.6).
Elemental analysis for C₂₃H₂₇ClN₄O₄S•1.5H₂O•0.5AcOEt Calculated (%): C, 53.42; H, 6.10; N, 9.97
Found (%): C, 53.68; H, 5.85; N, 10.28

### Example 147

### 2-(1-{3-[(6-Chloro-2-naphthyl)thio]-3-methylbutanoyl}-4-piperidinyl)-5-methyl-1,2-dihydro-3H-imidazo[1,5-c]imidazol-3-one hydrochloride

### 147a) 3-[(6-chloro-2-naphthyl)thio]-3-methylbutanoic acid

A solution of 6-chloronaphthalene-2-thiol (1.0 g), 3-methyl-2-butenoic acid (0.50 g) and triethylamine (0.35 mL) in THF (40 mL) was heated to reflux overnight. After the reaction solution was cooled to room temperature, the solvent was distilled off under reduced pressure and ethyl acetate was added to the residue. The mixed solution was washed with water and dried over anhydrous magnesium sulfate. The solvent was distilled off under reduced pressure and the residue was purified with a silica gel column (ethyl acetate/hexane = 3/7 to 1/1) to obtain the title compound (0.10 g, 7%) as white powder.
NMR (300 MHz, CDCl₃) δ: 1.46 (6H, s), 2.60 (2H, s), 7.45 (1H, dd, J = 1.8, 8.4), 7.63 (1H, dd, J = 1.8, 8.3), 7.72 (1H, d, J = 8.4), 7.77 7 (1H, d, J = 8.7), 7.83 (1H, d, J = 2.1), 8.07 (1H, s).

### 147b) 2-(1-{3-[(6-Chloro-2-naphthyl)thio]-3-methylbutanoyl}-4-piperidinyl)-5-methyl-1,2-dihydro-3H-imidazo[1,5-c]imidazol-3-one hydrochloride

From 3-[(6-chloro-2-naphthyl)thio]-3-methylbutanoic acid (0.10 g) obtained in Example 147a) and 5-methyl-2-(4-piperidinyl)-1,2-dihdyro-3H-imidazo[1,5-c]imidazol-3-one dihydrochloride (0.15 g) obtained in Example 69b), the title compound (0.13 g, 74%) was obtained as white powder in a similar manner to Example 145c).
NMR (300 MHz, CDCl₃) δ: 1.46 (3H, s), 1.49 (3H, s), 1.70 (1H, s), 1.92-1.99 (1H, m), 2.57-2.65 (1H, m), 2.70 (2H, s), 2.99 (3H, s), 3.11-3.19 (1H, m), 4.01-4.06 (1H, m), 4.10-4.23 (1H, m), 4.53 (2H, s), 4.87-4.91 (1H, m), 7.24 (1H, s), 7.46 (1H, dd, J = 1.8, 8.7), 7.59 (1H, dd, J = 1.8, 8.4), 7.72 (1H, d, J = 8.7), 7.77 (1H, d, J = 8.7), 7.83 (1H, d, J = 1.8), 8.04 (1H, s).
Elemental analysis for C₂₆H₂₉N₄O₂SCl•HCl•1.5H₂O
Calculated (%): C, 55.71; H, 5.93; N, 10.00
Found (%): C, 55.92; H, 6.02; N, 10.21

### Example 148

### 2-(1-{3-[(6-Chloro-2-naphthyl)sulfonyl]-3-methylbutanoyl}-4-piperidinyl)-5-methyl-1,2-dihydro-3H-imidazo[1,5-c]imidazol-3-one

Metachloroperbenzoic acid (0.10 g) was added to a solution of 2-(1-{3-[(6-chloro-2-naphthyl)thio]-3-methylbutanoyl}-4-piperidinyl)-5-methyl-1,2-dihydro-3H-imidazo[1,5-c]imidazol-3-one hydrochloride (0.13 g) obtained in Example 147b) in methanol (2.5 mL) at 0°C, and the mixture was stirred for 3 hours. To the reaction solution was added an aqueous sodium thiosulfate solution, and this was extracted with ethyl acetate. The extract was washed with an aqueous saturated sodium hydrogencarbonate solution and dried over anhydrous magnesium sulfate. The solvent was distilled off under reduced pressure and the residue was purified with a basic silica gel column (ethyl acetate) to obtain the title compound (0.09 g, 70%) as white powder.
NMR (300 MHz, CDCl₃) δ: 1.48 (3H, s), 1.56 (3H, s), 1.60-1.83 (2H, m), 1.86-1.99 (3H, m), 2.61 (3H, s), 2.64-2.68 (1H, m), 2.88-2.98 (2H, m), 3.18-3.26 (1H, m), 4.13-4.23 (2H, m), 4.28 (2H, s), 4.79-4.84 (1H, m), 6.70 (1H, t, J = 1.5), 7.59 (1H, dd, J = 2.1, 8.7), 7.86-7.96 (4H, m), 8.41 (1H, s).
Elemental analysis for C₂₆H₂₉N₄O₄SCl•0.5H₂O•AcOEt
Calculated (%): C, 57.54; H, 6.12; N, 8.95
Found (%): C, 57.82; H, 5.90; N, 9.22

### Example 149

### 2-[1-(3-{[2-(4-Chlorophenyl)ethyl]thio}propanoyl)-4-piperidinyl]-5-methyl-1,2-dihydro-3H-imidazo[1,5-c]imidazol-3-one hydrochloride

A mixture of 2-(4-chlorophenyl)ethyl 4-methylbenzenesulfonate (Schadt, F. L. et al., J. Am. Chem. Soc., 100, 228-246 (1978)) (1.2 g), 3-mercaptopropionic acid (0.35 mL) and a 1N aqueous sodium hydroxide solution (12 mL) was stirred at 100°C overnight. The reaction mixture was cooled to room temperature, acidified with 1N hydrochloric acid, and then extracted with ethyl acetate. The extract was dried over anhydrous magnesium sulfate. The solvent was distilled off under reduced pressure to obtain a crude product of 3-{[2-(4-chlorophenyl)ethyl]thio}propionic acid (0.26 g). From this carboxylic acid and 5-methyl-2-(4-piperidinyl)-1,2-dihydro-3H-imidazo[1,5-c]imidazol-3-one dihydrochloride (0.24 g) obtained in Example 69b), the title compound (0.06 g, 13%) was obtained as white powder in a similar manner to Example 145c).
NMR (300 MHz, CDCl₃) δ: 1.56-1.70 (2H, m), 1.79-1.96 (3H, m), 2.57-2.70 (5H, m), 2.76-2.91 (5H, m), 3.11-3.19 (1H, m), 3.92-3.97 (2H, m), 4.10-4.29 (3H, m), 4.79-4.84 (1H, m), 6.69-6.71 (1H, m), 7.11-7.16 (2H, m), 7.24-7.33 (2H, m). Elemental analysis for C₂₂H₂₇N₄O₂SCl•HCl•H₂O
Calculated (%): C, 52.69; H, 6.03; N, 11.17
Found (%): C, 52.75; H, 6.43; N, 11.57

### Example 150

### 2-[1-(3-{[2-(4-Chlorophenyl)ethyl]sulfonyl}propanoyl)-4-piperidinyl]-5-methyl-1,2-dihydro-3H-imidazo[1,5-c]imidazol-3-one

From 2-[1-(3-{ [2-(4-chlorophenyl)ethyl]thio}propanoyl)-4-piperidinyl]-5-methyl-1,2-dihydro-3H-imidazo[1,5-c]imidazol-3-one hydrochloride (0.29 g) obtained in Example 149, the title compound (0.17 g, 54%) was obtained as white powder in a similar manner to Example 148.
NMR (300 MHz, CDCl₃) δ: 1.61-1.76 (2H, m), 1.90-1.99 (2H, m), 2.61 (3H, s), 2.66-2.74 (1H, m), 2.85-2.96 (2H, m), 3.13-3.21 (3H, m), 3.25-3.46 (4H, m), 3.98-4.02 (1H, m), 4.17-4.23 (1H, m), 4.26 (2H, s), 4.75-4.80 (1H, m), 6.71 (1H, s), 7.18 (2H, d, J = 7.2), 7.30 (2H, d, J = 7.2). Elemental analysis for C₂₂H₂₇N₄O₄SCl•0.5H₂O
Calculated (%): C, 54.15; H, 5.78; N, 11.48
Found (%): C, 54.35; H, 6.01; N, 11.44

### Example 151

### Tert-butyl (1S)-1-{[(6-chloro-2-naphthyl)thio]methyl}-2-[4-(5-methyl-3-oxo-1H-imidazo[1,5-c]imidazol-2(3H)-yl)-1-piperidinyl]-2-oxoethylcarbamate

### 151a) Methyl (2S)-2-(tert-butoxycarbonylamino)-3-[(6-chloro-2-naphthyl) thio] propionate

6-Chloronaphthalne-2-thiol (4.3 g) was added to a suspension of sodium hydride (60% in oil: 0.88 g) in DMF (22 mL) at 0°C and the mixture was stirred at 0°C for 20 minutes. Then, a solution of N-(tert-butoxycarbonyl)-O-[(4-methylphenyl)sulfonyl]-D-serine methyl ester (E. M. Stocking, J. Chem. Soc. Perkin Trans. 1, 2443, (1997)) (8.2 g) in DMF (22 mL) was added dropwise and the mixture was stirred at 0°C for 2 hours. Water was added to the reaction mixture and this mixture was extracted with ether. The extract was washed with an aqueous potassium carbonate solution and an aqueous saturated sodium chloride solution, and dried over anhydrous magnesium sulfate. The solvent was distilled off under reduced pressure and the residue was purified with a silica gel column (ethyl acetate/hexane 1/10 to 3/10) to obtain the title compound (6.2 g, 71%) as a white solid.
NMR (300 MHz, CDCl₃) δ: 1.37 (9H, s), 3.46-3.55 (5H, m), 4.60-4.63 (1H, m), 5.36 (1H, d, J = 7.2), 7.25-7.50 (2H, m), 7.65-7.82 (4H, m).

### 151b) (2S)-2-(Tert-butoxycarbonylamino)-3-[(6-chloro-2-naphthyl)thio]propionic acid

A 1N aqueous sodium hydroxide solution (17 mL) was added to a solution of methyl (2S)-2-(tert-butoxycarbonylamino)-3-[(6-chloro-2-naphthyl)thio]propionate (6.2 g) obtained in Example 151a) in methanol (50 mL), and the mixture was stirred at room temperature for 1 hour. After the solvent was distilled off under reduced pressure, water was added to the residue and this was washed with ether. An aqueous layer was acidified with 1N hydrochloric acid and extracted with ethyl acetate. The extract was dried over anhydrous magnesium sulfate and the solvent was distilled off under reduced pressure to obtain the title compound (5.2 g, 88%) as a pale yellow oil.
NMR (300 MHz, CDCl₃) δ: 1.36 (9H, s), 3.48-3.50 (2H, m), 4.59-4.63 (1H, m), 5.34-5.36 (1H, m), 7.33-7.52 (2H, m), 7.65-7.84 (4H, m).

### 151c) Tert-butyl (1S)-1-{[(6-chloro-2-naphthyl)thio]methyl}-2-[4-(5-methyl-3-oxo-1H-imidazo[1,5-c]imidazol-2(3H)-yl)-1-piperidinyl]-2-oxoethylcarbamate

HOBt (1.7 g), triethylamine (3.5 mL) and 5-methyl-2-(4-piperidinyl)-1,2-dihydro-3H-imidazo[1,5-c]imidazol-3-one dihydrochloride (1.7 g) obtained in Example 69b) were added to a solution of (2S)-2-(tert-butoxycarbonylamino)-3-[(6-chloro-2-naphthyl)thio]propionic acid (2.8 g) obtained in Example 151b) in acetonitrile (36 mL) at 0°C and then WSC (2.1 g) was added. The mixture was stirred at room temperature overnight. The solvent was distilled off under reduced pressure and the residue was dissolved in ethyl acetate, washed with an aqueous sodium bicarbonate solution and an aqueous saturated sodium chloride solution, and dried over anhydrous magnesium sulfate. The solvent was distilled off under reduced pressure and the residue was purified with a basic silica gel column (ethyl acetate/hexane=1/4 to ethyl acetate). A product was recrystallized from ethyl acetate to obtain the title compound (1.9 g, 45%) as colorless needles.
NMR (300 MHz, CDCl₃) δ: 1.45 (9H, s), 1.49-1.81 (4H, m), 2.59 (3H, s), 2.96-3.08 (1H, m), 3.30-3.40 (2H, m), 3.90-3.99 (2H, m), 4.07-4.19 (3H, m), 4.60-4.91 (2H, m), 5.52 (1H, d, J = 8.0), 6.69 (1H, s), 7.41-7.48 (2H, m), 7.67-7.78 (3H, m), 7.85-7.90 (1H, m).
Elemental analysis for C₂₉H₃₉ClN₅O₄S•0.5H₂O
Calculated (%): C, 58.72; H, 5.95; N, 11.81
Found (%): C, 58.82; H, 6.11; N, 11.71

### Example 152

### Tert-butyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate

A solution of oxone (0.64 g) in water (3.0 mL) was added to a solution of tert-butyl (1S)-1-{[(6-chloro-2-naphthyl)thio]methyl}-2-[4-(5-methyl-3-oxo-1H-imidazo[1,5-c]imidazol-2(3H)-yl)-1-piperidinyl]-2-oxoethylcarbamate (0.51 g) obtained in Example 151c) in methanol (3.0 mL), and the mixture was stirred at room temperature for 1 hour. The solvent was distilled off under reduced pressure, and the residue was diluted with water and extracted with ethyl acetate. The extract was dried over anhydrous magnesium sulfate, and the solvent was distilled off under reduced pressure. The residue was purified with a basic silica gel column (ethyl acetate/hexane=1/4 to ethyl acetate) to obtain the title compound (0.51 g, 95%: 90%ee) as white powder.
NMR (300 MHz, CDCl₃) δ: 1.34 (9H, s), 1.92-2.15 (4H, m), 2.61 (3H, s), 3.13-3.61 (3H, m), 3.78-3.89 (1H, m), 4.11-4.41 (4H, m), 4.58-4.84 (1H, m), 5.22-5.35 (2H, m), 6.66-6.70 (1H, m), 7.57-7.62 (1H, m), 7.93-7.98 (4H, m), 8.50 (1H, s).
Elemental analysis for C₂₉H₃₄ClN₅O₆S•0.5H₂O•0.5AcOEt Calculated (%): C, 55.64; H, 5.87; N, 10.47
Found (%): C, 55.64; H, 6.11; N, 10.54

### Example 153

### N-((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)-2,2,2-trifluoroacetamide

Trifluoroacetic acid (2.0 mL) was added to a solution of tert-butyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate (0.51 g) obtained in Example 152 in dichloromethane (2.0 mL) at 0°C, and the mixture was stirred at room temperature overnight. The reaction solution was basified by addition of an aqueous saturated sodium hydrogencarbonate solution and potassium carbonate and then extracted with dichloromethane. The extract was dried over anhydrous magnesium sulfate, and the solvent was distilled off under reduced pressure. The residue was purified with a basic silica gel column (ethyl acetate/hexane = 1/4 to ethyl acetate) to obtain the title compound (0.13 g, 26%) as white powder.
NMR (200 MHz, CDCl₃) δ: 1.94-2.60 (3H, m), 2.66 (3H, s), 2.72-2.85 (1H, m), 3.27-3.88 (4H, m), 4.17-4.31 (4H, m), 4.56-4.83 (1H, m), 5.47-5.60 (1H, m), 6.67-6.71 (1H, m), 7.46-7.64 (2H, m), 7.74-7.99 (5H, m), 8.48-8.49 (1H, m). Elemental analysis for C₂₆H₂₅ClN₅O₅S•3H₂O
Calculated (%): C, 46.88; H, 4.69; N, 10.51
Found (%): C, 46.91; H, 4.95; N, 10.36

### Example 154

### 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrolchloride

A 40% solution of hydrogen chloride in ethanol (5.0 mL) was added to a solution of tert-butyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate (1.1 g) obtained in Example 152 in methanol (2.0 mL) at 0°C, and the mixture was stirred for 10 minutes. The solvent was distilled off under reduced pressure, and the residue was crystallized with ether to obtain the title compound (0.83 g, 77%) as white powder. NMR (300 MHz, DMSO-d₆) δ: 1.41-1.89 (3H, m), 2.15-2.37 (1H, m), 2.74-2.76 (3H, m), 3.16-3.33 (1H, m), 3.78-4.32 (5H, m), 4.52-4.57 (2H, m), 4.80-4.89 (1H, m), 7.50-7.53 (1H, m), 7.73-7.77 (1H, m), 7.96-8.07 (1H, m), 8.18-8.33 (3H, m), 8.64-8.93 (4H, m).
Elemental analysis for C₂₄H₂₆ClN₅O₄S•2HCl•2.5H₂O•0.1Et₂O Calculated (%): C, 45.76; H, 5.35; N, 10.94
Found (%): C, 45.64; H, 5.74; N, 11.12

### Example 155

### N-((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)acetamide

Triethylaimne (0.11 mL) and acetyl chloride (0.02 mL) were added to a solution of 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrolchloride (0.15 g) obtained in Example 154 in dichloromethane (2.0 mL) at 0°C, and the mixture was stirred for 2 hours. An aqueous saturated sodium hydrogencarbonate solution was added to the reaction mixture and this mixture was extracted with dichloromethane. The extract was dried over anhydrous magnesium sulfate, and solvent was distilled off under reduced pressure. The residue was purified by preparative high performance liquid chromatography to obtain the title compound (0.08 g, 54%) as a white solid.
NMR (300 MHz, CDCl₃) δ: 1.72-1.80 (5H, m), 1.86-2.17 (3H, m), 2.61-2.80 (4H, m), 3.16-3.32 (1H, m), 3.43-3.87 (2H, m), 4.18-4.39 (4H, m), 4.58-4.83 (1H, m), 5.52-5.69 (1H, m), 6.25-6.40 (1H, m), 6.66-6.70 (1H, m), 7.61 (1H, dd, J = 2.0, 8.8), 7.87-7.98 (4H, m), 8.50 (1H, s).
Elemental analysis for C₂₆H₂₈ClN₅O₅S•2.5H₂O•0.25AcOEt Calculated (%): C, 51.88; H, 5.64; N, 11.20
Found (%): C, 51.71; H, 5.26; N, 11.03

### Example 156

### Methyl N-((1S)-1-{[(6-chloro-2-naphthyl)sulfonyl]methyl}-2-[4-(5-methyl-3-oxo-1H-imidazo[1,5-c]imiadzol-2(3H)-yl)-1-piperidinyl]-2-oxoethyl)carbamate

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.10 g) obtained in Example 154 and methyl chloroformate (0.01 mL), the title compound (0.06 g, 61%) was obtained as a white solid in a similar manner to Example 155.
NMR (300 MHz, CDCl₃) δ: 1.63-2.02 (3H, m), 2.61 (3H, s), 2.67-2.81 (1H, m), 3.20-3.33 (1H, m), 3.43-3.55 (4H, m), 3.80-3.87 (1H, m), 4.18-4.39 (4H, m), 4.60-4.82 (1H, m), 5.30-5.40 (2H, m), 6.66-6.70 (1H, m), 7.60 (1H, dd, J = 2.1, 8.9), 7.87-7.97 (4H, m), 8.49 (1H, s).
Elemental analysis for C₂₆H₂₈ClN₅O₆S•1.5H₂O•0.25AcOEt Calculated (%): C, 52.04; H, 5.34; N, 11.24
Found (%): C, 51.82; H, 5.03; N, 11.06

### Example 157

### N-((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)methanesulfonamide

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.10 g) obtained in Example 154 and methanesulfonyl chloride (0.07 mL), the title compound (0.04 g, 41%) was obtained as a white solid in a similar manner to Example 155.
NMR (300 MHz, CDCl₃) δ: 1.61-1.91 (4H, m), 2.61 (3H, s), 2.63-2.80 (1H, m), 2.95-3.07 (3H, m), 3.21-3.77 (3H, m), 4.17-4.28 (4H, m), 4.59-4.81 (1H, m), 5.10-5.19 (1H, m), 5.50-5.65 (1H, m), 6.67-6.70 (1H, m), 7.60-7.63 (1H, m), 7.88-7.98 (4H, m), 8.49-8.51 (1H, m).
Elemental analysis for C₂₅H₂₈ClN₅O₆S•2H₂O•0.5AcOEt
Calculated (%): C, 48.10; H, 5.38; N, 10.39
Found (%): C, 48.04; H, 5.00; N, 10.37

### Example 158

### N-((1S)-1-{[(6-chloro-2-naphthyl)sulfonyl]methyl}-2-{4-(5-methyl-3-oxo-1H-imidazo[1,5-c]imidazol-2(3H)-yl)-1-piperidinyl}-2-oxoethyl)-4-methylbenzenesulfonamide

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.10 g) obtained in Example 154 and p-toluenesulfonyl chloride (0.03 g), the title compound (0.08 g, 70%) was obtained as a white solid in a similar manner to Example 155.
NMR (300 MHz, CDCl₃) δ: 1.68-1.94 (4H, m), 2.26-2.36 (1H, m), 2.43-2.45 (3H, m), 2.60-2.61 (3H, m), 2.82-3.37 (2H, m), 3.60-3.76 (1H, m), 4.09-4.24 (3H, m), 4.43-4.56 (1H, m), 4.49-4.98 (1H, m), 5.69-5.84 (1H, m), 6.14-6.71 (1H, m), 7.28-7.32 (3H, m), 7.59-7.96 (7H, m), 8.37-8.45 (1H, m). Elemental analysis for C₃₁H₃₂ClN₅O₆S•H₂O•0.5AcOEt
Calculated (%): C, 51.78; H, 4.83; N, 9.58
Found (%): C, 51.74; H, 4.87; N, 9.37

### Example 159

### N-((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)-N'-ethylurea

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.12 g) obtained in Example 154 and ethyl isocyanate (0.02 mL), the title compound (0.06 g, 52%) was obtained as a white solid in a similar manner to Example 155.
NMR (300 MHz, CDCl₃) δ: 0.96-1.04 (3H, m), 1.79-2.19 (3H, m), 2.60-2.79 (4H, m), 3.00-3.33 (3H, m), 3.46-3.81 (2H, m), 4.09-4.16 (2H, m), 4.24-4.29 (2H, m), 4.41-4.77 (3H, m), 5.44-5.61 (2H, m), 6.66-6.69 (1H, m), 7.56-7.61 (1H, m), 7.89-7.95 (4H, m), 8.48 (1H, s).
Elemental analysis for C₂₇H₃₁ClN₆O₅S•1.5H₂O•0.25AcOEt Calculated (%): C, 52.87; H, 5.70; N, 13.21
Found (%): C, 53.05; H, 5.48; N, 13.28

### Example 160

### N-((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)urea

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.12 g) obtained in Example 154 and trimethylsilyl isocyanate (0.03 mL), the title compound (0.07 g, 61%) was obtained as a white solid in a similar manner to Example 155.
NMR (300 MHz, CDCl₃) δ: 1.74-2.15 (4H, m), 2.60-2.79 (3H, m), 3.13-3.35 (1H, m), 3.46-3.56 (2H, m), 3.75-3.81 (1H, m), 4.11-4.43 (4H, m), 4.58-4.77 (3H, m), 5.37-5.58 (1H, m), 5.75-5.87 (1H, m), 6.66-6.68 (1H, m), 7.58-7.61 (1H, m), 7.89-7.96 (4H, m), 8.48 (1H, s).
Elemental analysis for C₂₅H₂₇ClN₆OₛS•0.75H₂O
Calculated (%): C, 52.44; H, 5.02; N, 14.68
Found (%): C, 52.45; H, 5.28; N, 14.40

### Example 161

### Ethyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.12 g) obtained in Example 154 and ethyl chlorocarbonate (0.03 mL), the title compound (0.12 g, 70%) was obtained as a white solid in a similar manner to Example 155.
NMR (300 MHz, CDCl₃) δ: 1.07-1.15 (3H, m), 1.85-2.07 (4H, m), 2.61 (3H, s), 2.66-2.81 (1H, m), 3.19-3.56 (2H, m), 3.80-4.44 (7H, m), 4.59-4.84 (1H, m), 5.30-5.41 (2H, m), 6.66-6.70 (1H, m), 7.53-7.62 (1H, m), 7.87-7.97 (4H, m), 8.49 (1H, s).
Elemental analysis for C₂₇H₃₀ClN₅O₆S•0.5H₂O•0.5AcOEt Calculated (%): C, 54.33; H, 5.50; N, 10.92
Found (%): C, 54.30; H, 5.45; N, 11.16

### Example 162

### N-((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)-2-methoxyacetamide

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.20 g) obtained in Example 154 and methoxyacetyl chloride (0.04 mL), the title compound (0.09 g, 44%) was obtained as a white solid in a similar manner to Example 155.
NMR (300 MHz, CDCl₃) δ: 1.83-2.17 (3H, m), 2.62-2.81 (4H, m), 3.22 (3H, s), 3.26-3.36 (1H, m), 3.43-3.95 (5H, m), 4.11-4.39 (4H, m), 4.60-4.84 (1H, m), 5.53-5.74 (1H, m), 6.69-6.71 (1H, m), 6.99-7.06 (1H, m), 7.59-7.63 (1H, m), 7.90-7.98 (4H, m), 8.50 (1H, s).
Elemental analysis for C₂₇H₃₀ClN₅O₆S•0.5H₂O
Calculated (%): C, 54.31; H, 5.23; N, 11.73
Found (%): C, 54.24; H, 5.01; N, 11.56

### Example 163

### Tert-butyl (1R)-1-{[(6-chloro-2-naphthyl)sulfonyl]methyl}-2-[4-(5-methyl-3-oxo-1H-imdazo[1,5-c]imidazol-2(3H)-yl)-1-piperidinyl]-2-oxoethylcarbamate

Tert-butyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate (2.01 g, 90%ee) obtained in Example152 was optically resolved using CHIRALCEL OD (hexane/isopropanol = 20/80) to obtain the title compound (0.15 g, 96.8%ee) as a colorless solid.
NMR (300 MHz, CDCl₃) δ: 1.35 (9H, s), 1.63-2.24 (4H, m), 2.57-2.82 (4H, m), 3.17-3.67 (8H, m), 3.82-3.89 (1H, m), 4.16-4.42 (4H, m), 4.61-4.86 (1H, m), 5.14-5.54 (2H, m), 6.96 (1H, s), 7.59-7.62 (1H, m), 7.90-7.97 (4H, m), 8.49 (1H, s).
Elemental analysis for C₂₉H₃₄ClN₅O₆S•H₂O•0.6CH₂Cl₂
Calculated (%): C, 51.89; H, 5.47; N, 10.22
Found (%): C, 52.02; H, 5.44; N, 9.99

### Example 164

### N-((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)benzamide

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.20 g) obtained in Example 154 and benzoyl chloride (0.05 mL), the title compound (0.08 g, 36%) was obtained as a white solid in a similar manner to Example 155.
NMR (300 MHz, CDCl₃) δ: 1.88-2.14 (4H, m), 2.61-2.79 (4H, m), 3.23-3.37 (1H, m), 3.58-4.02 (2H, m), 4.22-4.42 (4H, m), 4.64-4.88 (1H, m), 5.68-5.77 (1H, m), 6.69 (1H, s), 6.80-6.91 (1H, m), 7.26-7.30 (2H, m), 7.45-7.61 (4H, m), 7.80-7.93 (4H, m), 8.48-8.50 (1H, m).
Elemental analysis for C₃₁H₃₀ClN₅O₅S•O.5H₂O
Calculated (%): C, 59.18; H, 4.97; N, 11.13
Found (%): C, 58.96; H, 5.10; N, 10.89

### Example 165

### Phenyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.20 g) obtained in Example 154 and phenyl chloroformate (0.04 mL), the title compound (0.11 g, 52%) was obtained as a white solid in a similar manner to Example 155.
NMR (300 MHz, CDCl₃) δ: 1.55-2.14 (3H, m), 2.61 (3H, s), 2.66-2.83 (1H, m), 3.20-3.35 (1H, m), 3.54-3.91 (2H, m), 4.16-4.40 (4H, m), 4.63-4.85 (1H, m), 5.30-5.46 (1H, m), 5.85-5.99 (1H, m), 6.67 (1H, s), 6.81-6.91 (2H, m), 7.16-7.30 (4H, m), 7.56-7.61 (1H, m), 7.91-7.97 (4H, m), 8.51 (1H, s).
Elemental analysis for C₃₁H₃₀ClN₅O₆S•H₂O
Calculated (%): C, 56.92; H, 4.93; N, 10.71
Found (%): C, 57.29; H, 5.00; N, 10.34

### Example 166

### 3-(1-{3-[(6-Chloro-2-naphthyl)sulfonyl]propionyl}-4-hydroxy-4-piperidinyl)-5,6,7,8-tetrahydroimidazo[1,5-a]pyridine hydrochloride

### 166a) N-(2-pyridinyl)methylformamide

A solution of 2-aminomethylpyridine (20.0 g) in formic acid (56 mL) was refluxed for 3 hours. The solvent was distilled off under reduced pressure, and ice-water was added to the residue, which was adjusted to pH 10 with a 8N aqueous sodium hydroxide solution and then extracted with chloroform. The extract was washed with an aqueous saturated sodium chloride solution and dried over anhydrous magnesium sulfate. The solvent was distilled off under reduced pressure and the residue was distilled to obtain the title compound (14.69 g, 58%) as a yellow liquid.
NMR (200 MHz, CDCl₃) δ: 4.61 (2H, d, J = 5.2), 7.06 (1H, br), 7.18-7.30 (2H, m), 7.64-7.73 (1H, m), 8.33 (1H, s), 8.54 (1H, d, J = 4.8).

### 166b) Imidazo[1,5-a]pyridine

From N-(2-pyridinyl)methylformamide (10.0 g) obtained in Example 166a), the title compound (6.38 g 74%) was obtained as a pale yellow solid in a similar manner to Example 225b).
NMR (200 MHz, CDCl₃) δ: 6.50-6.57 (1H, m), 6.65-6.73 (1H, m), 7.41-7.46 (2H, m), 7.91 (1H, dd, J = 7.0, 1.2), 8.10 (1H, s).

### 166c) Tert-butyl 4-hydroxy-4-(imidazo[1,5-a]pyridine-3-yl)piperidine-1-carboxylate

A 2 M solution (35 mL) of phenyllithium in THF was added to a solution of imidazo[1,5-a]pyridine (5.91 g) obtained in Example 166b) in diethyl ether (60 mL)-THF (10 mL) at room temperature, and the mixture was stirred at room temperature for 3 hours under argon atmosphere. To the resulting red suspension was added tert-butyl 4-oxopiperidine-1-carboxylate (24.91 g) in portions at 0°C. The reaction solution was stirred at room temperature for 16 hours under argon atmosphere. Ice-water was added, and this was extracted with ethyl acetate. The extract was washed with an aqueous saturated sodium chloride solution and dried over anhydrous magnesium sulfate. The solvent was distilled off under reduced pressure, and the residue was purified with a silica gel column (ethyl acetate) to obtain the title compound (3.97 g, 25%) as a white solid. NMR (200 MHz, CDCl₃) δ: 1.45 (9H, s), 1.88-2.07 (2H, m), 2.12-2.37 (2H, m), 2.99 (1H, br), 3.29-3.51 (2H, m), 3.74-3.91 (2H, m), 6.48-6.56 (1H, m), 6.67-6.74 (1H, m), 7.21 (1H, s), 7.38-7.43 (1H, m), 8.53 (1H, dd, J = 7.4, 0.8).

### 166d) 3-(4-Hydroxy-4-piperidinyl)imidazo[1,5-a]pyridine dihydrochloride

From tert-butyl 4-hydroxy-4-(imidazo[1,5-a]pyridine-3-yl)piperidine-1-carboxylate (3.17 g) obtained in Example 166c), the title compound (2.55 g, 88%) was obtained as a white solid in a similar manner to Example 207c).
NMR (200 MHz, DMSO-d₆) δ: 2.21-2.36 (2H, m), 2.43-2.62 (2H, m), 3.16-3.38 (4H, m), 7.07 (1H, t, J = 4.2), 7.18 (1H, t, J = 4.4), 7.83 (1H, d, J = 6.2), 8.00 (1H, s), 8.88 (1H, d, J = 4.6), 9.24-9.57 (2H, br).

### 166e) 3-(1-{3-[(6-chloro-2-naphthyl)sulfonyl]propionyl}-4-hydroxy-4-piperidinyl)-5,6,7,8-tetrahydroimidazo[1,5-a]pyridine hydrochloride

From 3-(4-hydroxy-4-piperidinyl)imidazo[1,5-a]pyridine dihydrochloride (0.65 g) obtained in Example 166d) and 3-[2-(6-chloro-2-naphthyl)sulfonyl]propionate (0.45 g), the title compound (0.52 g, 70%) was obtained as a white solid in a similar manner to Example 207d).
NMR (200 MHz, DMSO-d₆) δ: 1.80-2.24 (4H, m), 2.77 (2H, t, J = 7.4), 3.07-3.30 (2H, m), 3.44-3.56 (1H, m), 3.64 (2H, t, J = 7.2), 3.79-3.95 (1H, m), 5.72 (1H, s), 6.60 (1H, t, J = 7.4), 6.74 (1H, dd, J = 8.8, 2.2), 7.28 (1H, s), 7.53 (1H, d, J = 8.8), 7.68 (1H, dd, J = 8.8, 2.2), 7.99 (1H, dd, J = 8.8, 1.6), 8.14-8.27 (3H, m), 8.58-8.64 (2H, m).

### Example 167

### 2-Methoxyethyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.40 g) obtained in Example 154 and methoxyethyl chlorocarbonate (0.19 mL), the title compound (0.20 g, 47%) was obtained as a white solid in a similar manner to Example 155.
NMR (300 MHz, CDCl₃) δ: 1.83-2.14 (6H, m), 2.61 (3H, s), 2.65-2.80 (1H, m), 3.31-3.32 (3H, m), 3.44-3.86 (5H, m), 4.25-4.41 (4H, m), 4.59-4.82 (1H, m), 5.26-5.50 (2H, m), 6.66-6.70 (1H, m), 7.58-7.62 (1H, m), 7.87-7.97 (4H, m), 8.48-8.49 (1H, m).
Elemental analysis for C₂₈H₃₂ClN₅O₇S•H₂O
Calculated (%): C, 52.87; H, 5.39; N, 11.01
Found (%): C, 52.60; H, 5.10; N, 10.91

### Example 168

### N-{(1S)-1-{[(6-chloro-2-naphthyl)sulfonyl]methyl}-2-[4-(5-methyl-3-oxo-1H-imidazo[1,5-c]imidazol-2(3H)-yl)piperidin-1-yl]-2-oxoethyl}isonicotinamide

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.20 g) obtained in Example 154 and isonicotinoyl chloride hydrochloride (0.06 mg), the title compound (0.08 g, 38%) was obtained as a white solid in a similar manner to Example 155.
NMR (300 MHz, DMSO-d₆) δ: 1.52-1.74 (4H, m), 2.57-2.72 (1H, m), 3.12-3.26 (7H, m), 3.89-4.03 (3H, m), 4.18-4.37 (2H, m), 5.26-5.39 (1H, m), 6.62-6.70 (1H, m), 7.30 (2H, d, J = 4.5), 7.61-7.64 (1H, m), 7.87-7.91 (1H, m), 8.00-8.04 (1H, m), 8.15 (1H, d, J = 8.7), 8.48 (2H, d, J = 5.7), 8.54 (1H, d,
J = 4.8), 8.98-9.00 (1H, m).
Elemental analysis for C₃₀H₂₉ClN₆O₅S•H₂O
Calculated (%): C, 56.38; H, 4.89; N, 13.15
Found (%): C, 56.76; H, 4.83; N, 13.31

### Example 169

### 2-(Benzyloxy)ethyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.60 g) obtained in Example 154 and 2-benzyloxyethyl chloroformate (0.38 mL), the title compound (0.50 g, 70%) was obtained as a white solid in a similar manner to Example 155.
NMR (300 MHz, CDCl₃) δ: 1.69-2.06 (4H, m), 2.61 (3H, s), 3.21-3.25 (1H, m), 3.42-3.53 (4H, m), 3.81-3.87 (1H, m), 3.95-4.28 (4H, m), 4.49 (2H, s), 4.58-4.82 (1H, m), 5.25-5.50 (2H, m), 6.66 (1H, s), 7.27-7.35 (6H, m), 7.57-7.61 (1H, m), 7.90-7.96 (4H, m), 8.48 (1H, s).
Elemental analysis for C₃₄H₃₆ClN₅O₇S
Calculated (%): C, 58.83; H, 5.23; N, 10.09
Found (%): C, 58.51; H, 5.37; N, 9.84

### Example 170

### 2-Chloroethyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate

From 2-(1-{(2S)-2-amino-3-[(6-chloro-2-naphthyl)sulfonyl]propanoyl)-4-piperidinyl}-5-methyl-1,2-dihydro-3H-imidazo[1,5-c]imidazol-3-one dihydrochloride (0.59 g) obtained in Example 154 and 2-chloroethyl chloroformate (0.11 mL), the title compound (0.43 g, 69%) was obtained as a white solid in a similar manner to Example 155.
NMR (300 MHz, CDCl₃) δ: 1.61-2.13 (4H, m), 2.60 (3H, s), 2.65-2.81 (1H, m), 3.19-3.34 (1H, m), 3.45-3.56 (3H, m), 3.80-3.87 (1H, m), 4.17-4.87 (5H, m), 4.59-4.82 (1H, m), 5.25-5.44 (1H, m), 5.61-5.71 (1H, m), 6.66-6.70 (1H, m), 7.59-7.62 (1H, m), 7.87-7.97 (4H, m), 8.50 (1H, s). Elemental analysis for C₂₇H₂₉Cl₂N₅O₆S
Calculated (%): C, 52.09; H, 4.70; N, 11.25
Found (%): C, 51.75; H, 4.80; N, 10.92

### Example 171

### 2-{1-[(2S)-3-[(6-chloro-2-naphthyl)sulfonyl]-2-(2-oxo-1,3-oxazolidin-3-yl)propanoyl]-4-piperidinyl}-5-methyl-1,2-dihydro-3H-imidazo[1,5-c]imidazol-3-one

Sodium hydride (60% in oil: 0.03 g) was added to a solution of 2-chloroethyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate (0.20 g) obtained in Example 170 in DMF (3 mL) at 0°C, and the mixture was stirred for 10 minutes. Water was added to the reaction mixture, and this was then extracted with dichloromethane. The organic layer was dried over anhydrous magnesium sulfate. The solvent was distilled off under reduced pressure, and the residue was purified by preparative high performance liquid chromatography to obtain the title compound (0.11 g, 60%) as a white solid.
NMR (300 MHz, CDCl₃) δ: 1.88-2.02 (3H, m), 2.56-2.79 (4H, m), 3.08-3.75 (5H, m), 3.93-4.44 (7H, m), 4.57-4.76 (1H, m), 5.41-5.61 (1H, m), 6.67 (1H, s), 7.60-7.63 (1H, m), 7.95-8.00 (4H, m), 8.52 (1H, s).
Elemental analysis for C₂₇H₂₈ClN₅O₆S•H₂O•AcOEt
Calculated (%): C, 48.50; H, 4.35; N, 9.75
Found (%): C, 48.11; H, 4.39; N, 10.15

### Example 172

### Tert-butyl ((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)methylcarbamate

Methyl iodide (0.31 mL) and sodium hydride (0.04 g) were added to a solution of tert-butyl (1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethylcarbamate (0.31 g) obtained in Example 152 in DMF (2.0 mL) at 0°C, and the mixture was stirred for 10 minutes. Water was added to the reaction mixture, and this was extracted with ethyl acetate. The extract was dried over anhydrous magnesium sulfate. The solvent was distilled off under reduced pressure, and the residue was purified by preparative high performance liquid chromatography to obtain the title compound (0.30 g, 96%) as white powder. NMR (300 MHz, CDCl₃) δ: 1.46-1.98 (16H, m), 2.61 (3H, s), 2.69-2.75 (3H, m), 2.89-3.73 (3H, m), 4.24-4.69 (5H, m), 5.67-5.82 (1H, m), 6.70 (1H, s), 7.57-7.61 (1H, m), 7.88-7.98 (4H, m), 8.51 (1H, s).
Elemental analysis, for C₃₀H₃₆ClN₅O₆S•0.2H₂O•0.5AcOEt Calculated (%): C, 56.70; H, 6.01; N, 10.33
Found (%): C, 56.91; H, 6.41; N, 10.03

### Example 173

### 2-(1-{(2S)-3-[(6-chloro-2-naphthyl)sulfonyl]-3-(methylamino)propanoyl}-4-piperidinyl)-5-methyl-1,2-dihydro-3H-imidazo[1,5-c]imidazol-3-one dihydrochloride

A 4N solution (3 mL) of hydrogen chloride in ethyl acetate was added to tert-butyl ((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)methylcarbamate (0.20 g) obtained in Example 172, and the mixture was stirred at room temperature for 10 minutes. The solvent was distilled off under reduced pressure to obtain the title compound (0.10 g, 49%) as a white solid.
NMR (300 MHz, DMSO-d₆) δ: 1.70-2.12 (2H, m), 2.32-2.40 (1H, m), 2.57-2.59 (3H, m), 2.75-2.77 (3H, m), 3.22-4.41 (8H, m), 4.53-4.60 (2H, m), 4.93-4.96 (1H, m), 7.49 (1H, s), 7.78 (1H, d, J = 8.9), 7.98-8.07 (1H, m), 8.20-8.36 (3H, m), 8.64-8.72 (1H, m), 9.84 (1H, s).
Elemental analysis for C₂₅H₂₈ClN₅O₄S•2HCl•1.5H₂O•1.0AcOEt Calculated (%): C, 48.51; H, 5.75; N, 9.75
Found (%): C, 48.29; H, 5.96; N, 9.92

### Example 174

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl cyclohexylcarbamate

### 174a) (2S)-3-[(6-chloro-2-naphthyl)sulfonyl]-2-hydroxypropionic acid

An solution of oxone (6.6 g) in water (150 mL) was added to a suspension of (2S)-3-[(6-chloro-2-naphthyl)thio]-2-hydroxypropionic acid (1.5 g) obtained in Example 136b) in acetone (150 mL) at 0°C, and the mixture was stirred at room temperature for 5 hours. A precipitate was filtered, washed with water, and dried to obtain the title compound (1.4 g, 82%) as white powder.
NMR (300 MHz, CD₃OD) δ: 3.65 (1H, dd, J = 14.7, 8.1), 3.76 (1H, dd, J = 14.7, 3.3), 4.59 (1H, dd, J = 8.1, 3.3), 7.62 (1H, dd, J = 8.9, 2.3), 7.94-8.09 (4H, m), 8.55 (1H, s).

### 174b) Tert-butyl 4-[5-({[(cyclohexylamino)carbonyl]oxy}methyl)-3-oxo-1H-imidazo[1,5-c]imidazol-2(3H)-yl]piperidine-1-carboxylate

Cyclohexyl isocyanate (0.61 mL) was added to a solution of tert-butyl 4-[5-(hydroxymethyl)-3-oxo-1H-imidazo[1,5-c]imidazol-2(3H)-yl]piperidine-1-carboxylate (0.20 g) obtained in Example 185b) in THF (5.0 mL) at room temperature, and the mixture was stirred at 50°C for 2 days. After the reaction mixture was cooled to room temperature, dichloromethane was added. This mixture was washed with an aqueous saturated sodium carbonate solution and dried over anhydrous magnesium sulfate. The solvent was distilled off under reduced pressure, and the residue was purified with a basic silica gel column (ethyl acetate) to obtain the title compound (0.28 g, 99%) as a colorless oil.
NMR (300 MHz, CDCl₃) δ: 1.05-1.41 (6H, m), 1.47 (9H, s), 1.50-1.71 (6H, m), 1.83-1.95 (4H, m), 2.77-2.86 (2H, m), 3.48-3.51 (1H, m), 4.25-4.32 (3H, m), 4.72 (1H, d, J = 7.8), 5.35 (2H, s), 6.85 (1H, t, J = 1.2).

### 174c) (3-Oxo-2-(4-piperidinyl)-2,3-dihydro-1H-imidazo[1,5-c]imidazol-5-yl)methyl cyclohexylcarbamate dihydrochloride

A 40% solution of hydrogen chloride in ethanol (2.0 mL) was added to a solution of tert-butyl 4-[5-({[(cyclohexylamino)carbonyl]oxy}methyl)-3-oxo-1H-imidazo[1,5-c]imidazol-2(3H)-yl]piperidine-1-carboxlate (0.28 g) obtained in Example 174b) in ethanol (2.0 mL), and the mixture was stirred at room temperature for 30 minutes. The solvent was distilled off under reduced pressure to obtain the title compound (0.26 g, quantitative) as a colorless solid.
NMR (300 MHz, DMSO-d₆) δ: 1.03-1.30 (5H, m), 1.52-2.13 (5H, m), 2.98-3.45 (4H, m), 4.08-4.16 (1H, m), 4.58 (2H, s), 5,25 (2H, s), 5.36-5.76 (4H, m), 7.22-7.34 (2H, m), 9.05-9.18 (2H, m).

### 174d) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl cyclohexylcarbamate

From [3-oxo-2-(4-piperidinyl)-2,3-dihydro-1H-imidazo[1,5-c]imidazol-5-yl]methyl cyclohexylcarbamate dihydrochloride (0.26 g) obtained in Example 174c) and (2S)-3-[(6-chloro-2-naphthyl)sulfonyl]-2-hydroxypropionic acid (0.21 g) obtained in Example 174a), the title compound (0.11 g, 34%) was obtained as a colorless solid in a similar manner to Example 128.
NMR (300 MHz, CDCl₃) δ: 1.09-1.31 (6H, m), 1.53-1.96 (8H, m), 2.70-2.88 (1H, m), 3.15-3.31 (1H, m), 3.44-3.52 (3H, m), 3.74-3.89 (1H, m), 4.20-4.35 (4H, m), 4.68-4.78 (2H, m), 5.00-5.06 (1H, m), 5.36 (2H, s), 6.87 (1H, s), 7.58-7.62 (1H, m), 7.91-7.98 (4H, m), 8.51 (1H, s).
Elemental analysis for C₃₁H₃₆ClN₅O₇S•0.5H₂O
Calculated (%): C, 55.81; H, 5.59; N, 10.50
Found (%): C, 55.93; H, 5.79; N, 10.15

### Example 175

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 1-acetylpiperidine-4-carboxylate

### 175a) Tert-butyl 4-[5-({[(1-acetyl-1-piperidinyl)carbonyl]oxy}methyl)-3-oxo-1H-imidazo[1,5-c]imidazol-2(3H)-yl]piperidine-1-carboxylate

WSC (0.23 g) was added to a solution of tert-butyl 4-[5-(hydroxymethyl)-3-oxo-1H-imidazo[1,5-c]imidazol-2(3H)-yl]piperidine-1-carboxylate (0.25 g) obtained in Example 185b), 1-acetylpiperidine-4-carboxylic acid (0.15 g) and DMAP (0.02 g) in dichloromethane (3.0 mL), and the mixture was stirred at room temperature for 2 hours. To the reaction mixture was added dichloromethane, and this was washed with an aqueous saturated sodium chloride solution and dried over anhydrous magnesium sulfate. The solvent was distilled off under reduced pressure to obtain the title compound as a colorless oil. This was used in the next reaction without purification.
LCMS (m/z) 490.2

### 175b) [3-Oxo-2-(4-piperidinyl)-2,3-dihydro-1H-imidazo[1,5-c]imidazol-5-yl)methyl 1-acetylpiperidine-4-carboxylate dihydrochloride

From tert-butyl 4-[5-({[(1-acetyl-1-piperidinyl)carbonyl]oxy}methyl)-3-oxo-1H-imidazo[1,5-c]imidazol-2(3H)-yl]piperidine-1-carboxylate obtained in Example 175a), the title compound (0.35 g, quantitative) was obtained as white powder in a similar manner to Example 174c) .
NMR (300 MHz, CDCl₃) δ: 1.37-2.14 (5H, m), 2.64-2.72 (4H, m), 2.96-3.19 (5H, m), 3.32-3.37 (2H, m), 3.72-3.76 (1H, m), 4.11-4.20 (2H, m), 4.58 (2H, s), 5.38 (2H, s), 6.97-7.00 (1H, m), 7.24 (1H, s), 9.14-9.27 (2H, m).

### 175c) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 1-acetylpiperidine-4-carboxylate

From [3-oxo-2-(4-piperidinyl)-2,3-dihydro-1H-imidazo[1,5-c]imidazol-5-yl]methyl 1-acetylpiperidine-4-carboxylate dihydrochloride (0.35 g) obtained in Example 175b) and (2S)-3-[(6-chloro-2-naphthyl)sulfonyl]-2-hydroxypropionic acid (0.26 g) obtained in Example 174a), the title compound (0.14 g, 27%) was obtained as white powder in a similar manner to Example 128.
NMR (300 MHz, CDCl₃) δ: 1.12-1.28 (2H, m), 1.61-2.14 (16H, m), 2.58-2.86 (4H, m), 3.08-3.80 (7H, m), 4.11-4.38 (6H, m), 4.70-4.78 (1H, m), 5.02-5.07 (1H, m), 5.39 (2H, s), 6.90 (1H, s), 7.59-7.62 (1H, m), 7.91-7.98 (4H, m), 8.51 (1H, s). Elemental analysis for C₃₂H₃₆ClN₅O₈S•0.5H₂O
Calculated (%): C, 55.29; H, 5.36; N, 10.07
Found (%): C, 55.43; H, 5.60; N, 9.92

### Example 176

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(2-oxo-1-pyrrolidinyl)propionate

### 176a) 3-(2-Oxo-1-pyrrolidinyl)propionic acid

Sodium hydroxide (0.06 g) and pyrrolidin-2-one (1.2 mL) were added to a solution of methyl acrylate (1.4 mL) in THF (100 mL), and the mixture was stirred at room temperature for 4 days. The solvent was distilled off under reduced pressure and to the residue were added methanol (150 mL) and a 1N aqueous sodium hydroxide solution (30 mL). The mixture was stirred at room temperature for 2 days. The solvent was distilled off under reduced pressure and the residue was acidified by addition of 1N hydrochloric acid and the extracted with dichloromethane. The extract was dried over anhydrous magnesium sulfate, and the solvent was distilled off under reduced pressure to obtain the title compound (1.1 g, 45%) as a colorless oil.
NMR (300 MHz, CDCl₃) δ: 2.13-2.23 (1H, m), 2.46-2.54 (2H, m), 2.60 (2H, t, J = 6.8), 3.49-3.57 (2H, m), 3.61 (2H, t, J = 6.8), 10.5 (1H, s).

### 176b) Tert-butyl 4-[3-oxo-5-({[3-(2-oxo-1-pyrrolidinyl)propanoyl]oxy}methyl)-1H-imidazo[1,5-c]imidazol-2(3H)-yl]piperidine-1-carboxylate

From tert-butyl 4-[5-(hydroxymethyl)-3-oxo-1H-imidazo[1,5-c]imidazol-2(3H)-yl]piperidine-1-carboxylate (0.34 g) obtained in Example 185b) and 3-(2-oxo-1-pyrrolidinyl)propionic acid (0.19 g) obtained in Example 176a), the title compound (0.26 g, 55%) was obtained as a colorless oil in a similar manner to Example 175a).
NMR (300 MHz, CDCl₃) δ: 1.47 (9H, s), 1.61-1.71 (2H, m), 1.84-2.05 (4H, m), 2.34 (2H, t, J = 8.3), 2.63 (2H, t, J = 6.8), 2.78-2.88 (2H, m), 3.43 (2H, t, J = 7.0), 3.59 (2H, t, J = 6.8), 4.05-4.34 (5H, m), 5.39 (2H, s), 6.87 (1H, s).

### 176c) [3-Oxo-2-(4-piperidinyl)-2,3-dihydro-1H-imidazo[1,5-c]imidazol-5-yl]methyl 3-(2-oxo-1-pyrrolidinyl)propionate

A 4N solution of hydrogen chloride in ethyl acetate (2 mL) was added to a solution of tert-butyl 4-[3-oxo-5-({[3-(2-oxo-1-pyrrolidinyl)propanoyl]oxy}methyl)-1H-imidazo[1,5-climidazol-2(3H)-yl]piperidine-1-carboxylate (0.26 g) obtained in Example 176b) in ethyl acetate (1.0 mL), and the mixture was stirred at room temperature for 20 minutes. The solvent was distilled off under reduced pressure, and an aqueous saturated sodium hydrogencarbonate solution and potassium carbonate were added to the residue, which was extracted with dichloromethane. The extract was dried over anhydrous magnesium sulfate. The solvent was distilled off under reduced pressure to obtain the title compound (0.13 g, 65%) as white powder.
NMR (300 MHz, CDCl₃) δ: 1.57-2.07 (7H, m), 2.35 (2H, t, J = 7.8), 2.62 (2H, t, J = 6.6), 2.74 (2H, dt, J = 2.2 and 7.8), 3.17-3.23 (2H, m), 3.43 (2H, t, J = 6.6), 3.59 (2H, t, J = 6.6), 3.96-4.12 (1H, m), 4.37 (2H, s), 5.39 (2H, s), 6.86 (1H, s).

### 176d) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(2-oxo-1-pyrrolidinyl)propionate

From [3-oxo-2-(4-piperidinyl)-2,3-dihydro-1H-imidazo[1,5-c]imidazol-5-yl]methyl 3-(2-oxo-1-pyrrolidinyl)propionate (0.13 g) obtained in Example 176c) and (2S)-3-[(6-chloro-2-naphthyl)sulfonyl]-2-hydroxypropionic acid (0.12 g) obtained in Example 174a), the title compound (0.07 g, 28%) was obtained as white powder in a similar manner to Example 89b).
NMR (300 MHz, CDCl₃) δ: 1.67-2.07 (7H, m), 2.35 (2H, t, J = 8.3), 2.62 (2H, t, J = 6.8), 2.71-2.85 (1H, m), 3.16-3.73 (6H, m), 4.10-4.37 (4H, m), 4.68-4.78 (1H, m), 5.00-5.08 (1H, m), 5.39 (2H, s), 6.71 (1H, s), 6.88 (1H, s), 7.58-7.62 (1H, m), 7.91-7.98 (4H, m), 8.51 (1H, s).
Elemental analysis for C₃₁H₃₄ClN₅O₈S•0.5H₂O•0.5AcOEt Calculated (%): C, 54.65; H, 5.42; N, 9.66
Found (%): C, 54.66; H, 5.64; N, 9.90

### Example 177

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl (2-oxo-1-pyrrolidinyl)acetate

### 177a) (2-Oxo-1-pyrrolidinyl)acetic acid

A 1N aqueous sodium hydroxide solution (11 mL) was added to a solution of methyl (2-oxo-1-pyrrolidinyl)acetate (1.4 mL) in methanol (20 mL), and the mixture was stirred at 70°C for 2 hours. The solvent was distilled off under reduced pressure, and the residue was diluted with water, washed with ether, and extracted with dichloromethane. The extract was dried over anhydrous magnesium sulfate, and the solvent was distilled off under reduced pressure to obtain the title compound (0.16 g, 11%) as a white solid.
NMR (300 MHz, CDCl₃) δ: 2.06-2.16 (2H, m), 2.47-2.56 (2H, m), 3.48-3.57 (2H, m), 4.09 (2H, s), 8.11 (1H, s).

### 177b) Tert-butyl 4-(3-oxo-5-{[(2-oxo-1-pyrrolidinyl)acetoxy]methyl}-1H-imidazo[1,5-c]imidazol-2(3H)-yl)piperidine-1-carboxylate

From tert-butyl 4-[5-(hydroxymethyl)-3-oxo-1H-imidazo[1,5-c]imidazol-2(3H)-yl]piperidine-1-carboxylate obtained in Example 185b) and (2-oxo-1-pyrrolidinyl)acetic acid (0.16 g) obtained in Example 177a), the title compound (0.41 g, quantitative) was obtained as a colorless solid in a similar manner to Example 175a).
NMR (300 MHz, CDCl₃) δ: 1.48 (9H, s), 1.61-1.89 (4H, m), 2.02-2.12 (3H, m), 2.41 (2H, t, J = 8.3), 2.79-2.87 (2H, m), 3.00-3.01 (1H, m), 3.51 (2H, t, J = 7.0), 4.06-4.34 (5H, m), 5.44 (2H, s), 6.87 (1H, s).

### 177c) [3-Oxo-2-(4-piperidinyl)-2,3-dihydro-1H-imidazo[1,5-c]imidazol-5-yl]methyl (2-oxo-1-pyrrolidinyl)acetate dihydrochloride

From tert-butyl 4-(3-oxo-5-{[(2-oxo-1-pyrrolidinyl)acetoxy]methyl}-1H-imidazo[1,5-c]imidazol-2(3H)-yl)piperidine-1-carboxylate (0.26 g) obtained in Example 177b), the title compound (0.37 g, quantitative) was obtained as white powder in a similar manner to Example 174c).
NMR (300 MHz, DMSO-d₆) δ: 1.91-2.28 (8H, m), 3.01-3.42 (7H, m), 4.11-4.18 (3H, m), 4.60 (2H, s), 5.46 (2H, s), 7.32 (1H, s) .

### 177d) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl (2-oxo-1-pyrrolidinyl)acetate

From [3-oxo-2-(4-piperidinyl)-2,3-dihydro-1H-imidazo[1,5-c]imidazol-5-yl]methyl (2-oxo-1-pyrrolidinyl)acetate dihydrochloride (0.37 g) obtained in Example 177c) and (2S)-3-[(6-chloro-2-naphthyl)sulfonyl]-2-hydroxypropionic acid (0.30 g) obtained in Example 174a), the title compound (0.09 g, 16%) was obtained as white powder in a similar manner to Example 128.
NMR (300 MHz, CDCl₃) δ: 1.67-2.12 (8H, m), 2.41 (2H, t, J = 8.3), 2.70-2.78 (1H, m), 3.17-3.26 (1H, m), 3.46-3.55 (4H, m), 4.08-4.36 (5H, m), 4.68-4.72 (1H, m), 5.04-5.08 (1H, m), 5.43 (2H, s), 6.87 (1H, s), 7.58-7.61 (1H, m), 7.91-7.97 (4H, m), 8.51 (1H, s).
Elemental analysis for C₃₀H₃₂ClN₅O₈S•0.5H₂O
Calculated (%): C, 54.01; H, 4.99; N, 10.50
Found (%): C, 54.12; H, 5.25; N, 10.27

### Example 178

### Methyl {1-{[(6-chloro-2-naphthyl)sulfonyl]methyl}-2-[4-(5-methyl-3-oxo-1H-imidazo[1,5-c]imidazol-2(3H)-yl)piperidine-1-yl]-2-oxoethyl}methylcarbamate

From methyl N-((1S)-1-{[(6-chloro-2-naphthyl)sulfonyl]methyl}-2-[4-(5-methyl-3-oxo-1H-imidazo[1,5-c]imidazol-2(3H)-yl)-1-piperidinyl]-2-oxoethyl)carbamate (0.39 g) obtained in Example 156 and methyl iodide (0.42 mL), the title compound(0.32 g, 79%) was obtained as a white solid in a similar manner to Example 172.
NMR (300 MHz, CDCl₃) δ: 1.86-1.98 (3H, m), 2.60 (3H, s), 2.68-2.75 (3H, m), 2.88 (3H, s), 2.96 (3H, s), 3.02-3.77 (2H, m), 4.23-4.69 (4H, m), 5.62-5.87 (1H, m), 6.69 (1H, s), 7.58-7.61 (1H, m), 7.92-8.02 (5H, m), 8.45-8.50 (1H, m). Elemental analysis for C₂₇H₃₀ClN₅O₆S•0.7AcOEt
Calculated (%): C, 55.09; H, 5.52; N, 10.78
Found (%): C, 55.47; H, 5.78; N, 10.70

### Example 179

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(2-oxo-1-pyrrolidinyl)propionate

### 179a) Methyl 3-(2-oxo-1-piperidinyl)propionate

Sodium hydroxide (0.16 g) and piperidin-2-one (4.1 g) were added to a solution of methyl acrylate (18 mL) in THF (200 mL), and the mixture was stirred at 80°C for 5 hours. The solvent was distilled off under reduced pressure, and dichloromethane was added to the residue, which was washed with an aqueous saturated sodium hydrogencarbonate solution and dried over anhydrous magnesium sulfate. The solvent was distilled off under reduced pressure to obtain the title compound (4.6 g, 62%) as a pale yellow oil.
NMR (300 MHz, CDCl₃) δ: 1.74-1.86 (4H, m), 2.34-2.39 (2H, m), 2.62 (2H, t, J = 7.0), 3.32-3.36 (2H, m), 3.62 (2H, t, J = 7.0), 3.68 (3H, s).

### 179b) 3-(2-Oxo-1-pyrrolidinyl)propionic acid

From methyl 3-(2-oxo-1-piperidinyl)propionate (4.6 g) obtained in Example 179a), the title compound (2.3 g, 55%) was obtained as pale light brown powder in a similar manner to Example 177a).
NMR (300 MHz, CDCl₃) δ: 1.77-1.87 (4H, m), 2.38-2.42 (2H, m), 2.64 (2H, t, J = 7.0), 3.30-3.41 (2H, m), 3.63 (2H, t, J = 7.0), 6.29 (1H, s).

### 179c) Tert-butyl 4-[3-oxo-5-({[3-(2-oxo-1-piperidinyl)propanoyl]oxy}methyl)-1H-imidazo[1,5-c]imidazol-2(3H)-yl]piperidine-1-carboxylate

From tert-butyl 4-[5-(hydroxymethyl)-3-oxo-1H-imidazo[1,5-c]imidazol-2(3H)-yl]piperidine-1-carboxylate (0.34 g) obtained in Example 185b) and 3-(2-oxo-1-pyrrolidinyl)propionic acid (0.20 g) obtained in Example 179b), the title compound (0.49 g, quantitative) was obtained as a colorless oil in a similar manner to Example 175a).

NMR (300 MHz, CDCl₃) δ: 1.47 (9H, s), 1.66-1.88 (2H, m), 2.32-2.38 (3H, m), 2.67 (2H, t, J = 7.0), 2.78-2.87 (2H, m), 3.33-3.36 (2H, m), 3.62 (2H, t, J = 6.8), 4.05-4.34 (5H, m), 5.38 (2H, s), 6.86 (1H, s).

### 179d) [3-Oxo-2-(4-piperidinyl)-2,3-dihydro-1H-imidazo[1,5-c]imidazol-5-yl]methyl 3-(2-oxo-1-piperidinyl)propionate dihydrochloride

From tert-butyl 4-[3-oxo-5-({[3-(2-oxo-1-piperidinyl)propanoyl]oxy}methyl)-1H-imidazo[1,5-c]imidazol-2(3H)-yl]piperidine-1-carboxylate (0.49 g) obtained in Example 179c), the title compound (0.46 g, 99%) was obtained as white powder in a similar manner to Example 174c).
NMR (300 MHz, DMSO-d₆) δ: 1.63-1.72 (5H, m), 2.06-2.20 (4H, m), 2.60-2.65 (2H, m), 2.97-3.52 (5H, m), 4.12-4.20 (2H, m), 4.63-4.66 (2H, m), 5.46 (2H, s), 7.46 (1H, s), 9.27-9.44 (2H, m).

### 179e) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(2-oxo-1-piperidinyl)propionate

From [3-oxo-2-(9-piperidinyl)-2,3-dihydro-1H-imidazo[1,5-c]imidazol-5-yl]methyl 3-(2-oxo-1-piperidinyl)propionate dihydrochloride (0.46 g) obtained in Example 179d) and (2S)-3-[(6-chloro-2-naphthyl)sulfonyl]-2-hydroxypropionic acid (0.35 g) obtained in Example 174a), the title compound (0.02 g, 2%) was obtained as white powder in a similar manner to Example 89b).
NMR (300 MHz, CDCl₃) δ: 1.76-2.11 (9H, m), 2.33-2.39 (2H, m), 2.65-2.91 (3H, m), 3.17-3.74 (7H, m), 4.05-4.36 (4H, m), 4.68-4.77 (1H, m), 5.05 (1H, s), 5.37 (2H, s), 6.86 (1H, s), 7.57-7.61 (1H, m), 7.93-7.96 (4H, m), 8.50 (1H, s).

### Example 180

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl (2-oxo-1-piperidinyl)acetate

### 180a) Tert-butyl 4-(3-oxo-5-{[(2-oxo-1-piperidinyl)acetoxy]methyl}-1H-imidazo[1,5-c]imidazol-2(3H)-yl)piperidine-1-carboxylate

From tert-butyl 4-[5-(hydroxymethyl)-3-oxo-1H-imidazo[1,5-c]imidazol-2(3H)-yl]piperidine-1-carboxylate (0.34 g) obtained in Example 185b) and (2-oxo-1-piperidinyl)acetic acid (Hassner, A. et al., J. Org. Chem., 57, 3070 (1992)) (0.19 g), the title compound (0.47 g, 99%) was obtained as a colorless oil in a similar manner to Example 175a).
NMR (300 MHz, CDCl₃) δ: 1.47 (9H, s), 1.62-1.66 (4H, m), 1.83-1.88 (5H, m), 2.39-2.42 (2H, m), 4.06-4.33 (6H, m), 5.44 (2H, s), 6.86 (1H, s).

### 180b) [3-Oxo-2-(4-piperidinyl)-2,3-dihydro-1H-imidazo[1,5-c]imidazol-5-yl]methyl (2-oxo-1-piperidinyl)acetate dihydrochloride

From tert-butyl 4-(3-oxo-5-{[(2-oxo-1-piperidinyl)acetoxy]methyl}-1H-imidazo[1,5-c]imidazol-2(3H)-yl)piperidine-1-carboxylate (0.47 g) obtained in Example 180a), the title compound (0.44 g, 98%) was obtained as white powder in a similar manner to Example 174c).
NMR (300 MHz, DMSO-d₆) δ: 1.68-1.78 (5H, m), 2.10-2.27 (4H, m), 2.97-3.05 (2H, m), 3.31-3.36 (4H, m), 4.12-4.20 (3H, m), 4.63 (2H, s), 5.49 (2H, s), 7.44 (1H, s), 9.26-9.41 (2H, m).

### 180c) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl (2-oxo-1-piperidinyl)acetate

From [3-oxo-2-(4-piperidinyl)-2,3-dihydro-1H-imidazo[1,5-c]imidazol-5-yl]methyl (2-oxo-1-piperidinyl)acetate dihydrochloride (0.44 g) obtained in Example 180b) and (2S)-3-[(6-chloro-2-naphthyl)sulfonyl]-2-hydroxypropionic acid (0.35 g) obtained in Example 174a), the title compound was obtained as white powder (0.06 g, 8%) in a similar manner to Example 89b).
NMR (300 MHz, CDCl₃) δ: 1.69-2.12 (7H, m), 2.39-2.43 (2H, m), 2.70-2.84 (1H, m), 3.16-3.54 (5H, m), 4.19-4.36 (6H, m), 4.68-4.77 (1H, m), 5.03-5.07 (1H, m), 5.44 (2H, s), 6.87 (1H, s), 7.58-7.62 (1H, m), 7.94-7.97 (4H, m), 8.51 (1H, s).

### Example 181

### 2-(1-{(2R)-3-[(6-chloro-2-naphthyl)sulfonyl]-2-hydroxypropanoyll-4-piperidinyl)-5-methyl-1,2-dihydro-3H-imidazo[1,5-c]imidazol-3-one

### 181a) Methyl (2R)-3-[(6-chloro-2-naphthyl)thio]-2-hydroxypropionate

From methyl (2S)-oxiran-2-carboxylate (2.6 g), the title compound (5.6 g, 74%) was obtained as a white crystal (hexane-ethyl acetate) in a similar manner to Example 136a). NMR (300 MHz, CDCl₃) δ: 3.13 (1H, d, J = 6.0), 3.36 (1H, dd, J = 14.1, 5.7), 3.48 (1H, dd, J = 14.1, 4.2), 4.46 (1H, ddd, J = 6.0, 5.7, 4.2), 7.41 (1H, dd, J = 6.9, 1.8), 7.51 (1H, dd, J = 6.9, 1.8), 7.67 (1H, d, J = 1.8), 7.70 (1H, d, J = 1.8), 7.77 (1H, d, J = 2.1), 7.84 (1H, d, J = 2.1).

### 181b) (2R)-3-[(6-chloro-2-naphthyl)thio]-2-hydroxypropionic acid

From methyl (2R)-3-[(6-chloro-2-naphthyl)thio]-2-hydroxypropionate (5.0 g) obtained in Example 181a), the title compound (4.7 g, 99%) was obtained as white powder in a similar manner to Example 136b).
NMR (300 MHz, CD₃OD) δ: 3.20 (1H, dd, J = 13.5, 3.3), 3.57 (1H, dd, J = 13.5, 3.3), 4.19 (1H, dd, J = 13.5, 3.3), 7.40 (1H, dd, J = 8.7, 2.1), 7.52 (1H, dd, J = 8.7, 2.1), 7.71 (1H, d, J = 8.7), 7.77 (1H, d, J = 8.7), 7.80 (1H, s), 7.87 (1H, s).

### 181c) 2-(1-{(2R)-3-[(6-chloro-2-naphthyl)thio]-2-hydroxypropanoyl}-4-piperidinyl)-5-methyl-1,2-dihydro-3H-imidazo[1,5-c]imidazol-3-one

From (2R)-3-[(6-chloro-2-naphthyl)thio]-2-hydroxypropionic acid (4.5 g) obtained in Example 181b), the title compound (5.6 g, 73%) was obtained as white powder in a similar manner to Example 136c).
NMR (300 MHz, CDCl₃) δ: 1.28-1.65 (2H, m), 1.66-1.88 (2H, m), 2.40-2.73 (1H, m), 2.58 (3H, s), 2.98-3.10 (1H, m), 3.20-3.44 (2H, m), 3.68-3.77 (1H, m), 3.80 (2H, s), 4.00-4.15 (1H, m), 4.19 (1H, s), 4.55-4.73 (2H, m), 6.67 (1H, s), 7.40-7.50 (2H, m), 7.66 (1H, s), 7.69 (1H, s), 7.70 (1H, s), 7.81 (1H, d, J = 5.4).

### 181d) 2-(1-{(2R)-3-[(6-chloro-2-naphthyl)sulfonyl]-2-hydroxypropanoyl}-4-piperidinyl)-5-methyl-1,2-dihydro-3H-imidazo[1,5-c]imidazol-3-one

From 2-(1-{(2R)-3-[(6-chloro-2-naphthyl)thio]-2-hydroxypropanoyl}-4-piperidinyl)-5-methyl-1,2-dihydro-3H-imidazo[1,5-c]imidazol-3-one (3.0 g) obtained in Example 181c), the title compound (2.2 g, 67%) was obtained as white powder in a similar manner to Example 136d).
NMR (300 MHz, CDCl₃) δ: 1.66-1.99 (4H, m), 2.61 (3H, s), 2.69-2.88 (1H, m), 3.14-3.33 (1H, m), 3.48 (2H, dd, J = 11.8, 5.6), 3.84 (1H, m), 4.07-4.22 (1H, m), 4.27 (2H, s), 4.65-4.88 (1H, m), 4.98-5.09 (1H, m), 6.71 (1H, s), 7.59 (1H, dd, J = 8.8, 1.0), 7.95 (3H, s), 7.96 (1H, d, J = 8.8), 8.51 (1H, s).
Elemental analysis for C₂₄H₂₅N₄O₅SCl
Calculated (%): C, 55.76; H, 4.87; N, 10.84
Found (%): C, 55.79; H, 4.92; N, 10.94

### Example 182

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl ethylcarbamate

### 182a) 2-(1-Benzyl-4-piperidinyl)-5-(hydroxymethyl)-1,2-dihydro-3H-imidazo[1,5-c]imidazol-3-one

2-(1-Benzyl-4-piperidinyl)-5-({[tert-butyl(dimethyl)silyl]oxy}methyl)-1,2-dihydro-3H-imidazo[1,5-c]imidazol-3-one (2.1 g) obtained in Example 137h) was dissolved in a mixed solvent of acetic acid (56 mL), THF (14 mL) and water (14 mL), and the solution was stirred at 60°C for 12 hours. THF was distilled off under reduced pressure, and the reaction solution was adjusted to about pH 8 with an aqueous saturated sodium hydrogencarbonate solution. The mixture solution was extracted with chloroform, and the extract was dried over anhydrous sodium sulfate and then concentrated under reduced pressure. The residue was washed with ethyl acetate to obtain the title compound (1.1 g, 70%) as a white solid.
NMR (300 MHz, CDCl₃) δ: 1.74-1.85 (4H, m), 2.09-2.18 (2H, m), 3.01 (2H, d, J = 12.0), 3.54 (2H, s), 3.92-4.03 (1H, m), 4.11 (1H, brs), 4.39 (2H, s), 4.86 (2H, s), 6.76 (1H, t, J = 1.5), 7.24-7.37 (5H, m).

### 182b) [2-(1-Benzyl-4-piperidinyl)-3-oxo-2,3-dihydro-1H-imidazo[1,5-c]imidazol-5-yl]methyl ethylcarbamate

Ethyl isocyanate (0.13 g) was added to a solution of 2-(1-benzyl-4-piperidinyl)-5-(hydroxymethyl)-1,2-dihydro-3H-imidazo[1,5-c]imidazol-3-one (0.5 g) obtained in Example 182a) in THF (30 mL), and the mixture was stirred at 50°C for 5 hours. The solvent was distilled off under reduced pressure, and the residue was washed with hexane to obtain the title compound (0.58 g, 95%) as a white solid.
NMR (300 MHz, CDCl₃) δ: 1.12 (3H, t, J = 7.2), 1.72-1.84 (4H, m), 2.12 (2H, dt, J = 12.0, 3.6), 3.00 (2H, d, J = 12.0), 3.19-3.28 (2H, m), 3.53 (2H, s), 3.93-4.04 (1H, m), 4.33 (2H, s), 4.76 (1H, brs), 5.37 (2H, s), 6.85 (1H, t, J = 1.5), 7.24-7.36 (5H, m).

### 182c) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl ethylcarbamate

A suspension of [2-(1-benzyl-4-piperidinyl)-3-oxo-2,3-dihydro-1H-imidazo[1,5-c]imidazol-5-yl]methyl ethylcarbamate (0.55 g) obtained in Example 182b), ammonium formate (0.87 g) and 10% palladium carbon (0.1 g) in methanol (50 mL) was heated to reflux for 2 hours. After the reaction solution was cooled to room temperature, insoluble substances were filtered off using Celite and the filtrate was concentrated under reduced pressure. Chloroform was added to the residue and insoluble substances were filtered off. The filtrate was concentrated again. WSC (0.38 g) was added to a solution of the resulting residue, (2S)-3-[(6-chloro-2-naphthyl)sulfonyl]-2-hydroxypropionic acid (0.42 g) obtained in Example 174a) and HOBt (0.31 g) in DMF (5 mL)-acetonitrile (30 mL), and the mixture was stirred at room temperature for 15 hours. The reaction solution was concentrated under reduced pressure, and diluted with ethyl acetate and an aqueous sodium hydrogencarbonate solution. An organic layer was separated, dried over anhydrous sodium sulfate and concentrated under reduced pressure. The residue was purified with a silica gel column (ethyl acetate/ethanol = 5/1) and further purified by reverse phase preparative HPLC to obtain the title compound (0.19 g, 24%) as a white solid.
NMR (300 MHz, CDCl₃) δ: 1.13 (3H, t, J = 11.4), 1.65-2.50 (4H, m), 2.63-2.81 (1H, m), 3.17-3.28 (3H, m), 3.48 (2H, dd, J = 18.9 and 8.7), 4.07-4.23 (1H, m), 4.31-4.35 (2H, s), 4.65-4.85 (2H, m), 5.00-5.06 (1H, m), 5.38(2H, s), 6.87 (1H, s), 7.60 (1H, dd, J = 11.4, 0.9), 7.94 (3H, s), 7.96 (1H, d,
J = 11.4), 8.51 (1H, s).
Elemental analysis for C₂₇H₃₀N₅O₇SCl•H₂O
Calculated (%): C, 52.13; H, 5.18; N, 11.26
Found (%): C, 52.13; H, 5.40; N, 11.05

### Example 183

### 2-(1-{(2S)-3-[(6-chloro-2-naphthyl)sulfonyl]-2-hydroxypropanoyl}-4-piperidinyl)-5-[(dimethylamino)methyl]-1,2-dihydro-3H-imidazo[1,5-c]imidazol-3-one

### 183a) 1-Trityl-1H-imidazol-4-carbaldehyde

Trityl chloride (42 g) was added to a suspension of 1H-imidazol-4-carbaldehyde (15 g) and triethylamine (23 mL) in dichloromethane (300 mL), and the mixture was stirred at room temperature for 15 hours. The reaction solution was diluted with dichloromethane and water. The organic layer was separated and dried over anhydrous sodium sulfate. The solvent was distilled off under reduced pressure to obtain the title compound (51 g, quantitative) as white powder. NMR (300 MHz, CDCl₃) δ: 7.07-7.13 (6H, m), 7.25-7.41 (9H, m), 7.52 (1H, d, J = 1.5), 7.60 (1H, d, J = 1.5), 9.86 (1H, s) .

### 183b) 1-Benzyl-N-[(1-trityl-1H-imidazol-4-yl)methyl]piperidine-4-amine

Sodium triacetoxyborohydride (7.5 g) was added to a solution of 1-trityl-1H-imidazol-4-carbaldehyde (105 g) obtained in Example 183a), 1-benzyl-4-piperidineamine (59 g) and acetic acid (20 mL) in dichloromethane (2L), and the mixture was stirred at room temperature for 15 hours. To the reaction solution was added a 1N aqueous sodium hydroxide solution to adjust the aqueous layer to about pH 14. After stirred for 1 hour, the reaction solution was extracted with dichloromethane, and the extract was washed with an aqueous saturated sodium chloride solution and dried over anhydrous magnesium sulfate. The solvent was distilled off under reduced pressure, and residue was recrystallized from hexane-ethyl acetate to obtain the title compound (146 g, 92%) as white crystals.
NMR (200 MHz, CDCl₃) δ: 1.45-1.65 (2H, m), 1.84-2.12 (4H, m), 2.57-2.68 (1H, m), 2.85-2.96 (2H, m), 3.57 (2H, s), 3.80 (2H, s), 6.75 (1H, d, J = 1.3), 7.09-7.16 (6H, m), 7.30-7.39 (14H, m), 7.41 (1H, d, J = 1.3).

### 183c) Tert-butyl (1-benzyl-4-piperidinyl)[(1-trityl-1H-imidazol-4-yl)methyl]carbamate

Di-tert-butyl dicarbonate (65 g) was added to a solution of 1-benzyl-N-[(1-trityl-1H-imidazol-4-yl)methyl]piperidine-4-amine (146 g) obtained in Example 183b) in ethanol (500 mL), and the mixture was stirred at room temperature for 15 hours. The solvent was distilled off under reduced pressure, and the residue was diluted with ethyl acetate and an aqueous saturated sodium chloride solution. The organic layer was separated, washed with water and an aqueous saturated sodium chloride solution, and dried over anhydrous magnesium sulfate. The solvent was distilled off under reduced pressure to obtain the title compound (175 g, quantitative) as white powder.
NMR (300 MHz, CDCl₃) δ: 1.28 (9H, s), 1.50 (2H, d, J = 11.4), 1.65-1.80 (2H, m), 1.98 (2H, t, J = 8.4), 2.87 (2H, d, J = 11.4), 3.45 (2H, s), 3.95-4.05 (1H, m), 4.29 (2H, s), 6.55 (1H, s), 7.09-7.14 (6H, m), 7.20-7.33 (15H, m).

### 183d) Tert-butyl (1-benzyl-4-piperidinyl)[(2-formyl-1-trityl-1H-imidazol-4-yl)methyl]carbamate

A solution of n-butyllithium in hexane (1.6 M, 890 mL) was added dropwise to a solution of tert-butyl (1-benzyl-4-piperidinyl)[(1-trityl-1H-imidazol-4-yl)methyl]carbamate (175 g) obtained in Example 183c) in THF (2 L) at -78°C under argon atmosphere. After the reaction solution was warmed to -40°C over 2 hours, DMF (110 mL) was added and the mixture was stirred at room temperature for 1 hour. Water (500 mL) was added to the reaction solution, and this was extracted with ether (2L). The extract was washed with water (500 mL x 3) and an aqueous saturated sodium chloride solution (500 mL) and dried over anhydrous magnesium sulfate. The solvent was distilled off under reduced pressure. The residue was recyrstallized from hexane-ether to obtain the title compound (85 g, 48%) as pale yellow powder.
NMR (300 MHz, CDCl₃) δ: 1.26 (9H, s), 1.42-1.54 (2H, m), 1.74-1.79 (2H, m), 1.95-1.99 (2H, m), 2.88 (2H, d, J = 11.1), 3.45 (2H, s), 3.98-4.03 (1H, m), 4.34 (2H, s), 6.85 (1H, s), 7.05-7.09 (6H, m), 7.20-7.35 (14H, m), 9.10 (1H, s).

### 183e) Tert-butyl 1-benzyl-4-piperidinyl({2-[(dimethylamino)methyl]-1-trityl-1H-imidazol-4-yl}methyl)carbamate

Sodium triacetoxyborohydride (1.5 g) was added to a solution of tert-butyl (1-benzyl-4-piperidinyl)[(2-formyl-1-trityl-1H-imidazol-4-yl)methyl]carbamate (3 g) obtained in Example 183d), a solution of dimethylamine in THF (2.0 M, 4.8 mL) and acetic acid (1 mL) in dichloroethane (100 mL), and the mixture was stirred at room temperature for 15 hours. The reaction solution was adjusted to about pH 12 by addition of an aqueous saturated potassium carbonate solution, and then extracted with dichloromethane. The extract was dried over anhydrous magnesium sulfate, and the solvent was distilled off under reduced pressure. The residue was purified with a basic silica gel column (hexane-ethyl acetate = 1/1) to obtain the title compound (2.4 g, 75%) as white powder.
NMR (300 MHz, CDCl₃) δ: 1.26 (9H, s), 1.45-1.51 (2H, d, J = 12.0), 1.82-2.02 (4H, m), 1.91 (6H, s), 2.65 (2H, s), 2.86 (2H, d, J = 9.6), 3.45 (2H, s), 3.92-3.97 (1H, m), 4.30 (2H, s), 6.54 (1H, s), 7.10-7.15 (6H, m), 7.15-7.31 (14H, m).

### 183f) 2-(1-Benzyl-4-piperidinyl)-5-[(dimethylamino)methyl]-1,2-dihydro-3H-imidazo[1,5-c]imidazol-3-one

A mixture of tert-butyl (1-benzyl-4-piperidinyl)({2-[(dimethylamino)methyl]-1-trityl-1H-imidazol-4-yl}methyl)carbamate (2.35 g) obtained in Example 183e) in trifluoroacetic acid (20 mL) and dichloromethane (60 mL) was stirred at room temperature for 2 hours. The reaction solution was diluted with water (80 mL), and dichloromethane was distilled off under reduced pressure. The aqueous layer was washed with ethyl acetate (150 mL), adjusted to pH 14 with potassium carbonate and then extracted with dichloromethane (150 mL x 2). The extract was dried over anhydrous magnesium sulfate, and the solvent was distilled off under reduced pressure. The residue was dissolved in THF (100 mL), and N,N'-carbonyldiimidazole (0.68 g) and GBU(1.28 g) were added. The mixture was stirred at room temperature for 15 hours. The solvent was distilled off under reduced pressure, and the residue was diluted with ethyl acetate and an aqueous saturated sodium chloride solution. An organic layer was separated and dried over anhydrous magnesium sulfate. The solvent was distilled off under reduced pressure, and the residue was purified with a basic silica gel column (ethyl acetate) to obtain the title compound (0.83 g, 67%) as pale yellow powder.
NMR (300 MHz, CDCl₃) δ: 1.71-1.82 (4H, m), 2.07-2.16 (2H, m), 2.36 (6H, s), 2.99 (2H, d, J = 12.0), 3.52 (2H, s), 3.80 (2H, s), 3.91-4.13 (1H, m), 4.30 (2H, s), 6.78 (1H, t, J = 1.5), 7.22-7.34 (5H, m).

### 183 g) 5-[(Dimethylamino)methyl]-2-(4-piperidinyl)-1,2-dihydro-3H-imidazo[1,5-c]imidazol-3-one

A suspension of 2-(1-benzyl-4-piperidinyl)-5-[(dimethylamino)methyl]-1,2-dihydro-3H-imidazo[1,5-c]imidazol-3-one (0.80 g) obtained in Example 183f), ammonium formate (1.4 g) and 10% palladium carbon (0.16 g) in methanol (40 mL) was heated to reflux for 2 hours. After the reaction solution was cooled to room temperature, insoluble substances were filtered using Celite and the filtrate was concentrated under reduced pressure. Chloroform was added to the residue and insoluble substances were filtered off. The filtrate was concentrated again to obtain the title compound (0.52 g, 87%) as pale yellow powder.
NMR (300 MHz, CDCl₃) δ: 1.54-1.75 (2H, m), 1.83-1.89 (2H, m), 2.37 (6H, s), 2.73 (2H, dt, J = 12.2 and 2.6), 3.19 (2H, d, J = 12.2), 3.96-4.12 (1H, m), 4.31 (2H, s), 6.79 (1H, s).

### 183h) 2-(1-{(2S)-3-[(6-chloro-2-naphthyl)sulfonyl]-2-hydroxypropanoyl}-4-piperidinyl)-5-[(dimethylamino)methyl]-1,2-dihydro-3H-imidazo[1,5-c]imidazol-3-one

WSC (0.36 g) was added to a mixture of 5-[(dimethylamino)methyl]-2-(4-piperidinyl)-1,2-dihydro-3H-imidazo[1,5-c]imidazol-3-one (0.45 g) obtained in Example 183 g), (2S)-3-[(6-chloro-2-naphthyl)sulfonyl]-2-hydroxypropionic acid (0.54 g) obtained in Example 174a) and HOBt(0.29 g) in DMF (5 mL) and acetonitrile (45 mL), and the mixture was stirred at room temperature for 15 hours. The solvent was distilled off under reduced pressure, and the residue was dissolved in chloroform, washed with an aqueous sodium hydrogencarbonate solution, and dried over anhydrous magnesium sulfate. The solvent was distilled off under reduced pressure, and the residue was purified with a basic silica gel column (ethyl acetate to ethyl acetate/ethanol = 20/1) to obtain the title compound (0.40 g, 42%) as white powder.
NMR (300 MHz, CDCl₃) δ: 1.60-2.04 (4H, m), 2.36 (6H, s), 2.62-2.90 (1H, m), 3.10-3.25 (1H, m), 3.43-3.53 (2H, m), 3.79 (2H, s), 4.07-4.31 (4H, m), 4.62-4.80 (1H, m), 5.02-5.08 (1H, m), 6.80 (1H, s), 7.59 (1H, dd, J = 8.8, 1.8), 7.94 (3H, s), 7.96 (1H, d, J = 8.8), 8.51 (1H, s). Elemental analysis for C₂₆H₃₀N₅O₅SCl•0.8H₂O
Calculated (%): C, 54.36; H, 5.54; N, 12.19
Found (%): C, 54.39; H, 5.61; N, 11.90

### Example 184

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl dimethylcarbamate

### 184a) [2-(1-benzyl-4-piperidinyl)-3-oxo-2,3-dihydro-1H-imidazo[1,5-c]imidazol-5-yl]methyl dimethylcarbamate

Sodium hydride (60% in oil: 62 mg) was added to a solution of 2-(1-benzyl-4-piperidinyl)-5-(hydroxymethyl)-1,2-dihydro-3H-imidazo[1,5-c]imidazol-3-one (0.27 g) obtained in Example 182a) and dimethylcarbamoyl chloride (0.17 g) in THF (20 mL) under ice-cooling, and the mixture was stirred for 3 hours under ice-cooling. To the reaction solution were added an aqueous saturated ammonium chloride solution and ethyl acetate. An organic layer was separated, washed with an aqueous sodium chloride solution, and dried over anhydrous magnesium sulfate. The solvent was distilled off, and the residue was purified with a basic silica gel column (ethyl acetate) to obtain the title compound (0.31 g, quantitative) as white powder.
NMR (200 MHz, CDCl₃) δ: 1.70-1.86 (4H, m), 2.08-2.19 (2H, m), 2.91 (6H, s), 2.99 (2H, d, J = 11.6), 3.53 (2H, s), 3.91-4.10 (1H, m), 4.34 (2H, s), 5.37 (2H, s), 6.84 (1H, t, J = 1.4), 7.23-7.38 (5H, m).

### 184b) [3-Oxo-2-(-4-piperidinyl)-2,3-dihydro-1H-imidazo[1,5-c]imidazol-5-yl]methyl dimethylcarbamate

From [2-(1-benzyl-4-piperidinyl)-3-oxo-2,3-dihydro-1H-imidazo[1,5-c]imidazol-5-yl]methyl dimethylcarbamate (0.30 g) obtained in Example 184a), the title compound (0.19 g, 82%) was obtained as white powder in a similar manner to Example 183g).
NMR (200 MHz, CDCl₃) δ: 1.80-1.96 (4H, m), 2.76-2.85 (2H, m), 2.92 (6H, s), 3.32 (2H, d, J = 12.4), 4.04-4.21 (1H, m), 4.38 (2H, s), 5.37 (2H, s), 6.86 (1H, s).

### 184c) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl dimethylcarbamate

From [3-oxo-2-(-4-piperidinyl)-2,3-dihydro-1H-imidazo[1,5-c]imidazol-5-yl]methyl dimethylcarbamate (0.48 g) obtained in Example 184b), the title compound (0.17 g, 18%) was obtained as white powder in a similar manner to Example 183h).
NMR (300 MHz, CDCl₃) δ: 1.62-2.04 (4H, m), 2.70-2.80 (1H, m), 2.85 (6H, s), 3.17-3.31 (1H, m), 3.45-3.53 (1H, m), 3.81 (1H, dd, J = 15.0 and 6.0), 4.09-5.04 (2H, m), 4.32 (1.4H, s), 4.35 (0.6H, s), 4.68-4.77 (1H, m), 5.03-5.07 (1H, m), 5.37 (2H, s), 6.87 (1H, s), 7.60 (1H, dd, J = 9.0, 1.2), 7.94 (3H, s), 7.96 (1H, d, J = 9.0), 8.51 (1H, s). Elemental analysis for C₂₇H₃₀N₅O₇SCl•0.5H₂O
Calculated (%): C, 52.89; H, 5.10; N, 11.42
Found (%): C, 52.83; H, 5.13; N, 11.15

### Example 185

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]iimdazol-5-yl]methyl 3-acetylaminopropionate

### 185a) 5-({[Tert-butyl(dimethyl)silyl]oxy}methyl)-2-(-4-piperidinyl)-1,2-dihydro-3H-imidazo[1,5-c]imidazol-3-one

From 2-(1-benzyl-4-piperidinyl)-5-({[tert-butyl(methyl)silyl]oxy}methyl)-1,2-dihydro-3H-imidazo[1,5-c]imidazol-3-one (4.0 g) obtained in Example 137h), the title compound (3.1 g, 98%) was obtained as white powder in a similar manner to Example 183g).
NMR (300 MHz, CDCl₃) δ: 0.13 (6H, s), 1.54-1.74 (2H, m), 1.81-1.88 (2H, m), 2.73 (2H, dt, J = 12.0, 2.6), 3.18 (2H, d, J = 12.0), 3.47 (2H, s), 3.97 -4.12 (1H, m), 4.32 (2H, d, J = 1.6), 4.32 (2H, d, J = 1.6), 4.92 (2H, s), 6.79 (1H, t, J = 1.6).

### 185b) Tert-butyl 4-[5-({[tert-butyl(dimethyl)silyl]oxy}methyl)-3-oxo-1H-imidazo[1,5-c]imidazol-2(3H)-yl]piperidine-1-carboxylate

Di-tert-butyl dicarbonate (1.3 g) was added to a solution of 5-({[tert-butyl(dimethyl)silyl]oxy}methyl)-2-(-4-piperidinyl)-1,2-dihydro-3H-imidazo[1,5-c]imidazol-3-one (1.9 g) obtained in Example 185a) in ethanol (50 mL), and the mixture was stirred at room temperature for 15 hours. Ethanol was distilled off under reduced pressure to obtain the title compound (2.4 g, quantitative) as white powder. NMR (300 MHz, CDCl₃) δ: 0.14 (6H, s), 0.92 (9H, s), 1.47 (9H, s), 1.59-1.73 (2H, m), 1.82-1.97 (2H, m), 2.83 (2H, t, J = 12.2), 3.64-3.77 (1H, m), 4.04-4.23 (2H, m), 4.31 (2H, s), 4.92 (2H, s), 6.80 (1H, s).

### 185c) Tert-butyl 4-[5-(hydroxymethyl)-3-oxo-1H-imidazo[1,5-c]imidazol-2(3H)-yl]piperidine-1-carboxylate

A solution of tert-butyl 4-[5-({[tert-butyl (dimethyl)silyl]oxy}methyl)-3-oxo-1H-imidazo[1,5-c]imidazol-2(3H)-yl]piperidine-1-carboxylate (2.4 g) obtained in Example 185b) and tetrabutylammonium fluoride (4.2 g) in THF was stirred at room temperature for 3 hours. The solvent was distilled off under reduced pressure, and the residue was dissolved in ethyl acetate. The ethyl acetate solution was washed with an aqueous sodium chloride solution, dried over anhydrous sodium sulfate, and concentrated under reduced pressure. The residue was purified with a basic silica gel column (ethyl acetate/ethanol = 40/1) and recrystallized from hexane-ethyl acetate to obtain the title compound (1.0 g, 55%) as a white crystal.
NMR (300 MHz, CDCl₃) δ: 1.48 (9H, s), 1.65 (2H, m), 1.86 (2H, d, J = 12.3), 2.83 (2H, d, J = 12.3), 4.04-4.18 (3H, m), 4.27 (1H, d, J = 12.3), 4.38 (2H, s), 4.86 (2H, d, J = 4.2), 6.77 (1H, s).

### 185d) Tert-butyl 4-[5-{[(3-acetylaminopropionyl)oxy]methyl}-3-oxo-1H-imidazo[1,5-c]imidazol-2(3H)-yl]piperidine-1-carboxylate

WSC (0.19 g) was added to a solution of tert-butyl 4-[5-(hydroxymethyl)-3-oxo-1H-imidazo[1,5-c]imidazol-2(3H)-yl]piperidine-1-carboxylate (0.34 g) obtained in Example 185c), 3-acetylaminopropionic acid (0.13 g), dimethylaminopyridine (0.12 g) and triethylamine (0.10 g) in THF (30 mL) and acetonitrile (30 mL), and the mixture was stirred at room temperature for 15 hours. The solvent was distilled off under reduced pressure, and the residue was diluted with ethyl acetate, washed successively with an aqueous saturated sodium hydrogencarbonate solution and an aqueous saturated sodium chloride solution, dried over anhydrous magnesium sulfate, and then concentrated under reduced pressure. The residue was purified with a basic silica gel column (ethyl acetate/hexane = 10/1 to ethyl acetate) to obtain the title compound (0.38 g, 85%) as white powder.
NMR (200 MHz, CDCl₃) δ: 1.48 (9H, s), 1.54-1.90 (4H, m), 1.98 (3H, s), 2.59 (2H, t, J = 5.8), 2.82 (2H, t, J = 12.2), 3.58 (2H, t, J = 5.8), 4.02-4.18 (1H, m), 4.28 (2H, d, J = 12.2), 4.37 (2H, s), 5.44 (2H, s), 6.85 (1H, t, J = 1.4).

### 185e) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-acetylaminopropionate

A 4N solution (4 mL) of hydrogen chloride in ethyl acetate was added to a solution of tert-butyl 4-[5-{[(3-acetylaminopropionyl)oxy]methyl}-3-oxo-1H-imidazo[1,5-c]imidazol-2(3H)-yl]piperidine-1-carboxylate (0.46 g) obtained in Example 185d) in ethyl acetate (10 mL), and the mixture was stirred at room temperature for 1 hour. The solvent was distilled off under reduced pressure, and the residue was suspended in a mixture of dichloromethane (40 mL) and DMF (10 mL). To this suspension were successively added triethylamine (0.49 mL), (2S)-3-[(6-chloro-2-naphthyl)sulfonyl]-2-hydroxypropionic acid (0.36 g) obtained in Example 174a), HOBt (0.17 g) and WSC (0.22 g), and the mixture was stirred at room temperature for 15 hours. The solvent was distilled off under reduced pressure, and the residue was diluted with chloroform and an aqueous saturated sodium hydrogencarbonate solution. An organic layer was separated, washed with an aqueous sodium chloride solution, dried over anhydrous magnesium sulfate, and concentrated under reduced pressure. The residue was purified with a basic silica gel column (ethyl acetate/ethanol = 10/1) and reverse phase preparative HPLC to obtain the title compound (98 mg, 25%) as white powder. NMR (200 MHz, CDCl₃) δ: 1.62-2.08 (4H, m), 1.98 (3H, s), 2.59 (2H, t, J = 6.0), 2.68-2.86 (1H, m), 3.15-3.35 (1H, m), 3.43-3.61(4H, m), 4.10-4.23 (2H, m), 4.35 (1.4H, s), 4.38 (0.6H, s), 4.65-4.80 (1H, m), 5.00-5.07 (1H, m), 5.44 (2H, s), 6.85 (1H, s), 7.62 (1H, dd, J = 8.8, 1.8), 7.94 (3H, s), 7.96 (1H, d, J = 8.8), 8.50 (1H, s)
Elemental analysis for C₂₉H₃₂N₅O₈SCI•2H₂O
Calculated (%): C, 51.06; H, 5.32; N, 10.27
Found (%): C, 51.29; H, 5.15; N, 10.11

### Example 186

### 2-(1-{(2S)-3-[(6-chloro-2-naphthyl)sulfonyl]-2-hydoroxypropanoyl}-4-piperidinyl)-5-(fluoromethyl)-1,2-dihydro-3H-imidazo[1,5-c]imidazol-3-one

### 186a) Tert-butyl 4-[5-(fluoromethyl)-3-oxo-1H-imidazo[1,5-c]imidazol-2(3H)-yl]piperidine-1-carboxylate

Diethylaminosulfur trifluoride (0.071 mL) was added to a solution of tert-butyl 4-[5-(hydroxymethyl)-3-oxo-1H-imidazo[1,5-c]imidazol-2(3H)-yl]piperidine-1-carboxylate (0.15 g) obtained in Example 185c) in dichloromethane (10 mL) under ice cooling, and the mixture was stirred at room temperature for 3 hours under argon atmosphere. The reaction solution was diluted with dichloromethane, washed with an aqueous saturated sodium hydrogencarbonate solution and an aqueous saturated sodium chloride solution, dried over anhydrous magnesium sulfate, and concentrated under reduced pressure. The residue was purified with a silica gel column (ethyl acetate) to obtain the title compound (70 mg, 46%) as pale yellow powder.
NMR (200 MHz, CDCl₃) δ: 1.48 (9H, s), 1.58-1.71 (2H, m), 1.87 (2H, t, J = 12.2), 2.84 (2H, t, J = 12.2), 4.08-4.19 (1H, m), 4.32 (2H, d, J = 12.2), 4.36 (2H, dd, J = 4.5, 1.2), 5.59 (2H, d, J = 48.0), 6.92 (1H, s).

### 186b) 2-(1-{(2S)-3-[(6-chloro-2-naphthyl)sulfonyl]-2-hydoroxypropanoyl}-4-piperidinyl)-5-(fluoromethyl)-1,2-dihydro-3H-imidazo[1,5-c]imidazol-3-one

From tert-butyl 4-[5-(fluoromethyl)-3-oxo-1H-imidazo[1,5-c]imidazol-2(3H)-yl]piperidine-1-carboxylate (0.20 g) obtained in Example 186a), the title compound (76 mg, 24%) was obtained as white powder in a similar manner to Example 185e).
NMR (200 MHz, CDCl₃) δ: 1.60-2.10 (4H, m), 2.70-2.88 (1H, m), 3.14-3.31 (1H, m), 3.48 (2H, dd, J = 12.6, 5.0), 3.68-3.87 (1H, m), 4.10-4.25 (2H, m), 4.35 (2H, d, J = 4.8), 4.65-4.81 (1H, m), 4.95-5.11 (1H, m), 5.59 (2H, d, J = 48.0), 6.93 (1H, s), 7.60 (1H, d, J = 9.4), 7.94 (3H, s), 7.96 (2H, d, J = 9.4), 8.51 (1H, s).
Elemental analysis for C₂₄H₂₄N₄O₅SClF•H₂O
Calculated (%): C, 52.13; H, 4.74; N, 10.13
Found (%): C, 52.42; H, 4.45; N, 10.29

### Example 187

### N-acetyl-L-valine [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl ester

### 187a) Tert-butyl 4-[5-{[(N-acetyl-L-valyl)oxy]methyl}-3-oxo-1H-imidazo[1,5-c]imidazol-2(3H)-yl]piperidine-1-carboxylate

A 40% solution of diethyl azodicarboxylate in toluene (2 mL) was added to a solution of tert-butyl 4-[5-(hydroxymethyl)-3-oxo-1H-imidazo[1,5-c]imidazol-2(3H)-yl]piperidine-1-carboxylate (0.80 g) obtained in Example 185c), N-acetyl-L-valine (0.73 g) and triphenylphosphine (1.2 g) in THF (30 mL), and the mixture was stirred at room temperature for 2 hours. The reaction solution was diluted with ethyl acetate and water and an organic layer was separated. The organic layer was washed with an aqueous saturated sodium chloride solution, dried over anhydrous magnesium sulfate, and concentrated under reduced pressure. The residue was purified with a basic silica gel column (ethyl acetate) to obtain the title compound (0.47 g, 42%) as white powder.
NMR (300 MHz, CDCl₃) δ: 0.89 (3H, d, J = 7.4), 0.93 (3H, d, J = 7.4), 1.48 (9H, s), 1.60-1.90 (4H, m), 2.03 (3H, s), 2.11-2.24 (1H, m), 2.83 (2H, t, J = 12.2), 4.05-4.30 (3H, m), 4.32-4.35 (2H, s), 6.64 (1H, dd, J = 8.8, 4.8), 5.35 (1H, d, J = 12.8), 5.48 (1H, d, J = 12.8), 6.88 (1H, s).

### 187b) N-acetyl-L-valine [2-(1-{(2S)-3-[(6-chloro-2-naphthyl)sulfonyl]-2-hydoroxypropanoyl}-4-piperidinyl)-3-oxo-2,3-dihydro-1H-imidazo[1,5-c]imidazol-5-yl]methyl ester

From tert-butyl 4-[5-{[(N-acetyl-L-valyl)oxy]methyl}-3-oxo-1H-imidazo[1,5-c]imidazol-2(3H)-yl]piperidine-1-carboxylate (0.45 g) obtained in Example 187a), the title compound (108 mg, 17%) was obtained as white powder in a similar manner to Example 185e).
NMR (200 MHz, CDCl₃) δ: 0.89 (3H, d, J = 7.4), 0.94 (3H, d, J = 7.4), 1.63-1.84 (2H, m), 1.85-2.10 (5H, m), 2.21 (1H, m), 2.74-2.90 (1H, m), 3.18-3.30 (1H, m), 3.43-3.58 (2H, m), 3.68-3.89 (1H, m), 4.08-4.32 (2H, m), 4.34-4.37 (2H, m), 4.64 (1H, dd, J = 9.2, 4.8), 4.68-4.80 (1H, m), 4.96-5.12 (1H, m), 5.35 (1H, d, J = 12.4), 5.49 (1H, d, J = 12.4), 6.05 (1H, d, J = 8.2), 6.88 (1H, s), 7.60 (1H, dd, J = 8.8, 1.8), 7.94 (3H, s), 7.96 (1H, d, J = 8.8), 8.51 (1H, s).

### Example 188

### [2-(1-{(2S)-3-[(6-chloro-2-naphtyl)sulfonyl]-2-hydoroxypropanoyl}-4-piperidinyl)-3-oxo-2,3-dihydoro-1H-imidazo[1,5-c]imidazol-5-yl]methyl isopropyl carbonate

### 188a) Tert-butyl 4-[5-{[(isoproxycarbonyl)oxy]methyl}-3-oxo-1H-imidazo[1,5-c]imidazol-2(3H)-yl]piperidine-1-carboxylate

A 1.1 M solution (1.69 mL) of lithium bis(trimethylsilyl)amide in THF was added dropwise to a solution of tert-butyl 4-[5-(hydroxymethyl)-3-oxo-1H-imidazo[1,5-c]imidazol-2(3H)-yl]piperidine-1-carboxylate (0.52 g) obtained in Example 185c) in THF (30 mL) under ice-cooling, and the mixture was stirred for 10 minutes. Under ice cooling, isopropyl chlorocarbonate (0.35 mL) was added dropwise to the reaction solution and the mixture was stirred for 30 minutes under ice cooling. An aqueous saturated ammonium chloride solution was added dropwise to the reaction solution and the mixture was diluted with ethyl acetate. An organic layer was separated, washed with an aqueous saturated sodium chloride solution, dried over anhydrous magnesium sulfate, and concentrated under reduced pressure. The residue was purified with a silica gel column (ethyl acetate/hexane = 1/4) to obtain the title compound (0.48 g, 73%) as white powder.
NMR (200 MHz, CDCl₃) δ: 1.30 (6H, d, J = 6.3), 1.48 (9H, s), 1.56-1.69 (2H, m), 1.85 (2H, dd, J = 11.7, 2.1), 2.82 (2H, t, J = 11.7), 4.05-4.18 (1H, m), 4.27 (2H, d, J = 11.7), 4.32 (2H, d, J = 2.1), 4.88-4.96 (1H, m), 5.42 (2H, s), 6.87 (1H, t, J = 1.5).

### 188b) [2-(1-{(2S)-3-[(6-chloro-2-naphthyl)sulufonyl]-2-hydroxypropanoyl}-4-piperidinyl)-3-oxo-2,3-dihydoro-1H-imidazo[1,5-c]imidazol-5-yl]methyl isopropyl carbonate

From tert-butyl 4-[5-{[(isoproxycarbonyl)oxy]methyl}-3-oxo-1H-imidazo[1,5-c]imidazol-2(3H)-yl]piperidine-1-carboxylate (0.45 g) obtained in Example 188a), the title compound (120 mg, 18%) was obtained as white powder in a similar manner to Example 185e).
NMR (200 MHz, CDCl₃) δ: 1.31 (6H, d, J = 6.2), 1.63-1.83 (2H, m), 1.90-2.06 (2H, m), 2.64-2.86 (2H, m), 3.15-3.36 (2H, m), 3.44-3.54 (2H, m), 3.78 (2H, dd, J = 28.2, 7.0), 4.05-4.23 (2H, m), 4.31-3.34 (2H, m), 4.66-4.80 (1H, dd, J = 8.8, 2.0), 7.94 (3H, s), 7.96 (1H, d, J = 8.8), 8.51 (1H, s) .
Elemental analysis for C₂₈H₃₁N₆O₈SCl•0.2Et₂O
Calculated (%): C, 54.57; H, 5.25; N, 8.84
Found (%): C, 54.38; H, 5.36; N, 8.63

### Example 189

### 2-{1-[3-[(6-Chloro-2-napththyl)sulfonyl]-2-(hydroxymethyl)propanoyl]-4-piperidinyl}-5-methyl-1,2-dihydro-3H-imidazo[1,5-c]imidazol-3-one

### 189a) 3-[(6-Chloro-2-napththyl)thio]-2-(hydroxymethyl)propionic acid

Under argon atmosphere, triethylamine (1.5 mL) was added to a solution of 6-chloronaphthalene-2-thiol (1.9 g) and ethyl 2-(hydroxymethyl)acrylate (1.3 g) in dichloromethane (50 mL), and the mixture was stirred at room temperature for 15 hours. The reaction solution was diluted with dichloromethane, washed successively with water and an aqueous saturated sodium chloride solution, and dried over anhydrous magnesium sulfate. The solvent was distilled off under reduced pressure, and the residue was purified with a silica gel column (hexane/ethyl acetate = 4/1) to obtain a colorless oil. The resulting oil was dissolved in ethanol (20 mL), a 1N aqueous sodium hydroxide solution (10 mL) was added, and the mixture was stirred at room temperature for 15 hours. The reaction solution was concentrated under reduced pressure, and the residue was diluted with water, and washed with diethyl ether. An aqueous layer was adjusted to about pH 3 with 1N hydrochloric acid and then extracted with ethyl acetate. The extract was washed with an aqueous saturated sodium chloride, and then dried over anhydrous magnesium sulfate. The solvent was distilled off under reduced pressure and the residue was washed with hexane-diethyl ether to obtain the tile compound (0.86 g, 52%) as a white solid.
NMR (200 MHz, CDCl₃) δ: 2.74-2.86 (2H, m), 3.25 (1H, dd, J = 13.8, 8.0), 3.48 (1H, dd, J = 13.8, 5.8), 3.93 (2H, d, J = 5.2), 7.40 (1H, dd, J = 8.8, 1.8), 7.47 (1H, dd, J = 8.8, 1.8), 7.65 (1H, d, J = 3.6), 7.69 (1H, d, J = 3.6), 7.76 (2H, m).

### 189b) 2-{1-[3-[(6-Chloro-2-naphthyl)thio]-2-(hydroxymethyl)propanoyl]-4-piperidinyl}-5-methyl-1,2-dihydro-3H-imidazo[1,5-c]imidazol-3-one

From 3-[(6-chloro-2-napththyl)thio]-2-(hydroxymethyl)propionic acid (0.75 g) obtained in Example 189a) and 5-methyl-2-(4-piperidinyl)-1,2-dihydro-3H-imidazo[1,5-c]imidazol-3-one dihydrochloride (0.74 g) obtained in Example 69b), the title compound (0.62 g, 32%) was obtained as a white solid in a similar manner to Example 128.
NMR (200 MHz, CDCl₃) δ: 1.40-1.67 (2H, m), 1.68-1.85 (2H, m), 2.56-2.65 (1H, m), 2.59 (3H, s), 2.98-3.06 (1H, m), 3.07-3.47 (3H, m), 3.81-3.95 (4H, m), 4.07-4.16 (2H, m), 4.80 (1H, t, J = 13.5), 6.68 (1H, t, J = 1.5), 7.42-7.46 (2H, m), 7.69 (2H, dd, J = 8.7, 2.1), 7.81 (2H, dd, J = 8.7, 2.1).

### 189c) 2-{1-[3-[(6-Chloro-2-naphthyl)sulfonyl]-2-(hydroxymethyl)propanoyl]-4-piperidinyl}-5-methyl-1,2-dihydro-3H-imidazo[1,5-c]imidazol-3-one

A solution of oxone (1.5 g) in water (20 mL) was added to a solution of 2-{1-[3-[(6-chloro-2-naphthyl)thio]-2-(hydroxymethyl)propanoyl]-4-piperidinyl}-5-methyl-1,2-dihydro-3H-imidazo[1,5-c]imidazol-3-one (0.55 g) obtained in Example 189b) in methanol (20 mL), and the mixture was stirred at room temperature for 3 hours. An aqueous sodium thiosulfate solution was added to the reaction solution, and the mixture was stirred at room temperature for 30 minutes. Methanol was distilled off under reduced pressure, and the reaction solution was neutralized with an aqueous saturated sodium hydrogencarbonate solution and then extracted with chloroform. The extract was dried over anhydrous magnesium sulfate and the solvent was distilled off under reduced pressure. The residues was purified with a basic silica gel column (ethyl acetate) and recrystallized from ethanol-water to obtain the title compound (0.40 g, 68%) as a white solid.
NMR (300 MHz, CDCl₃) δ: 1.57-1.99 (3H, m), 2.09-2.22 (1H, m), 2.22-2.42 (0.5H, m), 2.61 (3H, s), 2.69-2.79 (0.5H, m), 3.14-3.44 (3H, m), 3.65-3.97 (4H, m), 4.06-4.17 (0.3H, m), 4.25-4.34 (3.7H, m), 4.58 (0.3H, d, J = 12.0), 4.85 (0.7H, d, J = 12.0), 6.65 (0.7H, s), 6.69 (0.3H, s), 7.61 (1H, dd, J = 9.0, 1.8), 7.87-7.96 (4H, m), 8.45 (0.3H, s), 8.49 (0.7H, s).
Elemental analysis for C₂₅H₂₇N₄O₅SCl•0.5H₂O
Calculated (%): C, 55.60; H, 5.23; N, 10.37
Found (%): C, 55.55; H, 5.14; N, 10.54

### Example 190

### 2-(1-{(2S)-3-[(6-chloro-2-naphthyl)sulfonyl]-2-hydroxypropanoyl}-4-piperidinyl)-5-(difluoromethyl)-1,2-dihydro-3H-imidazo[1,5-c]imidazol-3-one

### 190a) Tert-butyl 4-(5-formyl-3-oxo-1H-imidazo[1,5-c]imidazol-2(3H)-yl)piperidine-1-carboxylate

DMSO (0.78 g) was added dropwise to a solution of oxalyl chloride (1.3 g) in dichloromethane (100 mL) at -60°C, and the mixture was stirred for 10 minutes. To the reaction solution was added dropwise a solution of tert-butyl 4-[5-(hydroxymethyl)-3-oxo-1H-imidazo[1,5-c]imidazol-2(3H)-yl]piperidine-1-carboxylate (2.7 g) obtained in Example 185c) in dichloromethae (20 mL) at -60°C, and the mixture was stirred 10 minutes. Then, triethylamine (7.0 mL) was added dropwise at -60°C, the mixture was stirred at the same temperature for 10 minutes and then warmed to room temperature over 3 hours. The reaction solution was washed with water and dried over anhydrous magnesium sulfate. The solvent was distilled off under reduced pressure. The residue was purified with a silica gel column (EtOAc/EtOH = 30/1) to obtain the title compound (1.7 g, 62%,) as a pale yellow solid.
NMR (200 MHz, CDCl₃) δ: 1.48 (9H, s), 1.58-1.77 (2H, m), 1.79-1.98 (2H, m), 2.85 (2H, t, J = 12.4), 4.10-4.18 (1H, m), 4.28 (2H, d, J = 14.8), 4.48 (2H, s), 7.19 (1H, s), 10.1 (1H, s).

### 190b) Tert-butyl 4-[5-(difluoromethyl)-3-oxo-1H-imidazo[1,5-c]imidazol-2(3H)-yl]piperidine-1-carboxylate

Diethylaminosulfur trifluoride (0.48 mL) was added slowly dropwise to a solution of tert-butyl 4-(5-formyl-3-oxo-1H-imidazo[1,5-c]imidazol-2(3H)-yl)piperidine-1-carboxylate (0.4 g) obtained in Example 190a) in dichloromethane (20 mL), and the mixture was stirred at room temperature for 15 hours. The reaction mixture was diluted with dichloromethane and water. An organic layer was separated, dried over anhydrous sodium sulfate, and concentrated under reduced pressure. The residue was purified with a basic silica gel column (ethyl acetate /hexane = 4/1) to obtain the title compound (0.42 g, 98%) as a pale yellow solid.
NMR (300 MHz, CDCl₃) δ: 1.48 (9H, s), 1.65 (2H, dt, J = 12.6, 3.0), 1.88 (2H, d, J = 8.7), 2.84 (2H, t, J = 12.6), 4.09-4.17 (1H, m), 4.24-4.32 (2H, m), 4.39 (2H, dd, J = 3.9, 2.4), 6.95 (1H, t, J = 52.8), 6.97 (1H, t, J = 1.5). 190c) 2-(1-{(2S)-3-[(6-chloro-2-naphthyl)sulfonyl]-2-hydroxypropanoyl}-4-piperidinyl)-5-(difluoromethyl)-1,2-dihydro-3H-imidazo[1,5-c]imidazol-3-one

From tert-butyl 4-[5-(difluoromethyl)-3-oxo-1H-imidazo[1,5-c]imidazol-2(3H)-yl]piperidine-1-carboxolate (0.33 g) obtained in Example 190b), the title compound (0.20 g, 39%) was obtained as white crystals (ethanol-water) in a similar manner to Example 185e).
NMR (300 MHz, CDCl₃) δ: 1.42-1.86 (2H, m), 1.90-2.08 (2H, m), 2.72-2.86 (1H, m), 3.18-3.33 (1H, m), 3.41-3.54 (2H, m), 3.74 (0.4H, d, J = 6.3), 3.90 (0.6H, d, J = 6.3), 4.09-4.30 (2H, m), 4.39 (0.6H, s), 4.43 (0.4H, s), 4.73 (1H, t, J = 13.5), 5.02-5.09 (1H, m), 6.94 (1H, t, J = 52.8), 6.97 (1H, s), 7.60 (1H, dd, J = 9.0, 2.1), 7.94 (3H, s), 7.96 (1H, d, J = 9.0), 8.51 (1H, s).
Elemental analysis for C₂₄H₂₃N₄O₅SClF₂
Calculated (%): C, 52.13; H, 4.19; N, 10.13
Found (%): C, 52.03; H, 4.18; N, 9.99

### Example 191

### N-{[2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl}-N-methylacetamide

### 191a) Tert-butyl (1-benzyl-4-piperidinyl)({2-[(methylamino)methyl]-1-trityl-1H-imidazol-4-yl}methyl)carbamate

From tert-butyl (1-benzyl-4-piperidinyl)[(2-formyl-1-trityl-1H-imidazol-4-yl)methyl]carbamate (19.5 g) obtained in Example 183d) and a 2.0 M solution of methylamine in THF (18 mL), the title compound (19 g, 93%) was obtained as pale yellow powder in a similar manner to Example 138e). NMR (200 MHz, CDCl₃) δ: 1.28 (9H, s), 1.48-1.55 (2H, m), 1.81-1.97 (4H, m), 2.20 (3H, s), 2.87 (2H, d, J = 7.4), 2.89 (2H, s), 3.46 (2H, s), 3.84-4.04 (1H, m), 4.27(2H, s), 6.55 (1H, s), 7.08-7.17 (6H, m), 7.22-7.31 (16H, m).

### 191b) Tert-butyl (2-{[acetyl(methyl)amino]methyl}-1-trityl-1H-imidazol-4-yl)methyl(1-benzyl-4-piperidinyl)carbamate

Acetyl chloride (0.32 mL) was added dropwise to a solution of tert-butyl (1-benzyl-4-piperidinyl)({2-[(methylamino)methyl]-1-trityl-1H-imidazol-4-yl}methyl)carbamate (2.4 g) obtained in Example 191a) and triethylamine (0.62 mL) in THF (30 mL) under ice cooling, and the mixture was stirred at room temperature for 15 hours. The reaction mixture was concentrated, and ethyl acetate was added to the residue, which was washed with an aqueous saturated sodium chloride solution and dried over anhydrous sodium sulfate. The solvent was distilled off under reduced pressure, and the residue was purified with a basic silica gel column (ethyl acetate) to obtain the title compound (2.2 g, 87%) as colorless powder.
NMR (200 MHz, CDCl₃) δ: 1.26 (9H, s), 1.51 (2H, d, J = 11.0), 1.78-1.98 (4H, m), 1.98 (1.5H, s), 2.04 (1.5H, s), 2.53 (1.5 H, s), 2.65 (1.5H, s), 2.87 (2H, d, J = 11.0), 3.39 (1H, s), 3.45 (2H, s), 3.70 (1H, s), 3.85-4.01 (1H, m), 4.22 (2H, s), 6.65 (0.5H, s), 6.68 (0.5H, s), 7.10-7.22 (6H, m), 7.26-7.35 (1H, m).

### 191c) N-{[2-(1-benzyl-4-piperidinyl))-3-oxo-2,3-dihydro-1H-imidazo[1,5-c]imidazol-5-yl]methyl}-N-methylacetamide

From tert-butyl (2-{[acetyl(methyl)amino]methyl}-1-trityl-1H-imidazol-4-yl)methyl(1-benzyl-4-piperidinyl)carbamate (2.2 g) obtained in Example 191b), the title compound (0.75 g, 62%) was obtained as colorless powder in a similar manner to Example 183f).
NMR (200 MHz, CDCl₃) δ: 1.69-1.86 (4H, m), 2.06-2.15 (2H, m), 2.15 (1.5H, s), 2.31 (1.5H, s), 2.98 (1.5H, s), 3.01 (2H, d, J = 8.6), 3.12 (1.5H, s), 3.52 (2H, s), 3.89-4.10 (1H, m), 4.31 (1H, s), 4.35 (1H, s), 4.79 (1H, s), 4.92 (1H, s), 6.78 (0.5H, t, J = 1.4), 6.83 (0.5H, t, J = 1.4), 7.21-7.33 (5H, m).

### 191d) N-methyl-N-{[3-oxo-2-(4-piperidinyl)-2,3-dihydro-1H-imidazo[1,5-c]imidazol-5-ylmethyl}acetamide

From N-{[2-(1-benzyl-4-piperidinyl))-3-oxo-2,3-dihydro-1H-imidazo[1,5-c]imidazol-5-yl]methyl}-N-methylacetamide (0.70 g) obtained in Example 191c), the title compound (0.49 g, 86%) was obtained as colorless powder in a similar manner to Example 183g).
NMR (300 MHz, CDCl₃) δ: 1.59-1.75 (2H, m), 1.85-1.88 (2H, m), 2.16 (1.5H, s), 2.32 (1.5H, s), 2.69-2.80 (2H, m), 2.99 (1.5H, s), 3.13 (1.5H, s), 3.20 (2H, d, J = 11.1), 3.98-4.08 (1H, m), 4.34 (0.5H, s), 4.38 (0.5H, s), 4.80 (0.5H, s), 4.80 (0.5H, s), 4.93 (0.5H, s), 6.78 (0.5H, s), 6.84 (0.5H, s).

### 191e) N-{[2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methy]}-N-methylacetamide

WSC (0.36 g) was added to a mixture of N-methyl-N-{[3-oxo-2-(4-piperidinyl)-2,3-dihydro-1H-imidazo[1,5-c]imidazol-5-ylmethyl}acetamide (0.45 g) obtained in Example 191d), 2(S)-3-[(6-chloro-2-naphthyl)sulfonyl]-2-hydroxypropionic acid (0.58 g) obtained in Example 174a) and HOBt (0.28 g) in dichloromethane (40 mL) and acetonitrile (10 mL), and the mixture was stirred at room temperature for 15 hours. The solvent was distilled off under reduced pressure, and the residue was diluted with chloroform and an aqueous saturated sodium hydrogencarbonate solution. An organic layer was separated, washed with an aqueous saturated sodium chloride solution, and dried over anhydrous magnesium sulfate. The solvent was distilled off under reduced pressure, and the residue was purified with a basic silica gel column (ethyl acetate to ethyl acetate/ethanol = 10/1) to obtain the title compound (0.39 g, 66%) as white powder.
NMR (300 MHz, CDCl₃) δ: 1.40-1.86 (2H, m), 1.87-2.06 (2H, m), 2.15 (1.8H, s), 2.31 (1.2H, s), 2.68-2.83 (1H, m), 2.97 (1.2H, s), 3.13 (1.8H, s), 3.19-3.26 (1H, m), 3.48-3.56 (2H, m), 4.08-4.36 (4H, m), 4.63-4.75 (1H, m), 4.79 (1.2H, s), 4.91 (1.8H, s), 5.06 (1H, brs), 6.78 (1.8H, s), 6.84 (1.2H, s), 7.59 (1H, dd, J = 9.0, 1.5), 7.94 (3H, s), 7.96 (1H, d, J = 9.0), 8.51 (1H, s).
Elemental analysis for C₂₇H₃₀N₅O₆SCl•2H₂O
Calculated (%): C, 51.96; H, 5.49; N, 11.22
Found (%): C, 52.05; H, 5.23; N, 11.26

### Example 192

### N-{[2-(1-{(2S)-3-[(6-chloro-2-naphthyl)sulfonyl]-2-hydroxypropanoyl}-4-piperiidnyl)-3-oxo-2,3-dihydro-1H-imidazo[1,5-c]imidazol-5-yl]methyl}-N-methylmethanesulfonamide

### 192a) Tert-btuyl (1-benzyl-4-piperidinyl)[(2-{[methyl(methylsulfonyl)amino]methyl}-1-trityl-1H-imidazol-4-yl)methyl]carbamate

Methanesulfonyl chloride (0.35 mL) was added dropwise to a solution of tert-butyl (1-benzyl-4-piperidinyl)({2-[(methylamino)methyl]-1-trityl-1H-imidazol-4-yl}methyl)carbamate (2.4 g) obtained in Example 191a) and triethylamine (0.62 mL) in THF (30 mL), and the mixture was stirred at room temperature for 15 hours. The reaction mixture was concentrated under reduced pressure and diluted with ethyl acetate and water. An organic layer was separated, washed with an aqueous saturated sodium chloride solution, and dried over anhydrous magnesium sulfate. The solvent was distilled off under reduced pressure, and the residue was purified with a silica gel column (ethyl acetate-hexane = 4/1 to ethyl acetate) to obtain the title compound (1.2 g, 45%) as colorless powder.
NMR (200 MHz, CDCl₃) δ: 1.28 (9H, s), 1.54 (2H, d, J = 11.4), 1.70-2.00 (4H, m), 2.31 (3H, s), 2.86 (2H, d, J = 10.8), 3.15 (3H, s), 3.45 (2H, s), 3.70 (2H, s), 3.87-3.98 (1H, m), 4.20 (2H, s), 6.71 (1H, s), 7.09-7.16 (6H, m), 7.20-7.33 (15H, m).

### 192b) N-{[2-(1-benzyl-4-piperidinyl)-3-oxo-2,3-dihydro-1H-imidazo[1,5-c]imidazol-5-yl]methyl}-N-methylmethanesulfonamide

From tert-butyl (1-benzyl-4-piperidinyl)[(2-{[methyl(methylsulfonyl)amino]methyl}-1-trityl-1H-imidazol-4-yl)methyl]carbamate (1.2 g) obtained in Example 192a), the title compound (0.25 g, 38%) was obtained as white powder in a similar manner to Example 183f).
NMR (200 MHz, CDCl₃) δ: 1.75-1.86 (4H, m), 2.06-2.20 (2H, m), 3.01 (2H, d, J = 10.2), 2.95 (3H, s), 3.03 (3H, s), 3.53 (2H, s), 3.89-4.06 (1H, m), 4.34 (2H, s), 4.78 (2H, s), 6.83 (1H, t, J = 1.4), 7.23-7.35 (5H, m).

### 192c) N-methyl-N-{[3-oxo-2-(4-piperidinyl)-2,3-dihydro-1H-imidazo[1,5-c]imidazol-5-yl]methyl}methanesulfonamide

From N-{[2-(1-benzyl-4-piperidinyl)-3-oxo-2,3-dihydro-1H-imidazo[1,5-c]imidazol-5-yl]methyl}-N-methylmethanesulfonamide (0.25 g) obtained in Example 192b), the title compound (0.16 g, 83%) was obtained as colorless powder in a similar manner to Example 183g).
NMR (300 MHz, CDCl₃) δ: 1.68 (2H, dt, J = 12.0 and 4.2), 1.78-1.89 (2H, m), 2.75 (2H, t, J = 10.5), 2.95 (3H, s), 3.03 (3H, s), 3.24 (2H, d, J = 12.0), 3.96-4.10 (1H, m), 4.38 (2H, s), 4.79 (2H, s), 6.84 (1H, s).

### 192d) N-{[2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl}-N-methylmethanesulfonamide

From N-methyl-N-{[3-oxo-2-(4-piperidinyl)-2,3-dihydro-1H-imidazo[1,5-c]imidazol-5-yl]methyl}methanesulfonamide (0.15 g) obtained in Example 192c), the title compound (98 mg, 29%) was obtained as white powder in a similar manner to Example 191e).
NMR (300 MHz, CDCl₃) δ: 1.64-1.83 (2H, m), 1.90-2.06 (2H, m), 2.71-2.85 (1H, m), 2.97 (3H, s), 3.71-3.87 (1H, m), 4.09-4.29(2H, m), 4.32 (1.2H, s), 4.36 (0.8H, s), 4.74 (1H, t, J = 13.8), 4.79 (2H, s), 5.05 (1H, brs), 6.85 (1H, s), 7.60 (1H, dd, J = 8.7, 2.1), 7.95 (3H, s), 7.96 (1H, d, J = 8.7), 8.51 (1H, s).
Elemental analysis for C₂₆H₃₀N₅O₇S₂Cl•H₂O
Calculated (%): C, 48.63; H, 5.02; N, 10.91
Found (%): C, 48.76; H, 5.00; N, 10.85

### Example 193

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 4-(acetylamino)butanoate

### 193a) Tert-butyl 4-[5-({[4-(acetylamino)butanoyl]oxy}methyl)-3-oxo-1H-imidazo[1,5-c]imidazol-2(3H)-yl]piperidine-1-carboxylate

WSC (0.19 g) was added to a mixture of tert-butyl 4-[5-(hydroxymethyl)-3-oxo-1H-imidazo[1,5-c]imidazol-2(3H)-yl]piperidine-1-carboxylate obtained in Example 185c), 4-(acetylamino)butanoic acid (0.26 g) and dimethylaminopyridine (0.043 g) in dichloromethane (20 mL) and acetonitrile (20 mL), and the mixture was stirred at room temperature for 15 hours. The solvent was distilled off, and the residue was diluted with ethyl acetate, washed successively with an aqueous saturated sodium hydrogencarbonate solution and an aqueous saturated sodium chloride solution, and dried over anhydrous magnesium sulfate. The solvent was distilled off under reduced pressure, and the residue was purified with a basic silica gel column (ethyl acetate to ethyl acetate /ethanol = 19/1) to obtain the title compound (0.56 g, 68%) as white powder. NMR (200 MHz, CDCl₃) δ: 1.48 (9H, s), 1.63 (2H, dt, J = 12.4, 4.4), 1.84-1.92 (4H, m), 1.96 (3H, s), 2.43 (2H, t, J = 6.4), 2.83 (2H, t, J = 12.4), 3.20 (2H, dt, J = 6.4, 6.4), 4.02-4.17 (1H, m), 4.27 (2H, d, J = 13.2), 4.35 (2H, d, J = 1.2), 5.38 (2H, s), 6.22 (1H, brs), 6.86 (1H, t, J = 1.2).

### 193b) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 4-(acetylamino)butanoate

A 40% solution (10 mL) of hydrogen chloride in ethanol was added to tert-butyl 4-[5-({[4-(acetylamino)butanoyl]oxy}methyl)-3-oxo-1H-imidazo[1,5-c]imidazol-2(3H)-yl]piperidine-1-carboxylate (0.50 g) obtained in Example 193a), and the mixture was stirred at room temperature for 2 hours. The solvent was distilled off under reduced pressure, and the residue was suspended in a mixed solvent of dichloromethane (40 mL) and acetonitrile (20 mL). DBU (0.52 mL), (2S)-3-[(6-chloro-2-naphthyl)sulfonyl]-2-hydroxypropionic acid (0.41 g) obtained in Example 174a), HOBt (0.20 g) and WSC (0.25 g) were added successively to the suspension, and the mixture was stirred at room temperature for 15 hours. The solvent was distilled off under reduced pressure, and the residue was diluted with chloroform and an aqueous saturated hydrogencarbonate solution. An organic layer was separated, and dried over anhydrous magnesium sulfate. The solvent was distilled off under reduced pressure, and the residue was purified with a basic silica gel column (ethyl acetate to ethyl acetate/methanol = 19/1) to obtain the title compound (100 mg, 15%) as white powder.
NMR (200 MHz, CDCl₃) δ: 1.67-2.04 (6H, m), 1.95 (3H, s), 2.43 (2H, t, J = 7.0), 2.68-2.85 (2H, m), 3.15-3.30 (1H, m), 3.29 (2H, dt, J = 6.4, 6.4), 3.47-3.55 (2H, m), 4.10-4.23 (1H, m), 4.35 (1.2H, s), 4.38 (0.8H, s), 4.67-4.80 (1H, m), 5.01-5.07 (1H, m), 5.38 (2H, s), 6.20 (1H, brs), 6.87 (1H, s), 7.69 (1H, dd, J = 8.8, 1.8), 7.94 (3H, s), 7.96 (1H, d, J = 8.8), 8.51 (1H, s).
Elemental analysis for C₃₀H₃₄N₅O₈SCl•H₂O
Calculated (%): C, 53.13; H, 5.35; N, 10.33
Found (%): C, 53.13; H, 5.46; N, 10.54

### Example 194

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 5-(benzoylamino)pentanoate

### 194a) Tert-butyl 4-[5-({[5-(benzoylamino)pentanoyl]oxy}methyl)-3-oxo-1H-imidazo[1,5-climidazol-2(3H)-yl]piperidine-1-carboxylate

From tert-butyl 4-[5-(hydroxymethyl)-3-oxo-1H-imidazo[1,5-c]imidazol-2(3H)-yl]piperidine-1-carboxylate (0.6 g) obtained in Example 185 c) and 5-(benzoylamino)pentanoic acid (0.51 g), the title compound (0.57 g, 71%) was obtained as white powder in a similar manner to Example 193a).
NMR (300 MHz, CDCl₃) δ: 1.47 (9H, s), 1.53-1.84 (8H, m), 2.42 (2H, t, J = 6.9), 2.75 (2H, t, J = 12.3), 3.43 (2H, dt, J = 6.6 and 6.6), 3.99-4.12 (1H, m), 4.23 (2H, d, J = 11.7), 4.33 (2H, s), 5.37 (2H, s), 6.83 (1H, s), 6.87 (1H, t, J = 5.1), 7.37-7.50 (3H, m), 7.78-7.82 (2H, m).

### 194b) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 5-(benzoylamino)pentanoate

A 4N solution (10 mL) of hydrogen chloride in ethyl acetate was added to a solution of tert-butyl 4-[5-({[5-(benzoylamino)pentanoyl]oxy}methyl)-3-oxo-1H-imidazo[1,5-c]imidazol-2(3H)-yl]piperidine-1-carboxylate (0.54 g) obtained in Example 194a), and the mixture was stirred at room temperature for 3 hours. The solvent was distilled off under reduced pressure, and the residue was adjusted to pH 8 or above by addition of an aqueous saturated sodium hydrogencarbonate solution and then extracted with chloroform repeatedly. The extract was dried over anhydrous magnesium sulfate and concentrated under reduced pressure. WSC (0.23 g) was added to a solution of the residue obtained, (2S)-3-[(6-chloro-2-naphthyl)sulfonyl]-2-hydroxypropionic acid (0.38 g) obtained in Example 174a) and HOBt (0.18 g) in a mixed solvent of dichloromethane (20 mL)and acetonitrile (20 mL), and the mixture was stirred at room temperature for 15 hours. The solvent was distilled off under reduced pressure, and the residue was diluted with chloroform and an aqueous saturated sodium hydrogencarbonate solution. An organic layer was separated, dried over anhydrous magnesium sulfate, and concentrated under reduced pressure. The residue was purified with a basic silica gel column (ethyl acetate to ethyl acetate/methanol = 9/1) to obtain the title compound (180 mg, 24%) as white powder.
NMR (300 MHz, CDCl₃) δ: 1.58-1.83 (6H, m), 1.85-2.00 (2H, m), 2.5 (2H, d, J = 6.9), 2.69 (1H, dt, J = 12.0, 12.0), 3.08-3.23 (1H, m), 3.41-3.53 (4H, m), 3.91 (1H, brs), 4.07-4.20 (2H, m), 4.30 (1.2H, s), 4.34 (0.8H, s), 4.69 (1H, t, J = 13.5), 4.98-5.08 (1H, m), 5.37 (2H, s), 6.51 (1H, brs), 6.84 (1H, s), 7.38-7.50 (3H, m), 7.58 (1H, dd, J = 8.7, 2.1), 7.76-7.80 (2H, m), 7.93 (3H, s), 7.94 (1H, d, J = 8.7), 8.49 (1H, s).
Elemental analysis for C₃₆H₃₈N₅O₈SCl•H₂O
Calculated (%): C, 57.33; H, 5.35; N, 9.29
Found (%): C, 57.17; H, 5.35; N, 9.24

### Example 195

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(butyrylamino)propionate

### 195a) Tert-butyl 3-(butyrylamino)propionate

Under ice-cooling, butanoyl chloride (0.85 g) was added dropwise to a solution of tert-butyl 3-aminopropionate dihydrocholoride (1.82 g) and triethylamine (2.8 mL) in dicholoromethane (50 mL), and the mixture was stirred at room temperature for 15 hours. To the reaction solution was added an aqueous saturated sodium hydrogencarbonate solution, and an organic layer was separated, washed with an aqueous saturated sodium chloride solution, and dried over anhydrous magnesium sulfate. The solvent was distilled off under reduced pressure to obtain the title compound (1.96 g, 91%) as a colorless oil.
NMR (200 MHz, CDCl₃) δ: 0.94 (3H, t, J = 7.4), 1.45 (9H, s), 1.64 (2H, quintet, J = 7.4), 2.14 (2H, t, J = 7.4), 2.45 (2H, t, J = 6.0), 3.45 (2H, dt, J = 6.0 and 6.0).

### 195b) Tert-butyl 4-[5-({[(3-butyrylamino)propionyl]oxy}methyl)-3-oxo-1H-imidazo[1,5-c]imidazol-2(3H)-yl]piperidine-1-carboxylate

Concentrated hydrochloric acid (3 mL) was added to tert-butyl 3-(butyrylamino)propionate (0.52 g) obtained in Example 195a), and the mixture was stirred at room temperature for 6 hours. To the reaction solution were added toluene and THF, and the mixture was concentrated under reduced pressure to obtain a pale yellow oil. WSC (0.35 g) was added to a solution of the resulting oil, tert-butyl 4-[5-(hydroxymethyl)-3-oxo-1H-imidazo[1,5-c]imidazol-2(3H)-yl]piperidine-1-carboxylate (0.61 g) obtained in Example 185c) and dimethylaminopyridine (0.048 g) in dichloromethane (30 mL), and the mixture was stirred at room temperature for 15 hours. The reaction solution was diluted with dichloromethane and an aqueous saturated sodium chloride solution, and an organic layer was separated and dried over anhydrous magnesium sulfate. The solvent was distilled off under reduced pressure, and the residue was purified with a basic silica gel column (ethyl acetate to ethyl acetate-methanol = 19/1) to obtain the title compound (0.75 g, 70%) as white powder.
NMR (300 MHz, CDCl₃) δ: 0.93 (3H, t, J = 7.5), 1.47 (9H, s), 1.54-1.72 (4H, m), 1.86 (2H, d, J = 13.2), 2.16 (2H, t, J = 7.5), 2.58 (2H, t, J = 5.4), 2.82 (2H, t, J = 12.6), 3.57 (2H, dt, J = 5.4 and 5.4), 4.03-4.15 (1H, m), 4.28 (2H, d, J = 10.5), 4.36 (1H, s), 5.43 (2H, s), 6.84 (1H, s).

### 195c) [2-(1-{(2S)-3-[(6-chloro-2-naphtyl)sulfonyl]-2-hydroxypropanoyl}-4-piperidinyl)-3-oxo-2,3-dihydro-1H-imidazo[1,5-c]imidazol-5-yl]methyl 3-(butyrylamino)propionate

From tert-butyl 4-[5-({[(3-butyrylamino)propionyl]oxy}methyl)-3-oxo-1H-imidazo[1,5-c] imidazol-2(3H)-yl]-piperidine-1-carboxylate (0.65 g) obtained in Example 195b), the title compound (65 mg, 7%) was obtained as white powder in a similar manner to Example 194b).
NMR (300 MHz, CDCl₃) δ: 0.93 (3H, t, J = 7.5), 1.65-1.86 (4H, m), 1.92-2.05 (2H, m), 2.16 (2H, t, J = 7.5), 2.59 (2H, t, J = 6.3), 2.75 (2H, dt, J = 13.8 and 13.8), 3.13-3.30 (1H, m), 3.49-3.59 (4H, m), 4.09-4.24 (2H, m), 4.34 (1.2H, s), 4.38 (0.8H, s), 4.72 (1H, J = 13.8), 5.07-5.09 (1H, m), 5.42 (2H, s), 6.84 (1H, s), 7.59 (1H, dd, J = 9.0 and 2.1), 7.94 (3H, s), 7.96 (1H, d, J = 9.0), 8.51 (1H, s). Elemental analysis for C₃₁H₃₆N₅O₈SCl•0.5H₂O
Calculated (%): C, 54.50; H, 5.46; N, 10.25
Found (%): C, 54.56; H, 5.75; N, 10.19

### Example 196

### 3-(1-{3-[(6-Chloro-2-naphtyl)sulfonyl]propionyl}-4-piperidinyl)-5,6,7,8-tetrahydroimidazo[1,5-a]pyridine hydrochloride

From 3-[(6-chloro-2-naphtyl)sulfonyl]propionic acid and 3-(4-piperidinyl)-5,6,7,8-tetrahydroimidazopyridine dihydrochloride obtained in Example 225c), the title compound (76%) was obtained as white crystals in a similar manner to Example 207d).
NMR (200 MHz, DMSO-d₆) δ: 1.42-1.68 (2H, m), 1.68-2.03 (6H, m), 2.47-2.68 (1H, m), 2.68-2.87 (3H, m), 2.99-3.20 (1H, m), 3.24-3.54 (2H, m), 3.65 (2H, t, J = 5.8), 3.88-4.03 (1H, m), 4.14 (2H, t, J = 5.8), 4.31-4.48 (1H, m), 7.34 (1H, s), 7.73 (1H, dd, J = 8.8, 2.2), 8.01 (1H, dd, J = 8.8, 2.0), 8.18-8.32 (3H, m), 8.67 (1H, s).

### Example 197

### 2-(1-{(2S)-3-[(6-chloro-2-naphthyl)sulfonyl]-2-hydroxypropanoyl}-4-piperidinyl)-5,7-dimethyl-1,2-dihydro-3H-imidazo[1,5-c]imidozol-3-one

### 197a) 2-(1-Benzyl-4-piperidinyl)-5,7-dimethyl-1,2-dihydro-3H-imidazo[1,5-c]imidazol-3-one

From 2,4-dimethyl-1H-imidazole-5-carbaldehyde (1.5 g) and 1-benzyl-4-piperidineamine (2.3 g), the title compound (2.9 g, 74%) was obtained as white powder in a similar manner to Example 69a).
NMR (300 MHz, CDCl₃) δ: 1.75-1.85 (4H, m), 2.06-2.17 (4H, m), 2.56 (3H, s), 2.98 (2H, d, J = 12.0), 3.52 (3H, s), 3.89-4.00 (1H, m), 4.21 (2H, s), 7.22-7.37 (5H, m).

### 197b) 5,7-Dimethyl-2-(4-piperidinyl)-1,2-dihydro-3H-imidazo[1,5-c]imidazol-3-one

From 2-(1-benzyl-4-piperidinyl)-5,7-dimethyl-1,2-dihydro-3H-imidazo[1,5-c]imidazol-3-one (0.77 g) obtained in Example 197a), the title compound (0.53 g,95%) was obtained as white powder in a similar manner to Example 183g).
NMR (300 MHz, CDCl₃) δ: 1.55-1.75 (2H, m), 1.80-1.87 (2H, m), 2.16 (3H, s), 2.57 (3H, s), 2.72 (2H, dt, J = 10.0, 2.6), 3.19 (2H, d, J = 12.0), 3.94-4.11 (1H, m), 4.23 (2H, s).

### 197c) 2-(1-{(2S)-3-[(6-chloro-2-naphthyl)sulfonyl]-2-hydroxypropanoyl}-4-piperidinyl)-5,7-dimethyl-1,2-dihydro-3H-imidazo[1,5-c]imidazol-3-one

From 5,7-dimethyl-2-(4-piperidinyl)-1,2-dihydro-3H-imidazo[1,5-c]imidazol-3-one (0.74 g) obtained in Example 197b), the title compound (0.30 g, 27%) was obtained as white powder in a similar manner to Example 191e).
NMR (300 MHz, CDCl₃) δ: 1.60-1.86 (2H, m), 1.88-2.15 (2H, m), 2.14 (3H, s), 2.57 (3H, s), 2.71-2.85 (1H, m), 3.16-3.31 (1H, m), 3.47 (2H, dd, J = 16.5, 5.7), 3.75-3.84 (1H, m), 4.07-4.22 (4H, m), 4.71 (1H, t, J = 12.0), 5.03 (1H, s), 7.58 (1H, dd, J = 9.0, 2.1), 7.93 (3H, s), 7.95 (1H, d, J = 9.0), 8.50 (1H, s).
Elemental analysis for C₂₅H₂₇N₄O₅SCl•1.7H₂O•0.3EtOAc Calculated (%): C, 53.51; H, 5.62; N, 9.53
Found (%): C, 53.65; H, 5.63; N, 9.27

### Example 198

### 2-(1-{(2S)-3-[(6-chloro-2-naphtyl)sulfonyl]-2-hydroxypropanoyl}-4-piperidinyl)-5-(methoxymethyl)-1,2-dihydro-3H-imidazo[1,5-c]imidazol-3-one

### 198a) Benzyl 4-[5-({[tert-butyl(dimethyl)silyl]oxy}methyl)-3-oxo-1H-imidazo[1,5-c]imidazol-2(3H)-yl]piperidine-1-carboxylate

A solution of benzyl chloroformate (1.5 mL) in THF (30 mL) was added to a solution of 5-({[tert-butyl(dimethyl)silyl]oxy}methyl)-2-(4-piperidinyl)-1,2-dihydro-3H-imidazo[1,5-c]imidazol-3-one (3.3 g) obtained in Example 185a) and triethylamine (1.5 mL) in THF (50 mL), and the mixture was stirred at room temperature for 15 hours. The solvent was distilled off under reduced pressure, and ethyl acetate was added to the residue, which was washed with water and an aqueous saturated sodium chloride solution and dried over anhydrous magnesium sulfate. The solvent was distilled off under reduced pressure to obtain the title compound (4.6 g, quantitative) as a pale yellow oil.
NMR (200 MHz, CDCl₃) δ: 0.14 (6H, s), 0.92 (9H, s), 1.64 (2H, ddd, J = 12.0, 12.0 and 4.0), 1.86 (2H, d, J = 9.4), 2.90 (2H, t, J = 12.0), 4.06-4.22 (1H, m), 4.28 (2H, s), 4.28-4.39 (2H, m), 4.92 (2H, s), 5.14 (2H, s), 6.81 (1H, s), 7.33-7.40 (5H, m).

### 198b) Benzyl 4-[5-(hydroxymethyl)-3-oxo-1H-imidazo[1,5-c]imidazol-2(3H)-yl]piperidine-1-carboxylate

From benzyl 4-[5-({[tert-butyl(dimethyl)silyl]oxy}methyl)-3-oxo-1H-imidazo[1,5-c]imidazol-2(3H)-yl]piperidine-1-carboxylate (4.6 g) obtained in Example 198a), the title compound (3.3 g, 94%) was obtained as white powder in a similar manner to Example 185c).
NMR (300 MHz, CDCl₃) δ: 1.58-1.75 (2H, m), 1.88 (2H, d, J = 11.1), 2.91 (2H, t, J = 12.6), 4.06 (1H, t, J = 6.9), 4.07-4.18 (1H, m), 4.30-4.40 (4H, m), 4.86 (2H, d, J = 6.9), 5.15 (1H, s), 6.78 (1H, t, J = 1.8), 7.29-7.42 (5H, m).

### 198c) Benzyl 4-[5-(methoxymethyl)-3-oxo-1H-imidazo[1,5-c]imidazol-2(3H)-yl]piperidine-1-carboxylate

Benzyl 4-[5-(hydroxymethyl)-3-oxo-1H-imidazo[1,5-c]imidazol-2(3H)-yl]piperidine-1-carboxylate (1.2 g) obtained in Example 198b) and sodium hydride (60% in oil: 0.072 g) were suspended in THF (40 mL) under ice-cooling and methyl iodide (0.19 mL) was added. The mixture was stirred at room temperature for 2 hours. The reaction solution was poured into an aqueous saturated ammonium chloride solution, and an organic layer was separated, washed with an aqueous saturated sodium chloride solution, and dried over anhydrous magnesium sulfate. The solvent was distilled off under reduced pressure, and the residue was purified with a basic silica gel column (ethyl acetate) to obtain the title compound (0.50 g, 43%) as white powder. NMR (300 MHz, CDCl₃) δ: 1.60-1.76 (2H, m), 1.87 (2H, d, J = 11.4), 2.90 (2H, d, J = 11.4), 3.44 (3H, s), 4.07-4.18 (1H, m), 4.28-4.40 (2H, m), 4.29 (2H, s), 4.70 (2H, s), 5.13 (2H, s), 6.82 (1H, s), 7.27-7.40 (5H, m).

### 198d) 5-(Methoxymethyl)-2-(4-piperidinyl)-1,2-dihydro-3H-imidazo[1,5-c]imidazol-3-one

A suspension of benzyl 4-[5-(methoxymethyl)-3-oxo-1H-imidazo[1,5-c]imidazol-2(3H)-yl]piperidine-1-carboxylate (0.50 g) obtained in Example 198c) and 10% palladium carbon (100 mg) in methanol (30 mL) was stirred at room temperature for 15 hours under hydrogen atmosphere. The reaction solution was filtered using Celite, and the filtrate was concentrated under reduced pressure to obtain the title compound (300 mg, 92%) as white powder.
NMR (200 MHz, CD₃OD) δ: 2.10-2.22 (4H, m), 3.10-3.25 (2H, m), 3.33 (3H, s), 3.47 (3H, s), 3.54 (2H, d, J = 12.6), 4.10-4.30 (1H, m), 4.65 (2H, s), 7.20 (1H, s).

### 198e) 2-(1-{(2S)-3-[(6-chloro-2-naphthyl)sulfonyl]-2-hydroxypropanoyl}-4-piperidinyl)-5-(methoxymethyl)-1,2-dihydro-3H-imidazo[1,5-c]imidazol-3-one

From 5-(methoxymethyl)-2-(4-piperidinyl)-1,2-dihydro-3H-imidazo[1,5-c]imidazol-3-one (0.30 g) obtained in Example 198d), the title compound (36 mg, 5%) was obtained as white powder in a similar manner to Example 191e).
NMR (300 MHz, CDCl₃) δ: 1.61-1.83 (2H, m), 1.93-2.05 (2H, m), 2.71-2.84 (1H, m), 3.15-3.27 (1H, m), 3.27- 3.52 (2H, m), 3.46 (3H, s), 3.72-3.90 (1H, m), 4.08-4.28 (2H, m), 4.31 (1.2H, s), 4.35 (0.8H, s), 4.65-4.80 (1H, m), 4.72 (2H, s), 5.00-5.08 (1H, m), 6.85 (1H, s), 7.60 (1H, dd, J = 9.0, 2.1), 7.95 (3H, s), 7.96 (1H, d, J = 9.0), 8.51 (1H, s). Elemental analysis for C₂₅H₂₇N₄O₆SCl•0.5H₂O
Calculated (%): C, 54.00; H, 5.08; N, 10.08
Found (%): C, 54.24; H, 5.15; N, 10.21

### Example 199

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(hexanoylamino)propionate

### 199a) Tert-butyl 3-(hexanoylamino)propionate

From tert-butyl 3-aminopropionate dihydrochloride (1.82 g) and hexanoyl chloride (1.1 g), the title compound (1.5 g, 86%) was obtained as a colorless oil in a similar manner to Example 195a).
NMR (300 MHz, CDCl₃) δ: 0,89 (3H, t, J = 6.9), 1.23-1.35 (4H, m), 1.45 (9H, s), 1.56-1.66 (2H, m), 2.15 (2H, t, J = 7.8), 2.44 (2H, t, J = 6.3), 3.47 (2H, dt, J = 6.3, 6.3), 6.07 (1H, brs).

### 199b) Tert-butyl 4-[5-({[3-(hexanoylamino)propionyl]oxy}methyl)-3-oxo-1H-imidazo[1,5-c]imidazol-2(3H)-yl]piperidine-1-carboxylate

From tert-butyl 3-(hexanoylamino)propionate (0.60 g) obtained in Example 199a) and tert-butyl 4-[5-(hydroxymethyl)-3-oxo-1H-imidazo[1,5-c]imidazol-2(3H)-yl]piperidine-1-carboxylate (0.61 g) obtained in Example 185c), the title compound (0.80 g, 64%) was obtained as white powder in a similar manner to Example 195b).
NMR (300 MHz, CDCl₃) δ: 0.86 (3H, t, J = 6.9), 1.23-1.29 (4H, m), 1.45 (9H, s), 1.57-1.68 (4H, m), 1.84 (2H, d, J = 12.0), 2.15 (2H, t, J = 7.5), 2.56 (2H, t, J = 8.4), 2.80 (2H, t, J = 13.5), 3.51-3.59 (2H, m), 4.01-4.14 (1H, m), 4.25 (2H, d, J = 10.5), 4.34 (2H, s), 5.41 (2H, s), 6.82 (1H, s).

### 199c) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(hexanoylamino)propionate

From tert-butyl 4-[5-({[3-(hexanoylamine)propionyl]oxy}methyl)-3-oxo-1H-imidazo[1,5-climidazol-2(3H)-yl]piperidine-1-carboxylate (0.40 g) obtained in Example 199b), the title compound (0.15 g, 21%) was obtained as white powder in a similar manner to Example 194b).
NMR (300 MHz, CDCl₃) δ: 0.89 (3H, t, J = 6.9), 1.26-1.35 (4H, m), 1.57-1.67 (2H, m), 1.70-1.87 (2H, m), 1.96-2.07 (2H, m), 2.17 (2H, t, J = 7.5), 2.59 (2H, t, J = 6.0), 2.71-2.85 (1H, m), 3.17-3.32 (1H, m), 3.45-3.60 (4H, m), 3.69-3.88 (1H, m), 3.17-3.32 (1H, m), 3.45-3.60 (4H, m), 3.69-3.88 (1H, m), 4.09-4.26 (2H, m), 4.35 (1.4H, s), 4.39 (0.6H, s), 4.69-4.80 (1H, m), 5.03-5.09 (1H, m), 5.44 (2H, s), 6.76 (1H, brs), 6.85 (1H, s), 7.60 (1H, dd, J = 9.0, 1.8), 7.95 (3H, s), 7.96 (1H, d, J = 9.0), 8.51 (1H, s). Elemental analysis for C₃₃H₃₀N₅O₈SCl•H₂O
Calculated (%): C, 55.03; H, 5.88; N, 9.72
Found (%): C, 55.13; H, 6.01; N, 9.84

### Example 200

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-ethyl-N-propionylamino)propionate

### 200a) Tert-butyl 3-(ethylamino)propionate

A 2.0 M solution (30 mL) of ethylamine in THF was added to a solution of tert-butyl acrylate (5.0 g) in THF (30 mL), and the mixture was stirred at room temperature for 3 days. The reaction solution was concentrated under reduced pressure, and the residue was purified with a basic silica gel column (hexane-ethyl acetate = 19/1 to 1/4) to obtain the title compound (4.9 g, 73%) as a colorless oil.
NMR (300 MHz, CDCl₃) δ: 1.11 (3H, t, J = 6.9), 1.45 (9H, s), 2.44 (2H, t, J = 6.6), 2.66 (2H, q, J = 6.9), 2.84 (2H, t, J = 6.6).

### 200b) Tert-butyl 3-(N-ethyl-N-propionylamino)propionate

From tert-butyl 3-(ethylamino)propionate (1.0 g) obtained in Example 200a) and propanoyl chloride (0.67 g), the title compound (1.2 g, 91%) was obtained as a pale yellow oil in a similar manner to Example 195a).
NMR (300 MHz, CDCl₃) δ: 1.15 (3H, t, J = 7.5), 1.17 (3H, t, J = 7.5), 1.44 (5.5H, s), 1.45 (3.5H, s), 2.32 (1.2H, q, J = 7.5), 2.36 (0.8H, q, J = 7.5), 2.46-2.54 (2H, m), 3.31-3.41 (2H, m), 3.55 (2H, t, J = 7.5).

### 200c) Tert-butyl 4-[5-({[3-(N-ethyl-N-propionylamino)propionyl]oxy}methyl)-3-oxo-1H-imidazo[1,5-c]imidazol-2(3H)-yl]piperidine-1-carboxylate

From tert-butyl 3-(N-ethyl-N-propionylamino)propionate (0.40 g) obtained in Example 200b) and tert-butyl 4-[5-(hydroxymethyl)-3-oxo-1H-imidazo[1,5-c]imidazol-2(.3H)-yl]piperidine-1-carboxylate (0.50 g) obtained in Example 185c), the title compound (0.51 g, 70%) was obtained as white powder in a similar manner to Example 195b).
NMR (300 MHz, CDCl₃) δ: 1.07-1.20 (6H, m), 1.47 (9H, s), 1.59-1.72 (2H, m), 1.87 (2H, d, J = 11.4), 2.28-2.37 (2H, m), 2.60-2.69 (2H, m), 2.83 (2H, t, J = 12.0), 3.36 (2H, t, J = 7.2, 7.2), 3.56-3.63 (2H, m), 4.04-4.15 (1H, m), 4.27 (2H, d, J = 12.6), 4.36-4.39 (2H, m), 5.36 (1.2H, s), 5.40 (0.8H, s), 6.85 (0.6H, s), 6.87 (0.4H, s).

### 200d) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-ethyl-N-propionylamino)propionate

From tert-butyl 4-[5-({[3-(N-ethyl-N-propionylamino)propionyl]oxy}methyl)-3-oxo-1H-imidazo[1,5-c]imidazol-2(3H)-yl]piperidine-1-carboxylate (0.45 g) obtained in Example 200c), the title compound (0.17 g, 42%) was obtained as white powder an in Example 194b).
NMR (300 MHz, CDCl₃) δ: 1.07-1.18 (6H, m), 1.66-1.90 (2H, m), 1.92-2.08 (2H, m), 2.28-2.36 (2H, m), 2.63-2.88 (3H, m), 3.25-3.30 (1H, m), 3.35 (2H, q, J = 7.2), 3.45-3.63 (4H, m), 3.74-3.94 (1H, m), 4.08-4.28 (2H, m), 4.33 (1.4H, s), 4.36 (0.6H, s), 4.65-4.83 (1H, m), 4.98-5.10 (1H, m), 5.37 (1.4H, s), 5.41 (0.6H, s), 6.87 (1H, s), 7.60 (1H, dd, J = 8.7, 1.4), 7.94 (3H, s), 7.95 (1H, d, J = 8.7), 8.51 (1H, s). Elemental analysis for C₃₂H₃₈N₅O₈SCl•2H₂O
Calculated (%): C, 53.07; H, 5.85; N, 9.67
Found (%): C, 53.37; H, 5.75; N, 9.68

### Example 201

### [2-(1-{(2S)-3-[(6-choloro-2-naphtyl)sulfonyl]-2-hydroxypropanoyl}-4-piperidinyl)-3-oxo-2,3-dihydro-1H-imidazo[1,5-c]imidazol-5-yl]methyl 3-(N-butyryl-N-ethylamino)propionate

### 201a) Tert-butyl 3-(N-butyryl-N-ethylamino)propionate

From tert-butyl 3-(ethylamino)propionate (1.0 g) obtained in Example 200a) and butanoyl choloride (0.88 g), the title compound (1.3 g, 97%) was obtained as a pale yellow oil in a similar manner to Example 195a).
NMR (300 MHz, CDCl₃) δ: 0.93-1.02 (3H, m), 1.11 (1.2H, t, J = 7.2), 1.17 (1.8H, t, J = 7.2), 1.44 (6H, s), 1.46 (3H, s), 1.63-1.74 (2H, m), 2.25-2.33 (2H, m), 2.41-2.54 (2H, m), 3.32-3.41 (2H, m), 3.54 (2H, t, J = 6.9).

### 201b) Tert-butyl 4-[5-({[3-(N-butyryl-N-ethylamino)propionyl]oxy}methyl)-3-oxo-1H-imidazo[1,5-c]imidazol-2(3H)-yl]piperidine-1-carboxylate

From tert-butyl 3-(N-butyryl-N-ethylamino)propionate (0.40 g) obtained in Example 201a) and tert-butyl 4-[5-(hydroxymethyl)-3-oxo-1H-imidazo[1,5-c]imidazol-2(3H)-yl]piperidine-1-carboxylate obtained in Example 185c), the title compound was obtained as white powder (0.54 g, 73%) in a similar manner to Example 195b).
NMR (300 MHz, CDCl₃) δ: 0.94 (3H, t, J = 7.5), 1.09 (1.2H, t, J = 6.9), 1.18 (1.8H, t, J = 6.9), 1.47 (9H, s), 1.58-1.71 (4H, m), 1.87 (2H, d. J = 10.8), 2.24-2.30 (2H, m), 2.61-2.69 (2H, m), 2.83 (2H, t, J = 12.6), 3.31-3.40 (2H, m), 3.56-3.63 (2H, m), 4.05-4.13 (1H, m), 4.27 (2H, d, J = 10.8), 4.34-4.39 (2H, m), 5.36 (1.2H, s), 5.40 (0.8H, s), 6.85 (0.6H, s), 6.87 (0.4H, s).

### 201c) [2- (1-{(2S) -3- [(6-chloro-2-naphthyl) sulfonyl] -2-hydroxypropanoyl}-4-piperidinyl)-3-oxo-2,3-dihydro-1H-imidazo[1,5-c]imidazol-5-yl]methyl 3-(N-butyryl-N-ethylamino)propionate

From tert-butyl 4-[5-({[3-(N-butyryl-N-ethylamino)propionyl]oxy}methyl)-3-oxo-1H-imidazo[1,5-c]imidazol-2(3H)-yl]piperidine-1-carboxylate (0.54 g) obtained in Example 201b), the title compound (0.18 g, 25%) was obtained as white powder in a similar manner to Example 194b).
NMR (300 MHz, CDCl₃) δ: 0.94 (3H, t, J = 7.2), 1.90-2.09 (2H, m), 2.24-2.30 (2H, m), 2.61-2.88 (3H, m), 3.15-3.40 (2H, m), 3.44-3.64 (4H, m), 3.72-3.91 (1H, m), 4.10-4.27 (2H, m), 4.30-4.38 (2H, m), 4.68-4.83 (1H, m), 4.98-5.13 (1H, m), 5.37 (1.4H, s), 5.41 (0.6H, s), 6.87 (1H, s), 7.60 (1H, dd, J = 8.7 and 2.1), 7.95 (3H, s), 7.96 (1H, d, J = 8.7), 8.51 (1H, s).
Elemental analysis for C₃₃H₄₀N₅O₈SCl•H₂O
Calculated (%): C, 55.03; H, 5.88; N, 9.72
Found (%): C, 54.95; H, 6.06; N, 9.73

### Example 202

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-acetyl-N-methylamino)propionate

### 202a) Tert-butyl 3-(methylamino)propionate

From tert-butyl acrylate (5.0 g) and methylamine (30 mL), the title compound (3.1 g, 49%) was obtained as a colorless oil in a similar manner to Example 200a).
NMR (300 MHz, CDCl₃) δ: 1.43 (9H, s), 2.40 (3H, s), 2.40 (2H, t, J = 8.4), 2.78 (2H, t, J = 8.4).

### 202b) Tert-butyl 3-(N-acetyl-N-methylamino)propionate

From tert-butyl 3-(methylamino)propionate (0.90 g) obtained in Example 202a) and acetyl chloride (0.50 g), the title compound (1.0 g, 89%) was obtained as a pale yellow oil in a similar manner to Example 195a).
NMR (300 MHz, CDCl₃) δ: 1.45 (6H, s), 1.46 (3H, s), 2.07 (2H, s), 2.14 (1H, s), 2.49 (2H, t, J = 6.9), 2.91 (1.2H, s), 3.03 (1.8H, s), 3.56-3.62 (2H, m).

### 202c) Tert-butyl 4-[5-({[3-(N-acetyl-N-methylamino)propionyl]oxy}methyl)-3-oxo-1H-imidazo[1,5-c]imidazol-2(3H)-yl]piperidine-1-caboxylate

From tert-butyl 3-(N-acetyl-N-methylamino)propionate (0.40 g) obtained in Example 202b) and tert-butyl 4-[5-(hydroxymethyl)-3-oxo-1H-imidazo[1,5-c]imidazol-2(3H)-yl]piperidine-1-carboxylate (0.50 g) obtained Example 185c), the title compound (0.40 g, 59%) was obtained as white powder in a similar manner to Example 195b).
NMR (300 MHz, CDCl₃) δ: 1.45 (9H, s), 1.56-1.73 (2H, m), 1.85 (2H, d, J = 12.0), 2.03 (2H, s), 2.07 (1H, s), 2.55-2.68 (2H, m), 2.80 (1H, s), 2.86 (2H, s), 2.98-3.05 (2H, m), 3.55-3.69 (2H, m), 3.98-4.17 (1H, m), 4.18-4.32 (2H, m), 4.33 (2H, s), 5.37 (2H, s), 6.85 (1H, s).

### 202d) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-acetyl-N-methylamino)propionate

From tert-butyl 4-[5-({[3-(N-acetyl-N-methylamino)propionyl]oxy}methyl)-3-oxo-1H-imidazo[1,5-c]imidazol-2(3H)-yl]piperidine-1-caboxylate (0.35 g) obtained in Example 202c), the title compound (5 mg, 1%) was obtained as white powder in a similar manner to Example 194b).
NMR (300 MHz, CDCl₃) δ: 1.60-1.88 (2H, m), 1.92-2.10 (5H, m), 2.65 (2H, t, J = 6.9), 2.70-2.84 (1H, m), 2.88 (1H, s), 3.05 (1H, s), 3.14-3.34 (1H, m), 3.44-3.55 (2H, m), 3.64 (2H, t, J = 7.2), 3.74-3.98 (1H, m), 4.08-4.28 (2H, m), 4.30-4.43 (2H, m), 4.66-4.83 (1H, m), 5.00-5.10 (1H, m), 5.38 (1.4H, s), 5.41 (0.6H, s), 6.87 (1H, s), 7.60 (1H, dd, J = 9.0, 1.8), 7.95 (3H, s), 7.96 (1H, d, J = 9.0), 8.51 (1H, s).

### Example 203

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-acetyl-N-ethylamino)propionate

### 203a) Tert-butyl 3-(N-acetyl-N-ethylamino)propionate

From tert-butyl 3-(ethylamino)propionate (1.0 g) obtained in Example 200a) and acetyl chloride (0.50 g), the title compound (1.1 g, 90%) was obtained as a pale yellow oil in a similar manner to Example 195a).
NMR (300 MHz, CDCl₃) δ: 1.12 (1.4H, t, J = 7.2), 1.18 (1.6H, t, J = 7.2), 1.44 (5H, s), 1.46 (4H, s), 2.08 (1.8H, s), 2.12 (1.2H, s), 2.50 (2H, q, J = 7.2), 3.37 (2H, quintet, J = 7.2), 3.55 (2H, t, J = 7.2).

### 203b) Tert-butyl 4-[5-({[3-(N-acetyl-N-ethylamino)propionyl]oxy}methyl)-3-oxo-1H-imidazo[1,5-c]imidazol-2(3H)-yl]piperidine-1-carboxylate

From tert-butyl 3-(N-acetyl-N-ethylamino)propionate (0.40 g) obtained in Example 203a) and tert-butyl 4-[5-(hydroxymethyl)-3-oxo-1H-imidazo[1,5-c]imidazol-2(3H)-yl]piperidine-1-carbpxylate (0.50 g) obtained in Example 185c), the title compound (0.27 g, 40%) was obtained as white powder in a similar manner to Example 195b).
NMR (300 MHz, CDCl₃) δ: 1.10 (1H, t, J = 7.2), 1.17 (2H, t, J = 7.2), 1.48 (9H, s), 1.55-1.72 (2H, m), 1.83-1.93 (2H, m), 2.07 (2H, s), 2.08 (1H, s), 2.61-2.70 (2H, m), 2.83 (2H, t, J = 12.3), 3.31-3.40 (2H, m), 3.56-3.64 (2H, m), 4.05 - 4.16 (1H, m), 4.21-4.33 (2H, m), 4.35 (2H, s), 5.37 (1.3H, s), 5.41 (0.7H, s), 6.87 (0.6H, s), 6.88 (0.4H, s).

### 203c) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-acetyl-N-ethylamino)propionate

From tert-butyl 4-[5-({[3-(N-acetyl-N-ethylamino)propionyl]oxy}methyl)-3-oxo-1H-imidazo[1,5-c]imidazol-2(3H)-yl]piperidine-1-carboxylate (0.27 g) obtained in Example 203b), the title compound (35 mg, 9%) was obtained as white powder in a similar manner to Example 194b).
NMR (300 MHz, CDCl₃) δ: 1.09 (1H, t, J = 7.2), 1.17 (2H, t, J = 7.2), 1.60-1.86 (2H, m), 1.90-2.06 (2H, m), 2.07 (2H, s), 2.08 (1H, s), 2.61-2.85 (3H, m), 3.17-3.40 (3H, m), 3.45-3.65 (4H, m), 3.80-4.01 (1H, m), 4.09-4.27 (2H, m), 4.30-4.40 (2H, m), 4.98-5.10 (1H, m), 4.65-4.80 (1H, m), 5.37 (1.4H, s), 5.41 (0.6H, s), 6.87 (1H, s), 7.60 (1H, dd, J = 9.0, 2.1), 7.94 (3H, s), 7.96 (1H, d, J = 9.0), 8.51 (1H, s).
Elemental analysis for C₃₁H₃₆N₅O₈SCl•2.5H₂O
Calculated (%): C, 51.77; H, 5.75; N, 9.74
Found (%): C, 51.55; H, 5.58; N, 9.72

### Example 204

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-butyryl-N-methylamino)propionate

### 204a) Tert-butyl 3-(N-butyryl-N-methylamino)propionate

From tert-butyl 3-(methylamino)propionate (0.90 g) obtained in Example 202a) and butanoyl chloride (0.67 g), the title compound (1.2 g, 93%) was obtained as a pale yellow oil in a similar manner to Example 195a).
NMR (300 MHz, CDCl₃) δ: 0.96 (3H, t, J = 7.2), 1.44 (6H, s), 1.46 (3H, s), 1.62-1.74 (2H, m), 2.27 (1.2H, t, J = 7.5), 2.34 (0.8H, t, J = 7.5), 2.49 (2H, t, J = 7.2), 2.92 (1.2H, s), 3.02 (1.8H, s), 3.56-3.63 (2H, m).

### 204b) Tert-butyl 4-[5-({[3-(N-butyryl-N-methylamino)propionyl]oxy}methyl)-3-oxo-1H-imidazo[1,5-c]imidazol-2(3H)-yl]piperidine-1-carboxylate

From tert-butyl 3-(N-butyryl-N-methylamino)propionate (0.50 g) obtained in Example 204a) and tert-butyl 4-[5-(hydroxymethyl)-3-oxo-1H-imidazo[1,5-c]imidazol-2(3H)-yl]piperidine-1-carboxylate (0.54 g) obtained in Example 185c), the title compound (0.30 g, 38%) was obtained as white powder in a similar manner to Example 195b).
NMR (300 MHz, CDCl₃) δ: 0.94 (3H, t, J = 7.4), 1.47 (9H, s), 1.54-1.78 (4H, m), 1.80-1.91 (2H, m), 2.21-2.30 (2H, m), 2.56-2.68 (2H, m), 2.70-2.90 (2H, m), 2.89 (1H, s), 3.03 (2H, s), 3.56-3.69 (2H, m), 4.00-4.18 (1H, m), 4.19-4.35 (2H, m), 4.33 (2H, s), 5.37 (1.4H, s), 5.40 (0.6H, s), 6.85 (1H, s).204c) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-butyryl-N-methylamino)propionate

From tert-butyl 4-[5-({[3-(N-butyryl-N-methylamino)propionyl]oxylmethyl)-3-oxo-1H-imidazo[1,5-c]imidazol-2(3H)-yl]piperidine-1-carboxylate (0.30 g) obtained in Example 204b), the title compound (55 mg, 8%) was obtained as white powder in a similar manner to Example 194b).
NMR (300 MHz, CDCl₃) δ: 0.94 (3H, t, J = 6.9), 1.56-1.89 (4H, m), 1.92-2.10 (2H, m), 2.22-2.29 (2H, m), 2.64 (2H, t, J = 6.9), 2.68-2.82 (1H, m), 2.89 (1H, s), 3.03 (2H, s), 3.12-3.26 (1H, m), 3.45-3.55 (2H, m), 3.64 (2H, t, J = 6.9), 4.10-4.23 (2H, m), 4.32-4.40 (2H, m), 4.65-4.80 (1H, m), 5.01-5.08 (1H, m), 5.37 (1.4H, s), 5.40 (0.6H, s), 6.87 (1H, s), 7.59 (1H, dd, J = 9.0, 2.1), 7.94 (3H, s), 7.96 (1H, d, J = 9.0), 8.51 (1H, s).
Elemental analysis for C₃₂H₃₈N₅O₈SCl•H₂O
Calculated (%): C, 54.42; H, 5.71; N, 9.92
Found (%): C, 54.61; H, 5.71; N, 9.69

### Example 205

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-hexanoyl-N-methylamino)propionate

### 205a) Tert-butyl 3-(N-hexanoyl-N-methylamino)propionate

From tert-butyl 3-(methylamino)propionate (0.9 g) obtained in Example 202a) and hexanoyl chloride (0.85 g), the title compound (1.2 g, 83%) was obtained as a pale yellow oil in a similar manner to Example 195a).
NMR (300 MHz, CDCl₃) δ: 0.90 (3H, t, J = 6.9), 1.31-1.39 (4H, m), 1.44 (6H, s), 1.45 (3H, s), 1.57-1.69 (2H, m), 2.30 (1.2H, t, J = 7.5), 2.35 (0.8H, t, J = 7.5), 2.48 (2H, t, J = 6.9), 2.91 (1.2H, s), 3.02 (1.8H, s), 3.56-3.62 (2H, m) .

### 205b) Tert-butyl 4-[5-({[3-(N-hexanoyl-N-methylamino)propionyl]oxy}methyl)-3-oxo-1H-imidazo[1,5-c]imidazol-2(3H)-yl]piperidine-1-carboxylate

From tert-butyl 3-(N-hexanoyl-N-methylamino)propionate (0.50 g) obtained in Example 205a) and tert-butyl 4-[5-(hydroxymethyl)-3-oxo-1H-imidazo[1,5-c]imidazol-2(3H)-yl]piperidine-1-carboxylate (0.54 g) obtained in Example 185c), the title compound (0.31 g, 37%) was obtained as white powder in a similar manner to Example 195b).
NMR (200 MHz, CDCl₃) δ: 0.87 (3H, t, J = 6.6), 1.26-1.35 (4H, m), 1.45 (9H, s), 1.51-1.72 (2H, m), 1.76-1.87 (2H, m), 2.24 (2H, t, J = 7.6), 2.61 (2H, t, J = 6.8), 2.80 (2H, t, J = 12.0), 3.61 (2H, t, J = 7.0), 3.99-4.15 (1H, m), 4.21-4.32 (2H, m), 4.32 (2H, s), 5.34 (1.4H, s), 5.38 (0.6H, s), 6.83 (1H, s).

### 205c) [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-hexanoyl-N-methylamino)propionate

From tert-butyl 4-[5-({[3-(N-hexanoyl-N-methylamino)propionyl]oxy}methyl)-3-oxo-1H-imidazo[1,5-c]imidazol-2(3H)-yl]piperidine-1-carboxylate (0.30 g) obtained in Example 205b), the title compound was obtained in a similar manner to Example 194b).
LCMS (M+H⁺) 716

### Example 206

### [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(N-acetyl-N-propylamino)propionate

### 206a) Tert-butyl 3-(propylamino)propionate

Propylamine (2.8 g) was added to a solution of tert-butyl acrylate (5.0 g) in THF (40 mL), and the mixture was stirred at room temperature for 24 hours. The reaction solution was concentrated under reduce pressure, and the residue was purified with a basic silica gel column (hexane to hexane-ethyl acetate = 3/2) to obtain the title compound (0.69 g, 10%) as a colorless oil.
NMR (300 MHz, CDCl₃) δ: 0.92 (3H, t, J = 7.5), 1.45 (9H, s), 1.46-1.55 (2H, m), 2.43 (2H, t, J = 6.6), 2.58 (2H, t, J = 7.5), 2.83 (2H, t, J = 6.6).

### 206b) Tert-butyl 3-(N-acetyl-N-propylamino)propionate

From tert-butyl 3-(propylamino)propionate (0.69 g) obtained in Example 206a) and acetyl chloride (0.35 g), the title compound (0.82 g, 97%) was obtained as a colorless oil in a similar manner to Example 195a).
NMR (300 MHz, CDCl₃) δ: 0.89 (1H, t, J = 7.5), 0.92 (2H, t, J = 7.5), 1.43 (6H, s), 1.46 (3H, s), 1.50-1.67 (2H, m), 2.07 (2H, s), 2.12 (1H, s), 2.48 (0.7H, t, J = 7.0), 2.52 (1.3H, t, J = 7.0), 3.20-3.31 (2H, m), 3.55 (2H, t, J = 7.0).

### 206c) Tert-butyl 4-[5-({[3-(N-acetyl-N-propylamino)propionyl]oxy}methyl)-3-oxo-1H-imidazo[1,5-c]imidazol-2(3H)-yl]piperidine-1-carboxylate

From tert-butyl 3-(N-acetyl-N-propylamino)propionate (0.60 g) obtained in Example 206b) and tert-butyl 4-[5-(hydroxymethyl)-3-oxo-1H-imidazo[1,5-c]imidazol-2(3H)-yl]piperidine-1-carbpxylate (0.54 g) obtained in Example 185c), the title compound (0.66 g, 84%) was obtained as white powder in a similar manner to Example 195b).
NMR (300 MHz, CDCl₃) δ: 0.87 (1H, t, J = 7.2), 0.91 (2H, t, J = 7.2), 1.48 (9H, s), 1.55-1.74 (4H, m), 1.86 (2H, d, J = 12.0), 2.06 (2H, s), 2.09 (1H, s), 2.61-2.70 (2H, m), 2.75-2.88 (2H, m), 3.25 (2H, t, J = 7.8), 3.56-3.63 (2H, m), 4.04-4.15 (1H, m), 4.20-4.40 (4H, m), 5.37 (1.4H, s), 5.41 (0.6H, s), 6.86 (1H, s).

### 206d) [2-(1-{(2S)-3-[(6-chloro-2-naphthyl)sulfonyl]-2-hydroxypropionyl}-4-piperidinyl)-3-oxo-2,3-dihydro-1H-imidazo[1,5-c]imidazol-5-yl]methyl 3-(N-acetyl-N-propylamino)propionate

From tert-butyl 4-[5-({[3-(N-acetyl-N-propylamino)propionyl]oxy}methyl)-3-oxo-1H-imidazo[1,5-c]imidazol-2(3H)-yl]piperidine-1-carboxylate (0.50 g) obtained in Example 206c), the title compound (0.27 g, 39%) was obtained as white powder in a similar manner to Example 194b).
NMR (300 MHz, CDCl₃) δ: 0.82 (1H, t, J = 7.5), 0.92 (2H, t, J = 7.5), 1.49-1.90 (4H, m), 1.91-2.05 (2H, m), 2.06 (2H, s), 2.08 (1H, s), 2.61-2.85 (3h, m), 3.17-3.31 (3H, m), 3.45-3.63 (4H, m), 3.79-3.98 (1H, m), 4.08-4.26 (2H, m), 4.30-4.38 (2H, m), 4.73 (1H, t, J = 13.5), 5.01-5.09 (1H, m), 5.37 (0.3H, s), 5.41 (0.7H, s), 6.87 (1H, s), 7.60 (1H, dd, J = 9.0, 2.1), 7.95 (3H, s), 7.96 (1H, d, J = 9.0), 8.51 (1H, s).
Elemental analysis for C₃₂H₃₈N₅O₈SCl•H₂O
Calculated (%): C, 54.42; H, 5.71; N, 9.92
Found (%): C, 54.63; H, 5.87; N, 9.89

### Example 207

### 3-(1-{3-[(6-Chloro-2-naphthyl)sulfonyl]propionyl}-4-piperidinyl)imidazo[1,2-a]pyridine hydrochloride

### 207a) Tert-butyl 4-(2-oxoethyl)piperidine-1-carboxylate

From tert-butyl 4-(2-hydroxyethyl)piperidine-1-carboxylate (11.5 g), the title compound (10.91 g, 96%) was obtained as a pale yellow oil in a similar manner to Example 190a).
NMR (300 MHz, CDCl₃) δ: 1.07-1.28 (2H, m), 1.47 (9H, s), 1.63-1.74 (2H, m), 1.98-2.13 (1H, m), 2.39 (2H, d, J = 6.6), 2.68-2.84 (2H, m), 3.98-4.18 (2H, m), 9.78 (1H, s).

### 207b) Tert-butyl 4-(imidazo[1,2-a]pyridine-3-yl)piperidine-1-carboxylate

5,5-Dibromobarbituric acid (6.41 g) was added to a solution of tert-butyl 4-(2-oxoethyl)piperidine-1-carboxylate (10.2 g) obtained in Example 207a) in diethyl ether (100 mL), and the mixture was stirred at room temperature for 16 hours. The precipitate formed was filtered off, and the filtrate was washed successively with an aqueous saturated sodium hydrogencarbonate solution and an aqueous saturated sodium chloride solution, and dried over anhydrous magnesium sulfate. The solvent was distilled off under reduced pressure, and the residue was dissolved in ethanol (100 mL) and refluxed for 3 hours after addition of 2-aminopyridine (2.63 g). The solvent was distilled off under reduced pressure and water was added to the residue, which was washed with ethyl acetate. An aqueous layer was adjusted to pH 9 with 8N sodium hydroxide under ice-cooling and then extracted with ethyl acetate. The extract was washed with an aqueous saturated sodium chloride solution and dried over anhydrous magnesium sulfate. The solvent was distilled off under reduced pressure, and the residue was purified with a silica gel column (ethyl acetate/ethanol = 10/1) to obtain the title compound (8.11 g, 60%) as a pale yellow solid.
NMR (200 MHz, CDCl₃) δ: 1.49 (9H, s), 1.59-1.85 (2H, m), 1.98-2.17 (2H, m), 2.83-3.10 (3H, m), 4.18-4.38 (2H, m), 6.83 (1H, t, J = 6.4), 7.16 (1H, t, J = 8.0), 7.41 (1H, s), 7.62 (1H, d, J = 9.2), 7.97 (1H, d, J = 6.8).

### 207c) 3-(4-Piperidinyl)imidazo[1,2-a]pyridine dihydrochloride

Tert-butyl 4-(2-oxoethyl)piperidine-1-carboxylate (9.04 g) obtained in Example 207a) was dissolved in concentrated hydrochloric acid (49 mL), and stirred at room temperature for 10 minutes. To the reaction solution was added ethanol, and the mixture was concentrated under reduced pressure. The precipitated crystals were filtered and washed with ethanol and ether to obtain the title compound (7.92 g, 91%) as white crystals.
NMR (200 MHz, D₂O) δ: 1.94-2.22 (2H, m), 2.39-2.59 (2H, m), 3.23-3.48 (2H, m), 3.52-3.78 (3H, m), 7.48-7.61 (1H, m), 7.86 (1H, s), 7.98-8.04 (2H, m), 8.71 (1H, d, J = 7.0).

### 207d) 3-(1-{3-[(6-Chloro-2-naphthyl)sulfonyl]propionyl}-4-piperidinyl)imidazo[1,2-a]piperidine hydrochloride

From 3-(4-piperidinyl)imidazo[1,2-a]pyridine dihydrochloride obtained in Example 207c), 3-(1-{3-[(6-chloro-2-naphthyl)sulfonyl]propionyl}-4-piperidinyl)imidazo[1,2-a]pyridine was obtained in a similar manner to Example 19. This was dissolved in ethanol and concentrated hydrochloric acid was added. The solvent was then distilled off under reduced pressure. The residue was washed with ethanol-ether to obtain the title compound (yield 81%) as white powder.
NMR (300 MHz, DMSO-d₆) δ: 1.28-1.39 (1H, m), 1.39-1.68 (1H, m), 1.93-2.11 (2H, m), 2.62-2.84 (3H, m), 3.12-3.28 (1H, m), 3.32-3.57 (1H, m), 3.61-3.72 (2H, m), 3.88-4.01 (1H, m), 4.31-4.45 (1H, m), 7.54 (1H, t, J = 6.6), 7.73 (1H, dd, J = 7.4, 6.0), 7.93-8.04 (4H, m), 8.19 (1H, d, J = 8.7), 8.26-8.31 (2H, m), 8.68 (1H, s), 9.02 (1H, d, J = 6.9). Elemental analysis for C₂₅H₂₄ClN₃O₃S•HCl•H₂O
Calculated (%): C, 55.97; H, 5.07; N, 7.83
Found (%): C, 56.15, H, 5.06; N, 7.81.

### Example 208

### 3-(1-{3-[(6-Bromo-2-naphthyl)sulfonyl]propionyl}-4-piperidinyl)imidazo[1,2-a]pyridine hydrochloride

From 3-[(6-bromo-2-naphthyl)sulfonyl]propionic acid and 3-(4-piperidinyl)imidazo[1,2-a]pyridine dihydrochloride obtained in Example 207c), the title compound (yield 91%) was obtained as pale yellow powder in a similar manner to Example 207d).
NMR (200 MHz, DMSO-d₆) δ: 1.28-1.49 (1H, m), 1.49-1.70 (1H, m), 1.89-2.12 (2H, m), 2.61-2.89 (3H, m), 3.11-3.32 (1H, m), 3.32-3.57 (1H, m), 3.57-3.77 (2H, m), 3.88-4.04 (1H, m), 4.32-4.48 (1H, m), 7.51-7.57 (1H, m), 7.84 (1H, dd, J = 6.0, 1.4), 7.93-8.16 (4H, m), 8.16-8.32 (2H, m), 8.43 (1H, s), 8.67 (1H, s), 9.04 (1H, d, J = 4.6).

### Example 209

### 3-[1-[3-[(6-Chloro-2-naphthyl)sulfonyl]propionyl]-4-piperidinyl]-5,6,7,8-tetrahydroimidazo[1,2-a]pyridine hydrochloride

### 209a) Tert-butyl 4-(5,6,7,8-tetrahydroimidazo[1,2-a]pyridin-3-yl)piperidine-1-carboxylate

Platinum oxide (114 mg) was added to a solution of tert-butyl 4-(imidazo[1,2-a]pyridin-3-yl)piperidine-1-carboxylate obtained in Example 207b) in acetic acid (15 mL), and the mixture was stirred for 16 hours under hydrogen atmosphere. The catalyst was filtered off, and the filtrate was concentrated under reduced pressure.
After water was added to the residue, it was adjusted to pH 12 with 8N sodium hydroxide. The mixture was extracted with chloroform, and the extract was washed with an aqueous saturated sodium chloride solution and dried over anhydrous magnesium sulfate. The solvent was distilled off under reduced pressure to obtain the title compound (1.53 g, quantitative) as pale yellow powder.
NMR (200 MHz, CDCl₃) δ: 1.38-1.69 (11H, m), 1.81-2.22 (6H, m), 2.57-2.93 (3H, m), 3.11 (2H, t, J = 6.6), 3.95 (2H, t, J = 5.6), 4.12-4.36 (2H, m), 6.98 (1H, s).

### 209b) 3-(4-Piperidinyl)-5,6,7,8-tetrahydroimidazo[1,2-a]pyridine dihydrochloride

From tert-butyl 4-(5,6,7,8-tetrahydroimidazo[1,2-a]pyridin-3-yl)piperidine-1-carboxylate (1.53 g) obtained in Example 209a), the title compound (1.32 g, 95%) was obtained as white crystals in a similar manner to Example 207c).
NMR (200 MHz, D₂O) δ: 1.74-2.19 (6H, m), 2.20-2.37 (2H, m), 2.95-3.31 (5H, m), 3.50-3.66 (2H, m), 4.11 (2H, t, J = 5.4), 7.20 (1H, s).

### 209c) 3-[1-[3-[(6-Chloro-2-naphthyl)sulfonyl]propionyl]-4-piperidinyl]-5,6,7,8-tetrahydroimidazo[1,2-a]pyridine hydrochloride

From 3-(4-piperidinyl)-5,6,7,8-tetrahydroimidazo[1,2-a]pyridine dihydrochloride obtained in Example 209b), the title compound (yield 78%) was obtained as white powder in a similar manner to Example 207d).
NMR (200 MHz, DMSO-d₆) δ: 1.12-1.58 (2H, m), 1.76-2.08 (6H, m), 2.48-2.68 (1H, m), 2.75 (2H, t, J = 7.4), 2.84-3.19 (4H, m), 3.64 (2H, t, J = 7.0), 3.34-3.99 (1H, m), 4.00-4.12 (2H, m), 4.26-4.40 (1H, m), 7.34 (1H, s), 7.74 (1H, dd, J = 8.8, 2.2), 8.01 (1H, dd, J = 8.8, 1.8), 8.17-8.32 (3H, m), 8.67 (1H, s).
Elemental analysis for C₂₅H₂₈ClN₃O₃S•HCl•H₂O
Calculated (%): C, 55.55; H, 5.78; N, 7.77
Found (%): C, 55.72, H, 5.82; N, 7.75.

### Example 210

### 3-(1-{3-[(6-Bromo-2-naphthyl)sulfonyl]propionyl}-4-piperidinyl)-5,6,7,8-tetrahydroimidazo[1,2-a]pyridine hydrochloride

From 3-[(6-bromo-2-naphthyl)sulfonyl]propionic acid and 3-(4-piperidinyl)-5,6,7,8-tetrahydroimidazo[1,2-a]pyridine dihydrochloride obtained in Example 209b), the title compound (yield 84%) was obtained as white powder in a similar manner to Example 207d).
NMR (200 MHz, DMSO-d₆) δ: 1.17-1.38 (1H, m), 1.38-1.58 (1H, m), 1.74-2.07 (6H, m), 2.54-2.68 (1H, m), 2.75 (2H, t, J = 7.2), 2.83-3.18 (4H, m), 3.64 (2H, t, J = 7.8), 3.74-3.97 (1H, m), 3.97-4.11 (2H, m), 4.22-4.40 (1H, m), 7.34 (1H, s), 7.84 (1H, dd, J = 8.2, 2.0), 8.00 (1H, dd, J = 8.2, 2.0), 8.17-8.23 (2H, m), 8.43 (1H, s), 8.65 (1H, s).

### Example 211

### 3-(1-{3-[(5-Chloro-1H-indol-2-yl)sulfonyl]propionyl}-4-piperidinyl)-5,6,7,8-tetrahydroimidazo[1,2-a]pyridine hydrochloride

### 211a) Tert-butyl 3-[(5-chloro-1H-indol-2-yl)thio]propionate

5-Chloro-1,3-dihydro-2H-indol-2-one (25.82 g) and Lawesson's reagent (93.47 g) were added to pyridine (300 mL) and refluxed for 16 hours. The reaction solution was poured into ice-water (2L) and allowed to be stood at room temperature for 18 hours. The precipitate formed was filtered, washed with water and dried. Tert-butyl acrylate (22.6 mL) and triethylamine (21.5 mL) were added to a suspension of the resulting yellow solid in acetonitrile (200 mL) and refluxed for 1 hour. The solvent was distilled off under reduced pressure, and water was added to the residue, which was extracted with ethyl acetate. The extract was washed with an aqueous saturated sodium chloride solution and dried over anhydrous magnesium sulfate. The solvent was distilled off under reduced pressure. The residue was purified with a silica gel column (ethyl acetate/hexane = 1/4) to obtain the title compound (24.12 g, 50%) as a pale yellow solid.
NMR (300 MHz, CDCl₃) δ: 1.47 (9H, s), 2.55 (2H, t, J = 6.9), 3.03 (2H, t, J = 6.9), 6.58-6.59 (1H, m), 7.12-7.15 (1H, m), 7.24-7.27 (1H, m), 7.51-7.52 (1H, m), 8.73 (1H, br).

### 211b) Tert-butyl 3-[(5-chloro-1H-indol-2-yl)sulfonyl]propionate

Metachloroperbenzoic acid (3.08 g) was added to a solution of tert-butyl 3-[(5-chloro-1H-indol-2-yl)thio]propionate (1.56 g) obtained in Example 211a) in dichloromethane (15 mL) at 0°C, and the mixture was stirred at room temperature for 1 hour. The reaction solution was diluted with dichloromethane (50 mL), washed successively with an aqueous saturated sodium thiosulfate solution, an aqueous saturated sodium hydrogencarbonate solution and an aqueous saturated sodium chloride solution, and then dried over anhydrous magnesium sulfate. The solvent was distilled off under reduced pressure, and the residue was purified with a silica gel column (ethyl acetate/hexane = 1/4) to obtain the title compound (1.58 g, 92%) as a white solid.
NMR (200 MHz, CDCl₃) δ: 1.38 (9H, s), 2.72 (2H, t, J = 7.4), 3.56 (2H, t, J = 7.4), 7.13-7.14 (1H, m), 7.30-7.36 (1H, m), 7.40-7.44 (1H, m), 7.68-7.69 (1H, m), 9.52 (1H, br).

### 211c) Tert-butyl 2-{[3-(allyloxy)-3-oxopropyl]sulfonyl}-5-chloro-1H-indole-1-carboxylate

Concentrated hydrochloric acid (33 mL) was added to a solution of tert-butyl 3-[(5-chloro-1H-indol-2-yl)sulfonyl]propionate (13.75 g) obtained in Example 211b) in acetic acid (250 mL), and the mixture was stirred at 60°C for 1 hour. The solvent was distilled off under reduced pressure. The residue was dissolved in DMF (100 mL) and triethylamine (6.7 mL) and allyl bromide (10.4 mL) were added. The mixture was stirred at 60°C for 2 hours. The reaction solution was poured into ice-water and then extracted with ethyl acetate. The extract was washed with water and dried over anhydrous magnesium sulfate. The solvent was then distilled off under reduced pressure. Di-tert-butyl dicarbonate (8.73 g) was added to a solution of the resulting solid and 4-dimethylaminopyridine (4.89 g) in acetonitrile (150 mL), and the mixture was stirred for 1 hour. The solvent was distilled off under reduced pressure, and water was added to the residue, which was extracted with ethyl acetate. The extract was washed with an aqueous saturated sodium chloride solution and dried over anhydrous magnesium sulfate. The solvent was distilled off under reduced pressure. The residue was purified with a silica gel column (eluting solution; ethyl acetate/hexane = 1/4) to obtain the title compound (10.61 g, 62%) as a pale light brown solid.
NMR (200 MHz, CDCl₃) δ: 1.74 (9H, s), 2.90 (2H, t, J = 7.4), 4.03 (2H, t, J = 7.4), 4.47-4.52 (2H, m), 5.17-5.30 (2H, m), 5.72-5.93 (1H, m), 7.42-7.53 (2H, m), 7.64-7.65 (1H, m), 7.98 (1H, d, J = 9.2).

### 211d) 3-[(1-Tert-butoxycarbonyl-5-chloro-1H-indol-2-yl)sulfonyl]propionic acid

A solution of tert-butyl 2-{[3-(allyloxy)-3-oxopropyl]sulfonyl}-5-chloro-1H-indole-1-carboxylate (4.27 g) obtained in Example 211c), Meldrum's acid (2.16 g) and palladium(0)tetrakistriphenylphosphine (0.58 g) in THF (40 mL) was stirred at room temperature for 3 hours under argon. The solvent was distilled off under reduced pressure, and 1N hydrochloric acid (50 mL) was added to the residue, which was extracted with ethyl acetate. The extract was dried over anhydrous magnesium sulfate, and the solvent was distilled off under reduced pressure. Diisopropyl ether was added to the residue, and the precipitate formed was filtered to obtain the title compound (3.29 g, 85%) as a pale light brown solid.
NMR (200 MHz, DMSO-d₆) δ: 1.68 (9H, s), 2.71 (2H, t, J = 7.0), 3.96 (2H, t, J = 7.0), 7.57-7.62 (2H, m), 7.93-7.94 (1H, m), 8.05 (1H, d, J = 9.2).

### 211e) Tert-butyl 5-chloro-2-({3-oxo-3-[4-(5,6,7,8-tetrahydroimidazo[1,2-a]pyridin-3-yl)-1-piperidinyl]propyl}sulfonyl)-1H-indole-1-carboxylate

From 3-[(1-tert-butoxycarbonyl-5-chloro-1H-indol-2-yl)sulfonyl]propionic acid (0.78 g) obtained in Example 211d) and 3-(4-piperidinyl)-5,6,7,8-tetrahydroimidazo[1,2-a]pyridine dihydrochloride (0.67 g) obtained in Example 209b), the title compound (1.11 g, 92%) was obtained as pale yellow powder in a similar manner to Example 19.
NMR (200 MHz, CDCl₃) δ: 1.33-1.66 (2H, m), 1.74 (9H, s), 1.78-2.08 (6H, m), 2.62-2.77 (2H, m), 2.80-3.02 (4H, m), 3.02-3.23 (1H, m), 3.81 (2H, t, J = 5.2), 3.87-4.02 (1H, m), 4.02-4.12 (2H, m), 4.52-4.67 (1H, m), 6.66 (1H, s), 7.45 (1H, dd, J = 9.2, 2.2), 7.51 (1H, s), 7.64 (1H, d, J = 2.2), 8.00 (1H, d, J = 9.2) .

### 211f) 3-(1-{3-[(5-Chloro-1H-indol-2-yl)sulfonyl]propionyl}-4-piperidinyl)-5,6,7,8-tetrahydroimidazo[1,2-a]pyridine hydrochloride

From tert-butyl 5-chloro-2-({3-oxo-3-[4-(5,6,7,8-tetrahydroimidazo[1,2-a]pyridin-3-yl)-1-piperidinyl]propyl}sulfonyl)-1H-indole-1-carboxylate (0.68 g) obtained in Example 211e), the title compound (0.54 g, 90%) was obtained as pale light brown powder in a similar manner to Example 207c).
NMR (200 MHz, DMSO-d₆) δ: 1.16-1.57 (2H, m), 1.77-2.07 (6H, m), 2.47-2.68 (2H, m), 2.76 (2H, t, J = 7.2), 2.84-3.18 (3H, m), 3.66 (2H, t, J = 6.6), 3.81-3.97 (1H, m), 3.98-4.12 (2H, m), 4.26-4.39 (1H, m), 7.16 (1H, d, J = 1.6), 7.31-7.37 (2H, m), 7.53 (1H, d, J = 8.8), 7.74 (1H, br), 7.79 (1H, d, J = 1.8).
Elemental analysis for C₂₃H₂₇ClN₄O₃S•HCl•2H₂O
Calculated (%): C, 50.46; H, 5.89; N, 10.23
Found (%): C, 50.57, H, 5.82; N, 9.99.

### Example 212

### 3-(1-{3-[(5-chloro-1H-indol-2-yl)sulfonyl]propionyl}-4-piperidinyl)imidazo[1,2-a]pyridine hydrochloride

### 212a) Tert-butyl 5-chloro-2-({3-oxo-3-[4-(imidazo[1,2-a]pyridin-3-yl)-1-piperidinyl]propyl}sulfonyl)-1H-indole-1-carboxylate

From 3-[(1-tert-butoxycarbonyl-5-chloro-1H-indol-2-yl)sulfonyl]propionic acid (0.78 g) obtained in Example 211d) and 3-(4-piperidinyl)imidazo[1,2-a]pyridine dihydrochloride (0.66 g) obtained in Example 207c), the title compound (1.04 g, 91%) was obtained as pale yellow powder in a similar manner to Example 19.
NMR (200 MHz, CDCl₃) δ: 1.52-1.83 (2H, m), 1.75 (9H, s), 2.03-2.27 (2H, m), 2.71-2.92 (1H, m), 2.92-3.07 (2H, m), 3.07-3.20 (1H, m), 3.20-3.37 (1H, m), 3.93-4.17 (3H, m), 4.58-4.74 (1H, m), 6.82-6.90 (1H, m), 7.15-7.24 (1H, m), 7.41 (1H, s), 7.47 (1H, dd, J = 9.2, 2.2), 7.54 (1H, s), 7.54-7.67 (2H, m), 7.95-8.04 (2H, m)

### 212b) 3-(1-{3-[(5-Chloro-1H-indol-2-yl)sulfonyl]propionyl}-4-piperidinyl)imidazo[1,2-a]pyridine hydrochloride

From tert-butyl 5-chloro-2-({3-oxo-3-[4-(imidazo[1,2-a]pyridin-3-yl)-1-piperidinyl]propyl}sulfonyl)-1H-indole-1-carboxylate obtained in Example 212a), the title compound (0.23 g, 84%) was obtained as pale light brown powder in a similar manner to Example 207c).
NMR (200 MHz, DMSO-d₆) δ: 1.23-1.47 (1H, m), 1.47-1.63 (1H, m), 1.89-2.12 (2H, m), 2.60-2.74 (1H, m), 2.79 (2H, t, J = 7.0), 3.09-3.56 (2H, m), 3.68 (2H, t, J = 7.0), 3.88-4.03 (1H, m), 4.28-4.47 (1H, m), 7.18 (1H, m), 7.33 (1H, dd, J = 8.8, 2.2), 7.51-7.57 (2H, m), 7.89 (1H, d, J = 2.2), 7.91-8.04 (3H, m), 9.01 (1H, d, J = 7.0).

### Example 213

### 6-Chloro-2-({3-oxo-3-[4-(5,6,7,8-tetrahydroimidazo[1,2-a]pyridin-3-yl)-1-piperidinyl]propyl}sulfonyl)-1,2,3,4-tetrahydroisoquinoline hydrochloride

### 213a) 2-[(4-Chlorophenyl)methyl]aminoethanol hydrochloride

A solution of 4-chlorobenzaldehyde (14.06 g) and ethanolamine (6.18 g) in toluene (150 mL) was refluxed for 1 hour while produced water was removed, and the solvent was distilled off under reduced pressure. Sodium triacetoxyborohydride (42.39 g) was added to a solution of the residue in methanol (150 mL), and the mixture was stirred at room temperature for 1 hour. The solvent was distilled off under reduced pressure, and water was added to the residue, which was adjusted to pH 12 with a 8N aqueous sodium hydroxide solution and then extracted with chloroform. The extract was washed with an aqueous saturated sodium chloride solution and dried over anhydrous magnesium sulfate. The solvent was distilled off under reduced pressure. The residue was dissolved in ethanol (100 mL), and concentrated hydrochloric acid (8.2 mL) was added. The solvent was distilled off under reduced pressure. 2-Propanol-ether was added to the residue, and a precipitate was filtered and washed with ether to obtain the title compound (18.21 g, 82%) as white powder.
NMR (200 MHz, DMSO-d₆) δ: 2.93 (2H, br), 3.69 (2H, t, J = 3.6), 4.15 (2H, br), 5.27 (1H, br), 7.50 (2H, d, J = 8.8), 7.62 (2H, d, J = 8.8), 9.43 (2H, br).

### 213b) 6-Chloro-1,2,3,4-tetrahydroixoquinoline hydrochloride

A mixture of 2-[(4-chlorophenyl)methyl]aminoethanol hydrochloride (22.21 g) obtained in Example 213a), ammonium chloride (4.01 g) and aluminum chloride (26.67 g) was stirred at 180°C. After 40 minutes and 80 minutes, aluminum chloride (26.67 g and 53.34 g, respectively) was added to the reaction mixture and it was stirred at 180°C for 18 hours. Ice-water was added to the residue and the mixture was adjusted to pH 12 with a 8N aqueous sodium hydroxide solution and then extracted with chloroform. The extract was washed with an aqueous saturated sodium chloride solution and dried over anhydrous magnesium sulfate. The solvent was distilled off under reduced pressure. The residue was dissolved in ethanol (50 mL), and concentrated hydrochloric acid (8.2 mL) was added. The solvent was distilled off under reduced pressure. 2-Propanol-ether was added to the residue, and a precipitate was filtered and washed with ether to obtain the title compound (4.08 g, 20%) as pale yellow powder.
NMR (200 MHz, DMSO-d₆) δ: 3.01 (2H, t, J = 6.2), 3.35 (2H, t, J = 6.4), 4.21 (2H, s), 7.19-7.33 (3H, m), 9.80 (2H, br).

### 213c) Methyl 3-(chlorosulfonyl)propionate

A chlorine gas was bubbled through a solution of dimethyl 3,3'-dithiopropionate (23.83 g) in water (250 mL) at 5°C or lower for 4 hours under ice-cooling. The reaction solution was extracted with diethyl ether, and the extract was washed with an aqueous saturated sodium chloride solution and dried over anhydrous magnesium sulfate. The solvent was distilled off under reduced pressure, and the residue was distilled under reduced pressure to obtain the title compound (10.26 g, 55%) as a yellow liquid.
NMR (200 MHz, CDCl₃) δ: 3. 06 (2H, t, J = 7.6), 3.78 (3H, s), 4.01 (2H, t, J = 7.4).

### 213d) Methyl 3-[(6-chloro-1,2,3,4-tetrahydroisoquinoline-2(1H)-yl)sulfonyl]propionate

A solution of 6-chloro-1,2,3,4-tetrahydroisoquinoline hydrochloride (2.04 g) obtained in Example 213b), DBU (1.5 mL) and triethylamine (2.8 mL) in acetonitrile (10 mL) was added dropwise to a solution of methyl 3-chlorosulfonylpropionate (2.05 g) obtained in Example 213c) in acetonitrile (10 mL) at 0°C. The reaction mixture was stirred at 0°C for 30 minutes, and the solvent was distilled off under reduced pressure. Water was added to the residue, and this was extracted with chloroform. The extract was washed with an aqueous saturated sodium chloride solution, and dried over anhydrous magnesium sulfate. The solvent was distilled off under reduced pressure, and the residue was recrystallized from chloroform-ethanol to obtain the title compound (2.70 g, 85%) as pale light brown crystals.
NMR (200 MHz, CDCl₃) δ: 2.82 (2H, t, J = 7.2), 2.93 (2H, t, J = 5.8), 3.32 (2H, t, J = 7.8), 3.57 (2H, t, J = 5.8), 3.67 (3H, s), 4.44 (2H, s), 7.01 (1H, d, J = 8.2), 7.15 (1H, s), 7.17 (1H, d, J = 8.2).

### 213e) 3-[(6-Chloro-1,2,3,4-tetrahydroisoquinolin-2(1H)-yl)sulfonyl]propionic acid

Methyl 3-[(6-chloro-1,2,3,4-tetrahydroisoquinolin-2(1H)-yl)sulfonyl]propionate (1.59 g) obtained in Example 213d) was added to a 1N aqueous sodium hydroxide solution (15 mL), and the mixture was stirred at 100°C for 40 minutes. To the reaction solution was added 1N hydrochloric acid (15 mL) at 0°C, and the precipitate formed was filtered and washed with water and ether to obtain the title compound (0.38 g, 25%) as pale light brown powder.
NMR (200 MHz, DMSO-d₆) δ: 2.86 (2H, t, J = 7.5), 2.93 (2H, t, J = 6.0), 3.31 (2H, t, J = 7.5), 3.57 (2H, t, J = 6.0), 4.44 (2H, s), 7.01 (1H, d, J = 8.1), 7.14-7.25 (2H, m).

### 213f) 6-Chloro-2-({3-oxo-3-[4-(5,6,7,8-tetrahydroimidazo[1,2-a]pyridin-3-yl)-1-piperidinyl]propyl}sulfonyl)-1,2,3,4-tetrahydroisoquinoline hydrochloride

From 3-[(6-chloro-1,2,3,4-tetrahydroisoquinolin-2(1H)-yl)sulfonyl]propionic acid obtained in Example 213e) and 3-(4-piperidinyl)-5,6,7,8-tetrahydroimidazo[1,2-a]pyridine dihydrochloride obtained in Example 209b), the title compound (62%) was obtained as white powder in a similar manner to Example 207d).
NMR (200 MHz, DMSO-d₆) δ: 1.14-1.59 (2H, m), 1.78-2.07 (6H, m), 2.48-2.68 (1H, m), 2.75-2.84 (4H, m), 2.84-3.19 (6H, m), 3.65 (2H, t, J = 7.0), 3.34-3.99 (1H, m), 4.00-4.12 (2H, m), 4.26-4.40 (1H, m), 4.44 (2H, d, J = 6.8), 7.21-7.46 (4H, m).

### Example 214

### N-({1-[3-(6-chloro-2-naphthyl)sulfonyl]propionyl}-4-piperidinyl)-5,6,7,8-tetrahydroimidazo[1,2-a]pyridine-3-carboxamide

### 214a) 3-Trichloroacetylimidazo[1,2-a]pyridine

Trichloroacetyl chloride (15.65 mL) was added to a solution of imidazo[1,2-a]pyridine (5.52 g) and 4-dimethylaminopyridine (25.69 g) in THF (150 mL) at room temperature, and the mixture was refluxed for 16 hours while vigorously stirring. A precipitate was filtered, and the filtrate was washed with water and an aqueous saturated sodium chloride solution and dried over anhydrous magnesium sulfate. The solvent was distilled off under reduced pressure, and the residue was purified with a silica gel column (ethyl acetate) to obtain the title compound (11.70 g, 95%) as a pale yellow solid.
NMR (200 MHz, CDCl₃) δ: 7.26 (1H, dt, J = 7.0, 1.2), 7.65 (1H, ddd, J = 8.8, 7.0, 1.4), 7.89 (1H, td, J = 8.8, 1.4), 8.88 (1H, s), 9.62 (1H, td, J = 7.0, 1.4).

### 214b) Tert-butyl 4-{[(imidazo[1,2-a]pyridin-3-yl)carbonyl]amino}piperidin-1-carboxylate

Tert-butyl 4-aminopiperidine-1-carboxylate (2.40 g) and triethylamine (2.8 mL) were added to a solution of 3-trichloroacetylimidazo[1,2-a]pyridine (2.64 g) obtained in Example 214a) in acetonitrile (30 mL), and the mixture was stirred at 60°C for 3 hours. The solvent was distilled off under reduced pressure and water was added to the residue, which was extracted with ethyl acetate. The extract was washed with an aqueous saturated sodium chloride solution and dried over anhydrous magnesium sulfate. The solvent was distilled off under reduced pressure, and the residue was purified with a silica gel column (ethyl acetate/ethanol = 10/1) to obtain the title compound (2.96 g, 86%) as a white solid.
NMR (200 MHz, CDCl₃) δ: 1.32-2.63 (2H, m), 1.45 (9H, s), 1.95-2.12 (2H, m), 2.81-3.02 (2H, m), 4.01-4.31 (3H, m), 6.13 (1H, d, J = 8.0), 6.99 (1H, dt, J = 7.0, 1.0), 7.37 (1H, ddd, J = 8.8, 6.6, 1.2), 7.68 (1H, td, J = 8.8, 1.0), 8.04 (1H, s), 9.48 (1H, td, J = 7.0, 1.2).

### 214c) Tert-butyl 4-{[(5,6,7,8-tetrahydroimidazo[1,2-a]pyridin-3-yl)carbonyl]amino}piperidine-1-carboxylate

From tert-butyl 4-{[(imidazo[1,2-a]pyridin-3-yl)carbonyl]amino}piperidine-1-carboxylate (0.69 g) obtained in Example 214b), the title compound (0.66 g, 94%) was obtained as a white solid in a similar manner to Example 209a).
NMR (200 MHz, CDCl₃) δ: 1.18-1.52 (2H, m), 1.46 (9H, s), 1.81-2.07 (6H, m), 2.74-2.98 (4H, m), 3.93-4.19 (3H, m), 4.31 (2H, t, J = 5.4), 5.89 (1H, d, J = 8.2), 7.34 (1H, s).

### 214d) N-({1-[3-(6-chloro-2-naphthyl)sulfonyl]propionyl}-4-piperidinyl)-5,6,7,8-tetrahydroimidazo[1,2-a]pyridine-3-carboxamide

Starting from tert-butyl 4-{[(5,6,7,8-tetrahydroimidazo[1,2-a]pyridin-3-yl)carbonyl]amino}piperidine-1-carboxylate (0.70 g) obtained in Example 214c), the title compound (yield 82%) was obtained as pale yellow powder according to the same reactions as those described in Example 207c) and Example 19 successively.
NMR (200 MHz, DMSO-d₆) δ: 1.10-1.52 (2H, m), 1.64-1.93 (6H, m), 2.37-2.68 (2H, m), 2.68-2.83 (3H, m), 2.95-3.17 (1H, m), 3.20-3.41 (2H, m), 3.63 (2H, t, J = 7.6), 3.72-4.00 (2H, m), 4.17 (2H, t, J = 5.6), 7.50 (1H, s), 7.73 (1H, dd, J = 8.8, 2.2), 7.90 (1H, d, J = 7.8), 8.00 (1H, dd, J = 8.4, 1.8), 8.19 (1H, d, J = 8.8), 8.29 (1H, d, J = 9.0), 8.66 (1H, s).

### Example 215

### 3-(1-{3-[(3,5-Dichloro-1H-indol-2-yl)sulfonyl]propionyl}-4-piperidinyl)-5,6,7,8-tetrahydroimidazo[1,2-a]pyridine hydrochloride

### 215a) Tert-butyl 3-[(3,5-dichloro-1H-indol-2-yl)thio]propionate

N-chlorosuccinimide (4.81 g) was added to a solution of tert-butyl 3-[(5-chloro-1H-indol-2-yl)thio]propionate (9.36 g) obtained in Example 211a) in ethyl acetate (100 mL), and the mixture was stirred at room temperature for 1 hour. Water was added to the reaction solution, and an organic layer was separated, washed with an aqueous saturated sodium thiosulfate solution and an aqueous saturated sodium chloride solution, and then dried over anhydrous magnesium sulfate. The solvent was distilled off under reduced pressure to obtain the title compound (10.3 g, 99%) as a pale gray solid.
NMR (200 MHz, CDCl₃) δ: 1.47 (9H, s), 2.54 (2H, t, J = 6.6), 3.07 (2H, t, J = 7.0), 7.17-7.31 (2H, m), 7.52-7.59 (1H, m), 8.93 (1H, br).

### 215b) Tert-butyl 3-[(3,5-dichloro-2-indolyl)sulfonyl]propionate

From tert-butyl 3-[(3,5-dichloro-1H-indol-2-yl)thio]propionate (10.30 g) obtained in Example 215a), the title compound (11.15 g, 99%) was obtained as a white solid in a similar manner to Example 211b).
NMR (200 MHz, CDCl₃) δ: 1.38 (9H, s), 2.73 (2H, t, J = 7.6), 3.68 (2H, t, J = 7.2), 7.39-7.40 (2H, m), 7.70 (1H, s), 9.30 (1H, br).

### 215c) Tert-butyl 2-{[3-(allyloxy)-3-oxopropyl]sulfonyl}-3,5-dichloro-1H-indole-1-carboxylate

Concentrated hydrochloric acid (5.5 mL) was added to a solution of tert-butyl 3-[(3,5-dichloro-1H-indol-2-yl)sulfonyl]propionate (11.15 g, 29.47 mmol) obtained in Example 215b) in allyl alcohol (120 mL), and the mixture was stirred at 60°C for 1 hour. The solvent was distilled off under reduced pressure, and water was added to the residue, which was extracted with ethyl acetate. The extract was washed with an aqueous saturated sodium hydrogencarbonate solution and an aqueous saturated sodium chloride solution, and dried over anhydrous magnesium sulfate. The solvent was distilled off under reduced pressure. To a solution of the resulting residue and 4-dimethylaminopyridine (3.60 g) in acetonitrile (120 mL) was added dropwise di-tert-butyl dicarbonate (6.43 g) at room temperature, and the mixture was stirred at room temperature for 1 hour. The solvent was distilled off under reduced pressure, and water was added to the residue, which was extracted with ethyl acetate. The extract was washed with an aqueous saturated sodium chloride solution and dried over anhydrous magnesium sulfate. The solvent was distilled off under reduced pressure, and the residue was purified with a silica gel column (ethyl acetate/hexane = 1/4) to obtain the title compound (11.78 g, 86%) as a pale light brown solid.
NMR (200 MHz, CDCl₃) δ: 1.71 (9H, s), 3.01 (2H, t, J = 8.0), 4.02 (2H, t, J = 7.8), 4.55-4.59 (2H, m), 5.20-5.35 (2H, m), 5.77-5.97 (1H, m), 7.49 (1H, dd, J = 8.8, 2.2), 7.72 (1H, d, J = 2.2), 7.87 (1H, d, J = 8.8).

### 215d) 3-[(1-Tert-butoxycarbonyl-3,5-dichloro-1H-indol-2-yl)sulfonyl]propionic acid

From tert-butyl 2-{[3-(allyloxy)-3-oxopropyl]sulfonyl}-3,5-dichloro-1H-indol-1-carboxylate (2.16 g) obtained in Example 215c), the title compound (4.01 g, 95%) was obtained as a pale light brown solid in a similar manner to Example 211d).
NMR (200 MHz, CDCl₃) δ: 1.71 (9H, s), 3.01 (2H, t, J = 7.2) , 3.99 (2H, t, J = 7.6), 7.50 (1H, dd, J = 8.8, 2.2), 7.72 (1H, d, J = 2.2), 7.87 (1H, d, J = 8.8).

### 215e) Tert-butyl 3,5-dichloro-2-({3-oxo-3-[4-(5,6,7,8-tetrahydroimidazo[1,2-a]pyridin-3-yl)-1-piperidinyl]propyl}sulfonyl)-1H-indole-1-carboxylate

From 3-[(1-tert-butoxycarbonyl-3,5-dichloro-1H-indol-2-yl)sulfonyl]propionic acid (0.42 g) obtained in Example 215d) and 3-(4-piperidinyl)-5,6,7,8-tetrahydroimidazo[1,2-a]pyridine dihydrochloride (0.33 g) obtained in Example 209b), the title compound (0.494 g, 81%) was obtained as colorless powder in a similar manner to Example 19.
NMR (200 MHz, CDCl₃) δ: 1.50-1.68 (2H, m), 1.71 (9H, s), 1.77-2.08 (6H, m), 2.54-2.81 (1H, m), 2.83 (2H, t, J = 6.2), 2.93-3.24 (3H, m), 3.82 (2H, t, J = 6.2), 3.88-4.12 (3H, m), 4.12-4.28 (1H, m), 4.53-4.68 (1H, m), 6.67 (1H, s), 7.49 (1H, dd, J = 8.8, 2.2), 7.72 (1H, d, J = 2.2), 7.90 (1H, d, J = 8.8).

### 215f) 3-(1-{3-[(3,5-Dichloro-1H-indol-2-yl)sulfonyl]propionyl}-4-piperidinyl)-5,6,7,8-tetrahydroimidazo[1,2-a]pyridine hydrochloride

From tert-butyl 3,5-dichloro-2-({3-oxo-3-[4-(5,6,7,8-tetrahydroimidazo[1,2-a]pyridin-3-yl)-1-piperidinyl]propyl}sulfonyl)-1H-indole-1-carboxylate (0.31 g) obtained in Example 215e), the title compound (0.17 g, 62%) was obtained as white powder in a similar manner to Example 207c).
NMR (200 MHz, DMSO-d₆) δ: 1.01-1.32 (1H, m), 1.32-1.61 (1H, m), 1.73-2.11 (6H, m), 2.52-2.68 (1H, m), 2.83 (2H, t, J = 6.8), 2.93-3.19 (3H, m), 3.23-3.38 (1H, m), 3.74 (2H, t, J = 7.0), 3.79-3.95 (1H, m), 4.06 (2H, t, J = 5.8), 4.18-4.32 (1H, m), 7.34 (1H, s), 7.43 (1H, dd, J = 8.8, 2.2), 7.58 (1H, d, J = 8.8), 7.70 (1H, d, J = 2.2), 7.38 (1H, br) .

### Example 216

### 3-(1-{3-[(3,5-Dichloro-1H-indol-2-yl)sulfonyl]propionyl}-4-piperidinyl)imidazo[1,2-a]pyridine hydrochloride

### 216a) Tert-butyl 3,5-dichloro-2-({3-oxo-3-[4-(imidazo[1,2-a]pyridin-3-yl)-1-piperidinyl]propyl}sulfonyl)-1H-indole-1-carboxylate

From 3-[(1-tert-butoxycarbonyl-5-chloro-1H-indol-2-yl)sulfonyl]propionic acid (0.42 g) obtained in Example 211d) and 3-(4-piperidinyl)imidazo[1,2-a]pyridine dihydrochloride (0.33 g) obtained in Example 207c), the title compound (0.50 g, 83%) was obtained as colorless powder in a similar manner to Example 19.
NMR (200 MHz, CDCl₃) δ: 1.56-1.86 (2H, m), 1.74 (9H, s), 1.99-2.28 (2H, m), 2.73-2.91 (1H, m), 2.91-3.21 (3H, m), 3.21-3.39 (1H, m), 3.95-4.36 (3H, m), 4.60-4.75 (1H, m), 6.82-6.89 (1H, m), 7.15-7.22 (1H, m), 7.40 (1H, s), 7.49 (1H, dd, J = 8.8, 2.2), 7.65 (1H, dd, J = 8.8, 2.2), 7.72 (1H, d, J = 2.2), 7.90 (1H, d, J = 8.4), 7.96 (1H, d, J = 7.0).

### 216b) 3-(1-{3-[(3,5-Dichloro-1H-indol-2-yl)sulfonyl]propionyl}-4-piperidinyl)imidazo[1,2-a]pyridine hydrochloride

From tert-butyl 3,5-dichloro-2-({3-oxo-3-[4-(imidazo[1,2-a]pyridin-3-yl)-1-piperidinyl]propyl}sulfonyl)-1H-indole-1-carboxylate obtained in Example 216a), the title compound (0.13 g, 48%) was obtained as white powder in a similar manner to Example 207c).
NMR (200 MHz, DMSO-d₆) δ: 1.24-1.74 (2H, m), 1.88-2.12 (2H, m), 2.56-2.78 (1H, m), 2.86 (2H, t, J = 7.0), 3.08-3.57 (2H, m), 2.66-2.81 (2H, m), 2.85-4.02 (1H, m), 4.25-4.40 (1H, m), 7.43 (1H, dd, J = 8.8, 2.2), 7.50-7.60 (2H, m), 7.69 (1H, d, J = 2.2), 7.95-8.04 (3H, m), 9.02 (1H, d, J = 6.8), 9.38 (1H, br) .

### Example 217

### 3-(1-{3-[(5-Chloro-1,3-dihydro-2H-isoindol-2-yl)sulfonyl]propionyl}-4-piperidinyl)-5,6,7,8-tetrahydroimidazo[1,2-a]pyridine hydrochloride

### 217a) 5-Chloro-2-[(4-methylphenyl)sulfonyl]isoindoline

A suspension of 1-chloro-3,4-dimethylbenzene (10.85 g), N-bromosuccinimide (30.22 g) and 2,2'-azobisisobutyronitrile (0.63 g) in carbon tetrachloride (100 mL) was refluxed for 3 hours. To the reaction solution was added additional 2,2'-azobisbutyronitrile (0.63 g), and the mixture was further refluxed for 3 hours. A precipitate was filtered off, and the filtrate was concentrated under reduced pressure.
Paratoluenesulfonamide (15.9 g) and potassium carbonate (32.0 g) were added to a solution of the residue in DMF (250 mL), and the mixture was stirred at room temperature for 24 hours. The precipitate formed was filtered and dissolved in ethyl acetate. The ethyl acetate solution was washed with water and an aqueous saturated sodium chloride solution, and dried over anhydrous magnesium sulfate. The solvent was distilled off under reduced pressure, and the residue was crystallized from ethyl acetate-diethyl ether to obtain the title compound (14.25 g, 60%) as white crystals.
NMR (200 MHz, CDCl₃) δ: 2.41 (3H, s), 4.58 (4H, s), 7.07-7.22 (3H, m), 7.32 (2H, d, J = 8.0), 7.76 (2H, d, J = 8.0).

### 217b) 5-Chloroindoline hydrobromide

5-Chloro-2-[(4-methylphenyl)sulfonyl]isoindoline (13.85 g) obtained in Example 217a) was added to a 25% solution of hydrogen bromide in acetic acid (135 mL), and the mixture was stirred at room temperature for 16 hours. The solvent was distilled off under reduced pressure, and ethyl acetate was added to the residue. Insoluble substances were filtered and washed with diethyl ether to obtain the title compound (10.1 g, 96%) as a white solid. NMR (200 MHz, DMSO-d₆) δ: 4.52 (4H, br), 7.38-7.49 (2H, m), 7.52 (1H, s), 9.57 (2H, br).

### 217c) Methyl 3-[(5-chloro-1,3-dihydro-2H-isoindol-2-yl)sulfonyl]propionate

From 5-chloroisoindoline hydrobromide (7.04 g) obtained in Example 217b) and methyl 3-chlorosulfonylpropionate (6.72 g) obtained in Example 213c), the title compound (7.93 g, 87%) was obtained as a pale yellow solid in a similar manner to Example 213d).
NMR (200 MHz, CDCl₃) δ: 2.86 (2H, t, J = 7.4), 3.40 (2H, t, J = 7.2), 3.61 (3H, s), 4.69 (4H, s), 7.13-7.32 (3H, m). 217d) 3-[(5-Chloro-1,3-dihydro-2H-isoindol-2-yl)sulfonyl]propionic acid

From methyl 3-[(5-chloro-1,3-dihydro-2H-isoindol-2-yl)sulfonyl]propionate obtained in Example 217c), the title compound (1.65 g, 25%) was obtained as pale purple powder in a similar manner to Example 213e).
NMR (200 MHz, DMSO-d₆) δ: 2.67 (2H, t, J = 7.0), 3.41 (2H, t, J = 7.4), 4.65 (4H, s), 7.06-7.52 (3H, m). 217e) 3-(1-{3-[(5-Chloro-1,3-dihydro-2H-isoindol-2-yl)sulfonyl]propionyl}-4-piperidinyl)-5,6,7,8-tetrahydroimidazo[1,2-a]pyridine hydrochloride

From 3-[(5-chloro-1,3-dihydro-2H-isoindol-2-yl)sulfonyl]propionic acid obtained in Example 217d) and 3-(4-piperidinyl)-5,6,7,8-tetrahydroimidazo[1,2-a]pyridine dihydrochloride obtained in Example 209b), the title compound (72%) was obtained as white crystals in a similar manner to Example 207d).
NMR (200 MHz, DMSO-d₆) δ: 1.14-1.63 (2H, m), 1.77-2.08 (6H, m), 2.54-2.73 (1H, m), 2.80 (2H, t, J = 7.0), 2.96 (2H, t, J = 5.4), 3.01-3.22 (1H, m), 3.32-3.48 (2H, m), 3.69-4.02 (2H, m), 4.07 (2H, t, J = 5.4), 4.29-4.48 (1H, m), 4.66 (4H, s), 7.36-7.47 (4H, m).

### Example 218

### 3-(1-{3-[(5-Chloro-1,3-dihydro-2H-isoindol-2-yl)sulfonyl]propionyl}-4-piperidinyl)imidazo[1,2-a]pyridine hydrochloride

From 3-[(5-chloro-1,3-dihydro-2H-isoindol-2-yl)sulfonyl]propionic acid obtained in Example 217d) and 3-(4-piperidinyl)imidazo[1,2-a]pyridine dihydrochloride obtained in Example 207c), the title compound (54%) was obtained as white powder in a similar manner to Example 207d).
NMR (200 MHz, DMSO-d₆) δ: 1.52-1.76 (2H, m), 1.95-2.16 (2H, m), 2.65-2.90 (3H, m), 3.13-3.34 (1H, m), 3.38-3.72 (3H, m), 3.92-4.09 (1H, m), 4.37-4.52 (1H, m), 4.67 (4H, s), 7.36-7.44 (3H, m), 7.51-7.59 (1H, m), 7.92-7.99 (2H, m), 8.07 (1H, s), 9.05 (1H, d, J = 7.0).

### Example 219

### N-{[5-chloro-2-({3-oxo-3-[4-(5,6,7,8-tetrahydroimidazo[1,2-a]pyridine-3-yl)-1-piperidinyl]propyl}sulfonyl)-1H-indol-3-yl]methyl}-N-methylacetamide hydrochloride

### 219a) Tert-butyl 2-[(3-tert-butoxy-3-oxopropyl)thio]-5-chloro-1H-indole-1-carboxylate

Di-tert-butyl dicarbonate (6.55 g) was added dropwise to a solution of tert-butyl 3-[(5-chloro-1H-indol-2-yl)thio]propionate (6.24 g) obtained in Example 211a) and 4-dimethtylaminopyridine (3.67 g) in acetonitrile (65 mL) at room temperature, and the mixture was stirred at room temperature for 30 minutes. The solvent was distilled off under reduced pressure, water was added to the residue, which was extracted with ethyl acetate. The extract was washed successively with 1N hydrochloric acid, a 1N aqueous sodium hydroxide solution and an aqueous saturated sodium chloride solution, and dried over anhydrous magnesium sulfate. The solvent was distilled off under reduced pressure to obtain the title compound (8.22 g, quantitative) as a white solid.
NMR (200 MHz, CDCl₃) δ: 1.48 (9H, s), 1.70 (9H, s), 2.69 (2H, t, J = 7.8), 3.18 (2H, t, J = 7.2), 6.29 (1H, s), 7.14 (1H, dd, J = 8.8, 2.2), 7.37 (1H, d, J = 2.2), 7.92 (1H, d, J = 8.8).

### 219b) Tert-butyl 2-[(3-tert-butoxy-3-oxopropyl)thio]-5-chloro-3-formyl-1H-indole-1-carboxylate

(Chloromethylene)dimethylammonium chloride (6.40 g) was added to a solution of tert-butyl 2-[(3-tert-butoxy-3-oxopropyl)thio]-5-chloro-1H-indole-1-carboxylate (8.22 g) obtained in Example 219a) in acetonitrile (100 mL) at room temperature, and the mixture was stirred at room temperature for 16 hours. The solvent was distilled off under reduced pressure, and an aqueous saturated sodium hydrogencarbonate solution was added to the residue, which was extracted with ethyl acetate. The extract was washed with an aqueous saturated sodium chloride solution and dried over anhydrous magnesium sulfate. The solvent was distilled off under reduced pressure to obtain the title compound (8.80 g, quantitative) as a pale yellow solid.
NMR (200 MHz, CDCl₃) δ: 1.41 (9H, s), 1.74 (9H, s), 2.52 (2H, t, J = 7.0), 3.23 (2H, t, J = 7.0), 7.36 (1H, dd, J = 8.8, 2.2), 7.94 (1H, d, J = 8.8), 8.38 (1H, d, J = 2.2), 10.41 (1H, s).

### 219c) Tert-butyl 3-{[acetyl(methyl)amino]methyl}-2-[(3-tert-butoxy-3-oxopropyl)thio]-5-chloro-1H-indole-1-carboxylate

A 40% solution of methylamine in methanol (21 mL) was added dropwise to a solution of tert-butyl 2-[(3-tert-butoxy-3-oxopropyl)thio]-5-chloro-3-formyl-1H-indole-1-carboxylate (8.80 g) obtained in Example 219b) in chloroform (50 mL)-acetic acid (50 mL) at 0°C, and the mixture was stirred at room temperature for 30 minutes. Sodium triacetoxyborohydride (8.48 g) was added to the reaction solution in portions at room temperature, and the mixture was stirred at room temperature for 3 hours. The solvent was distilled off under reduced pressure, and water was added to the residue, which was extracted with ethyl acetate. The extract was washed with an aqueous saturated sodium hydrogencarbonate solution and an aqueous saturated sodium chloride solution, and dried over anhydrous magnesium sulfate. The solvent was distilled off under reduced pressure. 4-Dimethylaminopyridine (3.67 g) and triethylamine (5.6 mL) were added to a solution of the residue in acetonitrile (100 mL), and acetic anhydride (2.8 mL) was then added dropwise at room temperature. The reaction solution was stirred at room temperature for 30 minutes, and the solvent was distilled off under reduced pressure. Water was added to the residue and it was then extracted with ethyl acetate. The extract was washed successively with 1N hydrochloric acid, a 1N aqueous sodium hydroxide solution and an aqueous saturated sodium chloride solution, and dried over anhydrous magnesium sulfate. The solvent was distilled off under reduced pressure to obtain the title compound (9.94 g, quantitative) as yellow powder. NMR (200 MHz, CDCl₃) δ: 1.42 (9H, s), 1.72 (9H, s), 2.14 (3H, s), 2.44 (2H, t, J = 7.0), 2.81 (3H, s), 3.08 (2H, t, J = 6.8), 4.96 (2H, s), 7.29 (1H, dd, J = 8.4, 2.2), 7.71 (1H, d, J = 2.2), 7.97 (1H, d, J = 8.4).

### 219d) Tert-butyl 3-{[acetyl(methyl)amino]methyl}-2-[(3-tert-butoxy-3-oxoisopropyl)sulfonyl]-5-chloro-1H-indole-1-carboxylate

From tert-butyl 3-{[acetyl(methyl)amino]methyl}-2-[(3-tert-butoxy-3-oxopropyl)thio]-5-chloro-1H-indole-1-carboxylate (9.94 g) obtained in Example 219c), the title compound (10.6 g, quantitative) was obtained as a brown oil in a similar manner to Example 211b).
NMR (200 MHz, CDCl₃) δ: 1.42 (9H, s), 1.73 (9H, s), 2.19 (3H, s), 2.79 (2H, t, J = 7.2), 2.90 (3H, s), 4.00 (2H, t, J = 7.2), 5.23 (2H, s), 7.38-7.48 (2H, m), 7.80-7.84 (1H, m).

### 219e) Tert-butyl 3-{[acetyl(methyl)amino]methyl}-2-{[3-(allyloxy)-3-oxopropyl]sulfonyl}-5-chloro-1H-indole-1-carboxylate

From tert-butyl 3-{[acetyl(methyl)amino]methyl}-2-[(3-tert-butoxy-3-oxoisopropyl)sulfonyl]-5-chloro-1H-indole-1-carboxylate (10.6 g) obtained in Example 219d), the title compound (2.98 g, 29%) was obtained as a brown oil in a similar manner to Example 215c).
NMR (200 MHz, CDCl₃) δ: 1.71 (9H, s), 2.16 (3H, s), 2.90 (3H, s), 2.95 (2H, t, J = 7.8), 4.07 (2H, t, J = 7.8), 4.57 (2H, d, J = 5.8), 5.20 (2H, s), 5.26-5.36 (2H, m), 5.77-5.98 (1H, m), 7.41-7.47 (1H, m), 7.79-7.86 (2H, m).

### 219f) 3-{[3-{[Acetyl(methyl)amino]methyl}-1-(tert-butoxycarbonyl)-5-chloro-1H-indol-2-yl]sulfonyl}propionic acid

From tert-butyl 3-{[acetyl(methyl)amino]methyl}-2-{[3-(allyloxy)-3-oxopropyl]sulfonyl}-5-chloro-1H-indole-1-carboxylate (2.98 g) obtained in Example 219e), the title compound (1.73 g, 63%) was obtained as pale brown powder in a similar manner to Example 211d).
NMR (200 MHz, DMSO-d₆) δ: 1.67 (9H, s), 2.08 (3H, s), 2.76 (2H, t, J = 7.2), 2.85 (3H, s), 4.03 (2H, t, J = 7.0), 5.03 (2H, s), 7.60 (1H, dd, J = 8.8, 2.2), 7.77 (1H, d, J = 2.2), 7.90 (1H, d, J = 8.8).

### 219g) N-{[5-chloro-2-({3-oxo-3-[4-(5,6,7,8-tetrahydroimidazo[1,2-a]pyridin-3-yl)-1-piperidinyl]propyl}sulfonyl)-1H-indol-3-yl]methyl}-N-methylacetamide hydrochloride

From 3-{[3-{[acetyl(methyl)amino]methyl}-1-(tert-butoxycarbonyl)-5-chloro-1H-indol-2-yl]sulfonyl}propionic acid obtained in Example 219f) and 3-(4-piperidinyl)-5,6,7,8-tetrahydroimidazo[1,2-a]pyridine dihydrochloride obtained in Example 209b), the title compound (yield 30%) was obtained as colorless powder in a similar manner to Example 207b).
NMR (200 MHz, DMSO-d₆) δ: 1.15-1.56 (2H, m), 1.75-2.09 (6H, m), 2.09 (3H, s), 2.48-2.69 (2H, m), 2.77 (2H, t, J = 7.2), 2.83-3.18 (3H, m), 2.86 (3H, s), 3.68 (2H, t, J = 6.6), 3.82-3.98 (1H, m), 3.99-4.13 (2H, m), 4.25-4.40 (1H, m), 5.22 (2H, s), 7.32-7.38 (2H, m), 7.54 (1H, d, J = 8.8), 7.77 (1H, br), 7.80 (1H, d, J = 1.8).

### Example 220

### 2-{[5-Chloro-2-({3-oxo-3-[4-(5,6,7,8-tetrahydroimidazo[1,2-a]pyridin-3-yl)-1-piperidinyl]propyl}sulfonyl)-1H-indol-1-yl]-N,N-dimethylacetamide hydrochloride

### 220a) Tert-butyl 3-{5-chloro-1-[(dimethylcarbamoyl)methyl]-1H-indol-2-yl}thio]propionate

Sodium hydride (60% in oil: 0.96 g) was added to a solution of tert-butyl 3-[(5-chloro-1H-indolyl-2-yl)thio]propionate (6.24 g) obtained in Example 211a) in THF (65 mL) at 0°C, and the mixture was stirred at 0°C for 30 minutes under argon atmosphere. Then 2-chloro-N-N-dimethylacetamide (4.86 g) was added and the mixture was stirred at room temperature for 3 hours. Ice-water was added to the reaction solution and it was then extracted with ethyl acetate. The extract was washed with water and an aqueous saturated sodium chloride solution, and dried over anhydrous magnesium sulfate. The solvent was distilled off under reduced pressure, and the residue was purified with a silica gel column (ethyl acetate/hexane = 1/1) to obtain the title compound (2.89 g, 36%) as white solid.
NMR (200 MHz, CDCl₃) δ: 1.44 (9H, s), 2.49 (2H, t, J = 6.6), 2.89 (2H, t, J = 6.6), 2.95 (3H, s), 3.09 (3H, s), 5.00 (2H, s), 6.71 (1H, s), 7.00-7.14 (2H, m), 7.52 (1H, d, J = 1.8).

### 220b) Tert-butyl 3-{5-chloro-1-[(dimethylcarbamoyl)methyl]-1H-indol-2-yl}sulfonyl]propionate

From tert-butyl 3-{5-chloro-1-[(dimethylcarbamoyl)methyl]-1H-indol-2-yl}thio]propionate (3.82 g) obtained in Example 220a), the title compound (3.26 g, 79%) was obtained as a white solid in a similar manner to Example 211b).
NMR (200 MHz, CDCl₃) δ: 1.41 (9H, s), 2.65 (2H, t, J = 8.0), 2.98 (3H, s), 3.16 (3H, s), 3.52 (2H t, J = 7.4), 5.28 (2H, s), 7.14 (1H, d, J = 9.2), 7.23 (1H, s), 7.30 (1H, dd, J = 9.2, 2.2), 7.66 (1H, d, J = 2.2).

### 220c) 3-{5-Chloro-1-[(dimethylcarbamoyl)methyl]-1H-indol-2-yl}sulfonyl]propionic acid

2N hydrochloric acid (6.24 mL) was added to a solution of tert-butyl 3-{5-chloro-1-[(dimethylcarbamoyl)methyl]-1H-indol-2-yl}sulfonyl]propionate (3.26 g) obtained in Example 220b) in acetic acid (30 mL), and the mixture was stirred at 60°C for 1 hour. The solvent was distilled off under reduced pressure and the residue was washed with ether to obtain the title compound (2.46 g, 87%) as a white solid. NMR (200 MHz, DMSO-d₆) δ: 2.58 (2H, t, J = 4.8), 2.85 (3H, s), 3.14 (3H, s), 3.57 (2H, t, J = 4.8), 5.43 (2H, s), 7.28 (1H, s), 7.40 (1H, dd, J = 6.0, 1.4), 7.64 (1H, d, J = 6.0), 7.85 (1H, d, J = 1.4).

### 220d) 2-{[5-Chloro-2-({3-oxo-3-[4-(5,6,7,8-tetrahydroimidazo[1,2-a]pyridin-3-yl)-1-piperidinyl]propyl}sulfonyl)-1H-indol-1-yl]-N,N-dimethylacetamide hydrochloride

From 3-{5-chloro-1-[(dimethylcarbamoyl)methyl]-1H-indol-2-yl}sulfonyl]propionic acid obtained in Example 220c) and 3-(4-piperidinyl)-5,6,7,8-tetrahydroimidazo[1,2-a]pyridine dihydrochloride obtained in Example 209b), the title compound (42%) was obtained as white powder in a similar manner to Example 207d).
NMR (200 MHz, DMSO-d₆) δ: 1.18-1.55 (2H, m), 1.76-2.05 (6H, m), 2.46-2.70 (2H, m), 2.77 (2H, t, J = 7.2), 2.82-3.15 (3H, m), 2.83 (3H, s), 3.16 (3H, s), 3.68 (2H, t, J = 6.6), 3.83-3.99 (1H, m), 3.96-4.11 (2H, m), 4.25-4.40 (1H, m), 5.42 (2H, s), 7.18 (1H, d, J = 1.6), 7.32-7.38 (2H, m), 7.54 (1H, d, J = 8.8), 7.81 (1H, d, J = 1.8).

### Example 221

### 3-(1-{3-[(5-Chloro-3-methyl-1H-indol-2-yl)sulfonyl]propionyl}-4-piperidinyl)-5,6,7,8-tetrahydroimidazo[1,2-a]pyridine hydrochloride

### 221a) 5-Chloro-3 methyl-1,3-dihydro-2H-indol-2-one

Iron powder (10.5 g) was added to a solution of methyl 2-methyl-2-(2-nitro-5-chlorophenyl)acetate (11.4 g) in acetic acid (120 mL), and the mixture was stirred at 100°C for 1 hour. The solvent was distilled off under reduced pressure and water was added to the residue, which was extracted with ethyl acetate. The extract was washed with an aqueous saturated sodium chloride solution and dried over anhydrous magnesium sulfate. The solvent was distilled off under reduced pressure, and the residue was washed with hexane and diisopropyl ether to obtain the title compound (7.12 g, 84%) as a pale brown solid.
NMR (200 MHz, DMSO-d₆) δ: 1.33 (3H, d, J = 7.8), 3.44 (1H, dd, J = 7.8), 6.82 (1H, d, J = 8.4), 7.20 (1H, d, J = 8.4), 7.33 (1H, s), 10.44 (1H, br).

### 221b) Tert-butyl 3-[(5-chloro-3-methyl-1H-indol-2-yl)thio]propionate

From 5-chloro-3-methyl-1,3-dihydro-2H-indol-2-one (7.12 g) obtained in Example 221a), the title compound (5.05 g, 40%) was obtained as a pale brown solid in a similar manner to Example 211a).
NMR (300 MHz, CDCl₃) δ: 1.47 (9H, s), 2.47 (2H, t, J = 7.2), 2.95 (2H, t, J = 7.0), 7.13-7.17 (1H, m), 7.21-7.26 (1H, m), 7.48-7.49 (1H, m), 8.45 (1H, br).

### 221c) Tert-butyl 3-[(5-chloro-3-methyl-1H-indol-2-yl)sulfonyl]propionate

From tert-butyl 3-[(5-chloro-3-methyl-1H-indol-2-yl)thio]propionate (50.5 g) obtained in Example 221b), the title compound (4.82 g, 87%) was obtained as a pale brown solid in a similar manner to Example 211b).
NMR (200 MHz, CDCl₃) δ: 1.36 (9H, s), 2.56 (3H, s), 2.69 (2H, t, J = 7.4), 3.52 (2H, t, J = 7.4), 7.29-7.35 (2H, m), 7.63 (1H, s), 9.09 (1H, br).

### 221d) Tert-butyl 2-[(3-allyloxy-3-oxopropyl)sulfonyl]-5-chloro-3-methyl-1H-indole-1-carboxylate

From tert-butyl 3-[(5-chloro-3-methyl-1H-indol-2-yl)sulfonyl]propionate (4.82 g) obtained in Example 221c), the title compound (2.62 g, 44%) was obtained as a pale brown liquid in a similar manner to Example 211c).
NMR (200 MHz, CDCl₃) δ: 1.71 (9H, s), 2.59 (3H, s), 2.94 (2H, t, J = 7.2), 4.04 (2H, t, J = 7.4), 4.53-4.57 (2H, m), 5.20-5.34 (2H, m), 5.77-5.97 (1H, m), 7.43 (1H, dd, J = 8.8, 2.2), 7.62 (1H, d, J = 2.2), 7.85 (1H, d, J = 8.8).

### 221e) 3-{[1-(Tert-butoxycarbonyl)-5-chloro-3-methyl-1H-indol-2-yl]sulfonyl}proponic acid

From tert-butyl 2-[(3-allyloxy-3-oxopropyl)sulfonyl]-5-chloro-3-methyl-1H-indole-1-carboxylate (2.62 g) obtained in Example 221d), the title compound (1.50 g, 63%) was obtained as a plate light brown solid in a similar manner to Example 211d).
NMR (200 MHz, DMSO-d₆) δ: 1.62 (9H, s), 2.51 (3H, s), 2.73 (2H, t, J = 7.2), 3.91 (2H, t, J = 7.2), 7.56 (1H, dd, J = 8.8, 2.2), 7.86 (1H, d, J = 8.8), 7.92 (1H, d, J = 2.2).

### 221f) Tert-butyl 5-chloro-3-methyl-2-({3-oxo-3-[4-(5,6,7,8-tetrahydroimidazo[1,2-a]pyridin-3-yl)-1-piperidinyl]propyl}sulfonyl)-1H-indole-1-carboxylate

From 3-{[1-(tert-butoxycarbonyl)-5-chloro-3-methyl-1H-indol-2-yl]sulfonyl}proponic acid (0.80 g) obtained in Example 221e) and 3-(4-piperidinyl)-5,6,7,8-tetrahydroimidazo[1,2-a]pyridine dihydrochloride (0.33 g) obtained in Example 209b), the title compound (0.84 g, 71%) was obtained as colorless powder in a similar manner to Example 19.
NMR (200 MHz, CDCl₃) δ: 1.39-1.68 (2H, m), 1.69 (9H, s), 1.80-2.07 (6H, m), 2.54-2.79 (2H, m), 2.58 (3H, s), 2.86 (2H, t, J = 6.2), 2.93-3.07 (2H, m), 3.09-3.24 (1H, m), 3.81 (2H, t, J = 6.0), 3.87-4.28 (3H, m), 4.56-4.72 (1H, m), 6.67 (1H, s), 7.42 (1H, dd, J = 8.8, 2.2), 7.62 (1H, d, J = 2.2), 7.85 (1H, d, J = 8.8).

### 221g) 3-(1-{3-[(5-Chloro-3-methyl-1H-indol-2-yl)sulfonyl]propionyl}-4-piperidinyl)-5,6,7,8-tetrahydroimidazo[1,2-a]pyridine hydrochloride

From tert-butyl 5-chloro-3-methyl-2-({3-oxo-3-[4-(5,6,7,8-tetrahydroimidazo[1,2-a]pyridin-3-yl)-1-piperidinyl]propyl}sulfonyl)-1H-indole-1-carboxylate (0.30 g) obtained in Example 221f), the title compound (0.16 g, 61%) was obtained as white powder in a similar manner to Example 207c).
NMR (200 MHz, DMSO-d₆) δ: 1.08-1.58 (2H, m), 1.71-2.10 (6H, m), 2.49 (3H, m), 2.53-2.66 (1H, m), 2.75 (2H, t, J = 7.2), 2.83-3.04 (3H, m), 3.04-3.19 (1H, m), 3.61 (2H, t, J = 7.2), 3.77-3.92 (1H, m), 4.05 (2H, t, J = 5.2), 4.23-4.38 (1H, m), 7.29-7.35 (2H, m), 7.47 (1H, d, J = 8.8), 7.79 (1H, d, J = 2.2), 9.31 (1H, br).

### Example 222

### 3-(1-{3-[(5-Chloro-3-methyl-1H-indol-2-yl)sulfonyl]propionyl}-4-piperidinyl)imidazo[1,2-a]pyridine hydrochloride

### 222a) Tert-butyl 5-chloro-2-{[3-(4-imidazo[1,2-a]pyridin-3-yl)-1-piperidinyl]-3-oxopropionyl}sulfonyl)-3-methyl-1H-indole-1-carboxylate

From 3-{[1-(tert-butoxycarbonyl)-5-chloro-3-methyl-1H-indol-2-yl]sulfonyl}propionic acid (0.80 g) obtained in Example 221e) and 3-(4-piperidinyl)imidazo[1,2-a]pyridine dihydrochloride (0.66 g) obtained in Example 207c), the title compound (0.81 g, 78%) was obtained as colorless powder in a similar manner to Example 19).
NMR (200 MHz, CDCl₃) δ: 1.70 (9H, s), 1.77-1.88 (2H, m), 2.08-2.27 (2H, m), 2.60 (3H, s), 2.74-2.96 (1H, m), 2.96-3.21 (3H, m), 3.21-3.40 (1H, m), 3.96-4.35 (3H, m), 4.62-4.81 (1H, m), 6.81-6.88 (1H, m), 7.13-7.22 (1H, m), 7.41-7.46 (2H, m), 7.61-7.66 (2H, m), 7.86 (1H, d, J = 8.8), 7.96 (1H, d, J = 7.0).

### 222b) 3-(1-{3-[(5-Chloro-3-methyl-1H-indol-2-yl)sulfonyl]propionyl}-4-piperidinyl)imidazo[1,2-a]pyridine hydrochloride

From tert-butyl 5-chloro-2-{[3-(4-imidazo[1,2-a]pyridin-3-yl)-1-piperidinyl]-3-oxopropionyl}sulfonyl)-3-methyl-1H-indole-1-carboxylate (0.29 g) obtained in Example 222a), the title compound (0.20 g, 76%) was obtained as white powder in a similar manner to Example 207c).
NMR (200 MHz, DMSO-d₆) δ: 1.24-1.71 (2H, m), 1.88-2.28 (2H, m), 2.50 (3H, s), 2.57-2.71 (1H, m), 2.79 (2H, t, J = 7.0), 3.07-3.55 (2H, m), 3.63 (2H, t, J = 7.2), 3.84-3.99 (1H, m), 4.28-4.45 (1H, m), 7.31 (1H, dd, J = 8.8, 2.2), 7.45-7.57 (2H, m), 7.79 (1H, d, J = 2.2), 7.91-8.02 (3H, m), 9.02 (1H, d, J = 7.0), 9.37 (1H, br).

### Example 223

### 3-(1-{3-[(5-Chloro-1-methyl-1H-indol-2-yl)sulfonyl]propionyl}-4-piperidinyl)-5,6,7,8-tetrahydroimidazo[1,2-a]pyridine hydrochloride

### 223a) Tert-butyl 3-[(5-chloro-1-methyl-1H-indol-2-yl)thio]propionate

To a solution of tert-butyl 3-[(5-chloro-1H-indol-2-yl)thio]propionate obtained in Example 211a) in DMF (40 mL) were added methyl iodide (5.26 mL) was added at room temperature and then sodium hydride (60% in oil: 0.81 g) at 0°C, and the reaction mixture was stirred at room temperature for 16 hours. Ice-water was added to the reaction solution and it was then extracted with ethyl acetate. The extract was washed with water and an aqueous saturated sodium chloride solution and dried over anhydrous magnesium sulfate. The solvent was distilled off under reduced pressure, and the residue wad purified with a silica gel column (ethyl acetate/hexane = 1/4) to obtain the title compound (4.94 g, 82%) as a colorless solid.
NMR (200 MHz, CDCl₃) δ: 1.43 (9H, s), 2.51 (2H, t, J = 7.2), 2.96 (2H, t, J = 7.2), 3.79 (3H, s), 6.63 (1H, s), 7.06-7.24 (2H, m), 7.52 (1H, s).

### 223b) Tert-butyl 3-[(5-chloro-1-methyl-1H-indol-2-yl)sulfonyl]propionate

From tert-butyl 3-[(5-chloro-1-methyl-1H-indol-2-yl)thio]propionate (4.90 g) obtained in Example 223a), the title compound (1.87 g, 38%) was obtained as a white solid in a similar manner to Example 211b).
NMR (200 MHz, CDCl₃) δ: 1.26 (9H, s), 2.71 (2H, t, J = 7.2), 3.51 (2H, t, J = 7.2), 4.03 (3H, s), 7.21 (1H, s), 7.29-7.43 (2H, m), 7.67 (1H, s).

### 223c) 3-[(5-Chloro-1-methyl-1H-indol-2-yl)sulfonyl]propionic acid

From tert-butyl 3-[(5-chloro-1-methyl-1H-indol-2-yl)sulfonyl]propionate (1.87 g) obtained in Example 223b), the title compound(1.24 g, 79%) was obtained as a pale light brown solid in a similar manner to Example 220c).
NMR (200 MHz, DMSO-d₆) δ: 2.60 (2H, t, J = 7.0), 3.68 (2H, t, J = 7.0), 3.99 (3H, s), 7.23 (1H, s), 7.43 (1H, dd, J = 8.8, 2.2), 7.71 (1H, d, J = 8.8), 7.84 (1H, d, J = 2.2).

### 223d) 3-(1-{3-[(5-Chloro-1-methyl-1H-indol-2-yl)sulfonyl]propionyl}-4-piperidinyl)-5,6,7,8-tetrahydroimidazo[1,2-a]pyridine hydrochloride

From 3-[(5-chloro-1-methyl-1H-indol-2-yl)sulfonyl]propionic acid obtained in Example 223c) and 3-(4-piperidinyl)-5,6,7,8-tetrahydroimidazo[1,2-a]pyridine dihydrochloride obtained in Example 209b), the title compound (yield 76%) was obtained as white powder in a similar manner to Example 207d).
NMR (200 MHz, DMSO-d₆) δ: 1.27-1.37 (1H, m), 1.64-1.78 (1H, m), 1.78-2.07 (6H, m), 2.34-2.54 (1H, m), 2.78 (2H, t, J = 6.6), 2.87-3.12 (2H, m), 3.34-3.58 (2H, m), 3.61-3.94 (3H, m), 3.94-4.19 (3H, m), 4.00 (3H, s), 7.25 (1H, s), 7.36-7.43 (2H, m), 7.70 (1H, d, J = 8.8), 7.79 (1H, d, J = 2.2).

### Example 224

### 2-(1-{3-[(6-Chloro-2-naphthyl)sulfonyl]propionyl}-4-piperidinyl)-1,2,3,4-tetrahydrodipyrido[1,2-a;4',3'-d]imidazole dihydrochloride

### 224a) 2-(3-Oxobutyl)-1H-isoindol-1,3(2H)-dione

A solution of phthalimide (90.0 g), methyl vinyl ketone (51 mL) and 40% Triton B in methanol (15.3 mL) was added to ethyl acetate (400 mL), and the mixture was refluxed for 1 hour. The solvent was distilled off under reduced pressure, and the residue was recrystallized from ethanol to obtain the title compound (114.4 g, 86%) as colorless crystals.
NMR (200 MHz, CDCl₃) δ: 2.19 (3H, s), 2.88 (2H, t, J = 7.2), 3.96 (2H, t, J = 7.2), 7.70-7.74 (2H, m), 7.82-7.86 (2H, m).

### 224b) 2-(4-Bromo-3-oxobutyl)-1H-isoindol-1,3(2H)-dione

Bromine (40.0 g) was added dropwise to a suspension of 2-(3-oxobutyl)-1H-isoindol-1,3(2H)-dione (54.31 g) obtained in Example 224a) in methanol (300 mL) at 0°C, and the mixture was stirred at room temperature for 16 hours. Dichloromethane was added to the reaction solution to dissolve precipitates and 10 N sulfuric acid (50 mL) was then added. The mixture was stirred at room temperature for 16 hours. The reaction solution was concentrated under reduced pressure, and the residue was washed with hot methanol to obtain the title compound (36.1 g, 49%) as colorless crystals.
NMR (200 MHz, CDCl₃) δ: 3.12 (2H, t, J = 7.2), 3.92 (2H, s), 4.01 (2H, t, J = 7.2), 7.70-7.75 (2H, m), 7.83-7.87 (2H, m).

### 224c) 2-[2-(Imidazo[1,2-a]pyridin-2-yl)ethyl]-1H-isoindol-1,3(2H)-dione

A mixture of 2-(4-bromo-3-oxobutyl)-1H-isoindol-1,3(2H)-dione (29.61 g) obtained in Example 224b), 2-aminopyridine (9.41 g) and sodium hydrogencarbonate (8.40 g) in DMF (100 mL) was stirred at 100°C for 1 hour. Ice-water was added to the mixture, and the precipitate formed was filtered and washed with water to obtain the title compound (21.85 g, 75%) as brown powder.
NMR (200 MHz, CDCl₃) δ: 3.21 (2H, t, J = 7.2), 4.12 (2H, t, J = 7.2), 6.69-6.76 (1H, m), 7.08-7.16 (1H, m), 7.45 (1H, s), 7.51 (1H, d, J = 8.8), 7.68-7.74 (2H, m), 7.79-7.86 (2H, m), 8.04 (1H, d, J = 7.2).

### 224d) 2-(Imidazo[1,2-a]pyridin-2-yl)ethylamine dihydrochloride

A solution of 2-[2-(imidazo[1,2-a]pyridin-2-yl)ethyl]-1H-isoindol-1,3(2H)-dione (29.13 g) obtained in Example 224c) in 6N hydrochloric acid (500 mL) was refluxed for 3 hours. The reaction solution was cooled to room temperature, and the precipitated crystals were filtered and washed with ethanol to obtain the title compound (16.86 g, 72%) as pale brown crystals.
NMR (200 MHz, D₂O) δ: 3.28-3.43 (2H, m), 3.43-3.58 (2H, m), 7.42 (1H, t, J = 6.6), 7.78-7.98 (2H, m), 8.03 (1H, s), 8.62 (1H, d, J = 6.6).

### 224e) Tert-butyl 4-(1,2,3,4-tetrahydrodipyrido[1,2-a;4',3'-d]imidazol-2-yl)piperidine-1-carboxylate

Tert-butyl 4-oxopiperidine-1-carboxylate (1.99 g) was added to a solution of 2-(imidazo[1,2-a]pyridin-2-yl)ethylamine dihydrochloride (2.34 g) obtained in Example 224d) and DBU (3.0 mL) in ethanol (25 mL), and the mixture was refluxed for 2 hours. The reaction solution was cooled to room temperature and sodium triacetoxyborohydride (4.24 g) was added. The mixture was stirred at room temperature for 1 hour. Insoluble substances were filtered off and the filtrate was concentrated under reduced pressure. Under ice-cooling, 8N sodium hydroxide (20 mL) was added to the residue and it was then extracted with chloroform. The extract was washed with an aqueous saturated sodium chloride solution and dried over anhydrous magnesium sulfate. The solvent was distilled off under reduced pressure. A 37% aqueous formaldehyde solution (15 mL) was added to a solution of the resulting residue in acetic acid (50 mL), and the mixture was stirred at 100°C for 20 minutes. The solvent was distilled off under reduced pressure. Under ice-cooling, a 8N aqueous sodium hydroxide solution (20 mL) was added to the residue and it was then extracted with chloroform. The extract was washed with an aqueous saturated sodium chloride solution and dried over anhydrous magnesium sulfate. The solvent was distilled off under reduced pressure, and the residue wad purified with a silica gel column (ethyl acetate/ethanol/triethylamine = 20/1/0.5 to 10/1/0.5) to obtain the title compound (2.14 g, 60%) as colorless powder.
NMR (200 MHz, CDCl₃) δ: 1.48 (9H, s), 1.52-1.73 (2H, m), 1.84-2.00 (2H, m), 2.64-2.88 (3H, m), 2.88-3.07 (4H, m), 3.88 (2H, s), 4.12-4.31 (2H, m), 6.78 (1H, dt, J = 7.0, 1.0), 7.12 (1H, dt, J = 8.0, 1.0), 7.54 (1H, d, J = 8.0), 7.73 (1H, d, J = 7.0).

### 224f) 2-(4-Piperidinyl)-1,2,3,4-tetrahydrodipyrido[1,2-a;4',3'-d]imidazole trihydrochloride

From tert-butyl 4-(1,2,3,4-tetrahydrodipyrido[1,2-a;4',3'-d]imidazol-2-yl)piperidine-1-carboxylate (1.78 g) obtained in Example 224e), the title compound (1.66 g, 91%) was obtained as white crystals in a similar manner to Example 207c).
NMR (200 MHz, D₂O) δ: 1.92-2.19 (2H, m), 2.38-2.55 (2H, m), 3.08-3.32 (4H, m), 3.48-3.78 (5H, m), 4.53 (2H, s), 7.50 (1H, t, J = 6.6), 7.85-8.03 (2H, m), 8.46 (1H, d, J = 6.8).

### 224 g) 2-(1-{3-[(6-Chloro-2-naphthyl)sulfonyl]propionyl}-4-piperidinyl)-1,2,3,4-tetrahydodipyrido[1,2-a;4',3'-d]imidazole dihydrochloride

From 2-(4-piperidinyl)-1,2,3,4-tetrahydrodipyrido[1,2-a;4',3'-d]imidazole trihydrochloride obtained in Example 224f) and 3-[(6-chloro-2-naphthyl)sulfonyl]propionic acid, the title compound (75%) was obtained as white powder in a similar manner to Example 207d).
NMR (200 MHz, DMSO-d₆) δ: 1.44-1.92 (2H, m), 2.11-2.33 (2H, m), 2.41-2.62 (1H, m), 2.79 (2H, t, J = 7.4), 2.93-3.12 (2H, m), 3.12-3.94 (5H, m), 3.94-4.12 (2H, m), 4.32-4.50 (1H, m), 4.75 (2H, br), 7.58 (1H, dt, J = 7.0, 1.8), 7.74 (1H, d, J = 8.8, 2.2), 7.19-8.03 (3H, m), 8.19-8.32 (3H, m), 8.66 (1H, s), 8.78 (1H, d, J = 6.8).

### Example 225

### 3-(1-{3-[(5-Chloro-1H-indol-2-yl)sulfonyl]propionyl}-4-piperidinyl)-5,6,7,8-tetrahydroimidazo[1,5-a]pyridine hydrochloride

### 225a) N-(2-Pyridyl)methyl-4-pyridinecarboxamide

To a suspension of isonicotinoyl chloride hydrochloride (20.0 g) in dichloromethane (200 mL) were added dropwise at 0°C triethylamine (94 mL) and then 2-aminomethylpyridine (11.6 mL). After the reaction mixture was stirred at room temperature for 30 minutes, insoluble substances were filtered and the filtrate was concentrated under reduced pressure. Ethyl acetate was added to the residue and it was extracted with 6N hydrochloric acid (200 mL). Under ice-cooling, an aqueous layer was adjusted to pH 11 by addition of a 8N aqueous sodium hydroxide solution and then extracted with chloroform. The extract was washed with an aqueous saturated sodium chloride solution and dried over anhydrous magnesium sulfate. The solvent was distilled off under reduced pressure, and the residue was purified with a silica gel column (chloroform/methanol = 10: 1) to obtain the title compound (19.00 g, 79%) as a pale brown solid.
NMR (200 MHz, CDCl₃) δ: 4.75 (2H, d, J = 4.8), 7.21-7.25 (1H, m), 7.32 (1H, d, J = 8.2), 7.66-7.75 (3H, m), 8.12 (1H, br), 8.55 (1H, d, J = 5.2), 8.73 (2H, m).

### 225b) 3-(4-Pyridyl)imidazo[1,5-a]pyridine

Phosphorus oxychloride (24.9 mL) was added dropwise to a solution of N-(2-pyridylmethyl)-4-pyridinecarboxamide (19.00 g) obtained in Example 225a) in toluene (60 mL) at 0°C, and the mixture was refluxed for 3 hours. The solvent was distilled off under reduced pressure, and the residue was pored into ice-water and then stirred for 2 hours. Under ice-cooling, the reaction solution was adjusted to pH 11 by addition of a 8N aqueous sodium hydroxide solution, and then extracted with chloroform. The extract was washed with an aqueous saturated sodium chloride solution and dried over anhydrous magnesium sulfate. The solvent wad distilled off under reduced pressure, and the residue was purified with a silica gel column (ethyl acetate/ethanol = 10: 1) to obtain the title compound (14.01 g, 81%) as a pale brown solid.
NMR (200 MHz, CDCl₃) δ: 6.65-6.72 (1H, m), 6.78-6.86 (1H, m), 7.50-7.56 (1H, m), 7.61 (1H, s), 7.74 (2H, dd, J = 4.8, 2.0), 8.36 (1H, dd, J = 7.4, 1.2), 8.72 (2H, dd, J = 4.8, 2.0).

### 225c) 3-(4-Piperidinyl)-5,6,7,8-tetrahydroimidazo[1,5-a]pyridine dihydrochloride

A solution of 3-(4-pyridyl)imidazo[1,5-a]pyridine (5.13 g) obtained in Example 225b) in acetic acid (50 mL) was hydrogenated at 60°C under 100 atm hydrogen atmosphere after addition of rhodium carbon (500 mg). The catalyst was filtered off, and concentrated hydrochloric acid (4.3 mL) was added to the filtrate, which was concentrated under reduced pressure. The residue was washed with 2-propanol and diethyl ether to obtain the title compound (5.00 g, 68%) as white powder.
NMR (200 MHz, D₂O δ: 1.78-1.97 (2H, m), 1.97-2.17 (4H, m), 2.23-2.38 (2H, m), 2.84 (2H, t, J = 6.2), 3.12-3.34 (2H, m), 3.43-3.57 (1H, m), 3.57-3.71 (2H, m), 4.16 (2H, t, J = 6.2), 7.11 (1H, s).

### 225d) Tert-butyl 5-chloro-2-({3-oxo-3-[4-(5,6,7,8-tetrahydroimidazo[1,5-a]pyridin-3-yl)-1-piperidinyl]propyl}sulfonyl)-1H-indole-1-carboxylate

From 3-(4-piperidinyl)-5,6,7,8-tetrahydroimidazo[1,5-a]pyridine dihydrochloride (0.67 g) obtained in Example 225c) and 3-[(1-tert-butoxycarbonyl-5-chloro-1H-indol-2-yl)sulfonyl]propionic acid (0.78 g) obtained in Example 211d), the title compound (0.87 g, 76%) was obtained as brown powder in a similar manner to Example 19.
NMR (200 MHz, CDCl₃) δ: 1.55-2.06 (8H, m), 1.74 (9H, s), 2.67-2.98 (6H, m), 3.08-3.24 (1H, m), 3.85 (2H, t, J = 5.8), 3.90-4.19 (3H, m), 4.43-4.58 (1H, m), 6.65 (1H, s), 7.44 (1H, dd, J = 8.8, 2.2), 7.51 (1H, s), 7.64 (1H, d, J = 2.2), 8.02 (1H, d, J = 8.8).

### 225e) 3-(1-{3-[(5-Chloro-1H-indol-2-yl)sulfonyl]propionyl}-4-piperidinyl)-5,6,7,8-tetrahydroimidazo[1,5-a]pyridine hydrochloride

From tert-butyl 5-chloro-2-({3-oxo-3-[4-(5,6,7,8-tetrahydroimidazo[1,5-a]pyridin-3-yl)-1 piperidinyl]propyl}sulfonyl)-1H-indole-1-carboxylate (0.40 g) obtained in Example 225d), the title compound (0.28 g, 78%) was obtained as white crystals in a similar manner to Example 207c).
NMR (200 MHz, DMSO-d₆) δ: 1.42-1.68 (2H, m), 1.68-2.03 (6H, m), 2.48-2.68 (1H, m), 2.71-2.87 (3H, m), 2.99-3.23 (1H, m), 3.27-3.51 (2H, m), 3.67 (2H, t, J = 6.8), 3.89-4.05 (1H, m), 4.14 (2H, t, J = 6.6), 4.31-4.48 (1H, m), 7.18 (1H, s), 7.31-7.37 (2H, m), 7.55 (1H, d, J = 8.8), 7.81 (1H, d, J = 2.2).

### Example 226

### 2-(1-{3-[(6-Chloro-2-naphthyl)sulfonyl]propanoyl}-4-piperidinyl)-1,2,3,4,6,7,8,9-octahydrodipyrido[1,2-a;4',3'-d]imidazole dihydrochoride

### 226a) Tert-butyl 4-(1,2,3,4,6,7,8,9-octahydrodipyrido[1,2-a;4',3'-d]imidazol-2-yl)piperidine-1-carboxylate

From tert-butyl 4-(1,2,3,4-tetrahydrodipyrido[1,2-a;4',3'-d]imidazol-2-yl)piperidine-1-carboxylate (2.19 g) obtained in Example 224e), the title compound (2.21 g, quantitative) was obtained as colorless powder in a similar manner to Example 209a).
NMR (200 MHz, CDCl₃) δ: 1.44-1.65 (2H, m), 1.46 (9H, s), 1.79-2.04 (6H, m), 2.32-2.57 (1H, m), 2.59-2.78 (4H, m), 2.78-2.92 (4H, m), 3.58 (2H, s), 2.66-2.77 (2H, m), 4.09-4.28 (2H, m).

### 226b) 2-(4-Piperidinyl)-1,2,3,4,6,7,8,9-octahydrodipyrido[1,2-a;4',3'-d]imidazole trihydrochloride

From tert-butyl 4-(1,2,3,4,6,7,8,9-octahydrodipyrido[1,2-a;4',3'-d]imidazol-2-yl)piperidine-1-carboxylate (1.80 g) obtained in Example 226a), the title compound (1.59 g, 86%) was obtained as white crystals in a similar manner to Example 207c).
NMR (200 MHz, D₂O) δ: 1.91-2.28 (6H, m), 2.42-2.59 (2H, m), 3.03 (2H, t, J = 6.4), 3.10-3.31 (4H, m), 3.60-3.89 (4H, m), 3.89-4.09 (3H, m), 4.58 (2H, s).

### 226c) 2-(1-{3-[(6-Chloro-2-naphthyl)sulfonyl]propanoyl}-4-piperidinyl)-1,2,3,4,6,7,8,9-octahydrodipyrido[1,2-a;4',3'-d]imidazole dihydrochloride

From 3-[(6-chloro-2-naphthyl)sulfonyl]propionic acid and 2-(4-piperidinyl)-1,2,3,4,6,7,8,9-octahydrodipyrido[1,2-a;4',3'-d]imidazole trihydrochloride obtained in Example 226b), the title compound (51%) was obtained as white powder in a similar manner to Example 207d).
NMR (200 MHz, DMSO-d₆) δ: 1.43-1.70 (1H, m), 1.70-2.08 (5H, m), 2.08-2.29 (2H, m), 2.39-2.58 (2H, m), 2.78 (2H, t, J = 7.6), 2.91-3.27 (5H, m), 3.27-3.85 (4H, m), 3.85-4.18 (3H, m), 4.30-4.54 (3H, m), 7.74 (1H, dd, J = 8.4, 2.2), 8.01 (1H, dd, J = 8.4, 2.2), 8.19-8.32 (3H, m), 8.66 (1H, s).

### Example 227

### 2-(1-{3-[(5-Chloro-1H-indol-2-yl)sulfonyl]propionyl}-4-piperidinyl)-1,2,3,4,6,7,8,9-octahydrodipyrido[1,2-a;4',3'-d]imidazole dihydrochloride

From 3-[(1-tert-butoxycarbonyl-5-chloro-1H-indol-2-yl)sulfonyl]propionic acid obtained in Example 211d) and 2-(4-piperidinyl)-1,2,3,4,6,7,8,9-octahydropyrido[4',3': 4'5]imidazo[1,2-a]pyridine trihydrochloride obtained in Example 227b), the title compound (21%) was obtained as pale yellow powder in a similar manner to Example 207d). NMR (200 MHz, DMSO-d₆) δ: 1.42-1.71 (1H, m), 1.71-2.09 (5H, m), 2.09-2.28 (2H, m), 2.39-2.59 (2H, m), 2.77 (2H, t, J = 7.6), 2.92-3.27 (5H, m), 3.26-3.85 (4H, m), 3.85-4.19 (3H, m), 4.31-4.55 (3H, m), 7.32-7.38 (2H, m), 7.54 (1H, d, J = 8.8), 7.72 (1H, br), 7.80 (1H, d, J = 1.8).

### Formulation Example 1

A FXa inhibitor (e.g. deep venous thrombosis treating agent, cardiogenic cerebral infarction treating agent, etc.) containing a compound represented by the formula (I) of the present invention or a salt thereof as an active ingredient can be prepared, for example, by the following formulation.

In the following formulation, as ingredients (additives) other than an active ingredient, products listed in Japanese Pharmacopoeia, Japanese Pharmaceutical Codex or Japanese Pharmaceutical Excipients can be used.

| 1. Capsule | |
|---|---|
| (1) Compound obtained in Example 24 | 120 mg |
| (2) Lactose | 210 mg |
| (3) Microcrystalline cellulose | 27 mg |
| (4) Magnesium stearate | 3 mg |
| One capsule | 360 mg |
| (1), (2), (3) and 1/2 of (4) are mixed and then granulated. To this is added the remainder of (4), and the whole is encapsulated into a gelatin capsule. | |

| 2. Capsule | |
|---|---|
| (1) Compound obtained in Example 68 | 120 mg |
| (2) Lactose | 210 mg |
| (3) Microcrystalline cellulose | 27 mg |
| (4) Magnesium stearate | 3 mg |
| One capsule | 360 mg |
| (1), (2), (3) and 1/2 of (4) are mixed and then | |
| granulated. To this is added the remainder of (4), and the whole is encapsulated into a gelatin capsule. | |

| 3. Tablet | |
|---|---|
| (1) Compound obtained in Example 68 | 120 mg |
| (2) Lactose | 174 mg |
| (3) Cornstarch | 54 mg |
| (4) Microcrystalline cellulose | 10.5 mg |
| (5) Magnesium stearate | 1.5 mg |
| One tablet | 360 mg |
| (1), (2), (3), 2/3 of (4) and 1/2 of (5) are mixed and then granulated. The remainders of (4) and (5) are added to this granule, which is compressed into a tablet. | |

| 4. Tablet | |
|---|---|
| (1) Compound obtained in Example 72 | 120 mg |
| (2) Lactose | 174 mg |
| (3) Cornstarch | 54 mg |
| (4) Microcrystalline cellulose | 10.5 mg |
| (5) Magnesium stearate | 1.5 mg |
| One tablet | 360 mg |
| (1), (2), (3), 2/3 of (4) and 1/2 of (5) are mixed and then granulated. The remainders (4) and (5) are added to this granule, which is compressed into a tablet. | |

### Formulation Example 2

After 50 mg of the compound obtained in Example 69 was dissolved in 50 ml of Japanese Pharmacopoeia distilled water for injection, Japanese Pharmacopoeia distilled water for injection is further added such that the whole volume is 100 mL. This solution is filtered under sterilizing condition. One milliliter aliquot of this solution is filled into a vial for injection, lyophilized, and sealed.

### Experimental Example 1

### (1) Human activated blood coagulation factor X (FXa) inhibitory activity

Experimental method: 225 µl of a 0.05 M Tris-HCl buffer (pH 8.3) containing 0.145 M sodium chloride and 2 mM calcium chloride, 5 µl of a sample (a test compound was dissolved in dimethyl sulfoxide) and 10 µl of human FXa (0.3 unit/ml) were added to a 96-well microplate and incubated at 37°C for about 10 minutes. Then, 10 µl of a substrate (3 mM, S-2765) was added to the plate and incubated at 37°C for 10 minutes. After stopping the reaction by adding 25 µl of a 50 % aqueous acetic acid, a change in absorbance at 405 nm was measured with a spectrophotometer, and the concentration (IC₅₀) at which 50 % of FXa activity was inhibited was calculated.

### (2) In vitro coagulation time measuring method

### (2-1) Extrinsic coagulation time (PT) measuring method:

Extrinsic coagulation time was measured with an automatic blood coagulation time measuring apparatus (STA compact, DIAGNOSTICA STAGO) using a PT reagent (DIAGNOSTICA STAGO). To 97 µl of human normal plasma (fresh human plasma FFP, Sekisui Chemical Co., Ltd.), 3 µl of a drug was added and preincubated at 37°C for 4 minutes. To 50 µl of the said plasma, 100 µl of a rabbit brain-derived tissue thromboplastin solution was added and then, a time required for coagulation was measured. A drug dissolved in dimethyl sulfoxide (DMSO) was used. The concentration of a drug required for 2-fold extension of a coagulation time was calculated based on a coagulation time when DMSO was added in place of the drug.

### (2-2) Intrinsic coagulation time (APTT) measuring method:

Intrinsic coagulation time was measured with an automatical blood coagulation time measuring apparatus using STA-APTT-LT (DIAGNOSTICA STAGO). To 97 µl of human normal plasma, 3 µl of a drug was added. To 50 µl of the plasma, 50 µl of an activated partial thromboplatin solution was added and preincubated at 37°C for 4 minutes. Then, 50 µl of a 25 mmol/l CaCl₂ solution was added, and a time required for coagulation was measured. A drug dissolved in DMSO was used. The concentration of a drug required for 2-fold extension of a coagulation time was calculated as described in (2-1).

### (2-3) Thrombin coagulation time (TT) measuring method:

Thrombin coagulation time was measured with an automatic coagulation measuring apparatus using a fibrinogen reagent (DIAGNOSTICA STAGO). A fibrinogen reagent (containing thrombin) was dissolved in 5 mL of distilled water and then diluted 20-fold with a physiological saline supplemented with 0.5% bovine serum albumin. To 97 µl of human normal plasma (fresh human plasma FFP, Sekisui Chemical CO., Ltd.), 3 µl of a drug was added and preincubated at 37°C for 3 minutes. To 50 µl of the said plasma, 100 µl of a thrombin solution was added, and a time required for coagulation was measured. A drug dissolved in DMSO was used. The concentration of a drug required for 2-fold extension of a coagulation time was calculated as described in (2-1).

### (3) Ex vivo coagulation time measuring method (mouse)

### (3-1) Intravenous administration:

A male ICR mouse (25-35 g,CLEA Japan Inc.) was used. To a mouse anesthetized with pentobarbital (50 mg/kg, i.p.), 5 ml/kg of a drug was administered once via a tail vein. After 5 minutes from administration, 0.8 ml of blood was taken from an abdominal aorta or heart using 1/10 volume of 3.8% sodium citrate (Citral, Yamanouchi Seiyaku) and then centrifuged at 3000rpm for 15 minutes to obtain plasma. To 50 µl of the said plasma, 100 µl of a rabbit brain-derived tissue thromboplastin solution was added, and a time required for coagulation was measured. A coagulation time was measured with an automatic coagulation time measuring apparatus (STA compact) using a PT reagent (DIAGNOSTICA ATAGO). A drug dissolved in a mixed solution of dimethylacetamide and 1/10 N hydrochloric acid was used. A mixed solution of dimethylacetamide and 1/10 N hydrochloric acid was administered to a control group in place of the drug. The activity of the drug was expressed as the ratio (%) of a coagulation time of a drug-administered group to a coagulation time of a control group.

### (3-2) Oral administration:

A male ICR mouse (25-35 g, Nippon Crea) was used. To a mouse which had been fasted for 12 hours or longer, 5 ml/kg of a drug was forced to be orally administered. After an hour from administration, blood was taken from an abdominal aorta under pentobarbital (50 mg/kg, i.p.) anesthesia. A drug suspended in 0.5% methylcellulose was used, and 0.5 % methylcellulose in place of a drug was administered to a control group. Others were as described in (3-1).

### (4) In vivo antithrombotic activity measuring method

### (4-1) Rat arteriovenous shunt method:

The method was according to the method of Umetsu et al. (Thromb. Haemostas., 39, 74-73, (1978)). A male SD rat (200-350 g, Nippon Crea) was used. An extracorporeal circulation path made of a polyethylene tube provided with a silk thread was placed between the left jugular and right jugular vein of a mouse anesthetized with pentobarbital (50 mg/kg, i.p.). In order to prevent blood coagulation, the tube was previously filled with a physiological saline containing heparin (50U/ml). Blood was circulated for 15 minutes, during which the wet weight of a thrombus attached to the silk thread was measured. A drug was administered orally or intravenously. In the case of oral administration, a drug (2 ml/kg) suspended in 0.5% methylcellulose was administered under fasting and 0.5 % methylcellulose was administered to a control group instead of a drug. In the case of intravenously administration, a drug (1 ml/kg) dissolved in a physiological saline was administered via a tail vein, and a physiological saline was administered to a control group instead of a drug. The activity of the drug was calculated as the ratio (%) of a thrombus wet weight of a drug-administered group to a thrombus wet weight of a control group.

### (4-2) Rat abdominal vena cava partial ligation model

A male SD rat (200-400 g, Nippon Crea) was used. After the abdominal vena cava of a mouse anesthetized with pentobarbital (50 mg/kg, i.p.) was carefully peeled, two ligatures were put round a renal vein branched part of the abdominal vena cava and a place 1 cm downstream therefrom respectively so that all branches between them were ligated. A balloon catheter (Fogarty 2F, Baxter) was inserted via the left femoral vein and the balloon was then dilated with a 200-300 ml air to damage three times between the two ligatures. The balloon catheter was taken out. The ligature put round the renal vein branched part was tied with a 26G needle and the needle was then taken out, thereby a partial ligation was made. After 30 minutes, the other ligature was tied, and a thrombus formed between the two ligatures was carefully isolated. The wet weight of the thrombus was measured using an analysis balance equipped with a windscreen (BP11OS, Satorius). A drug was administered orally or intravenously as described in (4-1). The activity of the drug was calculated as described in (4-1).

### (4-3) Rat deep vein thrombosis (DVT) model

A male SD rat (200-350 g, Nippon Crea) was used. A polyethylene tube was inserted into the left femoral vein of a mouse anesthetized with pentobarbital (50 mg/kg, i.p.). A silk thread (length 5cm) connected to a guide wire was inserted into the polyethylene tube and the tube was filled with a physiological saline containing heparin (50U/ml) in order to prevent blood coagulation. After the polyethylene tube was inserted to reach the abdominal vena cava, the silk thread was allowed to be stood in the abdominal vena cava using the guide wire. After 30 minutes, heparin (200U/kg) was intravenously administered via a tail vein. After exsanguinations by cutting of an upper arm artery, the abdominal part was opened to take out the silk thread and the wet weight of thrombus attached thereto (including weight of silk thread) was measured. A drug was administered orally or intravenously as described in (4-1). The wet weight of only thrombus was calculated using the equation: (wet weight of thrombus attached to silk thread) - (wet weight measured of silk thread immersed in a venous blood sample collected using heparin). The activity of the drug was calculated as described in (4-1).

### Experimental results

Table 1 shows IC₅₀ values obtained in Experimental Example 1 (1). From this, it is clear that the compound of the present invention shows excellent FXa inhibitory activity.

**Table 1**

| Example No. | IC₅₀(nM) | Example No. | IC₅₀(nM) |
|---|---|---|---|
| 24 | 31 | 38 | 11 |
| 39 | 11 | 42 | 7.1 |
| 68 | 5.6 | 72 | 7.2 |
| 102 | 43 | 109 | 45 |

### Industrial Applicability

Compound (I) of the present invention or a salt thereof has excellent FXa inhibitory activity, has high safety as a drug, is useful as an anticoagulant which can be orally absorbed, and is advantageously used for preventing or treating various diseases based on thrombus or infarction.

## Claims

1. A compound represented by the formula (I): 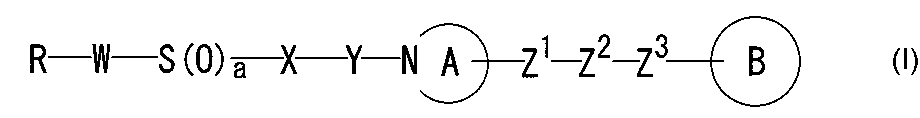 wherein R represents an optionally substituted cyclic hydrocarbon group or an optionally substituted heterocyclic group, W represents a bond or an optionally substituted divalent linear hydrocarbon group, X represents an optionally substituted divalent hydrocarbon group, Y represents -CO-, -S(O)-, -S(O)₂- or a bond, ring A represents an optionally substituted pyrrolidine ring, an optionally substituted piperidine ring or an optionally substituted perhydroazepine ring, Z¹ and Z³ independently represent a bond or an optionally substituted divalent linear hydrocarbon group, Z² represents -N(R¹)-, -0-, -S(O)-, -S(O)₂-, -CO-, -CH (R¹) - or a bond (R¹ represents a hydrogen atom, an optionally substituted hydrocarbon group, an optionally substituted acyl group, an optionally esterified carboxyl group or an optionally substituted carbamoyl group), ring B represents an optionally substituted imidazole ring, wherein a substituent which the optionally substituted imidazole ring represented by ring B may have may be taken together with R¹ to form an optionally substituted ring, and a represents 0, 1 or 2, or a salt thereof.

2. A prodrug of the compound according to claim 1.

3. The compound according to claim 1, wherein R is an optionally substituted aryl group.

4. The compound according to claim 1, wherein R is naphthyl optionally substituted with a halogen atom or indolyl optionally substituted with a halogen atom.

5. The compound according to claim 1, wherein W is a bond.

6. The compound according to claim 1, wherein X is an optionally substituted divalent linear hydrocarbon group.

7. The compound according to claim 1, wherein Y is -CO-.

8. The compound according to claim 1, wherein ring A is an optionally substituted piperidine ring.

9. The compound according to claim 1, wherein the formula: 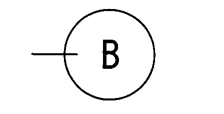 is the formula: 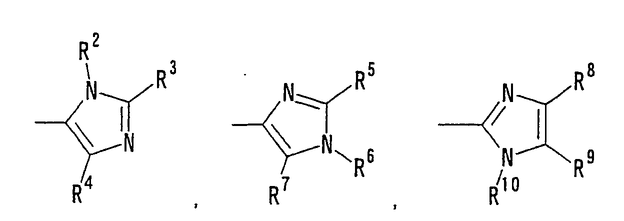 or 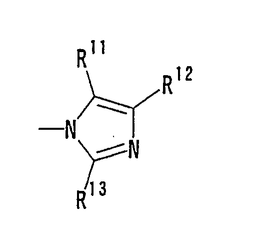 wherein R², R³, R⁴' R⁵, R⁶, R⁷, R⁸, R⁹, R¹⁰, R¹¹, R¹² and R¹³ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group or an optionally substituted amino group, or R² and R³, R⁵ and R⁶, R⁶ and R⁷, R⁸ and R⁹, R⁹ and R¹⁰, or R¹¹ and R¹² may be taken together to form an optionally substituted ring.

10. The compound according to claim 1, wherein the formula: 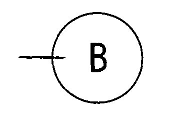 is the formula: 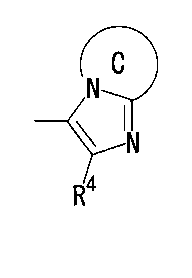 wherein ring C represents an optionally substituted nitrogen-containing heterocyclic ring, and other symbols are as defined in claim 9.

11. The compound according to claim 1, wherein a substituent which the optionally substituted imidazole ring represented by ring B may have and R¹ together do not form a ring.

12. The compound according to claim 1, wherein Z² is -N(R¹)- or -CH(R¹)- (R¹ is as defined in claim 1), and a substituent which the optionally substituted imidazole ring represented by ring B may have and R¹ are taken together to form an optionally substituted ring.

13. The compound according to claim 1, wherein the formula (I) is the formula (Ia): 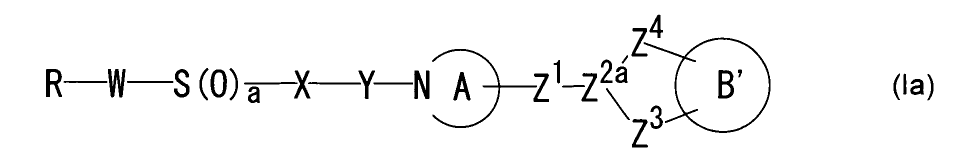 wherein ring B' represents an optionally further substituted imidazole ring, Z^{2a} represents N or CH, Z⁴ represents an optionally substituted divalent linear hydrocarbon group, and other symbols are as defined in claim 1.

14. The compound according to claim 13, wherein Z^{2a} is a nitrogen atom.

15. The compound according to claim 13, wherein Z³ and Z⁴ are independently a divalent linear hydrocarbon group optionally substituted with an oxo group.

16. The compound according to claim 1, wherein the formula (I) is the formula (Ib): 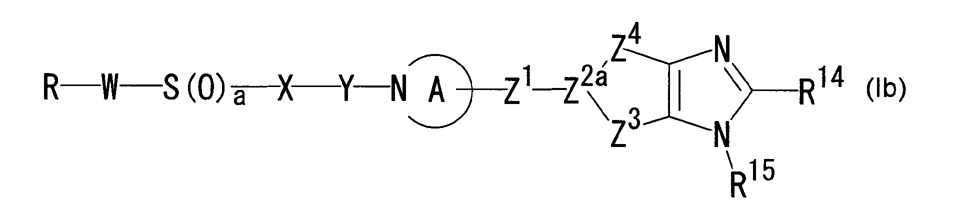 wherein R¹⁴ and R¹⁵ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group, or an optionally substituted amino group, or R¹⁴ and R¹⁵ may be taken together to form an optionally substituted ring, and other symbols are as defined in claim 1 or 13.

17. The compound according to claim 1, wherein the formula (I) is the formula (Ic): 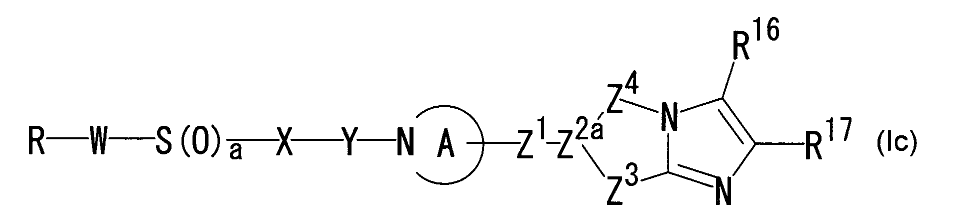 wherein R¹⁶ and R¹⁷ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group or an optionally substituted amino group, or R¹⁶ and R¹⁷ may be taken together to form an optionally substituted ring, and other symbols are as defined in claim 1 or 13.

18. The compound according to claim 1, wherein the formula (I) is the formula (Id): 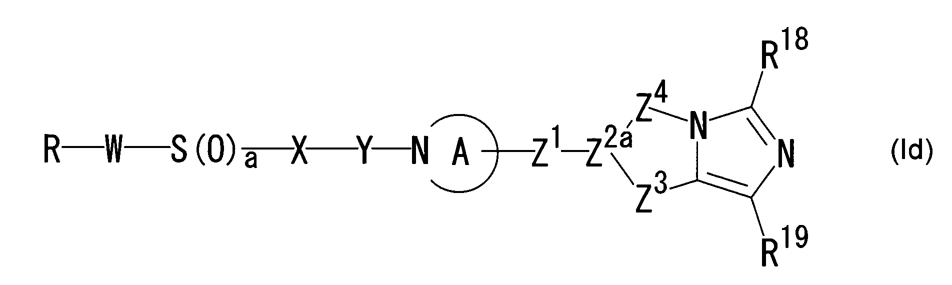 wherein R¹⁸ and R¹⁹ independently represent a hydrogen atom, an optionally substituted hydrocarbon group, an optionally substituted hydroxyl group, an optionally substituted thiol group, an optionally substituted alkylsulfinyl group, an optionally substituted alkylsulfonyl group, an optionally substituted acyl group, an optionally esterified carboxyl group, an optionally substituted carbamoyl group, or an optionally substituted amino group, and other symbols are as defined in claim 1 or 13.

19. The compound according to claim 1, wherein a is 2.

20. A compound selected from the group consisting of 7-(1-{3-[(6-chloro-2-naphthyl)sulfonyl]propanoyl}-4-piperidinyl)-3-methyl-6,7-dihydroimidazo[1,5-a]pyrazin-8(5H)-one, 7-(1-{3-[(6-choloro-2-naphthyl)sulfonyl]propanoyl}-4-piperidinyl)-1-methyl-6,7-dihydroimidazo[1,5-a]pyrazin-8(5H)-one, 2-(1-{3-[(6-choloro-2-naphthyl)sulfonyl]propanoyl}-4-piperidinyl)-5-methyl-1,2-dihydro-3H-imidazo[1,5-c]imidazol-3-one, 2-(1-{3-[(6-choloro-2-naphthyl)sulfonyl]propanoyl}-4-piperidinyl)-5,7-dimethyl-1,2-dihydro-3H-imidazo[1,5-c]imidazol-3-one, 2-(1-{3-[(7-choloro-2H-chromen-3-yl)sulfonyl]propanoyl}-4-piperidinyl)-5-methyl-1,2-dihydro-3H-imidazo[1,5-c]imidazol-3-one, 2-[1-(3-{[(E)-2-(4-cholorophenyl)vinyl]sulfonyl}propanoyl)-4-piperidinyl]-5-methyl-1,2-dihydro-3H-imidazo[1,5-c]imidazol-3-one, 2-(1-{3-[(5-chloro-1H-indol-2-yl)sulfonyl]propanoyl}-4-piperidinyl)-5-methyl-1,2-dihydro-3H-imidazo[1,5-c]imidazol-3-one, 2-(1-{3-[(6-chloro-2-naphthyl)sulfonyl]propanoyl}-4-piperidinyl)-5-(hydroxymethyl)-1,2-dihydro-3H-imidazo[1,5-c]imidazol-3-one, 2-(1-{(2S)-3-[(6-chloro-2-naphthyl)sulfonyl]-2-hydroxypropanoyl}-4-piperidinyl)-5-(hydroxymethyl)-1,2-dihydro-3H-imidazo[1,5-c]imidazol-3-one, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 1-acetylpiperidine-4-carboxylate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 3-(2-oxo-1-pyrrolidinyl)propionate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl (2-oxo-1-pyrrolidinyl)acetate, [2-(1-{(2S)-3-[(6-chloro-2-naphthyl)sulfonyl]-2-hydroxypropanoyl}-4-piperidinyl)-3-oxo-2,3-dihydro-1H-imidazo[1,5-c]imidazol-5-yl]methyl 4-(acetylamino)butanoate, and 2-(1-{(2S)-3-[(6-chloro-2-naphthyl)sulfonyl]-2-hydroxypropanoyl}-4-piperidinyl)-5,7-dimethyl-1,2-dihydro-3H-imidazo[1,5-c]imidazol-3-one or a salt thereof.

21. A pharmaceutical preparation which comprises the compound according to claim 1 or 2.

22. The pharmaceutical preparation according to claim 21, which is an anticoagulant.

23. The pharmaceutical preparation according to claim 21, which is an activated blood coagulation factor X inhibitor.

24. The pharmaceutical preparationn according to claim 21, which is an agent for preventing or treating myocardial infarction, cerebral infarction, deep venous thrombosis, pulmonary thromboembolism or arterioscleroticobliterans.

25. The pharmaceutical preparation according to claim 21, which is an agent for preventing or treating economy class syndrome, thromboembolism during or after an operation, or a secondary onset of deep venous thrombosis.

26. A process for preparing the compound according to claim 1, which comprises reacting a compound represented by the formula (II): 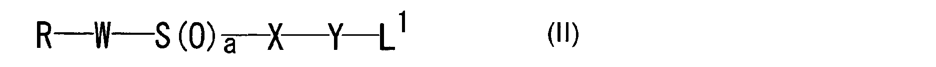 wherein L¹ represents a leaving group and other symbols are as defined in claim 1, or a salt thereof with a compound represented by the formula (III): 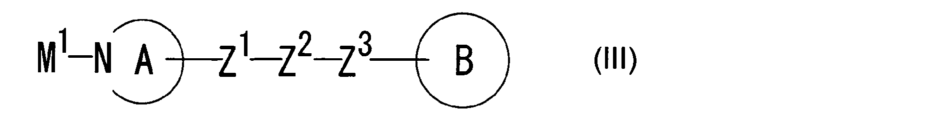 wherein M¹ represents a hydrogen atom, an alkaline metal, an alkaline earth metal or a leaving group, and other symbols are as defined in claim 1, or a salt thereof; or
reacting a compound represented by the formula (IV): 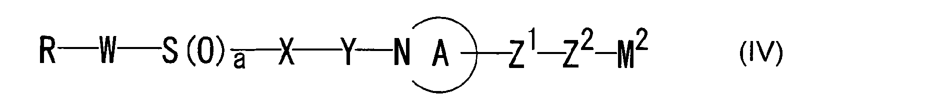 wherein M² represents a hydrogen atom, an alkaline metal, an alkaline earth metal or a leaving group, and other symbols are as defined in claim 1, or a salt thereof with a compound represented by the formula (V): 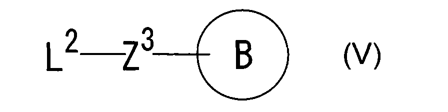 wherein L² represents a leaving group or a formyl group, and other symbols are as defined in claim 1, or a salt thereof; or
reacting a compound represented by the formula (Ie): 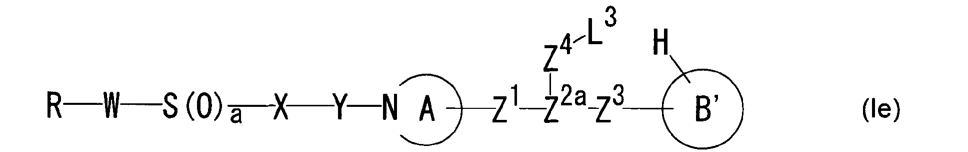 wherein L³ represents a leaving group and other symbols are as defined in claim 1 or 13, or a salt thereof with a base; or
reacting a compound represented by the formula (If): 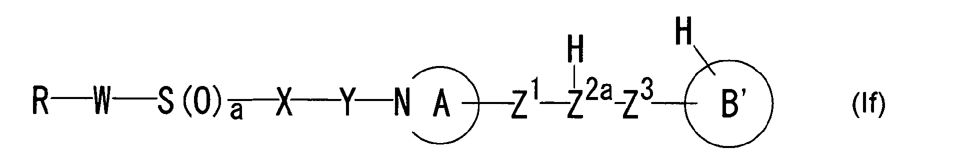 wherein symbols are as defined in claim 1 or 13, or a salt thereof with a compound represented by the formula (VI):
L⁴―Z⁴―L^{4,} (VI)
wherein L⁴ and L^{4'} represent a leaving group and other symbols are as defined in claim 13, or a salt thereof; or
oxidizing a compound represented by the formula (Ig): 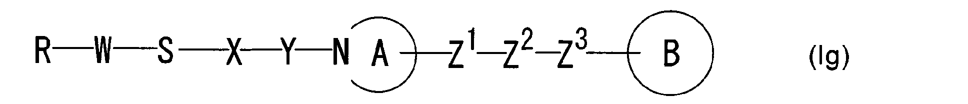 wherein symbols are as defined in claim 1, or a salt thereof, and optionally subjecting a compound obtained in the above reaction to hydrolysis, esterification, amidation, alkylation, acylation, reduction, oxidation or/and deprotection reaction.

27. A method for inhibiting blood coagulation in a mammal, which comprises administering an effective amount of the compound according to claim 1 or a prodrug thereof to said mammal.

28. A method for inhibiting activated blood coagulation factor X in a mammal, which comprises administering an effective amount of the compound according to claim 1 or a prodrug thereof to said mammal.

29. A method for preventing or treating myocardial infarction, cerebral infarction, deep venous thrombosis, pulmonary thromboembolism or arteriosclerotic obliterans in a mammal, which comprises administering an effective amount of the compound according to claim 1 or a prodrug thereof to said mammal.

30. Use of the compound according to claim 1 or a prodrug thereof for manufacture of a medicament for inhibiting blood coagulation.

31. Use of the compound according to claim 1 or a prodrug thereof for manufacture of a medicament for inhibiting activated blood coagulation factor X.

32. Use of the compound according to claim 1 or a prodrug thereof for manufacture a medicament for preventing or treating myocardial infarction, cerebral infarction, deep venous thrombosis, pulmonary thromboembolism or arteriosclerotic obliterans.
